# EUROPEAN PATENT SPECIFICATION

(11) **EP 0 341 104 B1**
(45) Date of publication and mention of the grant of the patent: **29.12.1993**
(21) Application number: 89400953.9
(22) Date of filing: 06.04.1989
(51) Int. Cl.: C07D 311/30, C07D 311/92, C07D 335/06, C07D 215/22, C07D 345/00, C07D 405/06, C07D 405/12, C07D 417/04, C07D 513/04, C07D 405/04, A61K 31/35

(54) **Substituted flavonoid compounds, their salts, their manufacture and medicines containing these materials**
Substituierte flavonoide Verbindungen, ihre Salze, ihre Herstellung und die enthaltende Arnzeizusammensetzungen
Composés flavonoides substitués, leurs sels, procédé de leur préparation et compositions pharmaceutiques les contenant

(30) Priority: 06.04.1988 US 178315
(43) Date of publication of application: 08.11.1989
(73) Proprietor: MERCK PATENT GmbH, 64293 Darmstadt (DE)
(72) Inventor: Briet, Philippe, F-69003 Lyon (FR); Berthelon, Jean-Jacques, F-69008 Lyon (FR); Collonges, François, F-01700 Beynost-Miribel (FR)
(74) Representative: Schüttler, Reinhard, Dr.

(56) References cited:
- EP-A- 0 123 113
- EP-A- 0 283 761
- EP-A-00 080 934
- DE-A- 3 517 950
- DE-B- 1 270 567
- CHEMICAL ABSTRACTS, vol. 104, no. 15, 14 April 1986, page 19, column 1, abstract no. 122574b, Columbus, Ohio, USA; G. ATASSI et al.: "Synthesis and antitumor ..." & J. Med. Chem.-Chim. Ther 1985, vol. 20, no. 5, pages 393-402
- CHEMICAL ABSTRACTS, vol. 107, no. 17, 26 October 1987, page 27, column 1, abstract no. 146923c, Columbus, Ohio, USA; M. CHING et al.: "Induction of natural Killer ..." Eur. J. Cancer Clin. Oncol. 1987, vol. 23, no. 7, pages 1047-1050
- PATENT ABSTRACTS OF JAPAN, vol. 10, no. 56, (C-331) (2113), 6 March 1986; & JP-A-60 199817 (RIKAGAKU KENKYUSHO) 09.10.1985
- PATENT ABSTRACTS OF JAPAN; vol. 8, no. 71 (C-217)(1508), 3 April 1984; & JP-A-58 225083 (NIPPON SHINYAKU K.K.) 27.12.1983
- PATENT ABSTRACTS OF JAPAN, vol. 9, no. 196 (C-297)(1919), 13 August 1985; & JP-A-60 64976 (HOKURIKU SEIYAKU K.K.) 13.04.1985
- 6015 JOURNAL OF THE CHEMICAL SOCIETY, Perkin Transactions I 1977, no. 9, pages 948-953; S. ANTUS et al.: "Synthesis of tachrosin ..."
- CHEMICAL ABSTRACTS, vol. 89, no. 13, 25 September 1978, page 867, column 2, abstract no. 108951n, Columbus, Ohio, USA; P. ROVERI et al.: "New alkylating cytostatically ...", & Arch. Pharm. (Weinheim, Ger.), 1978, vol. 311, no. 6, pages 465-468
- 499 JOURNAL OF HETEROCYCLIS CHEMISTRY, vol. 11, no. 4, August 1974, pages 469-470; I. LALEZARI et al.: "Selenium Heterocycles XIV. (1) 2,6-Diaryltetrahydroselenopyran-4-ones"
- PATENT ABSTRACTS OF JAPAN, vol. 9, no. 138 (C-286)(1861), 13 June 1985; & JP-A-60 23379 (SHINNIHON YAKUHIN K.K.) 05.02.85
- 2305 JOURNAL OF IMMUNOLOGY, vol. 140, No. 1, 9 May 1988, pages 3261-3265; R. H. WILTROUT et al.: "Flavone-8-acetic acid augments systemic Natural Killer cell activity and synergizes with IL-2 for treatment of murine renal cancer"

## Description

### BACKGROUND OF THE INVENTION

### Field of the Invention

This invention relates to substituted flavonoid compounds, and to these compounds used as medicaments.

### Discussion of the Background

U.S. 4,602,034 discloses (oxo-4-4H-(1)-benzopyran-8-yl) alkanoic acids and their derivatives, represented by the formula: 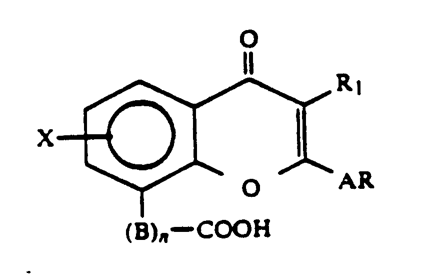
wherein, in the above formula, AR is hydrogen, a phenyl radical which may or may not be substituted, thenyl, furyl, naphthyl, a lower alkyl, cycloalkyl, aralkyl radical, B is a lower alkyl radical, R₁ is hydrogen or a phenyl radical, X is hydrogen or a lower alkyl or alkoxy radical, and n is equal to 1, as well as some salts, esters, amino esters and amides of these compounds.

Fifty-seven specific examples of this class of compounds are reported in U.S. 4,602,034. These compounds are disclosed to be useful in the control of tumors, however their anticancer activity reported is limited to P388 lymphocytic leukemia and carcinoma 38 of the colon.

Rubin et al in "Lancet", 8567, 11:1081-1082 (1987) disclose that flavone-8-acetic acid, one of the compounds disclosed by U.S. 4,602,034, inhibits ristocetin-induced platelet agglutination and prolongs bleeding time.

Wiltrout et al in "The Journal of Immunology", vol. 140, no. 9, pp. 1-5 (1988) disclose that flavone-8-acetic acid, the same compound discussed above, also augments systemic natural killer cell activity and synergizes with interleukin-2 (IL-2) for treatment of murine renal cancer.

In view of the wide variety of cancers found in animals, and in particular in humans, however there is a strongly felt need for other materials useful in the treatment of other types of cancers, e.g., pancreatic cancer, not to mention the fact that there is also a strongly felt need for new compounds possessing other desirable pharmaceutical properties, e.g., the property of inhibiting platelet agglutination. Such pharmaceutical properties would also include immunomodulatory properties.

### SUMMARY OF THE INVENTION

Accordingly, it is an object of this invention to provide a novel class of compounds possessing anticancer activity.

It is another object of this invention to provide a novel class of compounds possessing antipancreatic cancer activity.

It is another object of this invention to provide a novel class of compounds possessing immunomodulatory properties.

It is another object of this invention to provide a novel class of compounds possessing immunomodulatory properties where these properties include the property of stimulating the production of interferon (IFN).

It is another object of this invention to provide a novel class of compounds possessing immunomodulatory properties where these properties include the property of stimulating the formation of killer cells.

It is another object of this invention to provide a novel class of compounds possessing the property of inhibiting platelet agglutination.

It is another object of this invention to provide compounds possessing very favorable threshold values in the exploitation of their properties (threshold value being defined as the difference between the lowest level of administration of the compound at which the activity is observed and the level of administration at which the compound becomes toxic to the patient).

It is another object of this invention to provide pharmaceutical compositions containing at least one of the compounds provided by this invention.

The inventors have now discovered a class of compounds which satisfy all of the above objects of the invention and other objects which will become apparent from the description of the invention given hereinbelow. These compounds have the formula (I): 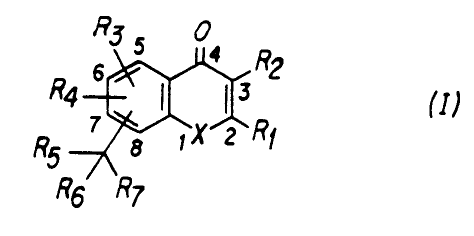
wherein:
X is N, O, Se, or S(O)ₙ, wherein n is 0, 1 or 2;
R₁ is phenyl; phenyl substituted by at least one member selected from the group consisting of halogens, C₁₋₁₂ alkyl, trifluoromethyl, hydroxyl, C₁₋₆ alkoxy, (̵C₁₋₆-alkylene)̵COOR₁₀, nitro, C₁₋₆(̵alkyl)̵carboylamino, benzoyl, C₁₋₆(̵alkyl)carboyl, CONR₁₀R₁₁, (where R₁₀ and R₁₁ are each independently H or C₁₋₆ alkyl), NR₁₀R₁₁, -N=N-NR₁₀R₁₁, phenyl, -O(̵C₁₋₆ alkalene)̵NR₁₀R₁₁, thiazolyl, and thiazolyl substituted by C₁₋₆ alkyl or amino; or R₁ is pyridyl; pyridyl substituted by at least one member selected from the group consisting of C₁₋₆ alkyls and halogens; trifluoromethyl; benzoyl or benzyl;
R₂ is H; phenyl; OH; C₁₋₃ alkyl; or C₁₋₃ alkoxy; or
R₁ and R₂ together form a naphthalene ring fused to the flavonoid nucleus;
R₃ is H; OH; or halogen;
R₄ is H;
R₅ is H; or C₁₋₃ alkyl;
R₆ is H; OH;
or -O-CO(̵C₁₋₆ alkyl);
or R₅ and R₆ together are a group =CR₁₀R₁₁, or a group =NOH, or a group =O or a group =CHR₁₂ (where R₁₂ is phenyl, pyridyl, phenyl substituted by at least one member selected from the group consisting of halogen atoms, trifluoromethyl and C₁₋₃ alkyls or pyrridyl substituted by at least one member selected from the group consisting of halogen atoms, trifluoromethyl and C₁₋₃ alkyls);
R₇ is H; COOR₁₀; -P(O)(OR₁₀R₁₁)₂; NR₁₃R₁₄ (where R₁₃ and R₁₄ are independently H; phenyl; phenyl substituted by a halogen atom or a C₁₋₃ alkyl group or a group -COOR₁₀, -CO-O-CH(CH₃)-COOR₁₀, morpholinyl, -C(CH₂OH)₂(CH₃), imidazolinyl, (̵C₁₋₆ alkylene)̵OH, (̵C₁₋₆ alkylene)̵COOR₁₀, or C₁₋₃ alkoxy, or wherein R₁₃ and R₁₄ together with the nitrogen atom to which they are both bound from an imidazole or a N(̵C₁₋₃ alkyl)̵piperazinyl); or
R₇ is -CO(̵C₁₋₆ alkyl); -S-(C₁₋₆ alkyl); -SH; -S-CO(̵C₁₋₃ alkyl); 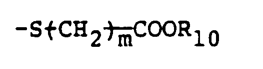
(with 0 < m < 6); -O(̵C₁₋₆ alkylene)̵NR₁₀R₁₁; -NR₁₀NR₁₀R₁₁; C₁₋₆ alkyl; thiazolyl; thiazolyl substituted by at least one member selected from the group consisting of -NH₂, C₁₋₃ alkyl, phenyl, and COOR₁₀; -NH-CO(̵C₁₋₃-alkyl); or (̵C₁₋₃-alkylene)̵CH(NH₂(COOH); or
-CR₅R₆R₇ is a group of the formula 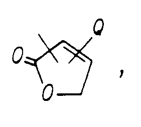
wherein Q is at least one member selected from the group consisting of H; COOR₁₀; phenyl; -O(̵C₁₋₃-alkylene)̵COOR₁₀; C₁₋₃ alkyl; -O-CS-NR₁₀R₁₁; -O(̵C₁₋₃-alkylene)̵NR₁₀R₁₁; OH; C₁₋₃ alkoxy; and NR₁₀R₁₁; or
wherein any two of R₃, R₄, R₅, R₆ and R₇ together form a benzene ring; or a benzene ring substituted by (̵C₁₋₃-alkalene)̵COOR₁₀; (̵C₁₋₃-alkyl)̵OH, COOR₁₀, or (̵C₁₋₃-alkylene)̵O-CO(̵C₁₋₃-alkyl); or a naphthalene system; or a naphthalene system substituted by (̵C₁₋₃-alkylene)̵COOR₁₀, (̵C₁₋₃-alky)̵OH, COOR₁₀, or (̵C₁₋₃-alkylene)̵O-CO(̵C₁₋₃-alkyl); and
physiologically acceptable salts thereof,
with the proviso that when R₂, R₃, R₄, R₅ and R₆ are all H and R₇ is COOR₁₀, R₁ is other than phenyl, 2-thenyl, 3-methoxy phenyl, 3,4-dimethoxy phenyl, 2-furyl, para-tolyl, 2-naphthyl, 4-methoxy phenyl, cyclohexyl, benzyl, or methyl.

### DETAILED DESCRIPTION OF THE PREFERRED EMBODIMENTS

The compounds of the present invention have been surprisingly discovered to possess anticancer activity, in particular antipancreatic cancer activity, and better threshold characteristics as compared to available compounds, namely those disclosed in U.S. 4,602,034. These compounds have further surprisingly been discovered to also possess immunomodulatory activity, in particular they stimulate the formation of interferon and of killer cells. And, it is believed that the compounds of the present invention inhibit platelet agglutination and prolong bleeding times. These compounds are therefore useful in the treatment of cancers, e.g., pancreatic cancers, and are believed to be useful in the suppression of clot formation.

In conjunction with Messrs. Robert H. Wiltrout and Ronald L. Hornung of the National Cancer Institute at Frederick, Maryland, U.S.A., the inventors have also found that the compounds of the present invention surprisingly potentiate the activity of interleukin-2 (IL-2).

The terms "alkyl", "alkylene", and "alkoxy" used in this document refer to linear or branched or cyclic, saturated or unsaturated alkyl, alkylene, or alkoxy groups unless otherwise specified.

The term "halogen" in this document refers to fluoro, chloro, bromo and iodo, preferably fluoro and chloro, and most preferably fluoro, unless otherwise specified.

The term "salt" is used in this document in accordance with its accepted definition to include all possibilites in which the compound of the invention is either the cationic or the anionic component of the salt. The compounds of the invention have acidic and/or basic functionalities which can of course be both present in the same molecule.

With acidic functionalities, the salts of the compounds are obtainable through reaction with either an organic or an inorganic base. Such bases include all bases known to be useful to make physiologically acceptable salts, for example, Na₂CO₃, NaHCO₃, KOH, NaOH, NH₃ and bases of the formula NR₂₇R₂₈R₂₉ where R₂₇, R₂₈ and R₂₉ are H, C₁₋₆ alkyl, C₁₋₆ hydroxyalkyl, etc.

With basic functionalities, the salts of the compounds are obtained by reaction with inorganic or organic acids. The acids which can be used are all the acids known to be useful to make physiologically acceptable salts, for example, HCl, HBr, HI, phosphoric acid, phosphonic acid, para-toluene sulfonic acid, formic acid, oxalic acid, fumaric acid, etc. These salt forms of the compounds are generally readily soluble in water, and permit administration of the compounds in solution to a patient.

The present invention also provides pharmaceutical compositions containing at least one of the present compounds. These pharmaceutical compositions are prepared in accordance with the general knowledge in the pharmaceutical art. They can be pharmaceutical compositions suitable for intravenous injection, oral administration, nasal administration (e.g. a nasal spray) or eye drops. The pH of these compositions is preferably at a value compatible with human administration, e.g. the pH is at a value of between 7 and 8.

These compounds can be administered following any protocol known in this art. For example, they can be administered intravenously at a dosage of 1 to 10 g m⁻² for a period of time of 1 to 24 hours or longer.

The compounds of the present invention, when not in solution in a pharmaceutically suitable carrier, are preferably lyopholized before storage. In lypholized form they are more easily dissolved in a pharmaceutical medium.

In a preferred embodiment, when R₁ is C₁₋₇ alkyl, the preferred alkyl groups are C₁₋₃ alkyl, e.g., methyl, ethyl, n-propyl or iso-propyl. When R₁ is a substituted phenyl group, the substituents are C₁₋₃ alkyl, halogen, trifluoromethyl, hydroxy, C₁₋₃ alkoxy or nitro. Phenyl substituted by one halogen atom is particularly preferred. When R₁ is a substituted pyrridyl, the same preferred substituents for phenyl as given above, are also preferred.

The groups R₂, R₃ and R₄ are preferrably H. Groups R₅ and R₆ are preferably =CR₁₀R₁₁ with R₁₀=R₁₁=H.

Preferably R₇ is a group which is metabolized in vivo to leave an acidic function on the flavonoid nucleus for R₇. Accordingly, -COOR₁₀, P(O)(OR₁₀R₁₁), -CH₂CH-COOR₁₀, and -CONR₁₀R₁₁, are preferred for R₇.

When R₄ and R₅ together form a benzene system, R₇ is preferably CH₂COOH.

When R₁ and R₂ together form a naphthylene system, preferably R₅ is hydrogen and R₇ is COOH.

When R₃ and R₄ form a benzene system, preferably R₅ is hydrogen and R₇ is COOH.

When R₅ and R₆ is =O, R₇ is preferably COOH, or CH₂CH₂COOH.

These compounds and their derivatives are particularily useful as antitumor agents, notably as antipancreatic cancer agents.

The compounds of formula (I) can be prepared in accordance with one of the methods of preparation generally outlined below which provides these compounds in good yields.

In a first process, a compound of (III) 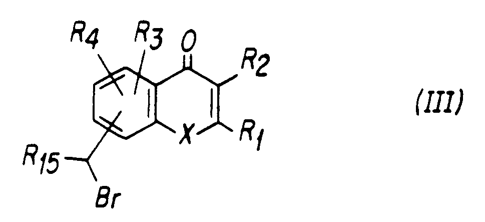
wherein R₁₅ represents 2H , O , =CR₁₀R₁₁ or =CHR₁₂, is reacted either with (1) alkaline nitrile component followed by hydrolysis, or (2) with an amine (NR₁₉R₂₀), or (3) with triethylphosphite followed by hydrolysis, or (4) with a compound of formula R₃₂-SH, or R₃₂-OH, wherein R₃₂ can be -CH₂COOEt or -CH₂CH₂-N(C₂H₅)₂.

In another process to obtain the compounds of formula (I), the compound of formula (III) is reacted either with (1) potassium acetate followed by hydrolysis, oxidation, bromination and condensation with thiourea, thioacetamide, thiobenzamide, 2-amino thiazole, 2-methylpyridine, 2-aminothiazole, or ethoxycarbonyl acetamide.

Compounds of formula (I) can also be obtained by reacting appropriate compounds of formula (I) wherein R₇ is COOH with an alpha halogenated ester followed by cyclization.

Compounds of formula (I) can also be obtained by reacting appropriate compounds of formula (III) with hexamethylene tetramine and condensation of the carbonylated compound obtained with thiosemicarbazide, hydrazinoimidazole, hydroxylamine, or malonic acid.

Compounds of formula (I) can also be obtained by reacting appropriate compounds of formula (II) wherein R₇ is COOH with bromine followed by potassium acetate with subsequent hydrolysis and then oxidation.

The compounds of formula (I) have been found to surprisingly possess antitumor activity. In particular, the compounds of the invention have been discovered to possess in vitro activity against a variety of tumors in accordance with the following method.

The compounds being tested are placed on a paper disk which is set in the middle of an agar-agar base in which a culture of the selected tumor has been placed. The activity is measured by examining the inhibition of growth of the tumor being cultured. The growth is measured as a function of units (1 unit = 25 microns) which are inhibited. These units represent the surface of the growth of the tumor culture. A product is considered to represent a notable level of activity if the number of zones which are inhibited is superior to 250. The tumors used in these tests were adinocarcinomic pancreatic PO3 and colon CO8.

In the tests run by the inventors, in the inhibition of tumor PO3 the compounds of formula 19, 58 and 70 at an application of 1000 micrograms per disk an inhibition value of from 900, 350 and 400, respectively. In the case of tumor CO8, the compounds of formula 31, 19 and 70 administered at 1000 micrograms per disk displayed an inhibition value of 500, 450 and 500, respectively.

Additionally, the compounds of the present invention demonstrated in animal studies, a surprising threshold level, i.e. a relationship between activity and toxicity which provides a therapeutic margin superior to a reference compound, in particular flavone-8-acetic acid. For example, flavone-8-acetic administered at 400 mg/kg, to a group of 10 mice, provided a mortality rate of 10 mice out of the 10 mice tested after 20 days. The compound of the present invention provided by formula 31 provided only 6 deaths out of 10 at an administration level of 400 mg/kg after 20 days. The compound of formula 29 provided 1 death out of 10 at an administration rate of 400 mg/kg after 20 days. And the compound of formula 58 resulted in no deaths of the group of 10 mice at an administration level of 400 mg/kg after 20 days.

In another of its embodiments, the present invention provides compounds of the formula (IV) 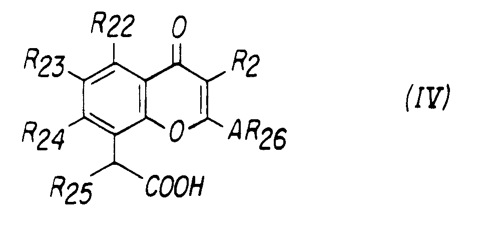
wherein:
AR₂₆ is phenyl, substituted phenyl or biphenylyl;
R₂ is hydrogen, hydroxyl, or C₁₋₃ alkoxy;
R₂₂ is hydrogen or hydroxyl;
R₂₃ is hydrogen, or fluoro;
R₂₄ is hydrogen, or hydroxy;
R₂₅ is hydrogen, 2-methylpyridyl, benzylidene, 4-methylenepyrridyl, or methylene; or
R₂₂ and R₂₃ together form a benzene ring fused to the flavonoid nucleus;
R₂₃ and R₂₄ form a benzene ring fused to the flavonoid nucleus; or
R₂₅ and R₂₄ form a benzene ring fused to the flavonoid nucleus.

These compounds possess an immunomodulating activity and in particular they stimulate the formation of interferon and of killer cells.

The compounds of (IV) can be obtained by the hydrolysis of the nitriles of formula (V): 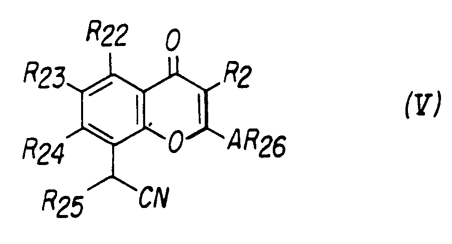
wherein AR₂₆, R₂, R₂₂, R₂₃, R₂₄ and R₂₅ are as defined above. The nitriles of formula (V) are obtained by the reaction of an alkali cyano compound with a compound of formula (VI): 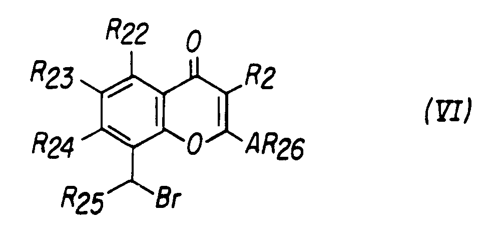
wherein AR ₂₆, R₂, R₂₂, R₂₃, R₂₄ and R₂₅ are as defined above.

With compounds of formula (IV) when R₂₅ is methylene or arylidene, the compounds are obtained by the reaction of compounds of formula (IV) wherein R₂₅ is hydrogen, with N,N,N,N-tetramethyldiaminomethane or with an aromatic aldehyde or an heteroaromatic aldehyde.

The compounds of formula (IV) have been discovered to surprisingly possess immunomodulating properties. The inventors have now discovered that the compounds of formula (IV) possessed a surprisingly high activity with the immune system and that in particular they stimulated the activity of killer cells and induce the formation of interferon (INF).

### Stimulation of the activity of killer cells:

The determination of the activity of killer cells was made in accordance with the following. Mice BALB/C were treated intraveneously, either with 0.25 ml of HBSS or with 200 mg/kg of a compound of formula (IV). Twenty four hours after administration of the compound, the spleens of the animals were reduced to a state of suspension. Debris and cellular wastes are eliminated by sedimentation and the red corpuscles are lysed with distilled water. The cellular suspension obtained was then filtered over sterile gauze and washed twice with HBSS.

Different quantities of splenic cells are incubated with 1 x 10⁴ tumored cells of the type YAC-1 stained with chromium 51. The length of incubation was 4 hours at 37°C in a RPMI 1640-type medium supplemented with 5% of FBS, pencillin (100 U/ml) streptomycin (100 µg/ml), L-glutamine (20 mM), sodium pyruvate (1 mM), and nonessential amino acids (0.1 mM) in a buffered medium. The floating bodies are removed and a count effectuated.

The results are expressed in lytic units (UL₁₀): where UL₁₀ is the quantity necessary to effectuate lysis of 1 x 10⁴ target cells. For example, it was discovered that the compound of formula 19, provided 80 UL. The compound of formula 25 provided 60 UL. The compound of formula 42 provided 110 UL. And the control animals treated with HBSS demonstrated no activity measureable in terms of lytic units (UL).

### Interferon induction:

Mice BALB/C received intravenously, either 0.25 ml of HBSS or 200 mg/kg of one compound of formula (IV). Interferon activity was determined by utilizing the method of viral vesicular stomatitis. A unit of IFN is the quantity of IFN in 1 ml of sample need to reduce the viral lysis by 50%. For example, it was discovered that with the compound of formulae 19, 25 and 67, an activity of 1000 units of IFN was obtained, whereas the control animals treated with HBSS demonstrated no induction in the production of IFN.

Other features of the invention will become apparent in the course of the following descriptions of exemplary embodiments which are given for illustration of the invention and are not intended to be limiting thereof.

### EXAMPLES

### Example 1

### a) OXO-1 PHENYL-3-1H-NAPHTO (2,1-b) PYRAN-5-ACETONITRILE

C₂₁H₁₃NO₂ MW=311,344

A mixture of 8.2 g (0.0224 mole) of bromomethyl-5-phenyl-3-[1H]-naphto(2.1-b)pyranol-1, of 4.9 g (0.031 mole) of tetraethylammonium cyanide in 250 ml of dichloroethane is stirred for 18 hours at room temperature. Evaporation is then carried out in a vacuum, the mixture is solidified using water, and the solid thus formed is filtered and dried. Weight obtained: 6.9 g (Yield: 98%); PFₖ=260°C; IR: Vc=o=7039 cm⁻¹, Vc=N; 2160 and 2220 cm⁻¹.

### b) OXO-1-PHENYL-3-(1H)-NAPHTO (2.1-b) PYRAN-5-ACETIC ACID

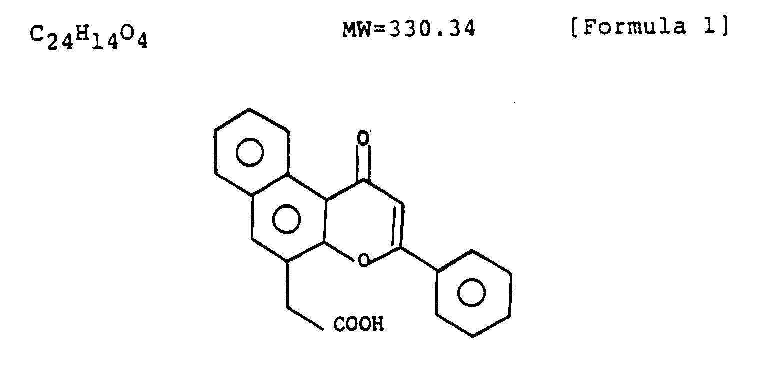

A mixture of 6.9 g (0.022 mole) of oxo-1-phenyl-3-[1H]-naphto(2.1-b)pyran-5-aceto-nitrile, 50 ml of acetic acid, 50 ml of water and 50 ml of H₂SO₄ in concentrated form is heated by reflux. The medium is then poured into water and frozen; the solid thus formed is centrifuged, dried, recrystallized in acetic acid. Weight obtained: 2.1 g (Yield: 28%); PF_{G}=291-293°C; IR: Vc=o (acid)=1700 cm⁻¹, Vc=o (pyrone)=1638 cm⁻¹; NMR (DMSO)δ in ppm in relation to TMS: 2H at 4.03 (s), 1H at 7.1 (s), 9H at 7.3 to 8.3 (n), 1H at 12.5 (exchangeable).

| Elemental analysis | | | |
|---|---|---|---|
| | C% | H% | 0% |
| calculated : | 76.35 | 4.27 | 19.37 |
| found : | 73.85 | 4.01 | |

Using the same technique the following compounds are prepared:

### OXO-4-PHENYL-2-4H-NAPHTO (2.3-b) PYRAN-1-ACETIC ACID

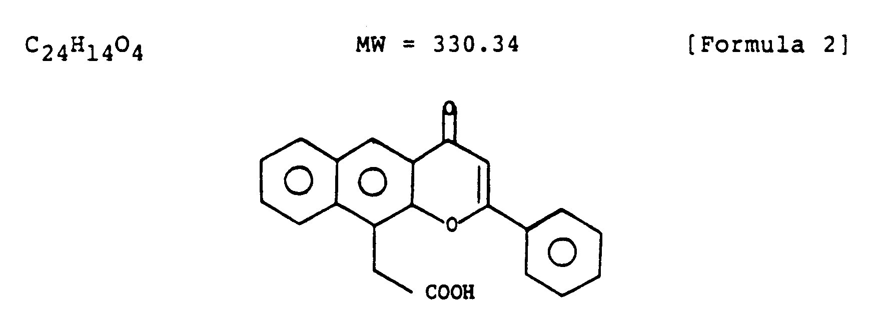

PF_{G} = 276-288°C; IR: Vc=o (acid) = 1720 cm⁻¹, VC=o (pyrone)=7610 cm⁻¹; NMR (DMSO)δ in ppm in relation to TMS: 2H at 4.4 (s), 1H at 6.97 (s), 9H at 7.2 to 8.5 (m), 1H at 8.62 (s), 1H at 12.5 (exchangeable).

| Elemental analysis | | | |
|---|---|---|---|
| | C% | H% | 0% |
| calculated : | 76.39 | 4.27 | 19.38 |
| found : | 79.89 | 4.39 | |

### OXO-4-PHENYL-2-4H-NAPHTO (1.2-b) PYRAN-10-ACETIC ACID

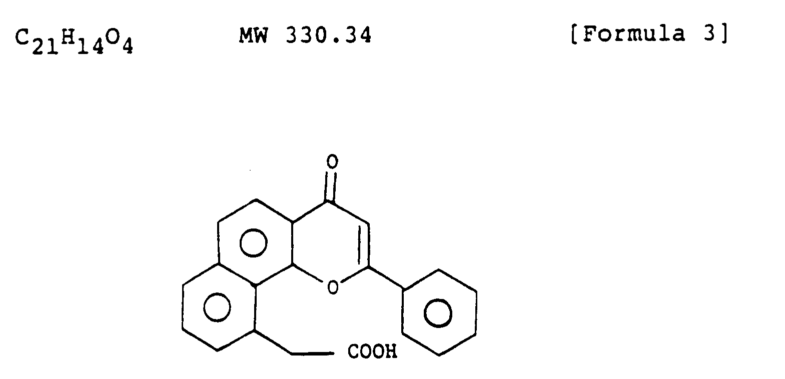

PF_{G}=259-261°C; IR: Vc=0 (acid)=1710 cm⁻¹, Vc=o (pyrone)=7630 cm⁻¹; NMR (DMSO)δ in ppm in relation to TMS: 2H at 4.45(s), 1 H at 6.9 (s), 10H at 7.3 to 8.3 (m), 1H at 12.2 (exchangeable).

| Elemental analysis | | | |
|---|---|---|---|
| | C% | H% | 0% |
| calculated : | 76.39 | 4.27 | 19.38 |
| found : | 76.24 | 4.07 | |

### METHOXY-3-OXO-4-PHENYL-2H-(1)-BENZOPYRAN-8-ACETIC ACID

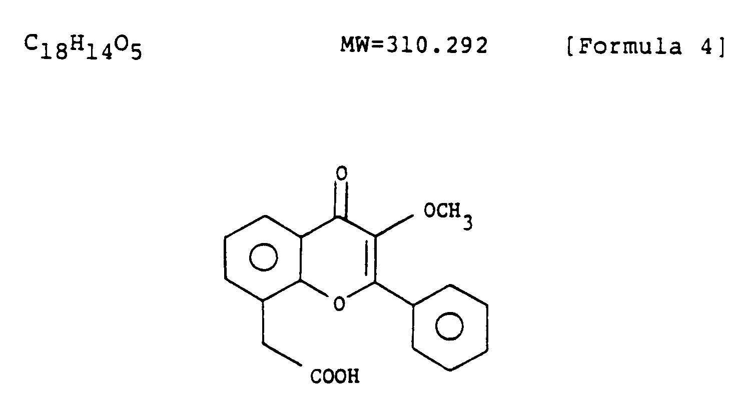

PF_{G}=187-192°C; IR Vc=o (acid)=1720 cm⁻¹, Vc=o (pyrone)=1610 cm⁻¹; NMR (DMSO)δ in ppm in relation to TMS: 3H at 3.8 (s), 2H at 4 (s), 8H at 7.4 to 8.3 (m), 1H 11.9 (exchangeable).

| Elemental analysis | | | |
|---|---|---|---|
| | C% | H% | 0% |
| calculated : | 69.67 | 4.55 | 23.78 |
| found : | 69.90 | 4.55 | |

### METHOXY-5-OXO-4-PHENYL-2-4H-(1)-BENZOPYRAN-8-ACETIC ACID

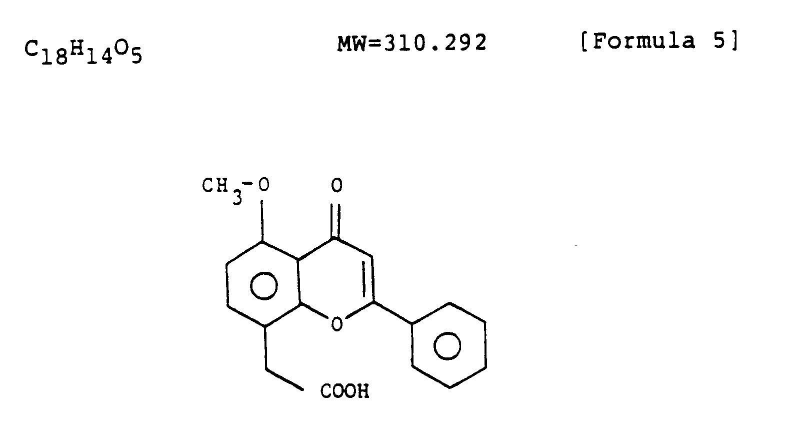

PF_{G}=245-248°C; IR vc=o (acid)=1720 cm⁻¹, Vc = o (pyrone) 1640 cm⁻¹; NMR (DMSO)δ in ppm in relation to TMS: 5H at 4 (s), 1H at 7.2 (s), 7H at 7.7 to 8.7 (m), 1H at 12.2 (exchangeable).

| Elemental analysis | | | |
|---|---|---|---|
| | C% | H% | 0% |
| calculated : | 69.67 | 4.55 | 29.78 |
| found : | 69.50 | 4.57 | |

### (METHOXY-2-PHENYL)-2-OXO-4-4H-(1) BENZOPYRAN-8-ACETIC ACID

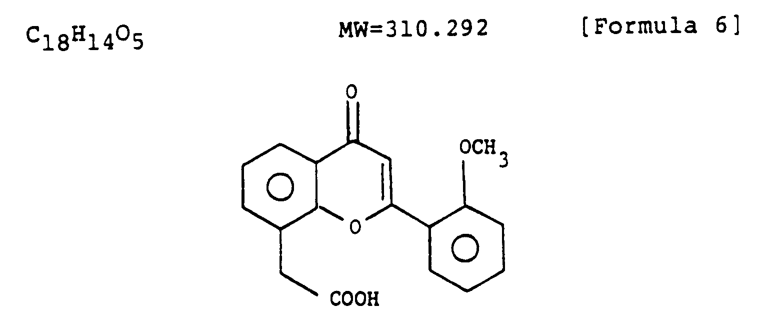

PF_{G}=203-205°C; IR vc=o (acid)=1730 cm⁻¹ ; Vc=o (pyrone)=1610 cm⁻¹; NMR (DMSO)δ in ppm in relation to TMS; 5H at 4 (s), 1H at 7 (s), 7H at 7.1 to 8.1 (m), 1H at 12.8 (exchangeable).

| Elemental analysis | | | |
|---|---|---|---|
| | C% | H% | 0% |
| calculated : | 69.67 | 4.55 | 25.78 |
| found : | 69.72 | 4.39 | |

### HYDROXY-3-OXO-4-PHENYL-2-4H-(1)-BENZOPYRAN-8-ACETIC ACID

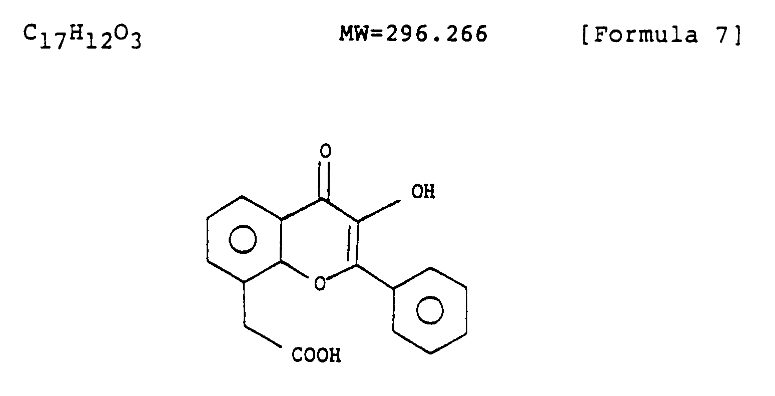

PF_{G}=221-223°C; IR Vc=o (acid)=1700 cm⁻¹, Vc=0 (pyrone)=1610 cm⁻¹; NMR (DMSO)δ in ppm in relation to the TMS; 2H at 4 (s), 8H) at 7.3-8.4 (m), 1H at 9.6 (exchangeable), 1H at 12.3 (exchangeable).

| Elemental analysis | | | |
|---|---|---|---|
| | C% | H% | 0% |
| calculated : | 68.92 | 4.08 | 27.00 |
| found : | 68.86 | 4.01 | |

### HYDROXY-5-OXO-4-PHENYL-2-4H-(1)-BENZOPYRAN-8-ACETIC ACID

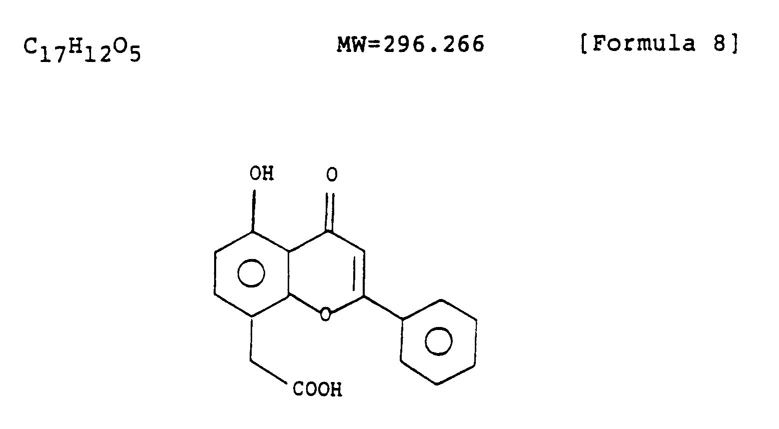

PF_{G}=233-238°C; IR Vc=o (acid)=1700 cm⁻¹, Vc=0 (pyrone)=1680 cm⁻¹; NMR (DMSO)δ in ppm in relation to TMS; 2H at 3.8 (s), 1H at 6.8 (d), 1H at 7.1 (s), 6H at 7.4 to 8.2 (m), 1H at 42.4 (exchangeable).

| Elemental analysis | | | |
|---|---|---|---|
| | C% | H% | 0% |
| calculated : | 68.92 | 4.08 | 27.00 |
| found : | 68.85 | 4.22 | |

### HYDROXY-7-OXO-4-PHENYL-2-4H-(1)-BENZOPYRAN-8-ACETIC ACID

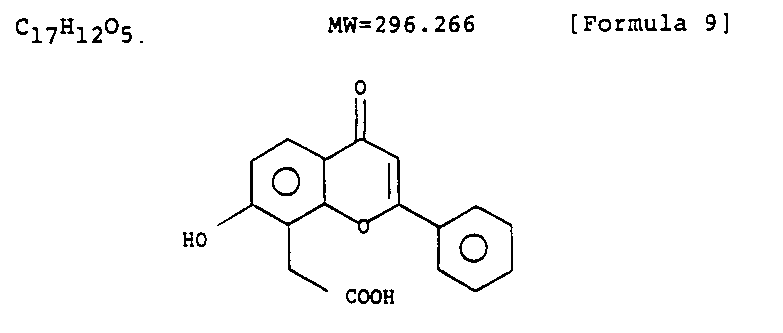

PF_{G}=227-238°C; IR Vc=o (acid)=1700 cm⁻¹, Vc=0 (pyrone)=1630 cm⁻¹; NMR (DMSO)δ in ppm in relation to TMS; 2H at 3.8 (s), 8H at 6.8 to 8.2 (m), 2H at 10.8 to 11.1 (exchangeable).

| Elemental analysis | | | |
|---|---|---|---|
| | C% | H% | 0% |
| calculated : | 68.92 | 4.08 | 27.00 |
| found : | 68.92 | 4.00 | |

### (HYDROXY-2-PHENYL)-2-OXO-4-4H-(1)-BENZOPYRAN-8-ACETIC ACID

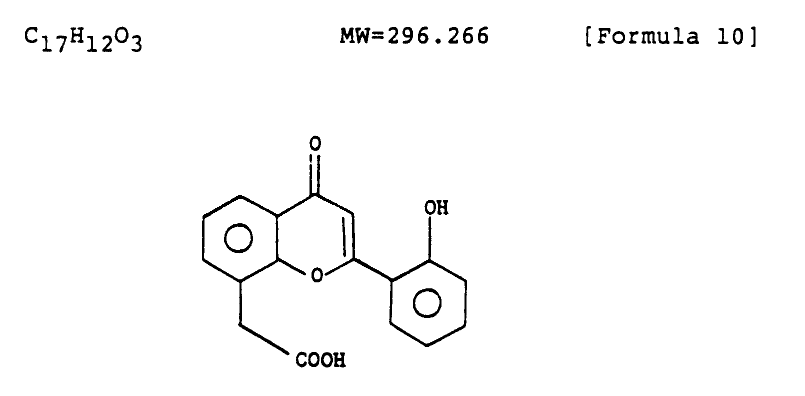

PF_{G}=288-292°C; IR Vc=o (acid)=1700 cm⁻¹, Vc=0 (pyrone)=1640 cm⁻¹; NMR (DMSO)δ in ppm in relation to TMS; 2H at 4 (s), 1H at 7 (S), 1H at 7 (s), 7H at 7.2 to 8.2 (m), 2H at 10.8 to 12.9 (exchangeable).

| Elemental analysis | | | |
|---|---|---|---|
| | C% | H% | 0% |
| calculated : | 68.92 | 4.08 | 27.00 |
| found : | 68.75 | 3.88 | |

### (HYDROXY-3-PHENYL)-2-OXO-4-4H-(1)-BENZOPYRAN-8-ACETIC ACID

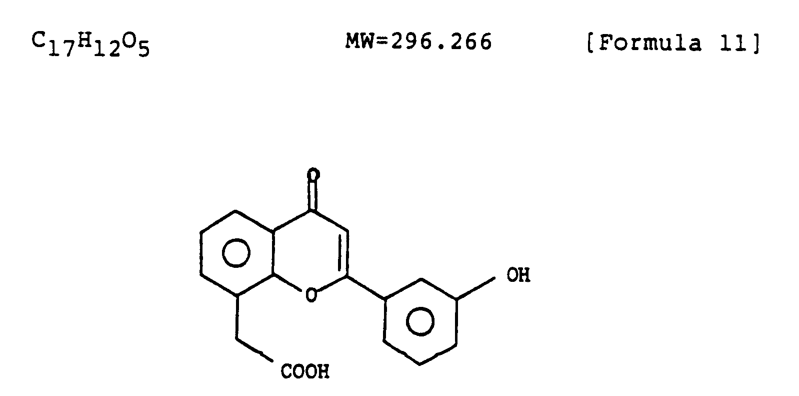

PF_{G}=259-288°C; IR Vc=o (acid)=1720 cm⁻¹, Vc=0 (pyrone)=1630 cm⁻¹; NMR (DMSO)δ in ppm in relation to TMS; 2H at 4.1 (s), 1H at 7 (s), 7H at 7.1 to 8.2 (m), 1H at 10 at 10 (exchangeable), 1H at 12.8 (exchangeable).

| Elemental analysis | | | |
|---|---|---|---|
| | C% | H% | 0% |
| calculated : | 68.92 | 4.08 | 27.00 |
| found : | 68.91 | 4.21 | |

### (HYDROXY-4-PHENYL)-2-OXO-4-4H-(1)-BENZOPYRAN-8-ACETIC ACID

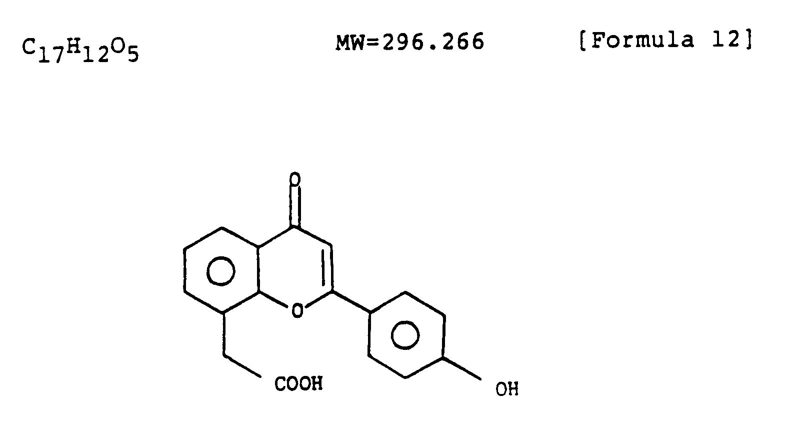

PF_{G}=261-268°C; IR Vc=o (acid)=1690 cm⁻¹, Vc=0 (pyrone)=1620 cm⁻¹; NMR (DMSO)δ in ppm in relation to TMS; 2H at 3.8 (s), 8H at 6.7 to 8 (m), 1H at 10.3 (exchangeable), 1H at 12.2 (exchangeable).

| Elemental analysis | | | |
|---|---|---|---|
| | C% | H% | 0% |
| calculated : | 68.92 | 4.08 | 27.00 |
| found : | 68.61 | 4.20 | |

### CHLOROHYDRATE OF (DIETHYLAMINOETHOXY-3-PHENYL)-2-OXO-4-4H- BENZOPYRAN-8-ACETIC ACID

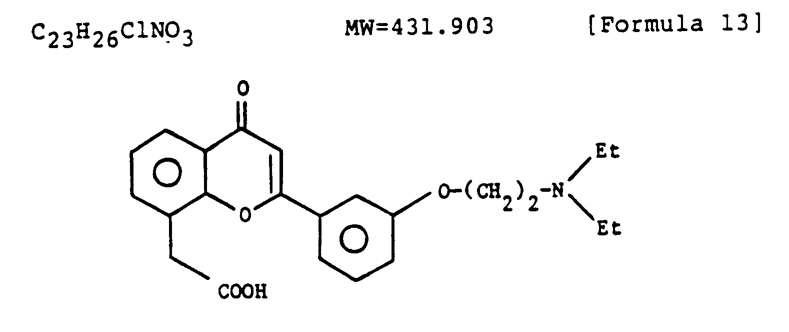

PF_{G}=176-179°C; IR Vc=o (acid)=1720 cm⁻¹, Vc=0 (pyrone)=1640 cm⁻¹; NMR (DMSO)δ in ppm in relation to TMS; 3H at 1.4 (t), 11H at 3 to 4.6 (m, of which 1H is interchangeable), 1H at 7.1 (s), 6H at 7.2 to 8.1 (m), 1H at 13.2 (interchangeable).

| Elemental analysis | | | | | |
|---|---|---|---|---|---|
| | C% | H% | Cl% | N% | 0% |
| calculated : | 63.96 | 6.07 | 8.21 | 3.24 | 18.32 |
| found : | 63.69 | 5.88 | 8.09 | 3.01 | |

### (PHENOXY-2-PHENYL-2-OXO-4-4H-(1)-BENZOPYRAN-8-ACETIC ACID

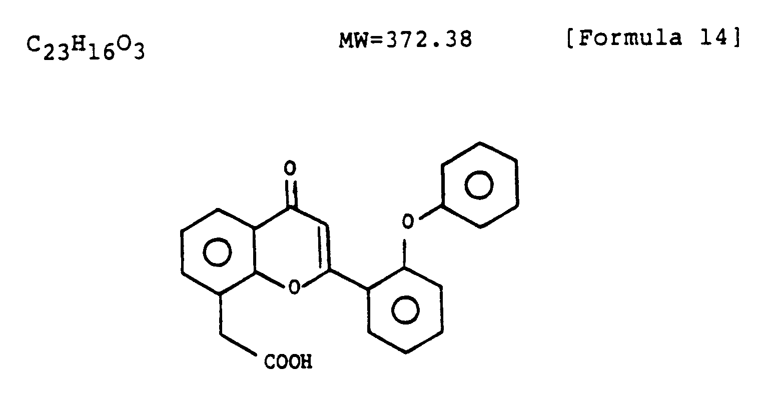

PF_{G}=218-220°C; IR Vc=o (acid)=1680 cm⁻¹, Vc=0 (pyrone)=1640 cm⁻¹; NMR (DMSO)δ in ppm in relation to TMS; 2H at 3.8 (s), 13H at 6.8 to 8 (m), 1H at 12.6 (interchangeable)

| Elemental analysis | | | |
|---|---|---|---|
| | C% | H% | 0% |
| calculated : | 74.19 | 4.33 | 21.48 |
| found : | 73.88 | 4.56 | |

### FLUORO-6-OXO-4-PHENYL-2-4H-(1)-BENZOPYRAN-8-ACETIC ACID

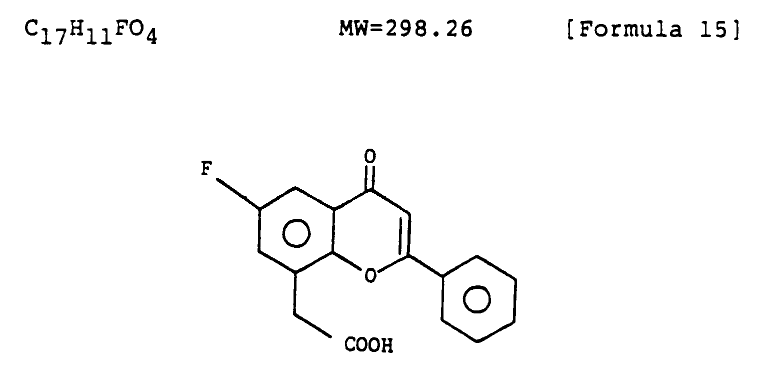

PF_{G}=225-239°C: IR Vc=o (acid)=1720 cm⁻¹, Vc=0 (pyrone)=1640 cm⁻¹; NMR (DMSO)δ in ppm in relation to TMS; 3H at 3 to 4 (m, of which 1H is interchangeable), 1H at 7 (s), 7H at 7, 1H at 8.4 (m).

| Elemental analysis | | | | |
|---|---|---|---|---|
| | C% | H% | F% | 0% |
| calculated : | 68.49 | 3.72 | 6.37 | 21.46 |
| found : | 68.42 | 3.92 | 6.28 | |

### (FLUORO-2-PHENYL)-2-OXO-4-4H-(1)-BENZOPYRAN-8-ACETIC ACID

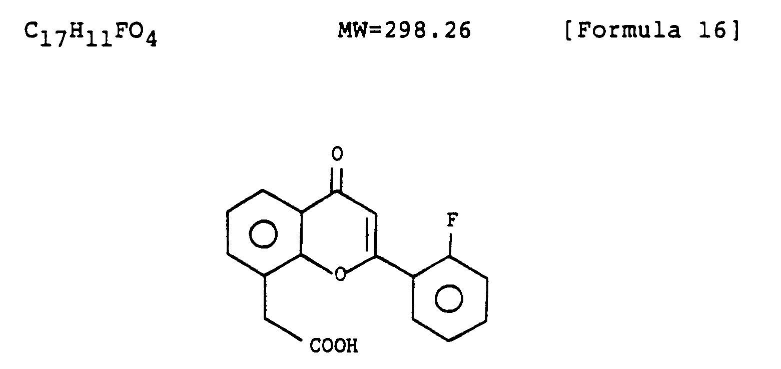

PF_{G}=193-199°C; IR Vc=o (acid)=1720 cm⁻¹, Vc=0 (pyrone)=1610 cm⁻¹ NMR (DMSO)δ in ppm in relation to TMS; 2H at 4 (s), 1H at 6.7 (s), 7H at 7.2 to 8.4 (m), 1H at 12.5 (interchangeable).

| Elemental analysis | | | | |
|---|---|---|---|---|
| | C% | H% | F% | 0% |
| calculated : | 68.49 | 3.72 | 6.37 | 21.46 |
| found : | 68.42 | 3.92 | 6.28 | |

### (FLUORO-PHENYL)-2-OXO-4H-(1)-BENZOPYRAN-8-ACETIC ACID

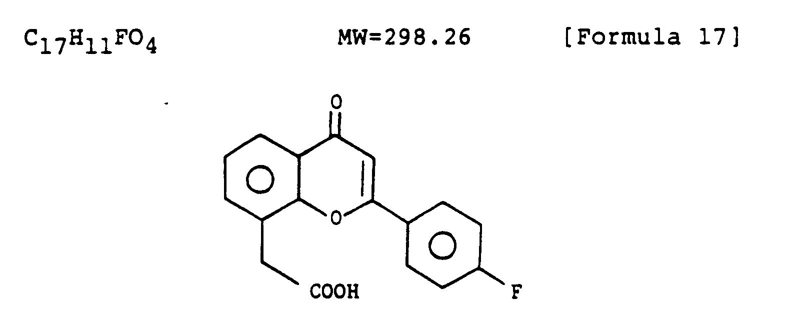

PF_{G}=215-217°C; IR Vc=o (acid)=1720 cm⁻¹, Vc=0 (pyrone)=1640 cm⁻¹; NMR (CF₃COOD)δ in ppm in relation to TMS; 2H at 4 (s), 8H at 7 to 9 (m).

| Elemental analysis | | | | |
|---|---|---|---|---|
| | C% | H% | F% | 0% |
| calculated : | 67.49 | 3.72 | 6.37 | 24.46 |
| found : | 68.54 | 3.80 | 6.33 | |

### (FLUORO-3-PHENYL)-2-OXO-4-4H-(1)-BENZOPYRAN-8-ACETIC ACID

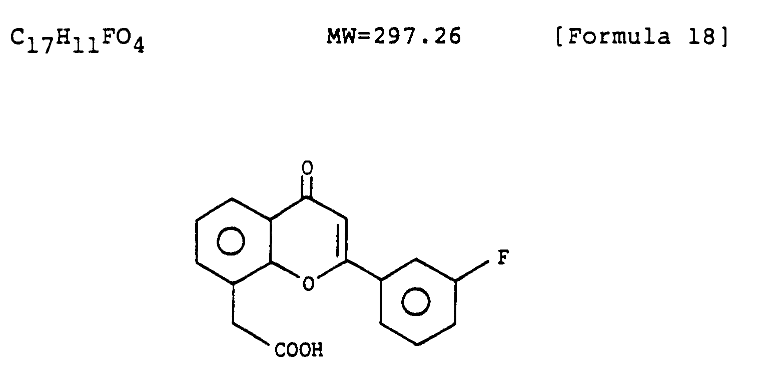

PF_{G}=201-203°C; IR Vc=o (acid)=1700 cm⁻¹, Vc=0 (pyrone)=1640 cm⁻¹; NMR (DMSO)δ in ppm in relation to TMS; 2H at 4 (s), 1H at 7.1 (s), 7H at 7.2 to 8 (m), 1H at 12.6 (interchangeable).

| Elemental analysis | | | | |
|---|---|---|---|---|
| | C% | H% | F% | 0% |
| calculated : | 68.49 | 3.72 | 6.37 | 21.46 |
| found : | 68.20 | 3.69 | 6.28 | |

### PHENYL-4-PHENYL)2-OXO-4-4H-(1)-BENZOPYRAN-8-ACETIC ACID

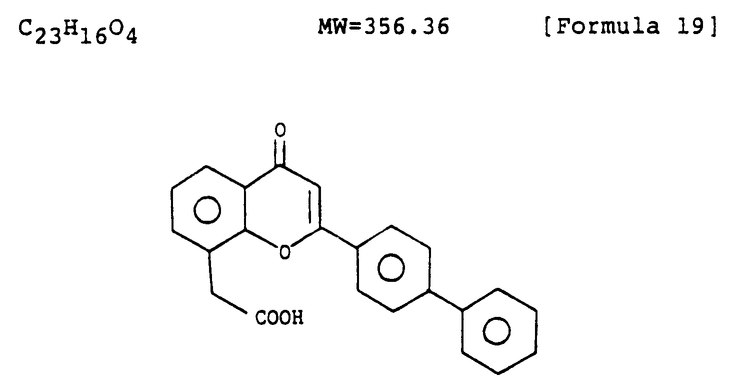

PF_{G}=229-231°C; IR Vc=o (acid)=1710 cm⁻¹, Vc=0 (pyrone)=1620 cm⁻¹; NMR (DMSO)δ in ppm in relation to TMS; 2H at 4 (s), 1H at 7 (s), 12H at 7.2 to 8.4 (m), 1H at 12.6 (interchangeable).

| Elemental analysis | | | |
|---|---|---|---|
| | C% | H% | 0% |
| calculated : | 77.51 | 4.53 | 17.96 |
| found : | 77.42 | 4.41 | |

### (CHLORO-4-PHENYL)-2-OXO-4-4H-(1)-BENZOPYRAN-8-ACETIC ACID

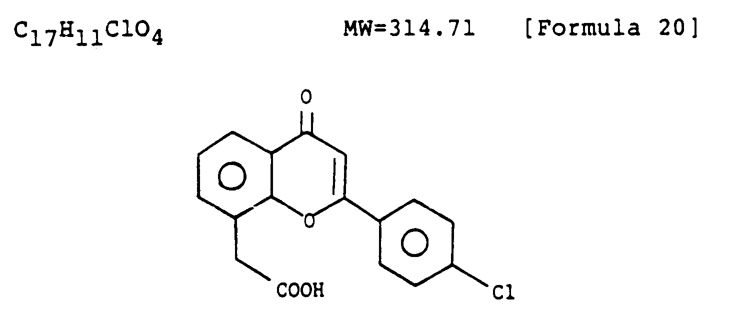

PF_{G}=238-242°C; IR Vc=o (acid)=1720 cm⁻¹, Vc=0 (pyrone)=1620 cm⁻¹; NMR (DMSO)δ in ppm in relation to TMS; 2H at 4 (s), 1H at 7 (s), 7H at 7.2 to 8.2 (m), 1H at 12.5 (interchangeable).

| Elemental analysis | | | | |
|---|---|---|---|---|
| | C% | H% | Cl% | 0% |
| calculated : | 64.87 | 3.52 | 11.27 | 20.34 |
| found : | 64.83 | 3.37 | 11.55 | |

### (CARBOXY-4-PHENYL)-2-OXO-4-4H-(1)-BENZOPYRAN-8-ACETIC ACID

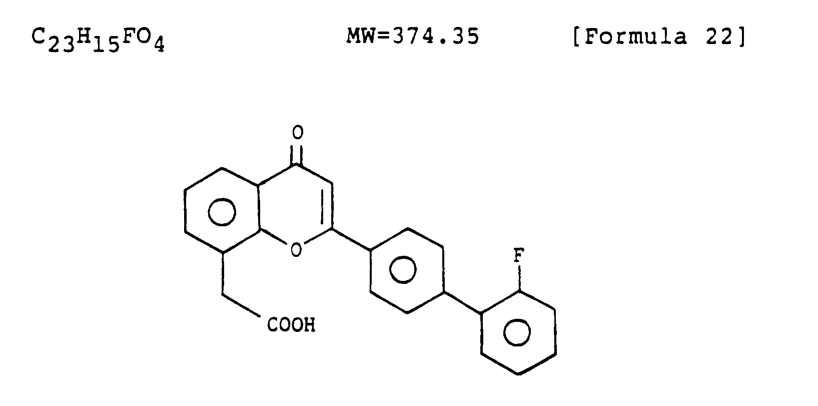

PF_{G}=312-314°C; IR Vc=o (acid)=1700-1720 cm⁻¹, Vc=0 (pyrone)=1640 cm⁻¹.

| Elemental analysis | | | |
|---|---|---|---|
| | C% | H% | 0% |
| calculated : | 66.67 | 3.73 | 29.69 |
| found : | 66.76 | 3.73 | |

### (FLUORO-2-PHENYL)-4-PHENYL)-2-OXO-4-4H-(1) BENZOPYRAN-8-ACETIC ACID

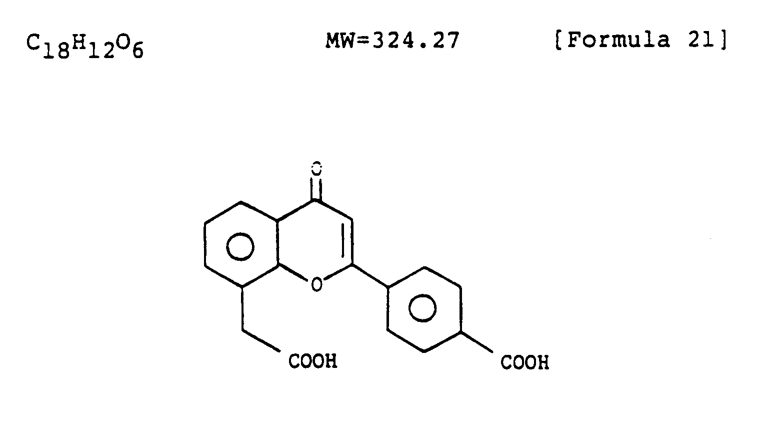

PF_{G}=226-228°C; IR Vc=o (acid)=1720 cm⁻¹, Vc=0 (pyrone)=1630 cm⁻¹; NMR (DMSO)δ in ppm in relation to TMS; 2H at 4 (S), 12H at 7 to 8.4 (m), 1H to 12.8 (interchangeable).

| Elemental analysis | | | | |
|---|---|---|---|---|
| | C% | H% | F% | 0% |
| calculated : | 73.79 | 4.04 | 5.08 | 17.10 |
| found : | 73.80 | 4.14 | 4.87 | |

### (NITRO-2-PHENYL)-2-OXO-4-4H-(1)-BENZOPYRAN-8-ACETIC ACID

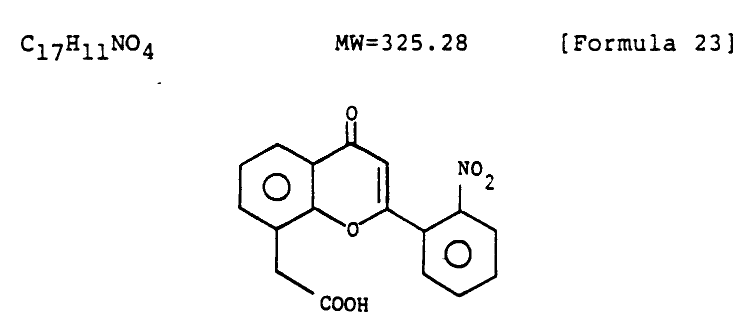

PF_{G}=180-182°C; IR Vc=o (acid)=1700 cm⁻¹, Vc=0 (pyrone)=1640 cm⁻¹; NMR (DMSO)δ in ppm in relation to TMS; 2H at 4 (s), 1H at 6.8 (s), 7H at 7.3 to 8.3 (m), 1H at 12.8 (interchangeable).

| Elemental analysis | | | | |
|---|---|---|---|---|
| | C% | H% | N% | 0% |
| calculated : | 62.77 | 3.41 | 4.31 | 29.51 |
| found : | 62.82 | 3.47 | 4.20 | |

### (NITRO-3-PHENYL)-2-OXO-4-4H-(1)-BENZOPYRAN-8-ACETIC ACID

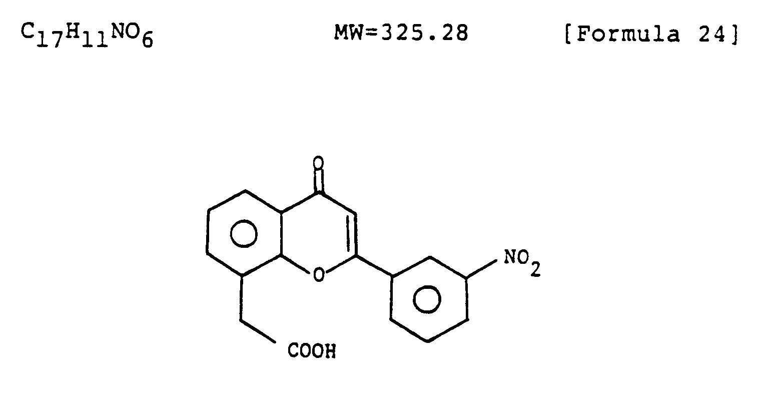

PF_{G}=203-208°C; IR Vc=o (acid)=1720 cm⁻¹, Vc=0 (pyrone)=1630 cm⁻¹; NMR (DMSO)δ in ppm in relation to TMS; 2H at 4 (s), 1H at 7.3 (s), 7H at 7.4 to 9 (m), 1H at 12.6 (interchangeable).

| Elemental analysis | | | | |
|---|---|---|---|---|
| | C% | H% | N% | 0% |
| calculated : | 62.77 | 3.41 | 4.31 | 29.51 |
| found : | 62.49 | 3.40 | 4.31 | |

### (NITRO-4-PHENYL)-2-OXO-4-4H-(1)-BENZOPYRAN-8-ACETIC ACID

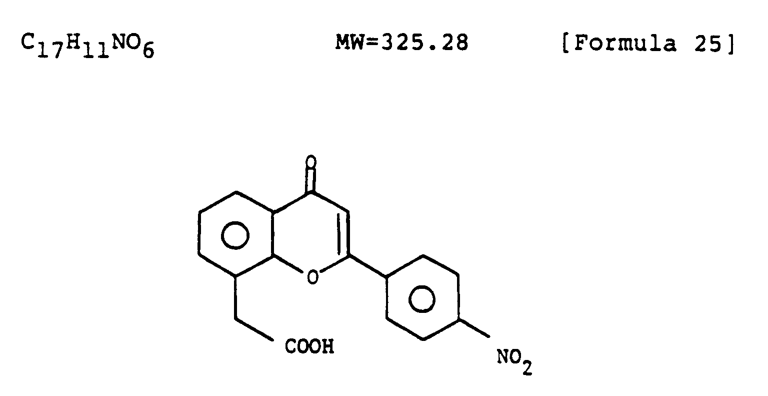

PF_{G}=242-244°C; IR Vc=o (acid)=1720 cm⁻¹, Vc=0 (pyrone)=1620 cm⁻¹; NMR (DMSO)δ in ppm in relation to TMS; 2H at 4 (s), 1H at 7 (s), 7H at 7.2 to 8.3 (m), 1H at 12.5 (interchangeable).

| Elemental analysis | | | | |
|---|---|---|---|---|
| | C% | H% | N% | 0% |
| calculated : | 62.77 | 3.41 | 4.31 | 29.51 |
| found : | 62.92 | 3.38 | 4.28 | |

### (AMINO-3-PHENYL)-2-OXO-4-4H-(1)-BENZOPYRAN-8-ACETIC ACID

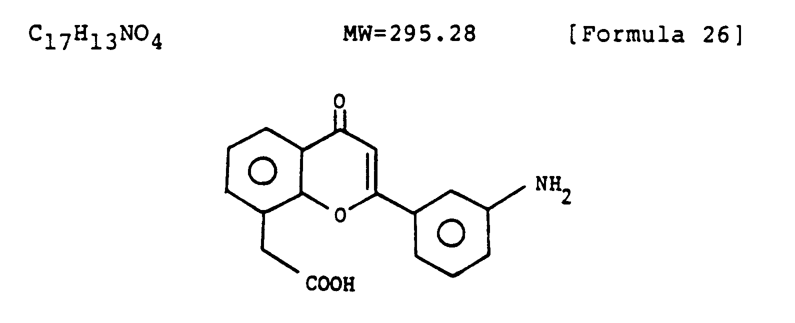

PF_{G}=227-139°C; IR Vc=o (acid)=1720 cm⁻¹, Vc=0 (pyrone)=1630 cm⁻¹; NMR (DMSO)δ in ppm in relation to TMS; 2H at 4 (s), 9H at 6.8 to 8 (m), 1H at 12.6 (interchangeable).

| Elemental analysis | | | | |
|---|---|---|---|---|
| | C% | H% | N% | 0% |
| calculated : | 69.14 | 4.44 | 4.74 | 21.67 |
| found : | 69.20 | 4.70 | 4.94 | |

### (AMINO-2-4-PHENYL)-2-OXO-4-4H-(1)-BENZOPYRAN-8-ACETIC ACID

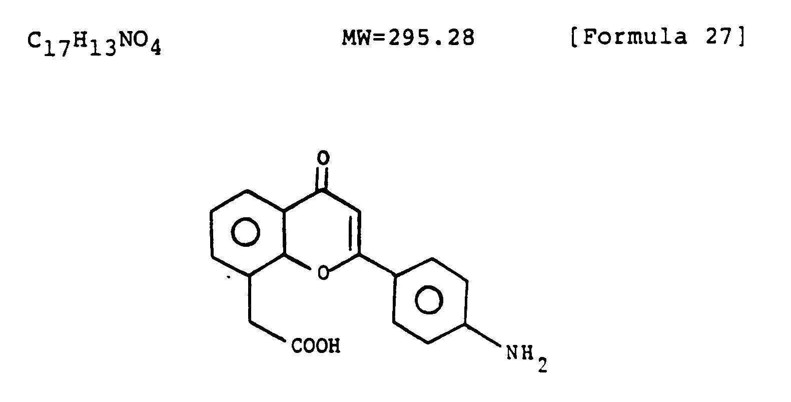

PF_{G}=189°C; IR Vc=o (acid)=1700 cm⁻¹, Vc=0 (pyrone)=1620 cm⁻¹.

| Elemental analysis | | | | |
|---|---|---|---|---|
| | C% | H% | N% | 0% |
| calculated : | 69.14 | 4.44 | 4.74 | 21.67 |
| found : | 69.00 | 4.48 | 4.66 | |

### OXO-4-PHENYL-2-4H-(1)-BENZOPYRAN-8-ACETIC ACID

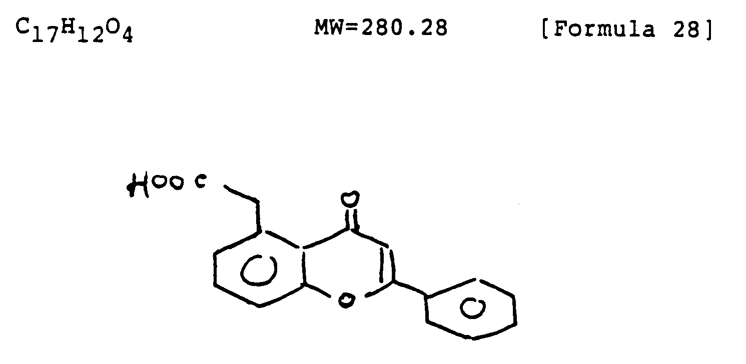

PF_{G}=240-242°C; IR Vc=o (acid)=1740 cm⁻¹, Vc=0 (pyrone)=1640 cm⁻¹; NMR (DMSO)δ in ppm in relation to TMS; 2H at 4 (s), 1H at 7 (s), 8H at 7.2 to 8.4 (m), 1H at 12.6 (interchangeable).

| Elemental analysis | | | |
|---|---|---|---|
| | C% | H% | 0% |
| calculated : | 72.85 | 4.32 | 22.83 |
| found : | 73.00 | 4.16 | |

### OXO-4-PHENYL-2-4H-(1) BENZOPYRAN-7-ACETIC ACID

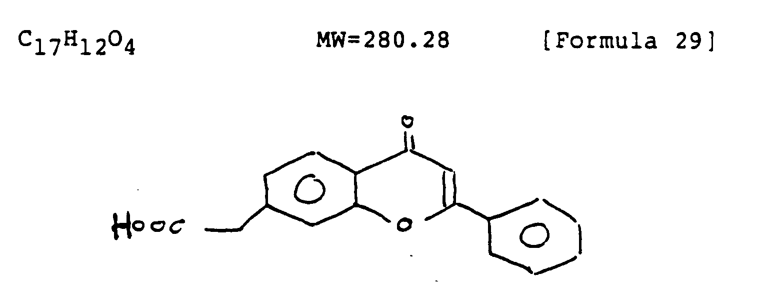

PF_{G}=237-239°C; IR Vc=o (acid)=1740 cm⁻¹, Vc=0 (pyrone)=1620 cm⁻¹; NMR (DMSO)δ in ppm in relation to TMS; 2H at 3.7 (s), 1H at 6.8 (s), 7H at 7.2 to 8 (m), 1H at 12.5 (interchangeable).

| Elemental analysis | | | |
|---|---|---|---|
| | C% | H% | 0% |
| calculated : | 72.85 | 4.32 | 22.83 |
| found : | 72.73 | 4.33 | |

### TRIFLUOROMETHYL-2-OXO-4-4H-(1)-BENZOPYRAN-8-ACETIC ACID

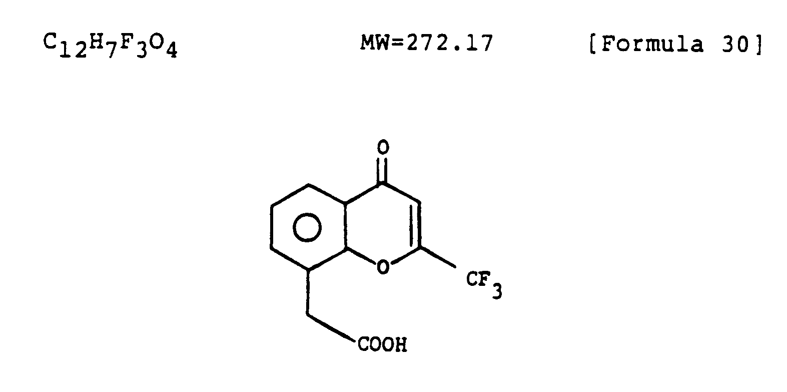

PF_{G}=141-143°C; IR Vc=o (acid)=1700 cm⁻¹, Vc=0 (pyrone)=1650 cm⁻¹.

| Elemental analysis | | | | |
|---|---|---|---|---|
| | C% | H% | F% | 0% |
| calculated : | 52.95 | 2.59 | 20.94 | 23.52 |
| found : | 52.72 | 2.64 | 20.35 | |

### OXO-4-PHENYL-2-4H-(1)-BENZOPYRAN-8-ACETIC ACID

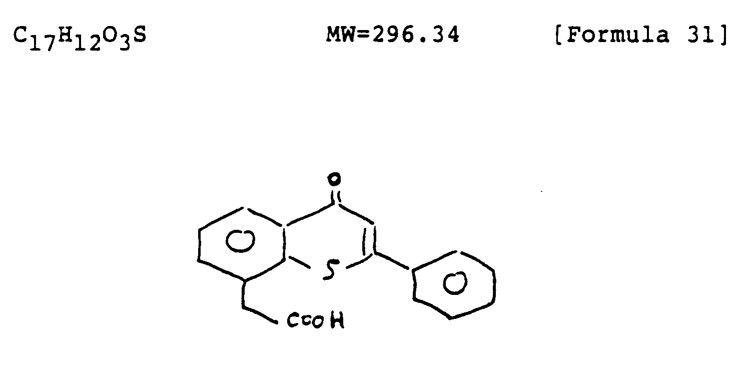

PF_{G}=198-200°C; IR Vc=o (acid)=1720 cm⁻¹, Vc=0 (pyrone)=1610 cm⁻¹; NMR (DMSO)δ in ppm in relation to TMS; 2H at 4 (s), 1H at 7.3 (s), 7H at 7.3 to 8.4 (m), 1H at 12.5 (s), 1H at 7.3 (s), 7H at 7.3 to 8.4 (m), 1H at 12.5 (interchangeable).

| Elemental analysis | | | | |
|---|---|---|---|---|
| | C% | H% | O% | S% |
| calculated : | 68.40 | 4.08 | 16.20 | 10.82 |
| found : | 69.04 | 4.29 | | 11.04 |

### OXO-4-PHENYL-2-4H-(1)-BENZOPYRAN-8-ACETIC ACID

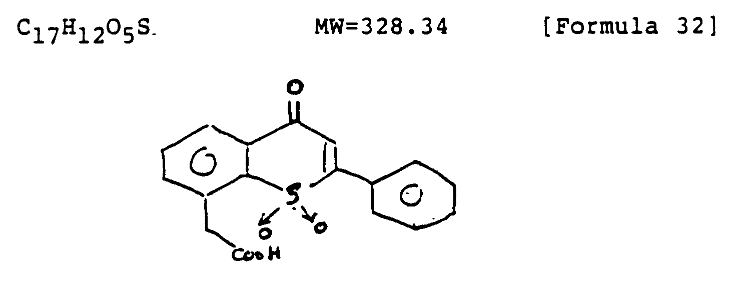

PF_{G}=184-187°C; IR Vc=o (acid)=1700 cm⁻¹, Vc=0 (pyrone)=1660 cm⁻¹; NMR (DMSO)δ in ppm in relation to TMS; 2H at 4 (s), 1H at 7 (s), 7H at 7.2 to 8.2 (m), 1H at 12.6 (interchangeable).

| Elemental analysis | | | | |
|---|---|---|---|---|
| | C% | H% | O% | S% |
| calculated : | 62.18 | 3.68 | 24.36 | 9.77 |
| found : | 62.29 | 3.68 | | 9.65 |

### OXO-4-PHENYL-2-DIHYDRO-1-4-QUINOLINE-8-ACETIC ACID

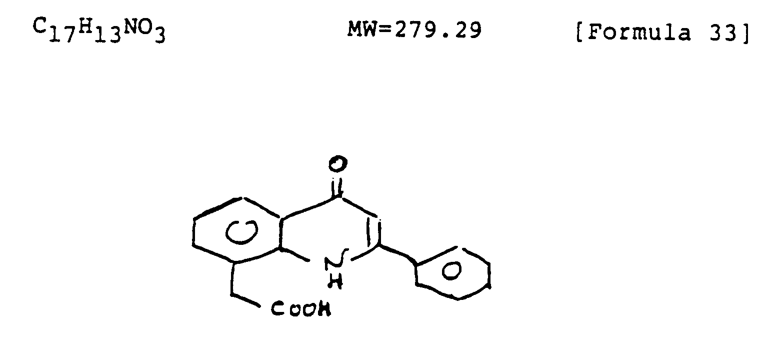

PF_{G}=236-238°C; IR Vc=o (acid)=1680 cm⁻¹, Vc=0 (pyrone)=1620 cm⁻¹; NMR (DMSO)δ in ppm in relation to TMS; 2H at 4 (s), 8H at 7 to 8.3 (m), 1H at 8.5 (interchangeable).

| Elemental analysis | | | | |
|---|---|---|---|---|
| | C% | H% | N% | 0% |
| calculated : | 73.11 | 4.69 | 5.01 | 17.18 |
| found : | 73.10 | 4.62 | 5.04 | |

### OXO-4-PHENYL-2-4H-(1)-BENZOSELENOPYRAN-8-ACETIC ACID

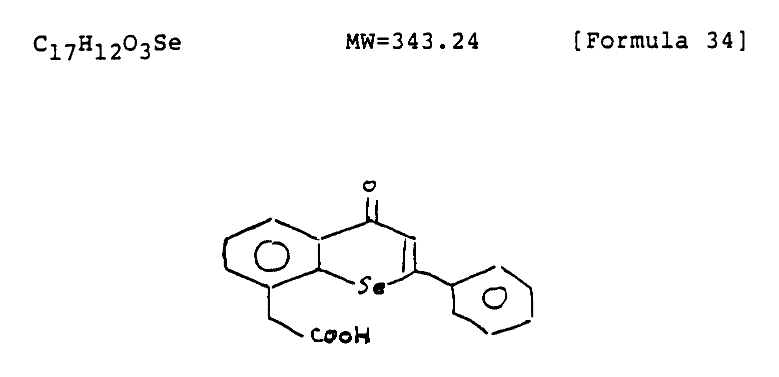

PF_{G}=182-184°C; IR Vc=o (acid)=1700 cm⁻¹, Vc=0 (pyrone)=1600 cm⁻¹; NMR (DMSO)δ in ppm in relation to TMS; 2H at 4 (s), 8H at 7.4 to 8.6 (m), 1H at 12.5 (interchangeable).

| Elemental analysis | | | | |
|---|---|---|---|---|
| | C% | H% | O% | Se% |
| calculated : | 59.49 | 3.52 | 13.98 | 23.00 |
| found : | 59.30 | 3.26 | | 22.91 |

### OXO-7-7H-BENZO(c)XANTHENYL-11-ACETIC ACID

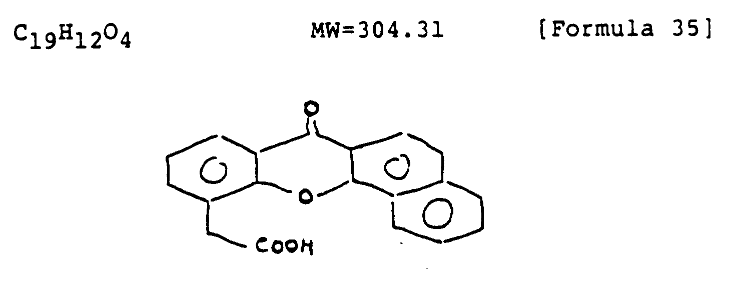

PF_{G}=270-272°C; IR Vc=o (acid)=1720 cm⁻¹, Vc=0 (pyrone)=1620 cm⁻¹; NMR (DMSO)δ in ppm in relation to TMS; 2H at 4 (s), 9H at 7.4 to 9.2 (m), 1H at 12.5 (interchangeable).

| Elemental analysis | | | |
|---|---|---|---|
| | C% | H% | 0% |
| calculated : | 74.99 | 3.97 | 21.03 |
| found : | 74.34 | 3.93 | |

### OXO-4-7-7H-DIBENZO(c,h)XANTHENYL-1-ACETIC ACID

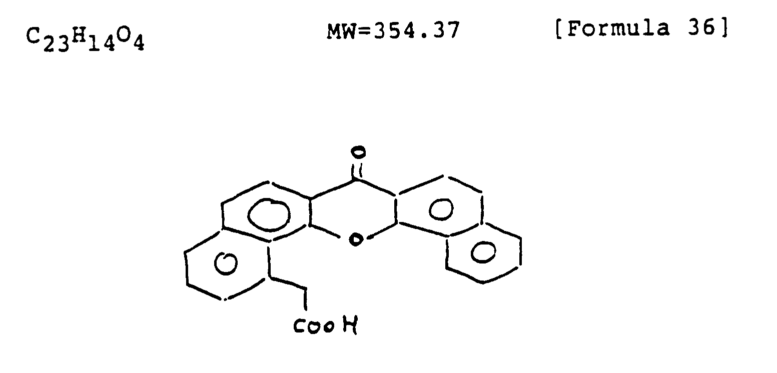

PF_{G}=276-278°C; IR Vc=o (acid)=1700 cm⁻¹, Vc=0 (pyrone)=1620 cm⁻¹; NMR (DMSO)δ in ppm in relation to TMS; 2H at 4 (s), 11H at 7.4 to 8.8 (m), 1H at 12.5 (interchangeable).

| Elemental analysis | | | |
|---|---|---|---|
| | C% | H% | 0% |
| calculated : | 77.96 | 3.98 | 18.06 |
| found : | 77.94 | 3.97 | |

### CARBOXYMETHYL-4-PHENYL)-2-4H-(1) BENZOPYRANONE-4

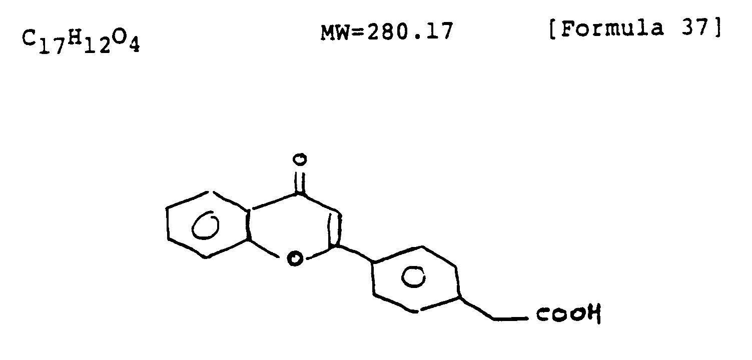

PF_{G}=204°C; IR Vc=o (acid)=1720 cm⁻¹, Vc=0 (pyrone)=1640 cm⁻¹.

| Elemental analysis | | | |
|---|---|---|---|
| | C% | H% | 0% |
| calculated : | 72.84 | 4.32 | 22.84 |
| found : | 72.08 | 4.33 | |

### (CARBOXYMETHYL-3-PHENYL)-2-4H-(1) BENZOPYRANONE-4

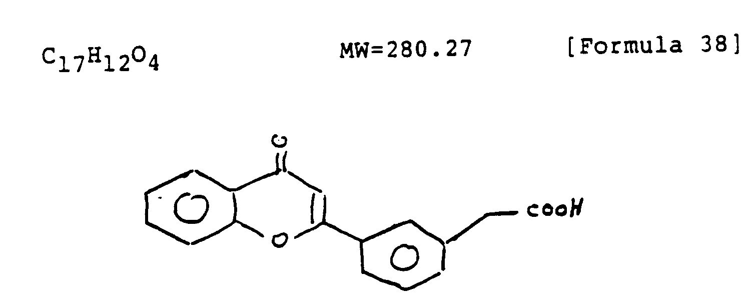

PF_{G}=181-183°C; IR Vc=o (acid)=1720 cm⁻¹, Vc=0 (pyrone)=1620 cm⁻¹; NMR (DMSO)δ in ppm in relation to TMS; 2H at 3.8 (s), 1H at 7 (s), 8H at 7.4 to 8.2 (m), 1H at 12.4 (interchangeable).

| Elemental analysis | | | |
|---|---|---|---|
| | C% | H% | 0% |
| calculated : | 72.84 | 4.32 | 22.84 |
| found : | 73.08 | 4.41 | |

### (CARBOXYMETHYL-2-PHENYL)-2-4H-(1) BENZOPYRANONE-4

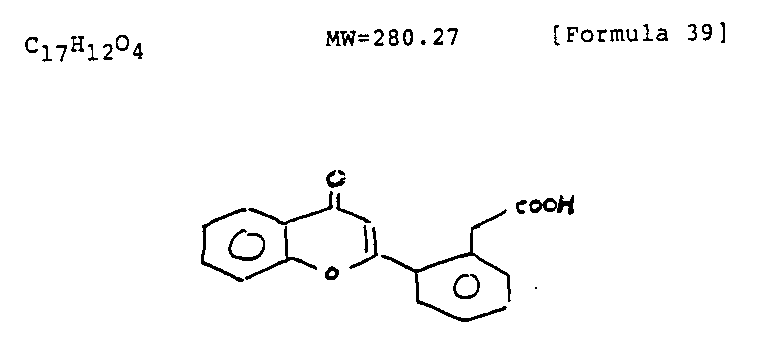

PF_{G}=179-181°C; IR Vc=o (acid)=1730 cm⁻¹, Vc=0 (pyrone)=1630 cm⁻¹; NMR (DMSO)δ in ppm in relation to TMS; 2H at 3.9 (s), 1H at 6.6 (s), 8H at 7.2 to 8.2 (m), 1H at 12.4 (interchangeable).

| Elemental analysis | | | |
|---|---|---|---|
| | C% | H% | 0% |
| calculated : | 72.84 | 4.32 | 22.84 |
| found : | 73.79 | 4.34 | |

### ((OXO-4-PHENYL-2-4H-(1) BENZOPYRAN-8-YL) METHYL) PHOSPHONATE OF DIETHYL

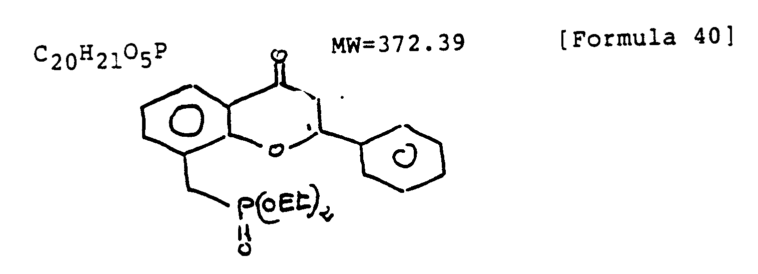

PF_{G}=107-109°C; IR Vc=0 (pyrone)=1640 cm⁻¹; NMR (CDCl₃)δ in ppm in relation to TMS; 6H at 1.2 (d), 2H at 3.57 (d), 4H at 3.7 to 4.4 (m), 1H at 6.85 (s), 8H at 7.2 to 8.4 (m).

| Elemental analysis | | | | |
|---|---|---|---|---|
| | C% | H% | O% | P% |
| calculated : | 64.51 | 5.69 | 21.48 | 8.32 |
| found : | 64.59 | 5.67 | | 8.17 |

### ((OXO-4-PHENYL-2-4H-(1) BENZOPYRAN-8-YL) METHYL) PHOSPHONIC ACID

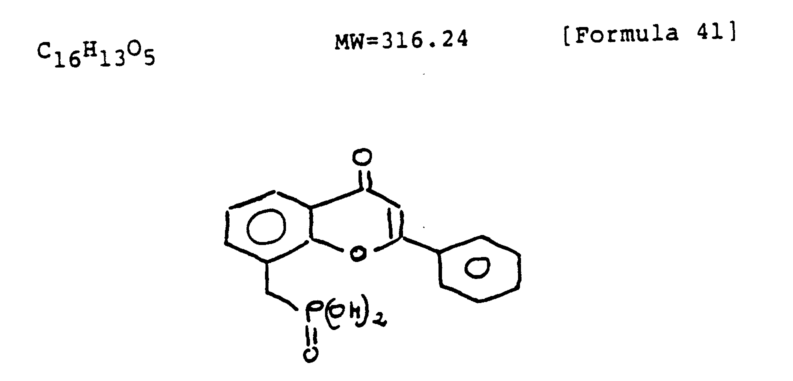

PF_{G}=331-334°C; IR V OH = 3400 to 2200 cm⁻¹, Vc=0 (pyrone)=1620 cm⁻¹; NMR (DMSO)δ in ppm in relation to TMS; 2H at 3.45 (d), 1H at 7.03 (s), 8H at 7.2 to 8.4 (m), 2H at 9.7 (interchangeable).

| Elemental analysis | | | | |
|---|---|---|---|---|
| | C% | H% | O% | P% |
| calculated : | 60.76 | 4.14 | 29.30 | 9.80 |
| found : | 60.77 | 4.17 | | 9.83 |

### Example 2

### (PHENYL-2-OXO-4-4H-(1) BENZOPYRAN-8-YL)-2-ACRYLIC ACID

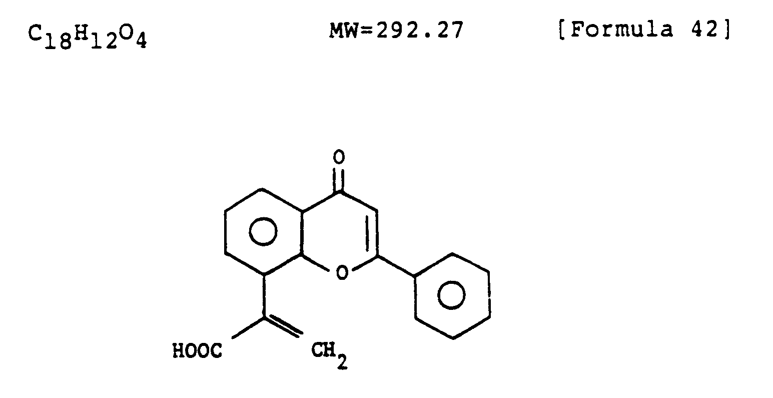

8.4 g (0.03 mole) of oxo-4-phenyl-2-4H-{4}-benzopyran-8-acetic and 81 ml of N,N,N′,N′ tetramethyldiaminomethane are mixed. 81 ml acetic acid are then added to the reaction mixture cooled in an ice bath. The temperature rises to 65°C, then falls to 20°C. Stirring continues for one (1) hour, then the mixture is poured into water. The solid formed is centrifuged, dried, and recrystallized in acetic acid. Weight obtained: 3.4 g (yield: 38.6%); PF_{G}=240-247°C; IR Vc=o (acid)=1689 cm⁻¹, Vc=0 (pyrone)=1620 cm⁻¹; NMR (DMSO)δ in ppm in relation to TMS; 2H at 6.3 (d), 1H at 7 (s), 8H at 7.3 to 8.2 (m), 1H at 13 (interchangeable).

| Elemental analysis | | | |
|---|---|---|---|
| | C% | H% | 0% |
| calculated : | 73.96 | 4.14 | 21.90 |
| found : | 74.21 | 4.05 | |

### Example 3

### PHENYL-3-(PHENYL-2-OXO-4-4H-[1] BENZOPYRAN-8-YL)-2-ACRYLIC ACID

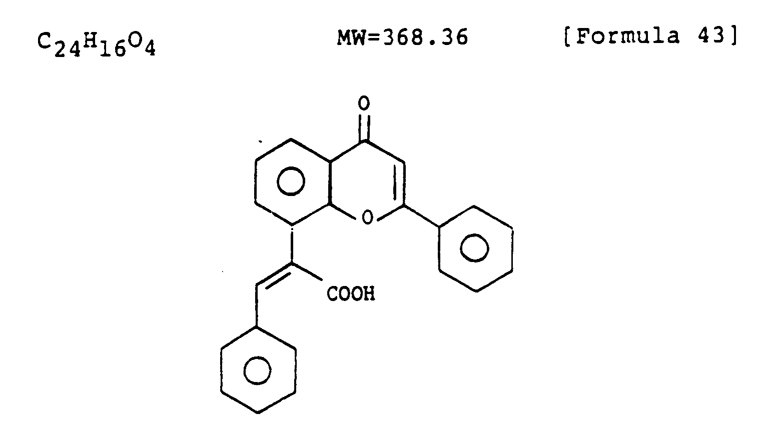

A mixture of 9.2 g (0.087 mole) of benzaldehyde, 16.8 g (0.06 mole) of oxo-4-phenyl-2-4H-[1]-benzopyranacetic acid, 30.9 ml of acetic anhydride, and 8.32 ml of triethylamine is refluxed for ten (10) minutes. The mixture is then poured into 30 ml of water. The precipitate formed is centrifuged, dried and recrystallized in acetic acid. Weight obtained: 9.8 g (yield: 44.3%); PF_{G}=215-220°C; IR Vc=o (acid)=1680 cm⁻¹, Vc=0 (pyrone)=1630 cm⁻¹ NMR (DMSO)δ in ppm in relation to TMS; 15H at 6.8 (m), 12.5 (interchangeable).

| Elemental analysis | | | |
|---|---|---|---|
| | C% | H% | 0% |
| calculated : | 78.25 | 4.39 | 17.31 |
| found : | 77.90 | 4.11 | |

Using the same technique, the following compounds were prepared:

### (BROMO-2-PHENYL)-3-(PHENYL-2-OXO-4-4H-[1] BENZOPYRAN-8-YL]-3-ACRYLIC ACID

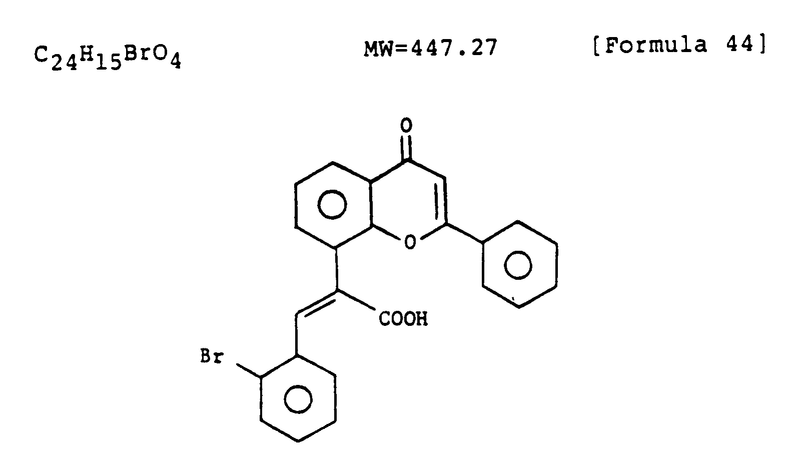

PF_{G}=217-219°C; IR Vc=o (acid)=1680 cm⁻¹, Vc=0 (pyrone)=1640 cm⁻¹; NMR (DMSO)δ in ppm in relation to TMS; 14H at 6.8 to 8.1 (m), 1H at 12.8 (interchangeable).

| Elemental analysis | | | | |
|---|---|---|---|---|
| | C% | H% | Br% | 0% |
| calculated : | 64.44 | 3.38 | 17.87 | 14.31 |
| found : | 64.29 | 3.37 | 17.58 | |

### (PYRIDINYL-4)-3-(PHENYL-2-OXO-4-4H-[1] BENZOPYRAN-8-YL)-3-ACRYLIC ACID

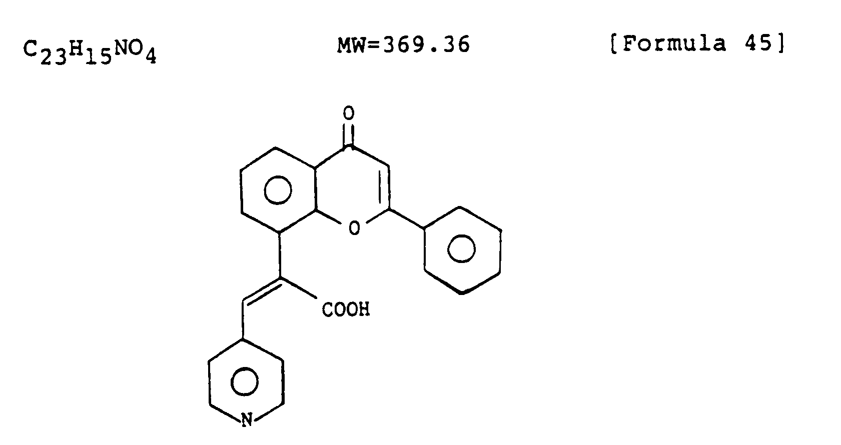

PF_{G}=272-283°C; IR Vc=o (acid)=1700 cm⁻¹, Vc=0 (pyrone)=1640 cm⁻¹; NMR (DMSO)δ in ppm in relation to TMS; 14H at 6.8 to 8.4 (m), 1H at 12.8 (interchangeable).

| Elemental analysis | | | | |
|---|---|---|---|---|
| | C% | H% | N% | 0% |
| calculated : | 74.79 | 4.09 | 3.79 | 17.33 |
| found : | 74.54 | 4.00 | 3.79 | |

### (PYRIDINYL-3)-3-(PHENYL-2-OXO-4-4H-[1] BENZOPYRAN-3-ACRYLIC ACID

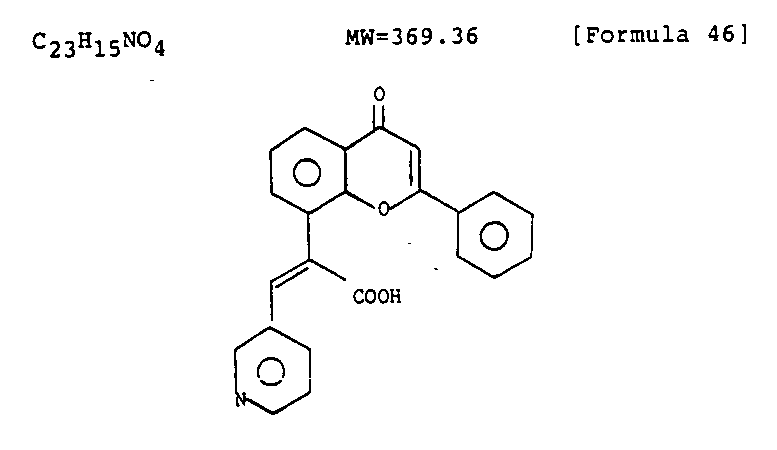

PF_{G}=118-124°C; IR Vc=o (acid)=1720 cm⁻¹, Vc=0 (pyrone)=1630 cm⁻¹; NMR (DMSO)δ in ppm in relation to TMS; 14H at 7 to 8.5 (m), 12.5 (interchangeable).

| Elemental analysis | | | | |
|---|---|---|---|---|
| | C% | H% | N% | 0% |
| calculated : | 74.79 | 4.09 | 3.79 | 17.33 |
| found : | 74.36 | 4.09 | 3.50 | |

### Example 4

### CHLOROHYDRATE OF [(METHYL-4-PIPERAZINYL) METHYL]-8-PHENYL-2-4H-[1] BENZOPYRANONE-4

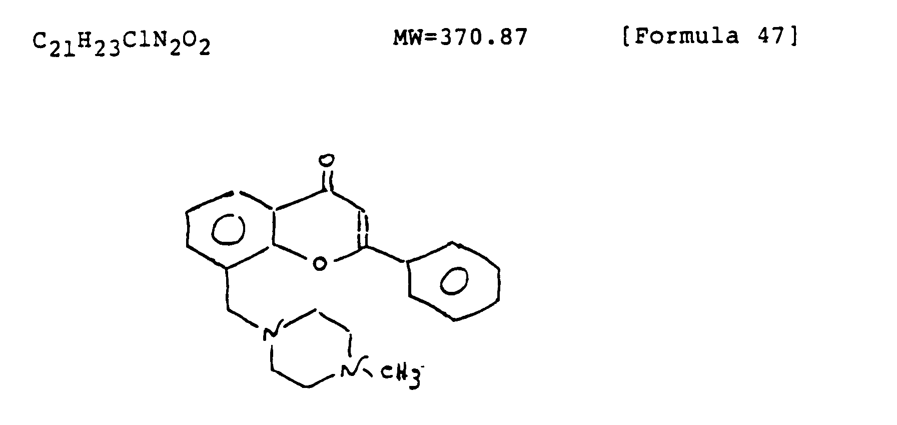

18.9 g (0.06 mole) of Bromomethyl-8-oxo-4-phenyl-2-4H-[1] Benzopyranone, 6.57 g (0.066 mole) of N-methyl piperazine, and 8.3 g (0.06 mole) of potassium carbonate in 200 ml of toluene are refluxed for 8 hours. Insolubles are filtered, and the solvent is evaporated in a vacuum. The solid obtained is recrystallized in hexane. Weight obtained: 9.69 g. PF_{G}=139°C; IR Vc=0 (pyrone)=1640 cm⁻¹. Using an HCl treatment in CHCl₃, the chlorohydrate is obtained: PF_{G}=244-246°C.

| Elemental analysis | | | | | |
|---|---|---|---|---|---|
| | C% | H% | Cl% | N% | O% |
| calculated : | 68.00 | 6.25 | 9.56 | 7.56 | 8.63 |
| found : | 68.34 | 5.86 | 9.80 | 7.61 | |

Using the same technique, the following compounds were prepared:

### BROMOHYDRATE OF N[IMIDAZOLINYL-2], N [(OXO-4-PHENYL-2-4H-[1] BENZOPYRAN-8-YL) METHYL]-DICHLORO-2-6-ANILINE

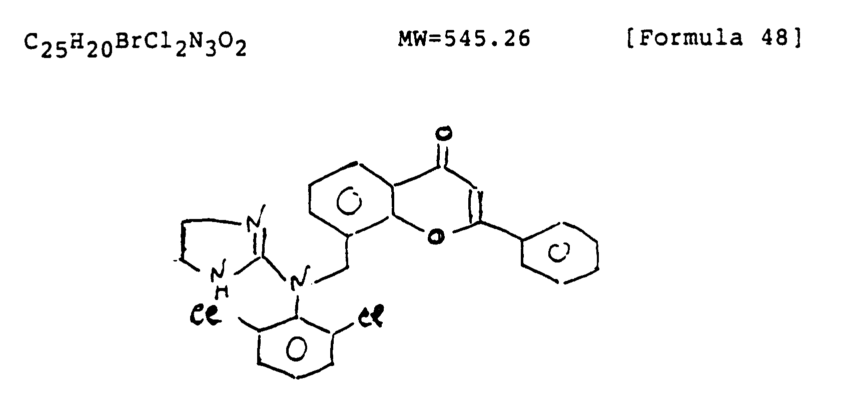

PF_{G}=289-290°C; IR Vc=o (pyrone)=1640 cm⁻¹; V NH=3000-3200 cm⁻¹; NMR (DMSO)δ in ppm in relation to TMS; 4H at 3.4 (s), 2H at 5.5 (s), 1H at 7 (s), 11H at 7.2 to 8.3 (m), 2H at 8.5 to 9.5 (interchangeable).

| Elemental analysis | | | | | | |
|---|---|---|---|---|---|---|
| | C% | H% | Br% | Cl% | N% | 0% |
| calculated : | 55.06 | 3.70 | 14.66 | 13.00 | 7.71 | 5.87 |
| found : | 55.14 | 3.63 | 14.56 | 13.09 | 7.07 | |

### [(OXO-4-PHENYL-2-4H-(1) BENZOPYRAN-8-YLO METHYL AMINO]-4-BENZOIC ACID

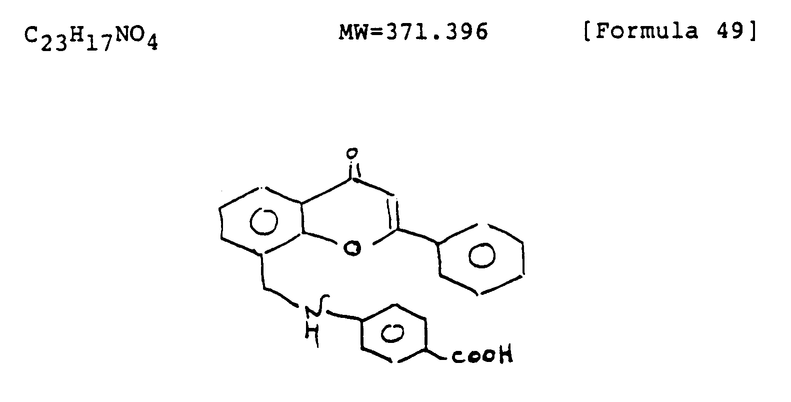

PF_{G}=269-271°C; IR Vc=o (acid)=1710 cm⁻¹, Vc=0 (pyrone)=1640 cm⁻¹; NMR (DMSO)δ in ppm in relation to TMS; 2H at 4.8 (m), 13H at 6.9 to 8.27 (m), 1H at 12.6 interchangeable).

| Elemental analysis | | | | |
|---|---|---|---|---|
| | C% | H% | N% | 0% |
| calculated : | 74.38 | 4.61 | 3.77 | 17.24 |
| found : | 74.08 | 4.59 | 3.91 | |

### N-[(OXO-4-PHENYL-2-4H-(1) BENZOPYRAN-8-YL)METHYL] N-METHYL, AMINO-4-BENZOIC ACID

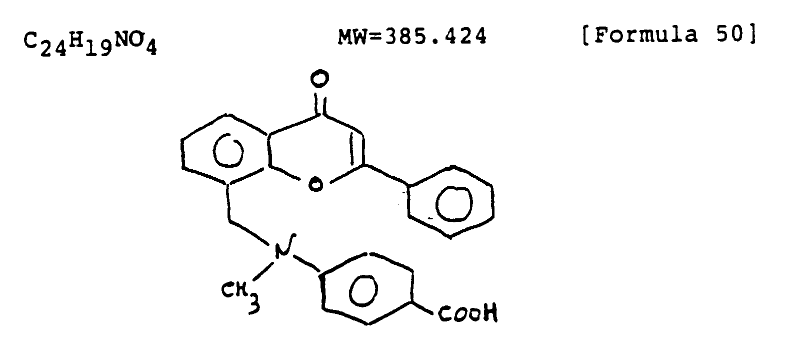

PF_{G}=260-262°C; IR Vc=o (acid)=1710 cm⁻¹, Vc=0 (pyrone)=1640 cm⁻¹; NMR (DMSO)δ in ppm in relation to TMS; 3H at 3.2 (s), 2H at 5 (s), 13H at 6.8 to 8.4 (m), 1H at 12.6 (interchangeable).

| Elemental analysis | | | | |
|---|---|---|---|---|
| | C% | H% | N% | 0% |
| calculated : | 74.79 | 4.97 | 3.63 | 16.60 |
| found : | 74.51 | 4.81 | 3.47 | |

### [(OXO-4-PHENYL-2-4H-[1] BENZOPYRAN-8-YLO METHYLAMINO]-3, METHYL-3, PROPANEDIOL-1-3

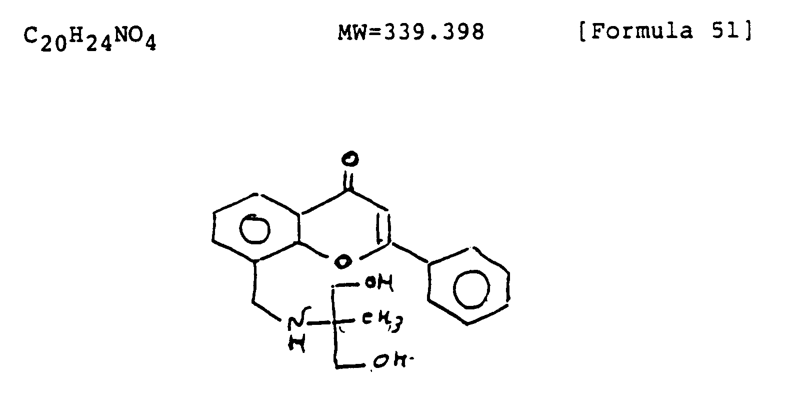

PF_{G}=150-152°C; IR Vc=o (pyrone)=1630 cm⁻¹, V OH=3380 cm⁻¹; NMR (DMSO)δ in ppm in relation to TMS; 3H at 1 (s), 4H at 3.2 (d), 2H at 4 (s), 2H at 4.5 (t, interchangeable), 1H at 7 (s), 8H at 7.2 to 8.2 (m).

| Elemental analysis | | | | |
|---|---|---|---|---|
| | C% | H% | N% | 0% |
| calculated : | 70.78 | 6.24 | 4.13 | 18.25 |
| found : | 70.51 | 6.42 | 4.37 | |

### CHLOROHYDRATE OF (AMINOMETHYL)-8-PHENYL-2-4H-[1] BENZOPYRANONE-4

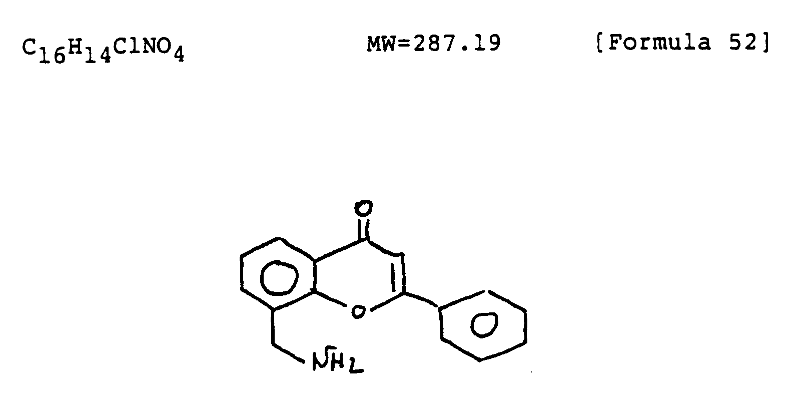

PF_{G}=275-279°C; IR V NH₃⁺ = 3100 to 2600 cm⁻¹; Vc - (pyrone)=1620 cm⁻¹; NMR (DMSO)δ in ppm in relation to TMS; 8H at 7.3 to 8.4 (m), 3H at 8.8 (interchangeable).

| Elemental analysis | | | | | |
|---|---|---|---|---|---|
| | C% | H% | Cl% | N% | 0% |
| calculated : | 64.76 | 5.1 | 11.95 | 4.72 | 13.48 |
| found : | 65.05 | 4.73 | 12.08 | 4.46 | |

### PHENYL-2-(TRIMETHOXY-3,4,5-PHENYLAMINOMETHYL)-8-4H-[1] BENZOPYRANONE-4

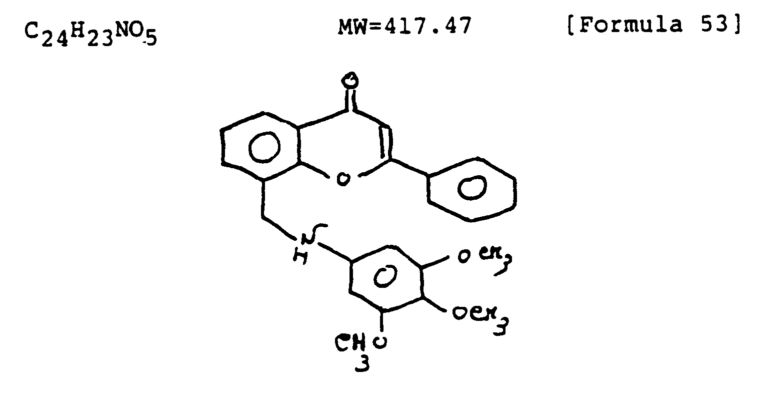

PF_{G}=219-2221C; IR V NH=3350 cm⁻¹, Vc=o (pyrone)=1620 cm⁻¹; NMR (CF₃COOD)δ in ppm in relation to TMS; 6H at 3.15 (s), 3H at 3.35 (s), 2H at 4.93 (s), 1H at 6.1 (s), 11H at 7 to 8.3 (m).

| Elemental analysis | | | | |
|---|---|---|---|---|
| | C% | H% | N% | 0% |
| calculated : | 74.93 | 5.55 | 3.35 | 19.16 |
| found : | 71.65 | 5.58 | 3.35 | |

### Example 5

### (ACETYLOXY-1-ETHYL)-8-PHENYL-2-4H-[1] BENZOPYRANONE-4

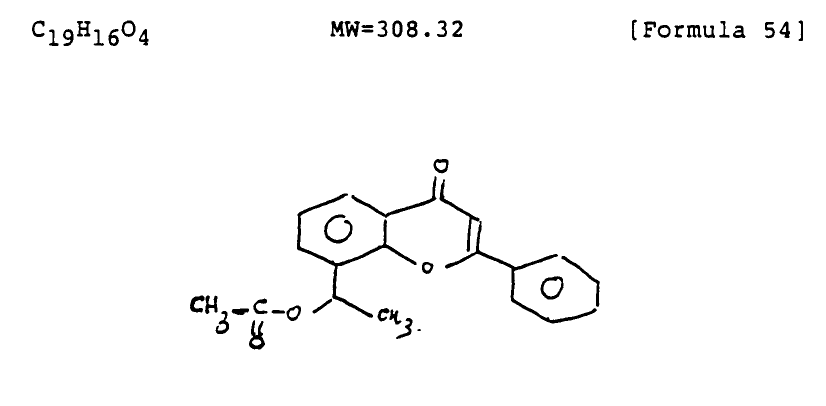

61.2 g (0.186 mole) of (bromo-1-ethyl)-8-phenyl-2-4H-[1] benzopyranone-4 and 20.1 g (0.204 mole) of potassium acetate in 290 ml of DMF are mixed and heated, with stirring to 45°C. Heating is stopped and the reaction mixture is returned to room temperature for 3 hours, with stirring. After one night at rest, the mixture is poured into ice water. The precipitate formed is filtered and recrystallized in alcohol. Weight obtained: 51 g (yield: 88.9%);
PF_{G}=137°C; IR Vc=o (ester) = 1740 cm⁻¹, Vc=o (pyrone)=1640 cm⁻¹; NMR (CDCl₃)δ in ppm in relation to TMS; 3H at 1.7 (d), 3H at 2.1 (s), 1H at 6.6 (g), 1H at 6.8, 8H at 7.2 to 8.4 (m).

### Example 6

### (HYDROXY-1-ETHYL)-8-PHENYL-2-4H-[1] BENZOPYRANONE-4

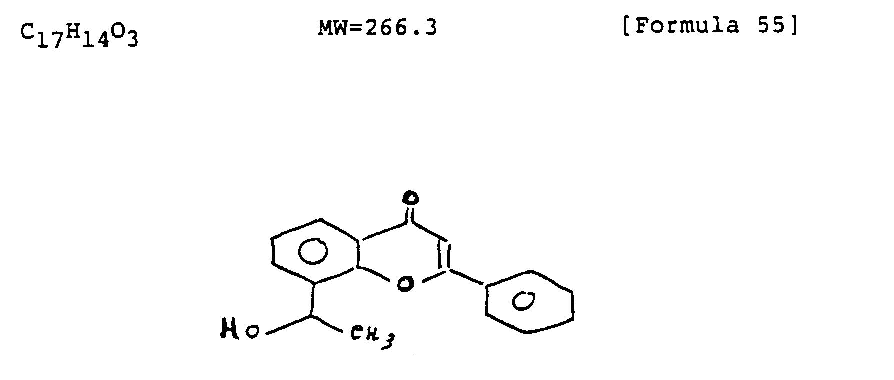

194.3 g (0.63 mole) of (acetoloxy-1-ethyl)-8-phenyl-2-4H-[1] benzopyranone-4 68.8 g (0.818 mole) of sodium bocarbonate are mixed in 239 ml of ethanol and 1628 ml of water. The mixture is kept under reflux for 5 hours. The mixture is heat-filtered, the filtrate is evaporated in a vacuum, the residue is taken up in water and recrystallized in toluene. Weight obtained: 152.9 g (yield: 91%);
PF_{G}=154-157°C; IR V OH=3350 cm⁻¹, Vc=0 (pyrone)=1620 cm⁻¹; NMR (CDCl₃)δ in ppm in relation to TMS; 3H at 1.62 (d), 1H at 2.8 (interchangeable).

| Elemental analysis | | | |
|---|---|---|---|
| | C% | H% | 0% |
| calculated : | 76.67 | 5.30 | 18.03 |
| found : | 76.50 | 5.19 | |

### Example 7

### ACETYL-8-PHENYL-2-4H-1 BENZOPYRANONE-4

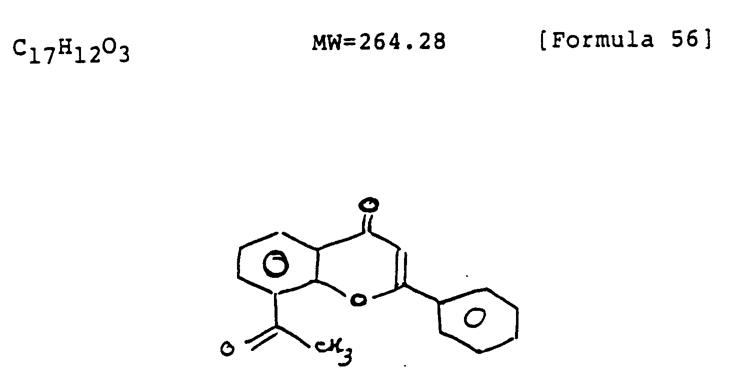

59.5 g (0.223 mole) of (hydroxy-1-ethyl)-8-phenyl-2-4H-[1] benzopyranone-4 are placed in 670 ml of dioxane. The medium is heated until a solution is obtained. This is then cooled to 20°C, and a reagent solution, prepared using 19.7 g (0.19 mole) of CrO₃, 50 ml of water, 13.6 ml of concentrated H₂SO₄ is added in a dropwise manner. This mixture is kept for three hours at room temperature while being stirred, the insoluble is filtered, the filtrate is evaporated in a vacuum and the residue obtained is recrystallized in methyl isobutylcetone. Weight obtained: 43.3 g (yield: 73.4%);
PF_{G}=125-126°C; IR Vc=o (cetone)=1675 cm⁻¹, Vc=0 (pyrone)=1690 cm⁻¹; NMR (CDCl₃)δ in ppm in relation to TMS; 3H at 2.8 (s), 1H at 6.8 (s), 8H at 7.3 to 8.6 (m).

| Elemental analysis | | | |
|---|---|---|---|
| | C% | H% | 0% |
| calculated : | 77.26 | 4.58 | 18.16 |
| found : | 77.23 | 4.53 | |

### Example 8

### (BROMOACETYL)-8-PHENYL-2-4H-[1] BENZOPYRANONE-4

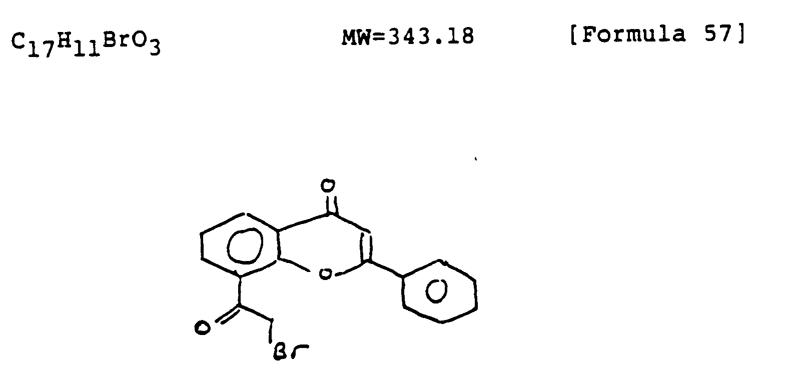

To a solution of 40 g (0.19 mole) of acetyl-8-phenyl-2-4H-[1] benzopyranone-4 in 750 ml of dioxane, 56.9 g (0.151 mole) of phenyltriethylammoniumtribromide are added. The mixture is stirred for 48 hours at room temperature, filtered, and the precipitate obtained is washed in water and recrystallized in acetone. Weight obtained: 42.9 g (yield: 82%);
PF_{G}=142°C; IR Vc=o -1630 cm⁻¹, NMR (CDCl₃)δ in ppm in relation to TMS; 2H at 4.64 (s), 1H at 6.8 (s), 8H at 7.2 to 8.6 (m).

### Example 9

### (AMINO-2-THIAZOL-4-YL)-8-PHENYL-2-4H-[1] BENZOPYRANONE-4

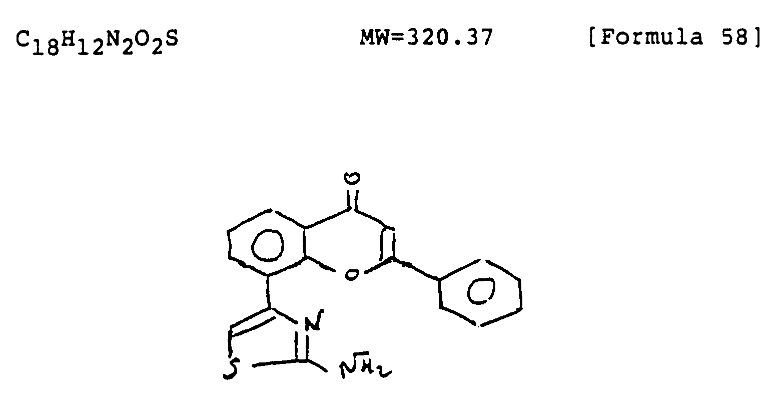

A mixture of 5 g (0.0146 mole) of (bromoacetyl)-8-phenyl-2-4H-[1] benzopyranone-4 and 2.22 g g (0.029 mole) thiourea in 100 ml of ethanol is heated for three hours under reflux, then poured into 200 ml of ice water. The precipitate formed is filtered, washed in water and recrystallized in a mixture of water and DMF. Weight obtained: 2.8 g (yield: 59%); IR V NH₂=3300 to 3350 cm⁻¹ Vc=o=1630 cm⁻¹; NMR (CDCl₃)δ in ppm in relation to TMS; 2H at 3.34 (interchangeable).

| Elemental analysis | | | | | |
|---|---|---|---|---|---|
| | C% | H% | N% | O% | S% |
| calculated : | 67.48 | 3.78 | 8.74 | 9.99 | 10.01 |
| found : | 67.57 | 3.65 | 8.84 | | 10.06 |

Using this same technique, the following compounds were prepared:

### [METHYL-2-THIAZOL-4YL)-8-PHENYL-2-4H-[1] BENZOPYRANONE-4

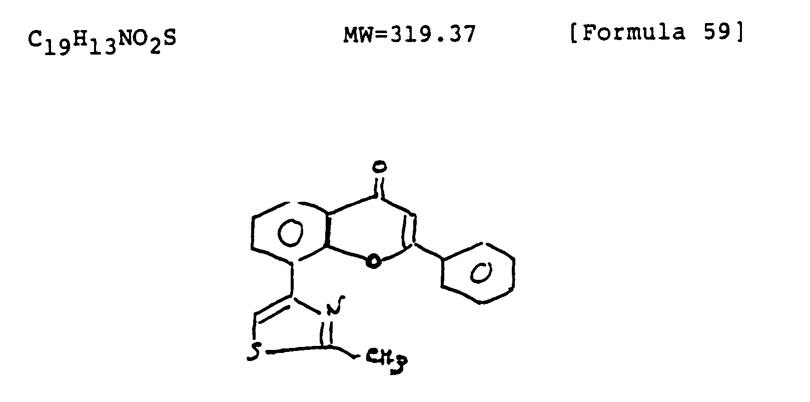

PF_{G}=148-153°C; IR Vc=o (acid)=1639 cm⁻¹, NMR (CDCl₃)δ in ppm in relation to TMS; 3H at 2.8 (s), 1H at 6.8 (s), 9H at 7.2 to 8.5 (m).

| Elemental analysis | | | | | |
|---|---|---|---|---|---|
| | C% | H% | N% | O% | S% |
| calculated : | 71.45 | 4.10 | 4.39 | 10.02 | 10.04 |
| found : | 71.39 | 4.03 | 4.36 | | 10.30 |

### (IMIDAZO [2,1-B] THIAZOL-6-YL)-8-PHENYL-2-4H-[1] BENZOPYRANONE-4

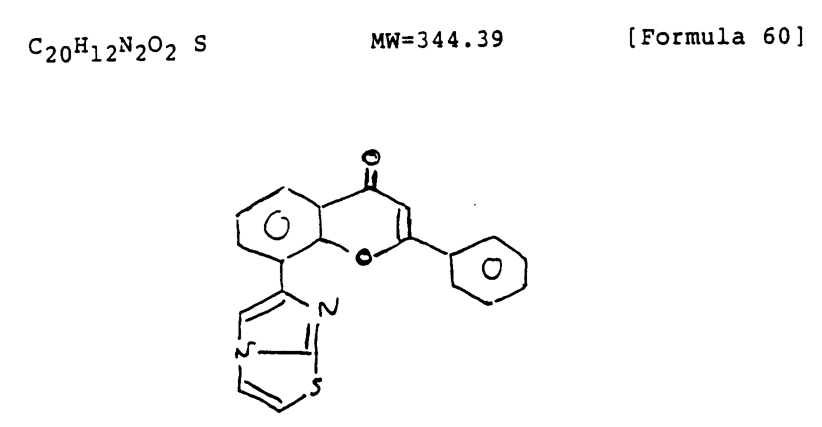

PF_{G}=229-233°C; IR Vc=o = 1630 cm⁻¹, NMR (DMSO + CF₃COOD)δ in ppm in relation to TMS; 1H at 7 (s), 11H at 7.4 to 8.8 (m).

| Elemental analysis | | | | | |
|---|---|---|---|---|---|
| | C% | H% | N% | 0% | S% |
| calculated : | 69.75 | 3.51 | 8.14 | 9.28 | 9.31 |
| found : | 69.50 | 3.59 | 8.01 | | 9.37 |

### [IMIDAZO [1,2-A] PYRIDIN-2-YL]-8-PHENYL-2-4H-[1] BENZOPYRANONE-4

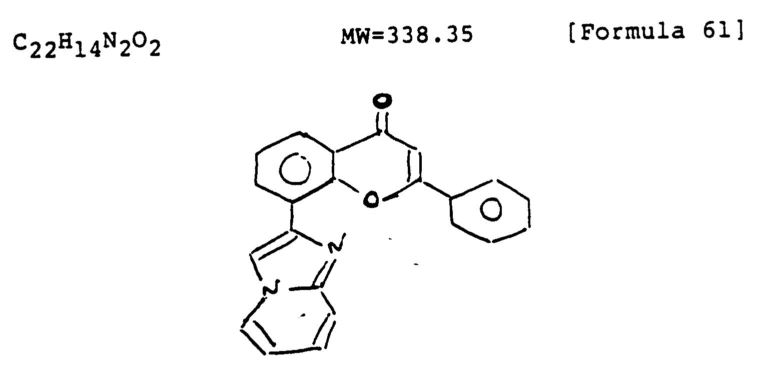

PF_{G}=203-205°C; IR Vc=o = 1635 cm⁻¹, NMR (CDCl₃)δ in ppm in relation to TMS; 1H at 6.8 (s), 13H at 7 to 8.7 (m).

| Elemental analysis | | | | |
|---|---|---|---|---|
| | C% | H% | N% | 0% |
| calculated : | 78.09 | 4.17 | 8.28 | 9.46 |
| found : | 78.16 | 4.12 | 8.26 | |

### (INDOLIZIN-2-YL)-8-PHENYL-2-4H-[1] BENZOPYRANONE-4

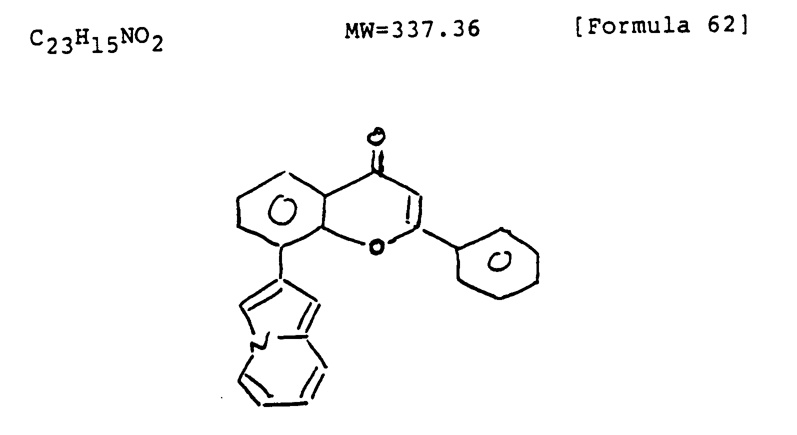

PF_{G}=204-207°C; IR Vc=o 1639 cm⁻¹, NMR (CDCl₃)δ in ppm in relation to TMS; 1H at 6.8 (s), 1H at 7.3 to 8.3 (m).

| Elemental analysis | | | | |
|---|---|---|---|---|
| | C% | H% | N% | 0% |
| calculated : | 81.88 | 4.48 | 4.15 | 9.49 |
| found : | 82.03 | 4.60 | 4.16 | |

### PHENYL-2-(PHENYL-2-THIAZOL-4-YL)-8-4H-[1] BENZOPYRANONE-4

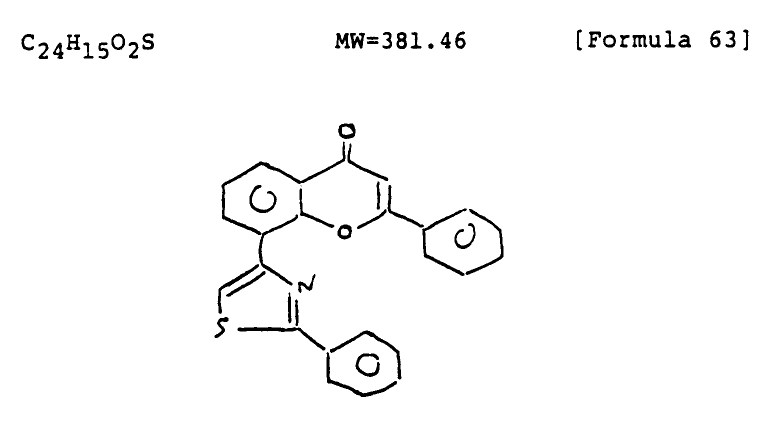

PF_{G}=199-202°C; IR Vc=o = 1650 cm⁻¹, NMR (CF₃COOD)δ in ppm in relation to TMS; 15H at 7.4 to 8.8.

| Elemental analysis | | | | | |
|---|---|---|---|---|---|
| | C% | H% | N% | 0% | S% |
| calculated : | 75.57 | 3.96 | 3.67 | 8.39 | 8.41 |
| found : | 75.42 | 4.03 | 3.64 | | 8.15 |

### (DIHYDRO-2-3-IMIDAZO [2,1-B] THIAZOL-6-YL)-8-PHENYL-2-4H-[1] BENZOPYRANONE-4

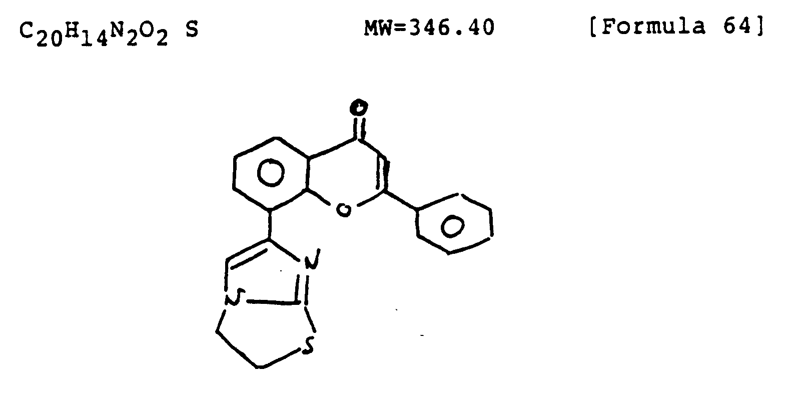

PF_{G}=226-230°C; IR Vc=o = 1635 cm⁻¹, NMR (DMSO)δ in ppm in relation to TMS; 4H at 4 to 5 (m), 1H at 7 (s), 9H at 7.4 to 8.3 (m).

| Elemental analysis | | | | | |
|---|---|---|---|---|---|
| | C% | H% | N% | 0% | S% |
| calculated : | 69.34 | 4.07 | 8.09 | 9.24 | 9.26 |
| found : | 69.21 | 4.19 | 8.32 | | 9.02 |

### EXAMPLE 10

### ACETOXYMETHYL-10-PHENYL-2-4H-NAPHTO [1,2-b] PYRANONE-4

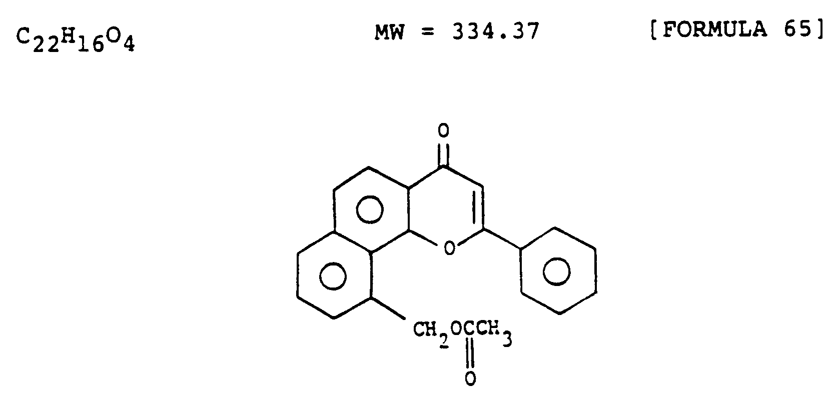

A mixture of 19.8 g (0.054 mole) of bromomethyl-10-phenyl-2-4H-naphtho[1,2-b]pyranone-4, 5.3 g (0.054 mole) of potassium acetate, and 110 ml of DMF is heated to 45° with stirring. This mixture is allowed to return to room temperature while still being stirred for one hour. It is poured into a mixture of water and ice, and the solid obtained is then filtered and used in the following step, without further purification. Weight obtained: 18.5 g (quantitative yield); PF_{G} = 170°C; IR Vc = o (ester) = 1740 cm⁻¹, Vc = o (pyrone) = 1635 cm⁻¹; NMR (CDCl₃) δ in ppm in relation to TMS: 3H at 2.1 (s), 2H at 5.9 (s), 1H at 6.9 (s), 10H at 7.2 to 8.6 (m).

### HYDROXYMETHYL-10-PHENYL-2-4H-NAPHTO[1,2-b]PYRANONE-4

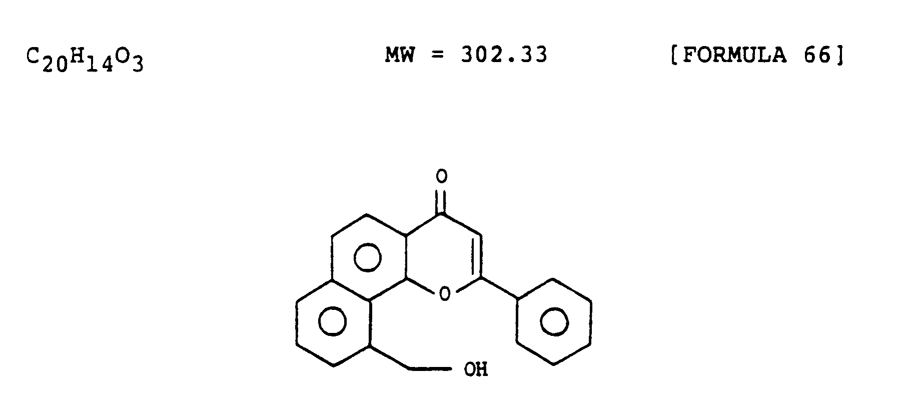

A mixture of 18.9 G (0.054 mole) of acetoxymethyl-10-phenyl-2-4H-naphto [1,2-b pyranone-4, 100 ml of ethanol and 39 g (0.07 mole) of potassium in tablet form is heated in a reflux for two hours. It is then poured into a water-ice mixture and acidified using 6N HCl. The precipitate obtained is filtered, dried, and used in the following step without further purification. Weight obtained: 16.2 g (yield = 99%): I_{R} V OH = 3400 cm⁻¹; V_{c} = o = 1630 cm⁻¹; NMR (DMSO) δ in ppm in relation to TMS: 1H at 3.5 (s, large), 2H at 5.4 (s), 1H at 7 (s), 10H at 7.2 to 8.4.

### OXO-4-PHENYL-2-4H-NAPHTO[1,2-b]PYRANONE-4

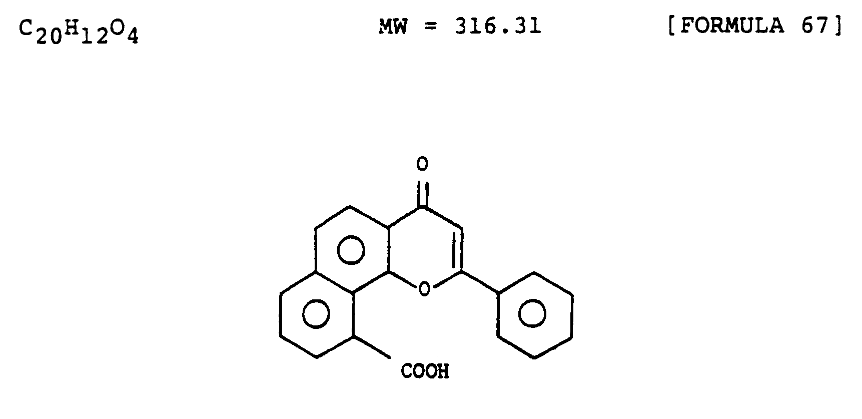

A mixture of 16.2 g (0.0536 mole) of hydroxymethyl-10-phenyl-2-4H-naphto [1,2-b] pyranone 4, 430 ml of pyridine, and 100 ml of water is heated to 60°C. 31.7 g (0.2 mole) of potassium permanganate is added over two hours in portions, then the mixture is heated for 4 hours in a reflux. It is then cooled, and treated with a watery solution of sodium metasulfite, until discoloration is obtained. It is poured into 1 liter of water, the insoluble is filtered, and the organic phase is poured off. After evaporation in a vacuum, the residue is taken up again by the water, acidified using 6N HCl. The precipitate obtained is filtered and recrystallized in acetic acid. Weight obtained: 1.1g (yield 6.5%); mp = 278-280°C; IR Vc = o (acid) = 1700 cm⁻¹, Vc = o (pyrone) = 1620 cm⁻¹; NMR (DMSO) δ in ppm in relation to TMS: 1H at 7.15 (s), 10H at 7.4 to 8.4 (m), 1H at 13.5 (interchangeable).

| Elemental Analysis | | | |
|---|---|---|---|
| | C% | H% | 0% |
| calculated | 75.96 | 3.82 | 20.24 |
| found | 75.58 | 3.77 | |

### EXAMPLE 11

### OXO-4-PHENYL-1-4H-[1]-BENZOPYRAN-8-ACETATE OF (ETHOXYCARBONYL)-1-ETHYL

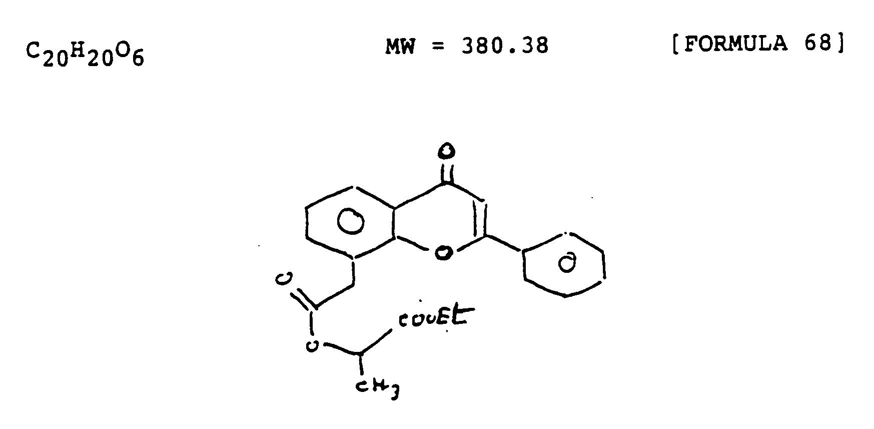

To a suspension of 30.6 g (0.109 mole) of oxo-4-phenyl-2-4H-1-benzopyran-8-acetic acid in 1.9 l of boiling ethanol is added dropwise a solution of 7.2 g (0.109 mole) of potassium in 100 ml of ethanol. The solution obtained is stirred for 30 minutes, allowed to return to room temperature, and evaporated in a vacuum. The residum is taken up using 300 ml of ethanol and evaporated in a vacuum, then taken up agin using 30 ml of benzene and evaporated in a vacuum. 546 ml of methyl isobutylketone (MIBK) is added to the residuum, followed by a solution of 21.7 g (0.12 mole) of ethyl α-bromopropionate in 55 ml of MIBK. This mixture is heated in a reflux for 3 hours; next, 12 g (0.066 mole) of ethyl α-bromopropionate is added before continuing heating for 5 hours in a reflux. Heat-filtratioin is carried out, and the filtrate is evaporated in a vacuum. The residuum is triturated in hexane in order to obtain a precipitate which is filtered, washed with hexane and recrystallized in isopropanol. Weight obtained: 36.2 g (yield: 87%); mp = 104-106°C; IR Vc = o (pyrone) = 1730 cm⁻¹, Vc = o (pyrone) = 1640 cm⁻¹; NMR (CDCl₃) δ in ppm in relation to TMS: 3H at 1.2 (t), 3H at 1.46 (d), 2H at 4.1 (s), 2H at 4.18 (q), 1H at 5.18 (q), 1H at 6.8 (s), 8H at 7.2 to 8.4 (m). (interchangeable).

### HYDROXY-4-METHYL-5-(OXO-4-PHENYL-2-4H-[1]-BENZOPYRAN-8-YL)-3-5H-FURANONE-2

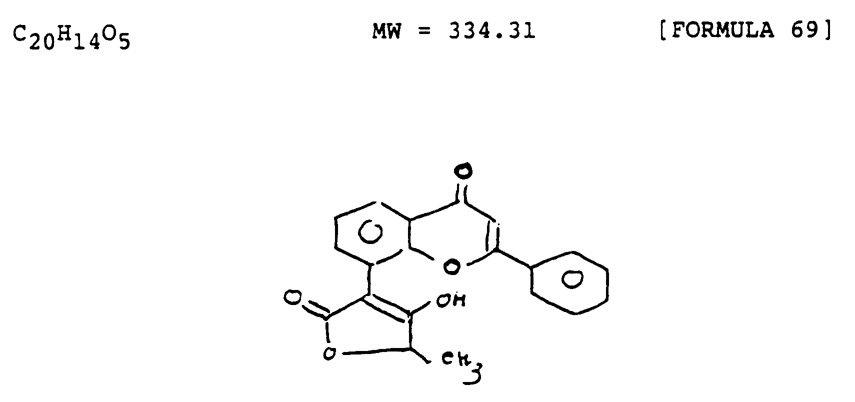

To a suspension of 2.62 g (0.109 mole) of sodium hydride in 226 ml of HMPT, is added dropwise to a solution of 41.7 g (0.109 mole) of oxo-4-phenyl-2-4H-[1]-benzopyran-8-acetate of (ethylcarbonyl)-1-ethyl in 260 ml of HMPT. This mixture is stirred overnight in an atmosphere of argon at room temperature, and is then carefully hydrolized using 2 l of 6N HCl. The precipitate obtained is filtered and recrystallized. Weight obtained: 28.3 g (yield 77%); mp = 265-268°C; IR V OH = 3400 to 2200 cm⁻¹, Vc = o (lactone) = 1740 cm⁻¹; Vc = o (pyrone) = 1600 cm⁻¹; NMR (DMSO) δ in ppm in relation to TMS: 3H at 1.6 (d), 1H at 5.2 (q), 1H at 7.1 (s), 9H at 7.2 to 8.6.

| Elemental Analysis | | | |
|---|---|---|---|
| | C% | H% | 0% |
| calculated | 71.85 | 4.22 | 23.93 |
| found | 71.55 | 4.11 | |

Using the same technique, the following compounds were obtained:

### (CHLORO-4-PHENYL)-5-HYDROXY-4-(OXO-4-PHENYL-2-4H-[1]-BENZOPYRAN-8-YL)-3-5H-FURANONE-2

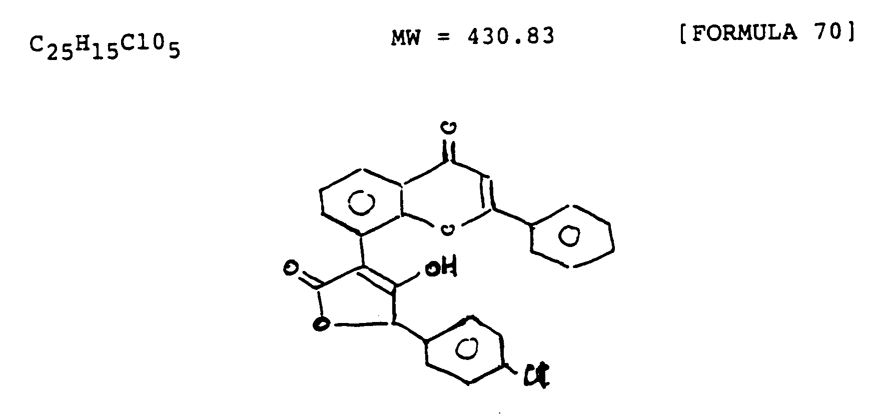

mp = 265-273°C; IR Vc = o (lactone) = 1750 cm⁻¹, Vc = o (pyrone) = 1660 cm⁻¹ NMR (DMSO) δ in ppm in relation to TMS: 1H at 6.16 (s), 1H at 7(s), 13H at 7.1 to 8.4.

| Elemental Analysis | | | | |
|---|---|---|---|---|
| | C% | H% | Cl% | 0% |
| calculated | 69.69 | 3.51 | 8.23 | 18.57 |
| found | 69.41 | 3.52 | 8.27 | |

### METHYL-3-HYDROXY-4-(OXO-4-PHENYL-2-4H-[1]-BENZOPYRAN-8-YL)-5-5H-FURANONE-2

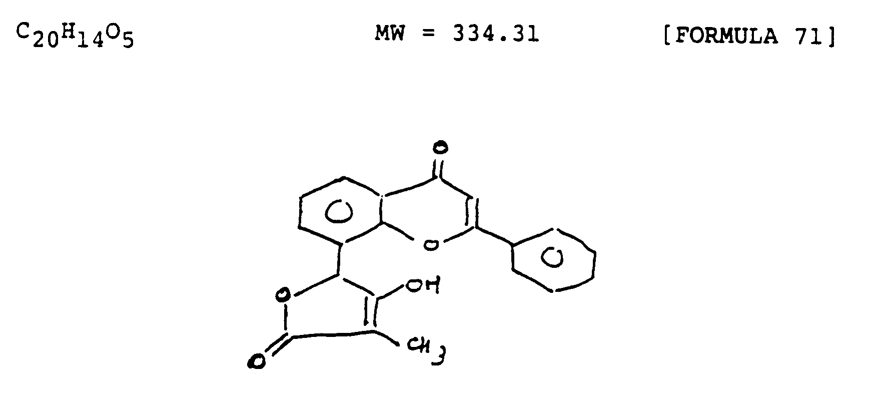

mp = 160° C; IR v c = o (lactone) = 1760 cm⁻¹, Vc=o (pyrone) = 1640 cm⁻¹; NMR (DMSO) δ in ppm in relation to TMS: 3H at 1.8 (s), 1H at 6.55 (s), 1H at 7.75 (s), 8H at 7.5 to 8.3 (m).

| Elemental Analysis | | | |
|---|---|---|---|
| | C% | H% | 0% |
| calculated | 71.85 | 4.22 | 23.93 |
| found | 71.80 | 4.22 | |

### EXAMPLE 12

### CHLORHYDRATE OF [(N,N-DIETHYLAMINO)-2-ETHOXY]-4-METHYL-5-[OXO-4-PHENYL-2-4H-[1]-BENZOPYRAN-8-YL]-3-5H-FURANONE-2

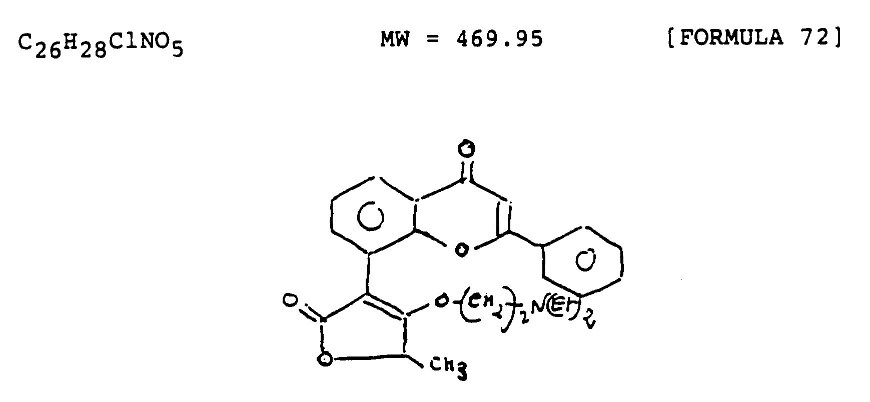

A mixture of 20 g (0.06 mole) of hydroxy-4-methyl-5-(oxo-4-phenyl-2-4H-[1]-benzopyran-8-yl)-3-5H-furanone-2, 9.93 g (0.72 mole) of potassium carbonate, and 0.36 g (0.002 mole) of potassium iodide in 490 ml of MIBK is heated for 1 hour at reflux. Next, a solution of 10.6 g (0.078 mole) of 2-(diethylamino)ethyl chloride in 90 ml of MIBK is added, and heating is continued for 7 hours. The minerals are heat filtered and the filtrate is evaporated in a vacuum. The residum is washed twice in hexane then solubilized in the minimum amount of acetone and diluted using hexane. A light insoluble is filtered, the filtrate is evaporated in a vacuum and the residuum is dissolved in 200 ml of ethanol. This product is cooled in an ice bath and HCl is bubbled through until a pH of 2 is achieved. By adding ether, a precipitate is obtained, which is filtered and recrystallized in an ethanol-ether mixture. Weight obtained: 16.9 g (yield 60%); mp = 168-169°C; IR Vc = o (lactone) = 1740 cm⁻¹, Vc = o (pyrone) = 1640 cm⁻¹; NMR (DMSO-CDCl₃) δ in ppm in relation to TMS: 6H at 0.9 (t), 3H at 1.5 (d), 6H at 2.6 to 3.3 (m), 2H at 4.2 (t), 1H at 5.2 (q), 1H at 6.75 (s), 8H at 7.3 to 8.2.

| Elemental Analysis | | | | | |
|---|---|---|---|---|---|
| | C% | H% | Cl% | N% | 0% |
| calculated | 66.45 | 6.00 | 7.55 | 2.98 | 17.02 |
| found | 66.30 | 6.20 | 7.55 | 2.83 | |

Using the same technique, the following compounds were obtained:

### [DIHYDRO-2-5-METHY-5-OXO-2-(OXO-4-PHENYL-2-4H-[1]-BENZOPYRAN-8-YL)-3-FURAN-4-YL] ETHYL OXYACETATE

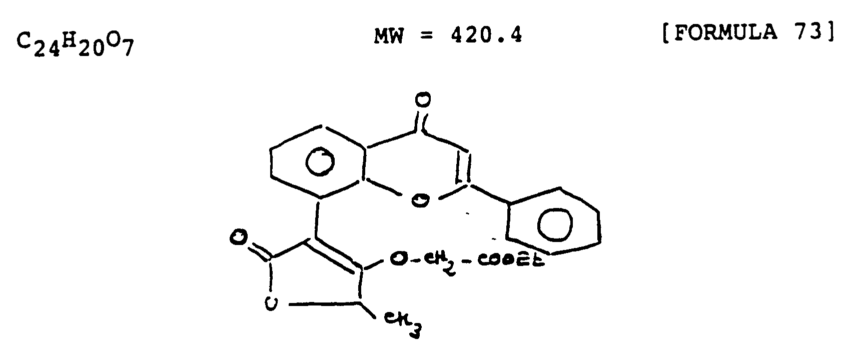

mp = 153°C; IR Vc = o NMR (ester and lactone) = 1755 cm⁻¹, Vc = o (pyrone) = 1640 cm⁻¹ NMR (CDCl₃) δ in ppm in relation to TMS: 3H at 1 (t), 3H at 1.7 (d), 2H at 3.9 (q), 2H at 4.5 (s), 1 H at 5.18 (q), 1H at 6.9 (s), 8H at 7.2 to 8.5.

### [DIHYDRO-2-5-METHYL-5-OXO-2-(OXO-4-PHENYL-2-4H-[1]-BENZOPYRAN-8-YL)-3-FURAN-4-YL] OXYACETIC ACID

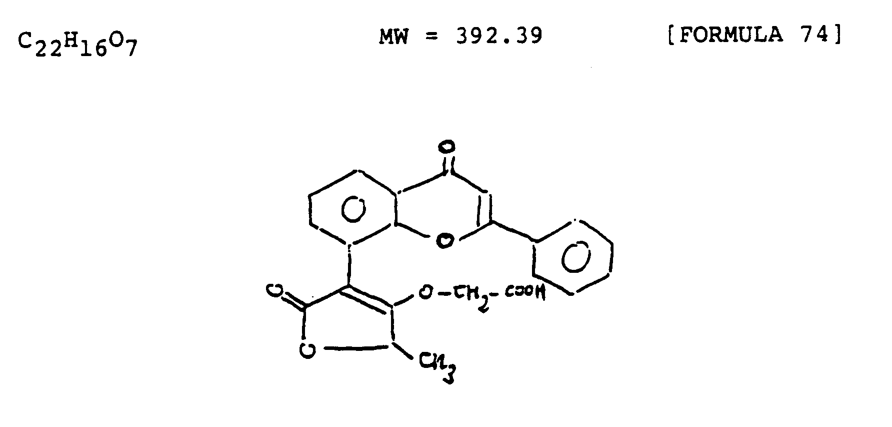

mp = 257-259°C; IR V OH = 2400 cm⁻¹, Vc = o (lactone) = 1740 cm⁻¹, Vc = o (acid) = 1710 cm⁻¹, Vc = o (pyrone) = 1620 cm⁻¹; NMR (DMSO) δ in ppm in relation to TMS: 3H at 1.6 (d), 1H at 4 (interchangeable), 2H at 4.66 (s), 1H at 5.4 (q), 1H at 7.08 (s), 8H at 7.2 to 8.4 (m).

| Elemental Analysis | | | |
|---|---|---|---|
| | C% | H% | 0% |
| calculated | 67.34 | 4.11 | 28.55 |
| found | 67.20 | 4.00 | |

### DIMETHYL CARBAMOTHIOATE OF O-[DIHYDRO-2,5-METHYL-5-OXO-2-(OXO-4-PHENYL-2-4H-[1]-BENZOPYRAN-8-YL)-3-FURN-4-YL]

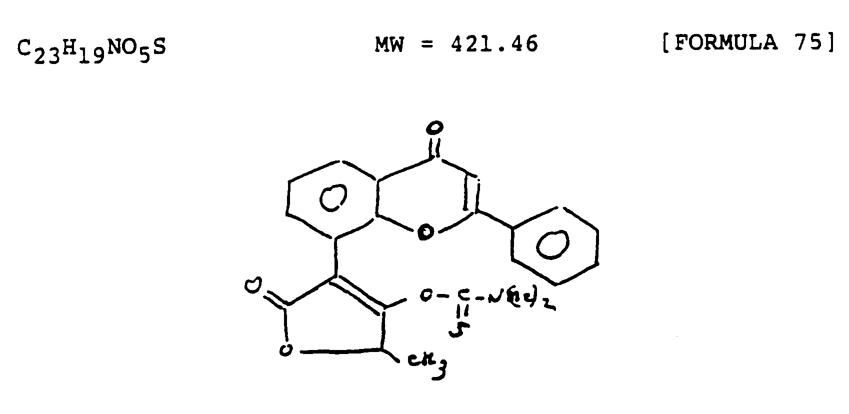

mp = 173-175°C; IR Vc = o (lactone) = 1740 cm⁻¹, Vc = o (pyrone) = 1630 cm⁻¹; NMR (CDCl₃) δ in ppm in relation to TMS: 3H at 1.66 (d), 6H at 2.8 (s), 1H at 6.16 (q), 1H at 6.8 (s), 8H at 7.2 to 8.4 (m).

| Elemental Analysis | | | | | |
|---|---|---|---|---|---|
| | C% | H% | N% | 0% | S% |
| calculated | 65.54 | 4.70 | 3.32 | 18.98 | 7.61 |
| found | 65.42 | 4.52 | 3.32 | | 7.64 |

### EXAMPLE 13

### ACETYLTHIMETHYL-8-OXO-4-PHENYL-2-4H-[1]-BENZOPYRANE

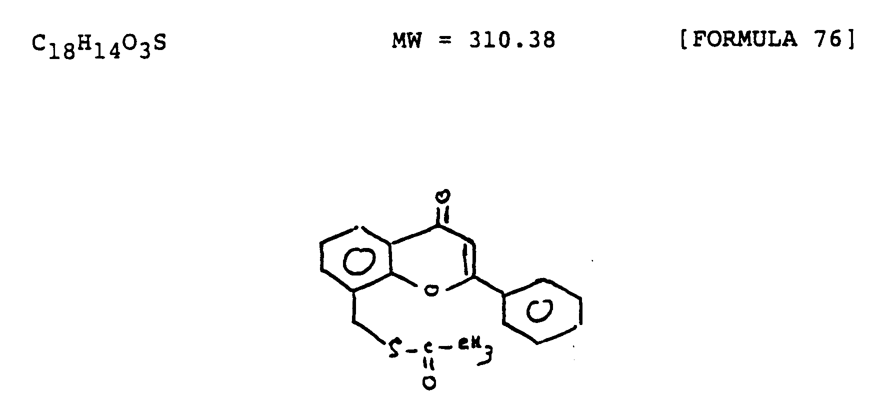

To a mixture of 17.4 g (0.152 mole) of potassium thioacetate in 120 ml of DMF is added 48 g (0.152 mole) of bromomethyl-8-phenyl-2-4H-[1]-benzopyranone-4, by portions while being stirred. This is stirred for 1 hour at room temperature and then poured into a water-ice mixture. The precipitate obtained is filtered and recrystallized in ethyl acetate. Weight obtained: 38 g (yield: 80%); mp = 160°C; IR Vc = o (ester) = 1690 cm⁻¹, Vc = o (pyrone) = 1655 cm⁻¹; NMR (CDCl₃) δ in ppm in relation to TMS: 3H at 2.4 (s), 2H at 4.5 (s), 1H at 6.9 (s), 8H at 7.2 to 8.4 (m).

### EXAMPLE 14

### MERCAPTOMETHYL-8-PHENYL-2-4H-[1]-BENZOPYRANONE-4

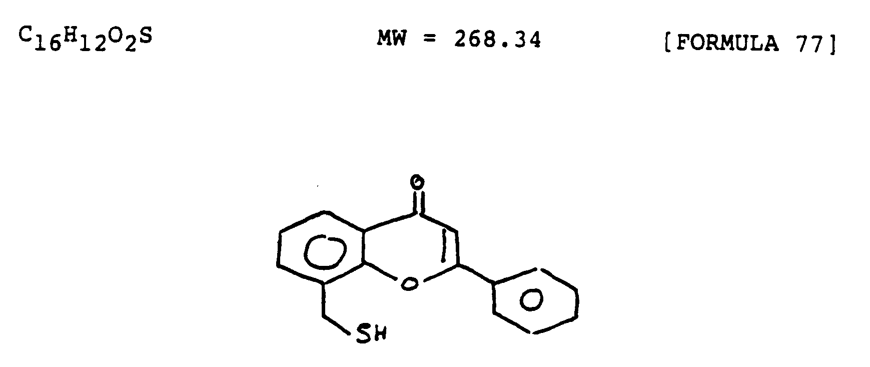

To a mixture of 38 g (0.122 mole) of thioacetylmethyl-8-phenyl-2-4H-[1]-benzopyranone-4 and 230 ml of ethanol are added at one time 150 ml of saturated ethanol in anhydrous HCl. This is heated for 18 hours in a reflux. This mixture is cooled and the precipitate obtained is heated and recrystallized in ethanol. Weight obtained: 39.7 g (yield: 97%); mp = 162°CJ; IR Vc = o = 1640 cm⁻¹ NMR (CDCl₃) δ in ppm in relation to TMS: 1H at 2 (t), 2H at 4.1 (d), 1H at 6.8 (s), 8H at 7.2 to 8.4 (m) (interchangeable).

| Elemental Analysis | | | | |
|---|---|---|---|---|
| | C% | H% | 0% | S% |
| calculated | 71.62 | 4.51 | 11.92 | 11.95 |
| found | 71.45 | 4.48 | | 11.84 |

Using the same technique, the following compounds were prepared:

### (OXO-4-PHENYL-2-4H-[1]-BENZOPYRAN-8-YL)-METHYL] METHYL THIOACETATE

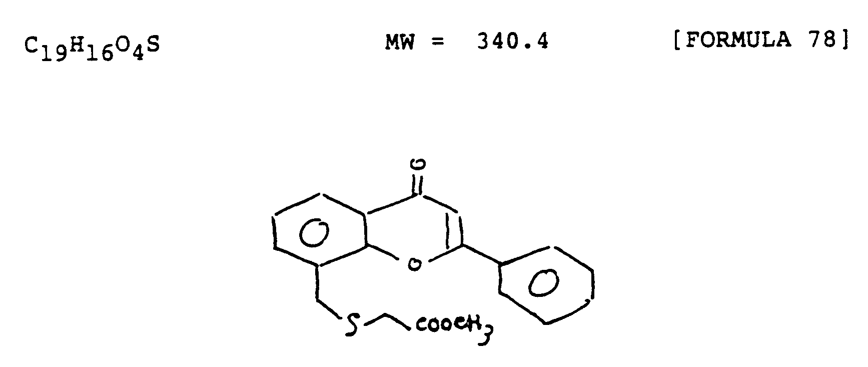

mp = 110°C; IR Vc = o (ester) = 1720 cm⁻¹, Vc = o (pyrone) = 1650 cm⁻¹ NMR (CDCl₃) δ in ppm in relation to TMS: 2H at 3.2 (s), 3H at 3.7 (s), 2H at 4.2 (s), 1H at 6.8 (s), 8 H at 7.2 to 8.4 (m).

### (OXO-4-PHENYL-2-4H-[1]-BENZOPYRAN-8-YL) METHLTHIOACETIC ACID

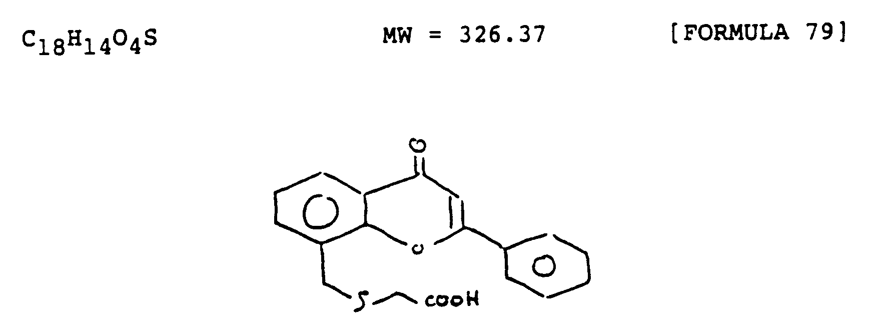

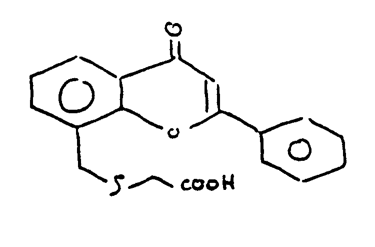

mp = 202-204°C; IR V OH = 3100-2400 cm⁻¹, IR Vc = o (acid) = 1720 cm⁻¹, Vc = o (pyrone) = 1640 cm⁻¹, NMR (DMSO) δ in ppm in relation to TMS: 2H at 3.2 (s), 2H at 4.25 (s), 1H at 6.8 (s), 9H at 7.2 to 8.4 (m).

| Elemental Analysis | | | | |
|---|---|---|---|---|
| | C% | H% | 0% | S% |
| calculated | 66.24 | 4.32 | 19.61 | 9.81 |
| found | 66.51 | 4.34 | | 10.11 |

### OXALATE OF DIETHYLAMINO-2-ETHOXYMETHYL)-8-PHENYL-2-4H-[1]-BENZOPYRANONE-4

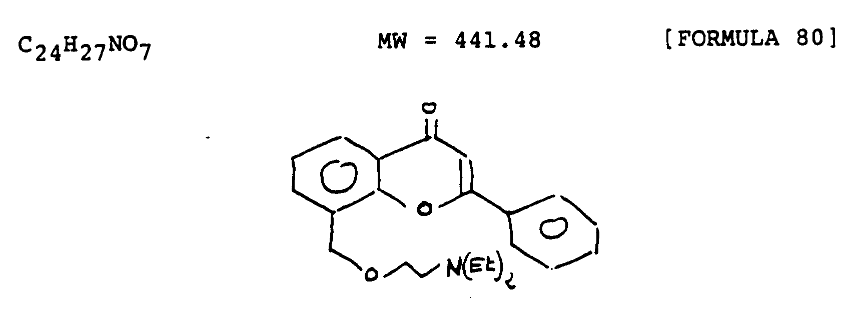

mp = 162-164°C; IR Vc = o = 1660 cm⁻¹; NMR (DMSO) δ in ppm in relation to TMS: 3H at 1.2 (t), 6H at 2.95 to 3.5 (m), 1H at 3.8 to 4.2 (m), 2H at 5.05 (s), 2H at 5.4 (interchangeable), 1H at 7.1 (s), 8H 7.5 to 8.5 (m).

| Elemental Analysis | | | | |
|---|---|---|---|---|
| | C% | H% | N% | 0% |
| calculated | 65.29 | 6.16 | 3.17 | 25.37 |
| found | 65.18 | 6.10 | 3.07 | |

### [[HYDROXY-2-(HYDROXYMETHYL)-1-ETHOXY] METHYL]-8-PHENYL-2-4H-[1]-BENZOPYRANONE-4

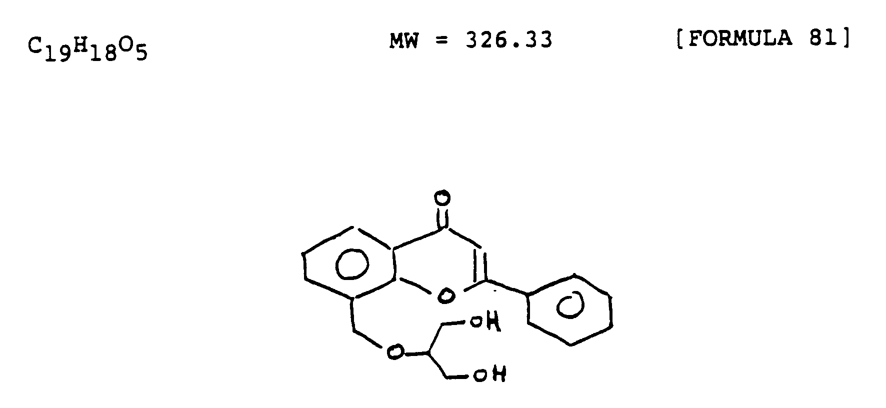

mp = 162°C; IR V OH = 3300 cm⁻¹; IR Vc = o 1620 cm⁻¹, NMR (DMSO) δ in ppm in relation to TMS: 5H at 3.3 to 3.7 (m), 2H at 4.5 (interchangeable), 2H at 5 (s), 1H at 6.93 (s), 8H at 7.2 to 8.2 (m).

| Elemental Analysis | | | |
|---|---|---|---|
| | C% | H% | 0% |
| calculated : | 69.93 | 5.56 | 24.51 |
| found : | 70.00 | 5.57 | |

### EXAMPLE 15

### OXO-4-PHENYL-2-4H-[1]-BENZOPYRAN-8-ACETAMIDE

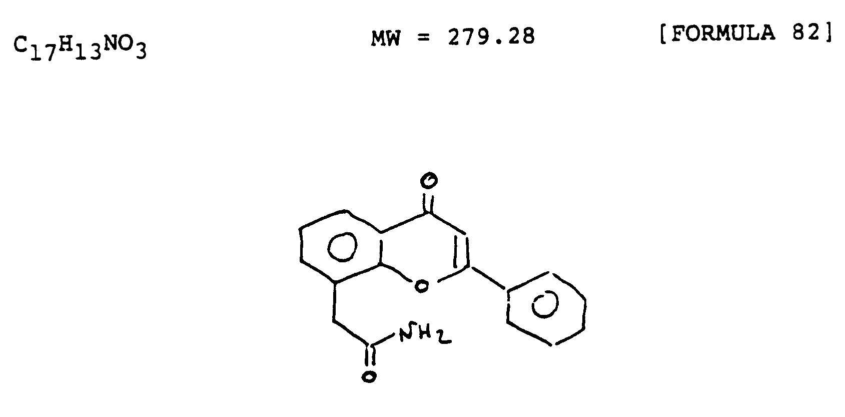

A suspension of 5 G (0.0178 mole) of oxo-4-phenyl-2-4H-[1]-benzopyran-8-acetic acid in 180 ml of dioxane is heated until it dissolves. A solution of 3.5 g (0.0124 mole) of N,N′-carboxyldiamidazol in 30 ml of dioxane is added and the mixture is heated for 1 hour to 80°C. It is then cooled to 20°C and approximately 10 ml (0.4 mole) liquified anhydrous ammonia at -33°C is slowly added. The mixture is stirred for 10 minutes at 20°C, then for 3 hours at 80°C. This is left overnight, filtered, washed with hexane, then with hot 5% sodium bicarbonate solution, then with water; it is next recrystallized in ethanol. Weight obtained:
3.3 g (yield 66%); mp = 232-258°C; IR V NH = 3370 to 3200 cm⁻¹; IR vc = o (acid) = 1660 cm⁻¹, Vc = o (pyrone) = 1630 cm⁻¹; NMR (CDCl₃ + CF₃COOD) δ in ppm in relation to TMS: 2H at 4.33 (s), 9H at 7.5 to 8.7 (m), 2H at 11.5.

| Elemental Analysis | | | | |
|---|---|---|---|---|
| | C% | H% | N% | 0% |
| calculated : | 73.13 | 4.69 | 5.01 | 17.19 |
| found : | 73.13 | 4.69 | 5.00 | |

### EXAMPLE 16

### OXO-4-PHENYL-2-4H-[1]-BENZOPYRAN-8-THIOACETIMIDE

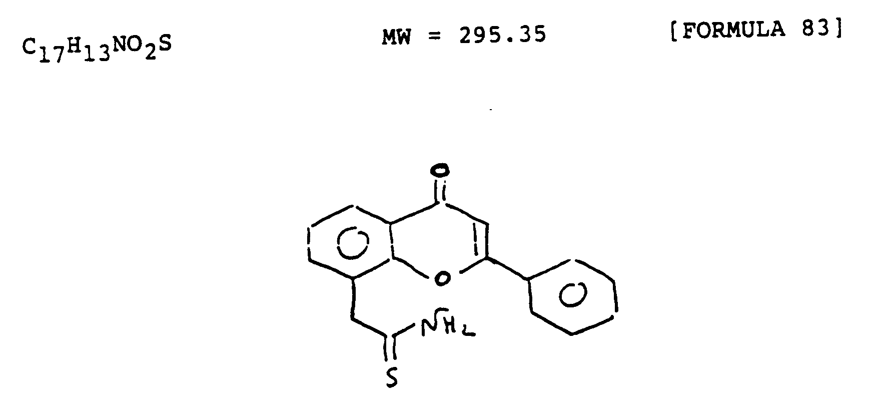

In a mixture of 60 g (0.229 mole) of oxo-4-phenyl-2-4H-[1]-benzopyran-8-acetonitrile, 16.2 ml (0.116 mole) of triethylamine and 900 ml of pyridine, a stream of H₂S is bubbled through for 3 hours. A nitrogen stream is then passed through this mixture and it is poured into 5 l of ice water, acidified to a pH 5-6 with HCl, filtered, washed in ether, dried, and crystallized in 6N DMF. Weight obtained:
24.7 g (yield: 36%); mp = 223-224°C; IR V NH = 3250 and 3080 cm⁻¹; IR Vc = o = 1620 cm⁻¹; NMR (DMSO) δ in ppm in relation to TMS: 2H at 4.15 (s), 1H at 6.9 (s), 8H at 7.2 to 8.4 (m), 2H at 9.4 (s).

| Elemental Analysis | | | | | |
|---|---|---|---|---|---|
| | C% | H% | N% | O% | S% |
| calculated : | 69.13 | 4.44 | 4.74 | 10.83 | 10.86 |
| found : | 69.22 | 4.38 | 4.80 | | 10.68 |

### EXAMPLE 17

### PHENYL-2-[(PHENYL-4-THIAZOL-2-YL)METHYL]-8-4H-[1]-BENZOPYRANONE-4

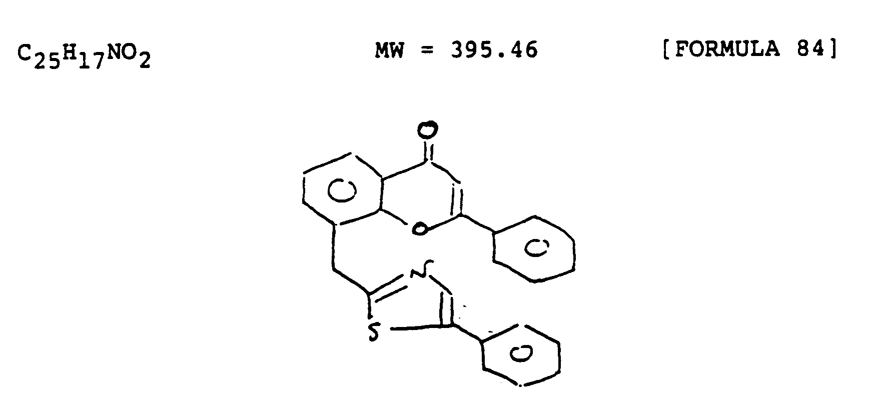

A mixture of 5 g (0.0169 mole) of oxo-4-phenyl-2-4H-[1]-benzopyran-8-thioacetamide, 4g (0.0203 mole) of d-bromoacetophenone and 120 ml of methoxyethanol is heated for five hours of reflux, then cooled and left overnight at -20°C. The solid obtained is filtered and recrystallized in MIBK then in acetone. Weight obtained: 3.3 g (yield: 49%); IR Vc = o 1620 cm⁻¹; NMR (CDCl₃) δ in ppm in relation to TMS: 2H at 4.7 (s), 1H at 6.75 (s), 14 H at 7.1 to 8.3 (m).

| Elemental Analysis | | | | | |
|---|---|---|---|---|---|
| | C% | H% | N% | O% | S% |
| calculated | 75.92 | 4.33 | 3.54 | 8.09 | 8.11 |
| found | 75.73 | 4.23 | 3.52 | | 8.31 |

Using this same technique, the following compounds were prepared:

### [(OXO-4-PHENYL-2-4H-[1]-BENZOPYRAN-8-YL) METHYL]-2-THIAZOL-4-ETHYL CARBOXYLATE

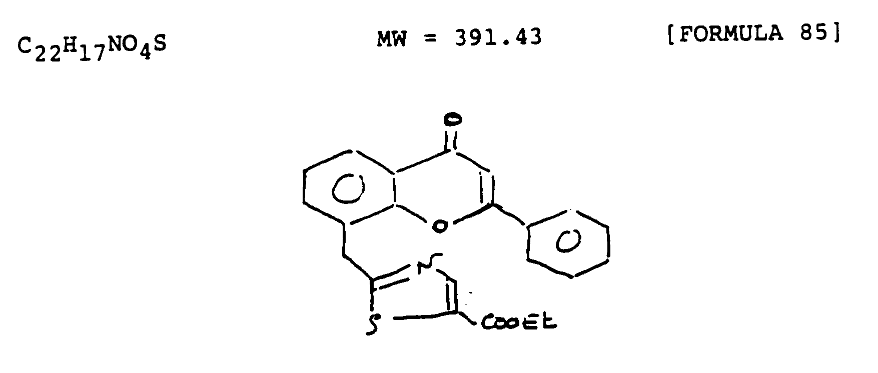

mp = 152-153°C; IR Vc = o (ester) = 1710 cm⁻¹, Vc = o (pyrone) = 1640 cm⁻¹; NMR (CDCl₃) δ in ppm in relation to TMS: 3H at 1.3 (t) 2H at 4.4 (q), 2H at 4.73 (s), 1H at 6.7 (s), 9H at 7.1 to 8.3 (m).

### [(OXO-4-PHENYL-2-4H-{12}-BENZOPYRAN-8-YL) METHYL-2-THIAZOL-4-CARBOXYLIC ACID

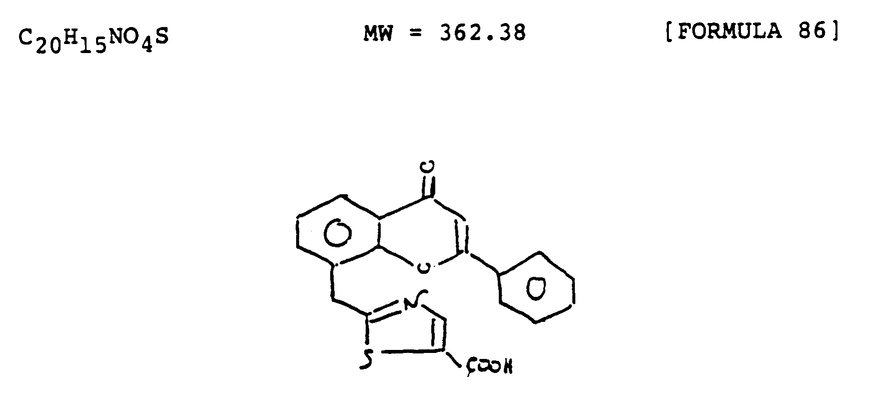

mp = 237-240°C; IR V OH = 3100 to 2400 cm⁻¹; IR Vc = o (acid) = 1720 cm⁻¹, Vc = o (pyrone) = 1620 cm⁻¹ NMR (DMSO) δ in ppm in relation to TMS: 1H at 4.7 (s), 1H at 6.9 (s), 8H at 7.2 to 8.15 (m), 1H at 8.2 (s).

| Elemental Analysis | | | | | |
|---|---|---|---|---|---|
| | C% | H% | N% | 0% | S% |
| calculated : | 66.10 | 3.61 | 3.85 | 17.61 | 8.82 |
| found : | 69.83 | 3.60 | 3.87 | | 8.60 |

### EXAMPLE 18

### [OXO-4-PHENYL-2-4H-[1]-BENZOPYRAN-8-YL)METHYLENE]-2-HYDRAZINE CARBOTHIOAMIDE

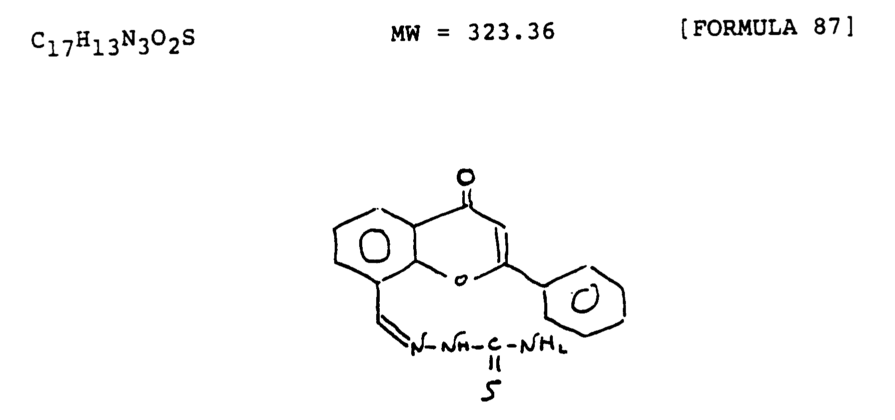

A suspension of 5 g (0.02 mole) of (oxo-4-phenyl-2-4H-[1]-benzopyran-8-yl) carboxaldehyde in 120 ml of dioxane was heated until dissolution. This was cooled to 25°C, a solution of 2 g (0.022 mole) of thiosemicarbazide in 40 ml of dioxane was added and this was heated for 5 minutes at 90°C, then left to return to 25°C while stirring. The precipitate was filtered and recrystallized in methoxyethanol. Weight obtained: 48 g (yield: 41%); mp = 258-262°C; IR V NH = 3400 to 3100 cm⁻¹, V C = O: 7640 cm⁻¹.

| Elemental Analysis | | | | | |
|---|---|---|---|---|---|
| | C% | H% | N% | O% | S% |
| calculated | 63.14 | 4.05 | 13.00 | 9.90 | 9.92 |
| found | 63.12 | 4.04 | 13.00 | | 9.87 |

Using the same method, the following compound was prepared:

### DIHYDRO-4,5-[1H]-IMIDAZO9LE-2-YL-HYDRAZONE BROMHYDRATE (OXO-4-PHENYL-2-4H-[1]-BENZOPYRAN-8-YL) CARBOXALDEHYDE

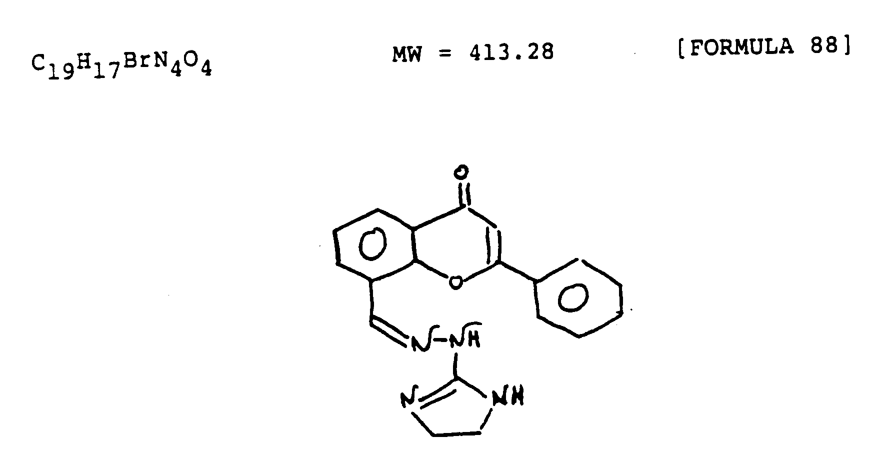

mp = 301-303°C; IR: V NH = 3300 cm⁻¹; V C = N - C = O = 1660 and 1640 cm⁻¹; NMR (DMSO) δ in ppm in relation to TMS: 2H to 3.4 (s), 4H to 3.8 (s), 1H to 7.1 (s), 10H from 7.3 to 9 (m of which 1H is exchangeable).

| Elemental Analysis | | | | | |
|---|---|---|---|---|---|
| | C% | H% | Br% | N% | O% |
| calculated : | 55.21 | 4.15 | 19.34 | 13.56 | 7.74 |
| found : | 54.96 | 4.09 | | 13.62 | |

### Example 19

### (OXO-4-TETRAHYDRO-2,3,5,6-4H-PYRAN-2-YL)-8-PHENYL-2-4H-[1]-BENZOPYRANONE-4

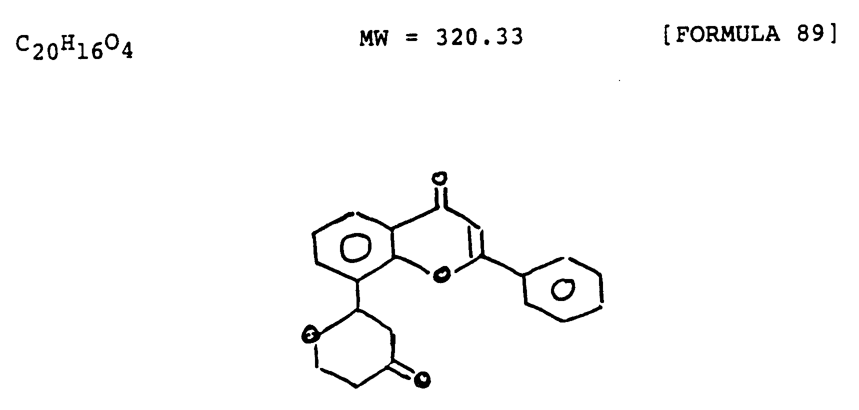

20 g (0.08 mole) of oxo-4-phenyl-2-4H-[1]-benzopyran-8-carboxaldehyde were added in parts to a mixture of 22.8 g (0.16 mole) of trimethylsilyloxy-2-butadiene-1-3 and 12 g (0.088 mole) of anhydrous ZnCl₂ in 500 ml of anhydrous dioxane. This was brought to reflux for 8 hours under nitrogen and then left under stirring for 48 hours at room temperature. A slight insoluble product was filtered and 1 liter of a solution of 5% NaHCO₃ was added to the filtrate. The insoluble product formed was filtered and the filtrate was extracted using ethyl acetate, then dried and evaporated under a vacuum. The residue was dissolved in 300 ml of methanol and brought to reflux for 3 hours. After having been cooled to 25°C, 3.6 ml of acetic acid were added and this was left under stirring for one night. This was evaporated under a vacuum and the residue was recrystallized in MIBK. Weight obtained: 9.2 g (yield: 30%); mp = 220-221°C; IR V C = O (pyranone) = 7695 cm⁻¹, V C = O (pyrone) = 1640 cm⁻¹; NMR (CDCl₃) δ in ppm in relation to TMS: 4H from 2.3 to 3.2 (m), 2H from 3.7 to 4.7 (m), 1H to 5.3 (dd), 1H to 6.8 (s), 8H from 7.1 to 8.3 (m).

| Elemental Analysis | | | |
|---|---|---|---|
| | C% | H% | 0% |
| calculated : | 74.98 | 5.03 | 19.98 |
| found : | 75.03 | 4.81 | |

### EXAMPLE 20

### (HYDROXY-4-TETRAHYDRO-3,4,5,6-2H-PYRAN-2-YL)-8-PHENYL-2-4H-[1]-BENZOPYRANONE-4

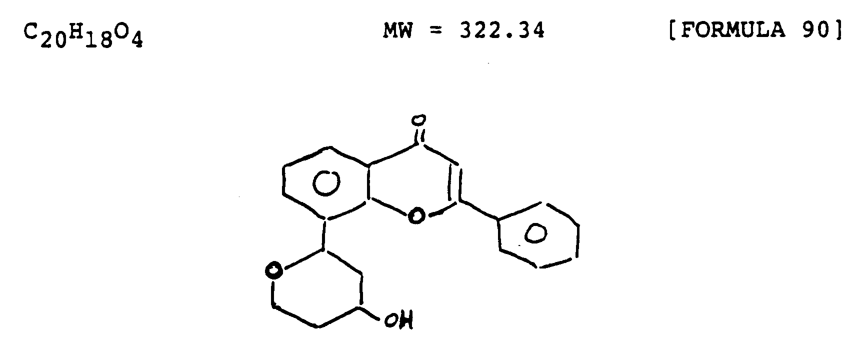

A mixture of 6.8 g (0.021 mole) of the compound of Example 18,116 ml of dioxane and 58 ml of methanol was heated until dissolution. It was cooled to 35°C and 0.9 g (0.023 mole) of NABH₄ were added in parts. This was then brought to reflux for 3 hours. After having been cooled, water was added and the precipitate obtained was filtered and recrystallized in isopropanol. Weight obtained: 3 g (yield: 43%); mp = 187-190°C; IR VOH 3400-3200 cm⁻¹; Vc = o = 1610 cm⁻¹ NMR CDCP₃) δ in ppm in relation to TMS: 4H at 1 to 2.76 m; 4H from 3.2 to 4.4 (m, of which 1H is exchangeable), 1H to 4.9 (dd) 1H to 6.8 (s), 8H from 7.2 to 8.2 (m).

| Elemental Analysis | | | |
|---|---|---|---|
| | C% | H% | 0% |
| calculated : | 74.52 | 5.63 | 19.85 |
| found : | 74.82 | 5.52 | |

### EXAMPLE 21

### OXO-4-(OXO-PHENYL-2-4H-[1]-BENZOPYRAN-8-YL)-4-BUTEN-2-OIC ACID

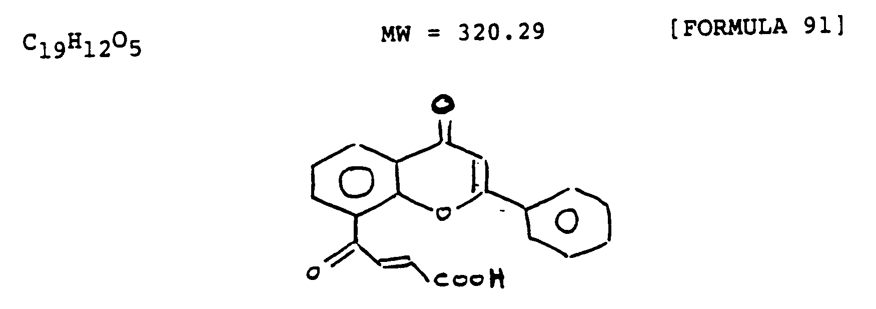

A mixture of 2 g (0.0076 mole) of acetyl-8-phenyl-2-4H-[1]-benzopyranone-4, 1.4 g (0.019 mole) of glyoxylic acid and 25 ml of acetic acid was brought to reflux for 2 hours. This was then poured into water and the precipitate formed was filtered. This was heat dissolved with a solution of 5% NaHCO₃ and acidified using acetic acid. The precipitate was filtered, washed with water and recrystallized in the dioxane-hexane mixture. Weight obtained: 0.5 g (yield: 20.6%); mp = 217-218°C; IR V C = O (acid) 1710 cm⁻¹, V C = O (ketone) = 1760 cm⁻¹, V C = O (pyrone) = 1620 cm⁻¹; NMR (DMSO) δ in ppm in relation to TMS 1H to 3.8 (exchangeable), 11H from 6.5 to 8.5 (m).

| Elemental Analysis | | | |
|---|---|---|---|
| | C% | H% | O% |
| calculated : | 71.24 | 3.78 | 24.98 |
| found : | 71.17 | 3.52 | |

### EXAMPLE 22

### (OXO-4-PHENYL-2-4H-[1]-BENZOPYRAN-8-YL)-2-HYDROXY-2-ACETIC ACID

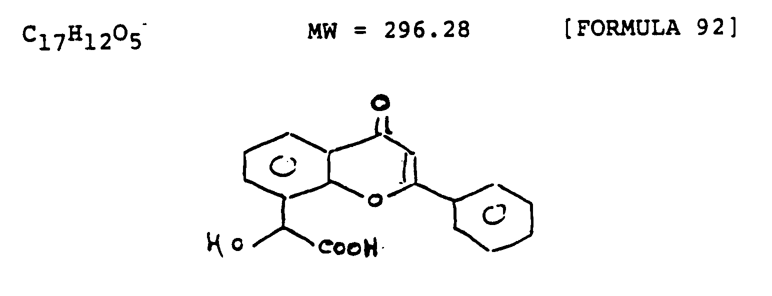

A mixture of 8.75 g (0.134 mole) of potassium cyanide, 125 ml of water, 1.25 l of dioxane, 53 g (0.5 mole) of Na₂CO₃ and 15.32 g (0.061 mole) of oxo-4-phenyl-2-4H-benzopyran-8-carboxaldehyde was stirred at room temperature for 1 hour. 75 ml of acetic acid were then added and this was stirred for 6 hours at room temperature. It was poured into 4 liters of water. The precipitate obtained was washed with water and recrystallized in an acetic acid-water mixture. Weight obtained: 2.5 g (Yield: 14%); IR V OH - 3450 cm⁻¹, V C = O (acid) = 1720 cm⁻¹, V C = O (pyrone) = 1620 cm⁻¹; NMR (DMSO) δ in ppm in relation to TMS: 1H at 3.4 (interchangeable), 1H at 5.6 (s), 1H at 7(s), 9H at 7.3 to 8.3 (m, of which 1H is interchangeable).

| Elemental Analysis | | | |
|---|---|---|---|
| | C% | H% | O% |
| calculated : | 68.92 | 4.08 | 27.00 |
| found : | 68.98 | 4.19 | |

### Example 23

### (OXO-4-PHENYL-2-4H-[1]-BENZOPYRAN-8-YL)-2-HYDROXY-2-ETHYL ACETATE

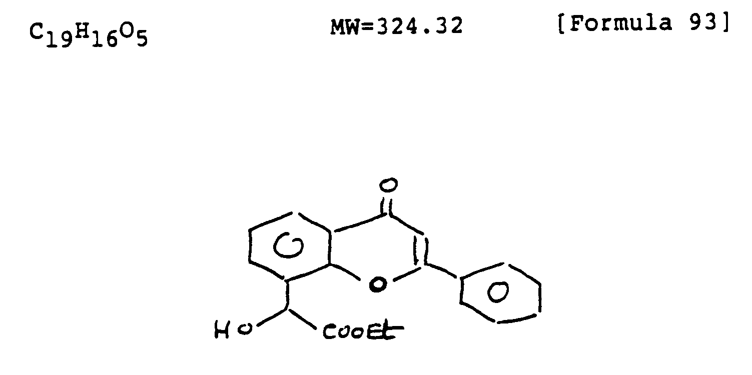

A mixture of 29 g (0.098 mole) of (oxo-4-phenyl-2-4H-[1]-benzopyran-8-yl)-2-hydroxy-2 acetic acid and 35 ml of concentrated H₂SO₄ in 585 ml of ethanol are brought to reflux for 5 hours. The mixture was then poured into water, extracted using ethyl acetate, dried, evaporated and the white solid obtained was recrystallized in MIBK-hexane. Weight obtained: 20.3 g (Yield: 64%); MP_{K}=135°C; IR: V OH=3420 cm⁻¹, V C=O (ester)=1730 cm⁻¹, V C=O (pyrone)=1640 cm⁻¹.

### Example 24

### (OXO-4-PHENYL-2-4H-[1]BENZOPYRAN-8-YL)-2-OXO-2-ETHYL ACETATE

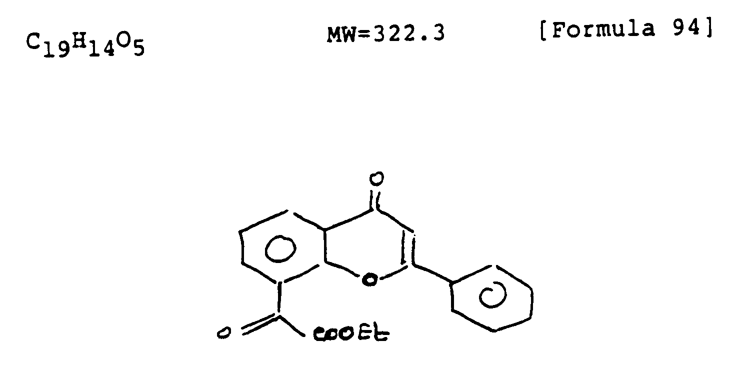

0.79 g (0.0077 mole) of CrO₃ and 0.84 g (0.0077 mole) of chlorotrimethylsilane were dissolved in 10 ml of methylene chloride. A solution of 2.5 g (0.0077 mole) of (oxo-4-phenyl-2-4H-[1]benzopyran-8-yl)-2-hydroxy-2-ethyl acetate in 20 ml of methylene chloride was added while cooling the red solution obtained. This was stirred at room temperature for 3 hours 50 minutes. The medium was then passed on a silica column and eluted with CHCl₃. This was evaporated and the residue was recrystallized in hexane. Weight obtained: 0.9 g (Yield: 36.3%); MP_{K}=85-90°C; IR V C=O (ester)=1730 cm⁻¹, V C=O (ketone)=1690 cm⁻¹ 1, V C=O (pyrone)=1640 cm⁻¹, NMR (CDCl₃), δ in ppm in relation to TMS: 3H to 1.3 (t), 2H to 4.3 (q), 1H to 6.8 (s), 8H to 7.25 to 8.7 (m).

### Example 25

### (OXO-4-PHENYL-2-4H[1]BENZOPYRAN-8-YL]-2-OXO-2-ACETATE ACID

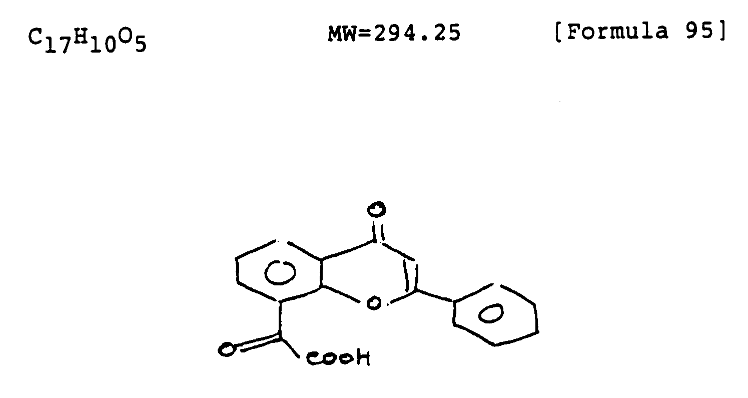

The mixture of 9.3 g (0.0288 mole) of (oxo-4-phenyl-2-4H-[1]benzopyran-8-yl)-2-oxo-2-ethyl acetate, 4.85 g (0.057 mole) of sodium bicarbonate, 150 ml of ethanol and 115 ml of water was refluxed for 4 hours 30 minutes. The ethanol was then evaporated, 150 ml of water were added, the mixture was acidified with 1/2 HCl and the precipitate obtained was filtered and recrystallized in dioxane. Weight obtained: 2.3 g (Yield 27%), MP_{G}=232-235°C, IR V C=O (acid)=1740 cm⁻¹, V C=O (ketone)= 1690 cm⁻¹, V C=O (pyrone)=1660 cm⁻¹. NMR (DMSO) δ in ppm in relation to TMS: 1H to 7.3 (s), 9H to 7.4 to 8.5 (m, 1H of which is exchangeable).

| Elemental analysis | | | |
|---|---|---|---|
| | C% | H% | 0% |
| calculated : | 69.39 | 3.91 | 27.19 |
| found : | 69.11 | 3.95 | |

### Example 26

### METHYL-2-(OXO-4-PHENYL-2-4H-[1]BENZOPYRAN-8-Y)-2-METHYL PROPANOATE

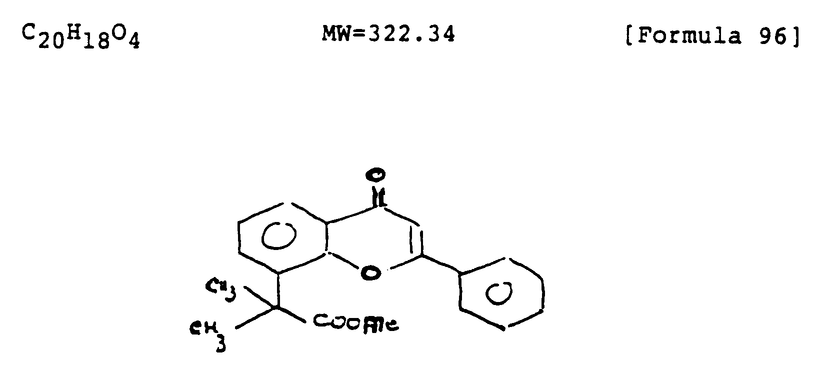

A solution of 6.7 g (0.023 mole) of oxo-4-phenyl-2-4H-[1]benzopyran-8-methyl acetate in 120 ml of DMF was added slowly to a suspension of 2.33 g (0.0485 mole of sodium hydride in 10 ml of DMF. This was stirred for one hour at room temperature, then 6.6 cm (0.1 mole) of methyl iodide in 5 ml of DMF was added dropwise. This was stirred for 6 hours at room temperature and then 6.6 ml of CIH₃ in 5 ml of DMF was added. This was stirred for one night, 15 ml of acetic acid were added, it was concentrated to 50 ml, water was added and the ethyl acetate was extracted. This was dried, evaporated under a vacuum and recrystallized in methanol. Weight obtained: 3.5 g (Yield 42%); MP_{K}=157°C; IR V C=O (ester)=1720 cm⁻¹, V C=O (pyrone)=1650 cm⁻¹; NMR (CDCl₃) δ in ppm in relation to TMS: 6H to 1.75 (s), 3H to 3.6 (s), 1H to 6.87 (s), 8H to 7.2 to 8.4 (m).

### Example 27

### METHYL-2-(OXO-4-PHENYL-2-4H-[1]BENZOPYRAN-8-YL)-2-PROPIONIC ACID

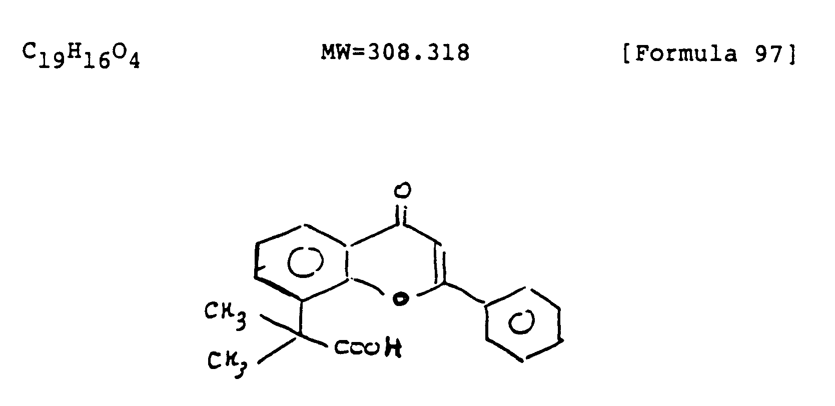

A mixture of 5.7 g (0.0177 mole) of methyl-2-(oxo-4-phenyl-2-4H-[1]benzopyran-8-yl)-2-methyl propanoate, 95 ml of acetic acid, 95 ml of concentrated sulfuric acid and 95 ml of concentrated hydrochloric acid were refluxed for 2 hours. This was then stirred for 12 hours at room temperature and brought again to reflux for 3 hours. It was cooled and the precipitate formed was filtered and stabilized in 250 ml of a 5% bicarbonate solution. It was acidified with 1/2 HCl and the precipitate was dried, washed with water and recrystallized in acetic acid.
Weight obtained: 3.1 g (Yield: 56.8%); MP_{G}=255-260; IR V C=O (acid)=1720 cm⁻¹, V C=O (pyrone)=1620 cm⁻¹; NMR (CF₃COOD) δ in ppm in relation to TMS: 6H to 2 (s), 9H from 7.6 to 8.6 (m), 1H to 11.7 (exchangeable).

| Elemental analysis | | | |
|---|---|---|---|
| | C% | H% | 0% |
| calculated : | 74.01 | 5.23 | 20.76 |
| found : | 73.93 | 5.25 | |

### Example 28

### OXO-4-PHENYL-2-4H[1]-BENZOPYRAN-8-CARBOXALDEHYDE OXIME (8)

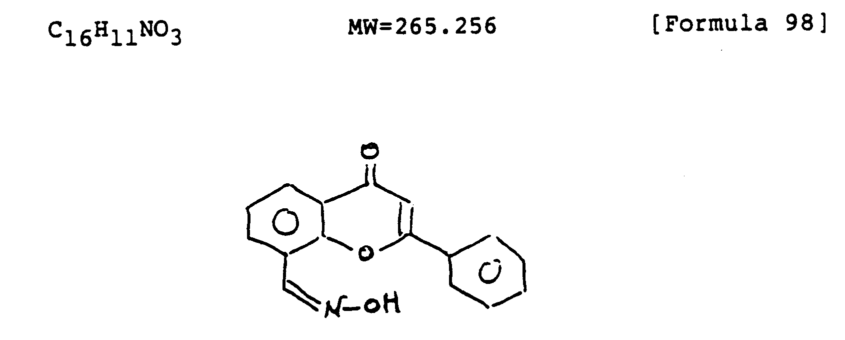

A mixture of 10 g (0.04 mole) of oxo-4-phenyl-2-4H-[1]benzopyran-8-carboxaldehyde, 3.7 g (0.054 mole) of hydroxylamine hydrochlorate, 7.1 g (0.10 mole) of sodium acetate, 20 ml of water and 40 ml of ethanol was brought to reflux for 1 hours. After cooling, the product formed was dried and recrystallized in dioxane. Weight obtained: 6.3 g (Yield: 59.4%); MP_{G}=230-238°C; IR V OH=3200 to 2800 cm⁻¹, V C=O; NMR (CF₃COOD), δ in ppm in relation to TMS: 10 H from 7.8 to 9.5 (m).

| Elemental analysis | | | | |
|---|---|---|---|---|
| | C% | H% | N% | 0% |
| calculated : | 72.44 | 4.18 | 5.28 | 18.10 |
| found : | 72.74 | 4.24 | 5.03 | |

Using the same method, the following compound was prepared:

### ACETYL-8-PHENYL-2-4H-[1]-BENZOPYRANONE-4- OXIME (8)

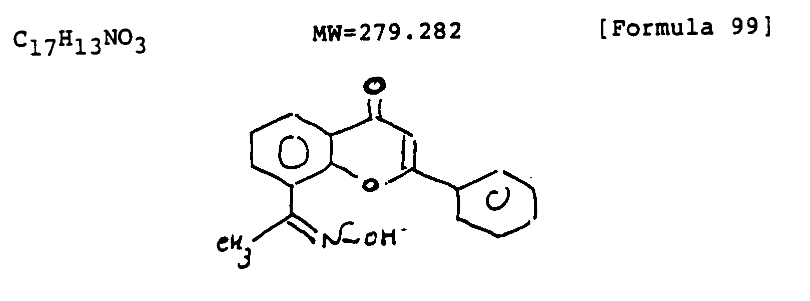

| Elemental analysis | | | | |
|---|---|---|---|---|
| | C% | H% | N% | 0% |
| calculated : | 73.10 | 4.69 | 5.02 | 17.19 |
| found : | 73.00 | 4.70 | 4.99 | |

### Example 29

### (MORPHOLIN-4-YL)-3-(OXO-4-PHENYL-2-4H-[1]-BENZOPYRAN-8-YL]-2-GLUTARONITRILE

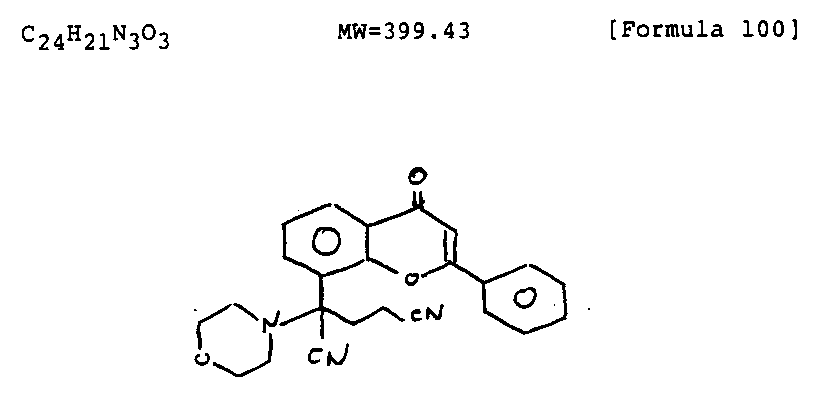

A solution of 1.33 g (0.025 mole) of acrylonitrile in 10 ml of dioxane was added dropwise to a mixture of 7 g (0.02 mole) of (morpholin-4-yl)- -(oxo-4-phenyl-2-4H-[1]benzopyran-8-yl)-2 acetonitrile. After 18 hours at room temperature, a slightly insoluble material was filtered and evaporated under a vacuum. The residue was recrystallized in isopropanol. Weight obtained: 3.1 g (Yield: 38.8%); MP_{K}=110°C; IR V C=N=2250 cm⁻¹, V C=1640 cm⁻¹; NMR (CDCl₃) δ in ppm in relation to TMS: 8H from 1.8 to 3.2 (m); 4H to 3.9 (t), 1H to 7 (s), 8H from 7.4 to 8.6 (m).

### Example 30

### OXO-4-(OXO-4-PHENYL-2-4H-[1]BENZOPYRAN-8-YL)-4-BUTYRIC ACID

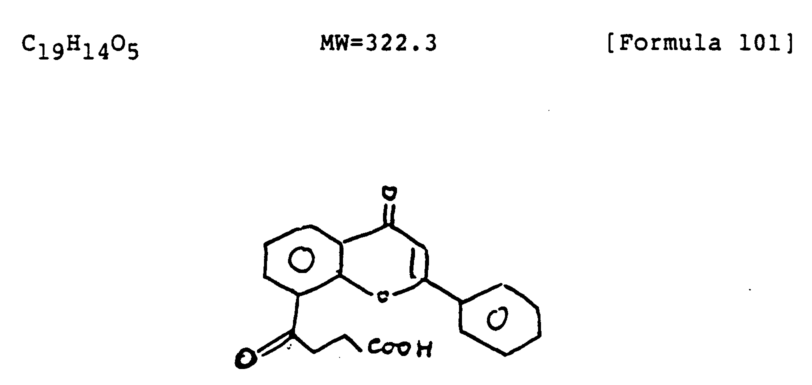

A mixture of 3 g (0.0075 mole) of the compounf od Example 29, 30 ml of 6N hydrochloric acid and 30 ml of acetic acid was refluxed for 4 hours. This was then poured into water and ice, the product was dried, it was replaced in a solution of 5% NaHCO₃ and acidified. The precipitate formed was dried and recrystallized in the MIBK-dioxane mixture. Weight obtained: 1.1 g (Yield: 45.5%); MP_{F}=207-209°C; IR V C= ) (acid=1720 cm⁻¹, V C=O (ketone)=1680 cm⁻¹, V C=O (pyrone)=1620 cm⁻¹; NMR DMSO) δ in ppm in relation to TMS: 4H from 2.4 to 3.6 (m), 1H to 7.1 (s), 8H from 7.4 to 8.4 (m), 1H to 12.1 (exchangeable).

| Elemental analysis | | | |
|---|---|---|---|
| | C% | H% | 0% |
| calculated : | 70.80 | 4.38 | 24.82 |
| found : | 70.50 | 4.43 | |

### Example 31

### HYDROXY-4-(OXO-4-PHENYL-2-4H-[1]BENZOPYRAN-8-Y)-BUTYRIC ACID

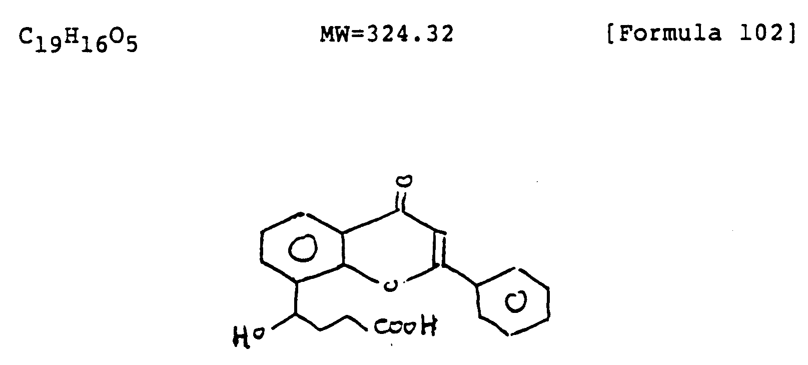

By treating 5 g (0.0155 mole) of the product of Example 30 with 9.5 g (0.0468 mole) of aluminum isopropylate in 100 ml of isopropanol and 40 ml of dioxane for 6 hours at reflux, 1.9 g of isopropyl hydroxy ester (MP_{K}=145°C) was obtained after recrystallization in hexane. This was placed in 10 ml of water, 17 m with 0.45 g of sodium bicarbonate. The medium was brought to reflux for 4 hours 30 minutes, evaporated and the residue was replaced in water. The insoluble material was filtered, acidified with acetic acid, dried, and recrystallized in dioxane. Weight obtained: 0.7 g MP_{G}=198-202°C; IR V OH=3350 cm⁻¹, V C=O (acid)=1700 cm⁻¹, V C=O (pyrone)=1620 cm⁻¹; NMR (DMSO) δ in ppm in relation to TMS: 4H from 1.7 to 2.9 (m), 2H from 5.3 to 5.8 (m, of which 1H is exchangeable), 1H to 7.1 (s), 8H from 7.4 to 8.4 (m), 1H to 11.8 (exchangeable).

| Elemental Analysis | | | |
|---|---|---|---|
| | C% | H% | 0% |
| calculated : | 70.36 | 4.97 | 24.67 |
| found : | 70.56 | 4.72 | |

### Example 32

### ACETAMIDO-2-ETHOXYCARBONYL-2-(OXO-4-PHENYL-2-4H-[1]BENZOPYRAN-8-YL)-2 ETHYL PROPIONATE

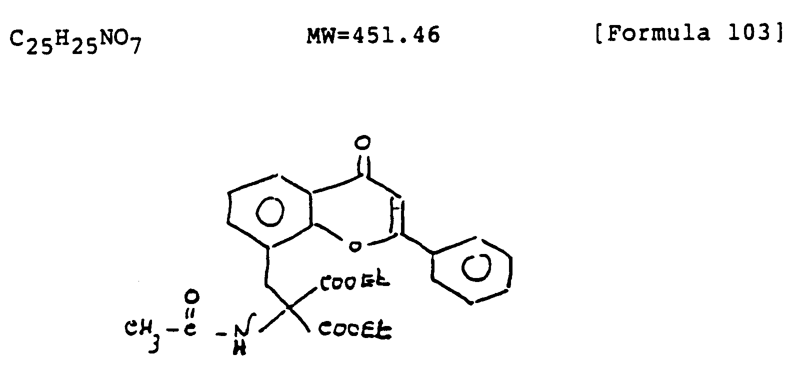

17.5 g (0.08 mole) of diethyl acetamidomalonate was added at 20°C in 20 minutes to a suspension of 3 g (0.08 mole) of sodium hydride in 100 ml of toluene. This was left under stirring for 1 hour, then 25 g (0.08 mole) of bromomethyl-8-phenyl-2-4H-[1]benzppyranone-4 were added in one hour. This was brought to a reflux for 8 hours and then hot filtered. the filtrate was evaporated under a vacum, the residue was replaced in water, the solid was dried and recrystallized in ethanol. Weight obtained:
24.4 g (Yield: 67.6%); MP_{K}=200°C; IR V NH=3370 cm⁻¹ V C=O (ester)=1720 cm⁻¹ and 1760 cm⁻¹, V C=O (amide)=7670 cm⁻¹, V C=O (pyrone)=1640 cm⁻¹.

### Example 33

### AMINO-2-(OXO-4-PHENYL-2-4H-[1]BENZOPYRAN-8-YL)-3-PROPIONIC ACID HYDROCHLORATE

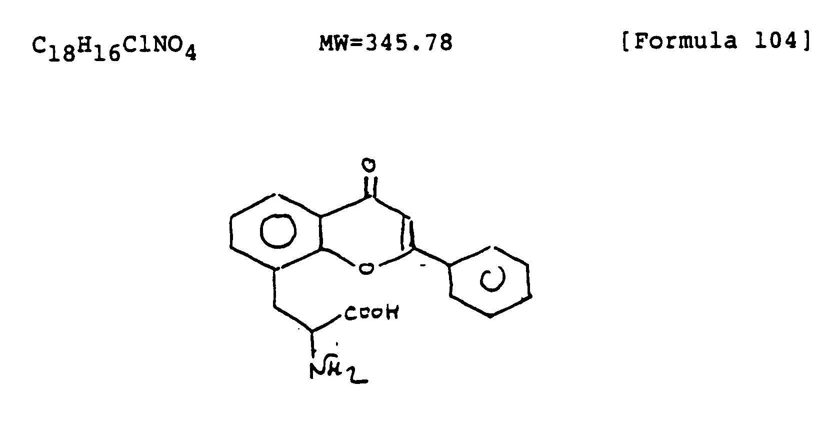

A mixture of 10 g (0.022 mole) of the compound of Example 32 and 400 ml of 1/2 HCl was brought to reflux for 4 hours. After a night of rest, the precipitate formed was dried and recrystallized in the ACOH-water mixture. Weight obtained: 4.4 g (Yield: 57.6%), MP_{G}=243°C; IR V OH, NH₃⁽⁺⁾=3500-2500 cm⁻¹, V C=O (acid)=1740 cm⁻¹, V C=O (pyrone)=1625 cm⁻¹; NMR (DMSO) δ in ppm in relation to TMS: 3H from 3.4 to 4.4 (m), 1H to 7.1 (s), 1H from 7.4 to 10 (m, of which 4H are exchangeable).

| Elemental analysis | | | | | |
|---|---|---|---|---|---|
| | C% | H% | Cl% | N% | O% |
| calculated : | 62.52 | 4.66 | 10.26 | 4.05 | 18.51 |
| found : | 62.66 | 4.89 | 10.35 | 4.11 | |

Following the experimental protocol outlined in Example 1 supra, the following compounds were prepared.

### 2-(2-AMINOPHENYL)-4-OXO-4H-[1]BENZOPYRAN-8-ACETIC ACID

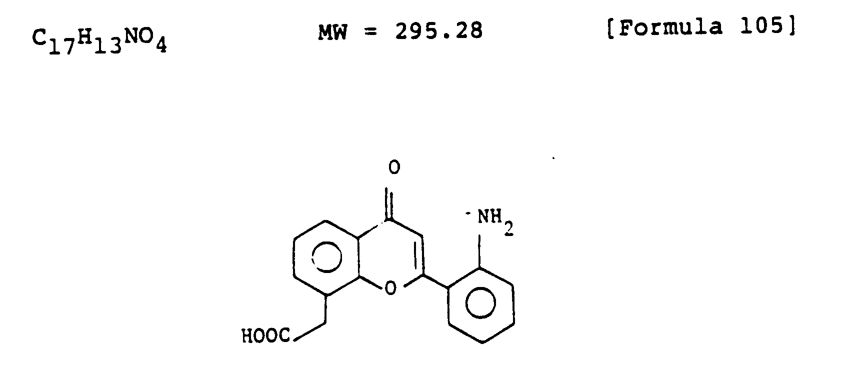

PF_{G} = 189° νC; IR νC = 0 (acid) 1710 cm⁻¹, νC = 0 (pyrone) = 1610 cm⁻¹

| Elemental Analysis | | | | |
|---|---|---|---|---|
| | C% | H% | N% | O% |
| calculated: | 69.14 | 4.44 | 4.74 | 21.61 |
| found: | 68.92 | 4.25 | 4.45 | |

### 2-(2-CHLOROPHENYL)-4-OXO-4H-[1]-BENZOPYRAN-8-ACETIC ACID

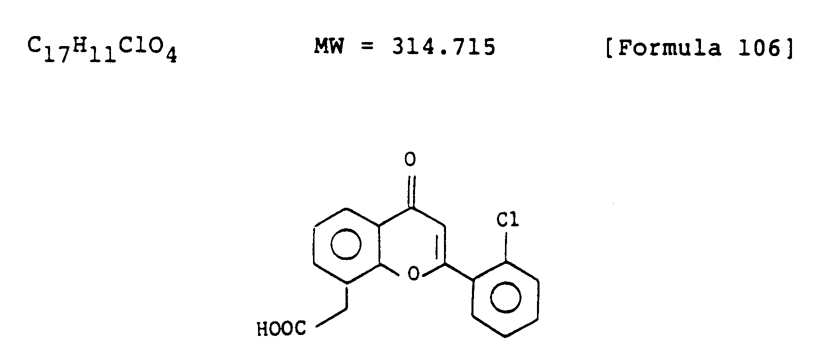

PF_{G} = 169°C; IR νC = 0 (acid) = 1710 cm⁻¹; νC = 0 (pyrone) = 1620 cm⁻¹; NMR (DMSO) δ in ppm relative to TMS: 2 H at 4.3 (s), 8 H from 7.5 to 8.7 (m), 1 H at 11.7 (exchangeable).

| Elemental Analysis | | | | |
|---|---|---|---|---|
| | C% | H% | Cl% | O% |
| calculated: | 64.87 | 3.52 | 11.27 | 20.34 |
| found: | 65.09 | 3.48 | 11.53 | |

### 2-(2-CHLOROPHENYL)-4-OXO-4H-[1]-BENZOPYRAN-8-ACETIC ACID

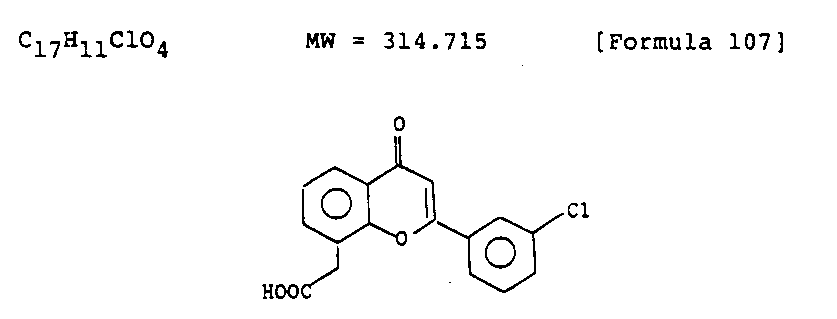

PF_{G} = 220-222°C; IR νC = 0 (acid) = 1730 cm⁻¹; νC = 0 (pyrone) = 1620 cm⁻¹; NMR (DMSO) δ in ppm relative to TMS: 2 H at 4 (s), 1 H from 7.5 (s), 7 H from 7.3 to 8.3 (m), 1 H at 13 (exchangeable).

| Elemental Analysis | | | | |
|---|---|---|---|---|
| | C% | H% | Cl% | O% |
| calculated: | 64.87 | 3.52 | 11.27 | 20.34 |
| found: | 64.59 | 3.53 | 11.28 | |

### 2-(2-ACETAMIDOPHENYL)-4-OXO-4H-[1]-BENZOPYRAN-8-ACETIC ACID

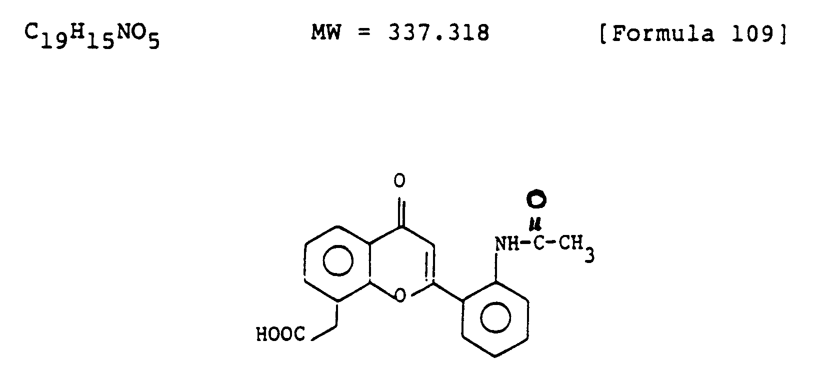

PF_{G} = 196-201°C; IR νC = 0 (acid) = 1720 cm⁻¹; νC = 0 amide) = 1660 cm⁻¹; νC = 0 (pyrone) = 1620 cm⁻¹; NMR (DMSO) δ in ppm relative to TMS: 3 H at 2 (s), 2 H at 3.8 (s), 1 H at 6.5 (s), 7 H from 7.3 to 8.2 (m), 1 H at 8.8 (exchangeable), 1 H at 11.5 (exchangeable).

| Elemental Analysis | | | | |
|---|---|---|---|---|
| | C% | H% | Cl% | O% |
| calculated: | 67.65 | 4.48 | 4.15 | 23.72 |
| found: | 67.42 | 4.24 | 4.11 | |

### 2-(4-ACETYLPHENYL)-4-OXO-4H-[1]-BENZOPYRAN-8-ACETIC ACID

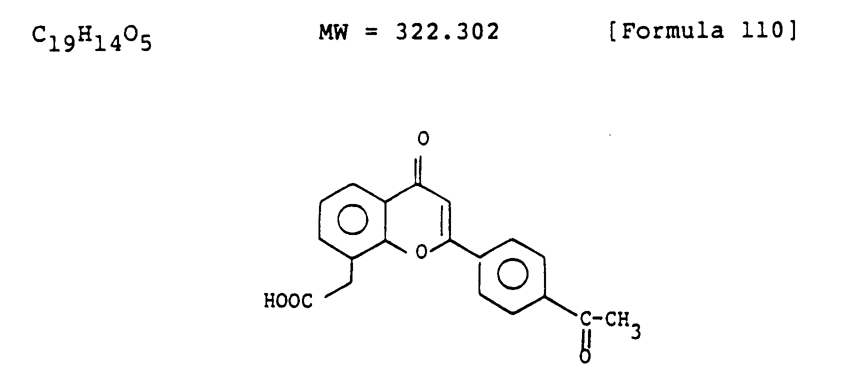

PF_{G} = 253-255°C; IR νC = 0 (pyrone) = 1620 cm⁻¹; NMR (CF₃COOD) δ in ppm relative to TMS: 3 H at 2.8 (s), 2 H at 4.3 (m), 8 H from 7.7 to 8.5 (m).

| Elemental Analysis | | | |
|---|---|---|---|
| | C% | H% | O% |
| calculated: | 70.80 | 4.38 | 24.82 |
| found: | 70.61 | 4.31 | |

### 2-(3-ACETAMIDOPHENYL)-4-OXO-4H-[1]-BENZOPYRAN-8-ACETIC ACID

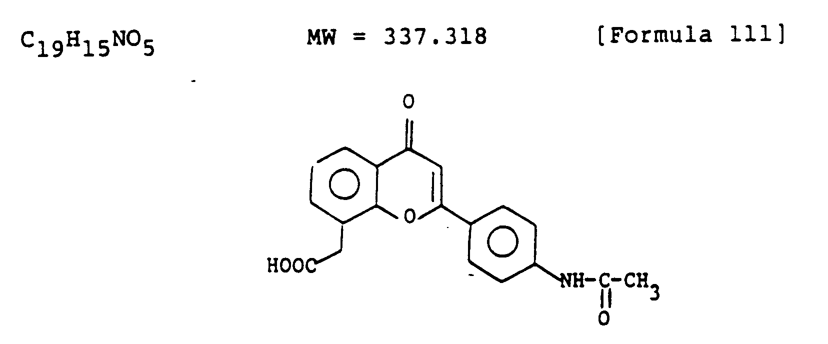

PF_{G} = 284-288°C IR νC = 0 (acid) = 1720 cm⁻¹; νC = 0 (amide + pyrone) = 1630 cm⁻¹; NMR (DMSO) δ in ppm relative to TMS: 3 H at 2 (s), 2 H at 4 (s), 1 H at 6.8 (s), 7 H from 7.1 to 8.1 (m), 1 H at 9.8 (exchangeable), 1 H at 12 (exchangeable).

| Elemental Analysis | | | | |
|---|---|---|---|---|
| | C% | H% | N% | O% |
| calculated: | 67.65 | 4.48 | 4.15 | 23.72 |
| found: | 67.70 | 4.40 | 4.12 | |

### 2-(2-DIETHYLAMINOETHOXYPHENYL]-4-OXO-4H-[1]-BENZOPYRAN-8-ACETIC ACID

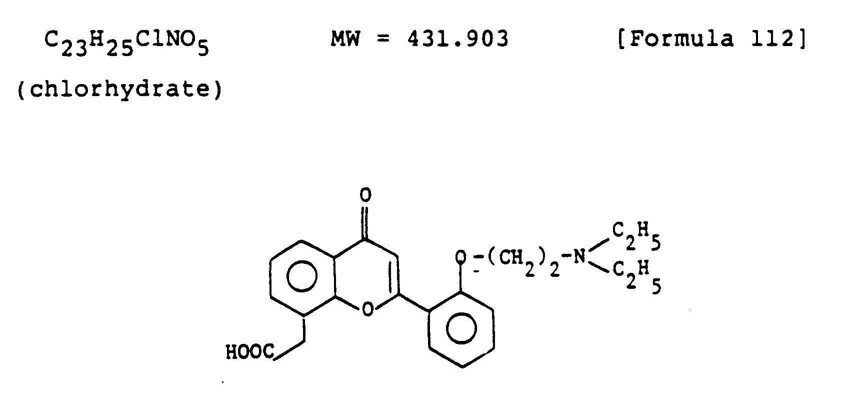

PF_{G} = 1780-182°C C₁(chlorhydrate); IR νC = 0 (acid) = 1720 cm⁻¹; νC = 0 (pyrone) = 1640 cm⁻¹; NMR (DMSO) δ in ppm relative to TMS: 6 H at 1 (t), 6 H from 2.8 to 3.8 (m), 1 H from 3.9 to 4.2 (m), 8 H from 6.8 to 8 (m), 1 H at 11 (exchangeable).

| Elemental Analysis | | | | | |
|---|---|---|---|---|---|
| | C% | H% | Cl% | N% | O% |
| calculated: | 63.96 | 6.07 | 8.21 | 3.24 | 18.52 |
| found: | 63.96 | 6.12 | 8.19 | 3.24 | |

### 2-(3-NITRO-4-CHLOROPHENYL)-4-OXO-4H-[1]-BENZOPYRAN-8-ACETIC ACID

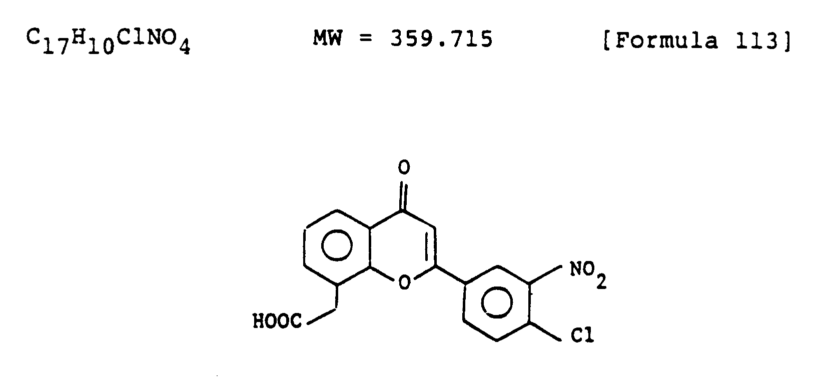

PF_{G} = 232 - 234°C; IR νC = 0 (acid) = 1720 cm⁻¹; νC = 0 (pyrone) = 1640 cm⁻¹; NMR (DMSO) δ in ppm relative to TMS: 2 H at 4 (s), 7 H from 7 to 8.8 (m), 1 H at 12.1 (exchangeable).

| Elemental Analysis | | | | | |
|---|---|---|---|---|---|
| | C% | H% | Cl% | N% | O% |
| calculated: | 56.76 | 2.80 | 9.86 | 3.89 | 26.69 |
| found: | 56.82 | 2.69 | 9.78 | 3.90 | |

### 2-(2,4-DIMETHOXYPHENYL)-4-OXO-4H-[1]-BENZOPYRAN-8-ACETIC ACID

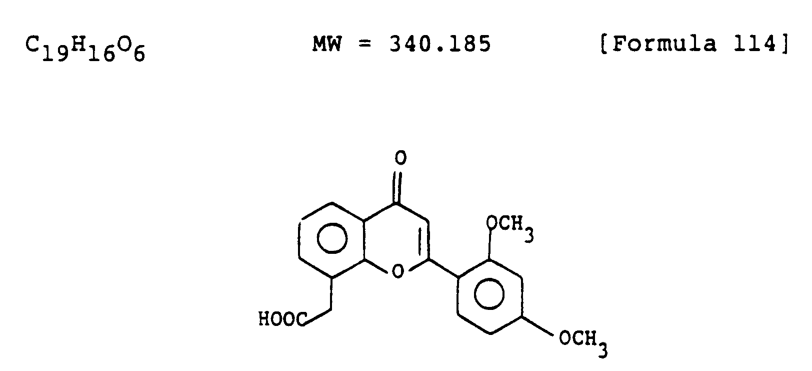

PF_{G} = 225-227°C; IR νC = 0 (acid) = 1710 cm⁻¹; νC = 0 (pyrone) = 1620 cm⁻¹; NMR (DMSO) δ in ppm relative to TMS: 2 H at 3.5 (s), 3 H at 3.9 (s), 3 H at 4 (s), 7 H from 6.8 to 8, 1 H at 12.2 (exchangeable).

| Elemental Analysis | | | |
|---|---|---|---|
| | C% | H% | O% |
| calculated: | 67.05 | 4.74 | 28.21 |
| found: | 67.23 | 4.63 | |

### 2-(4-DIMETHYLAMINOETHOXYPHENYL)-4-OXO-4H-[1]-BENZOPYRAN-8-ACETIC ACID

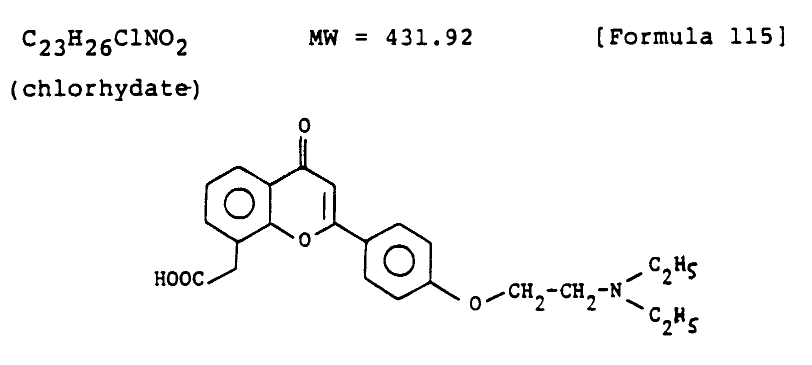

PF_{G} = 194-199°C (chlorhydate); IR νC = 0 (acid) = 1720 cm⁻¹; νC = 0 (pyrone) = 1630 cm⁻¹; NMR (DMSO) δ in ppm relative to TMS: 8 H from 3 to 4.7 (m), 8 H from 6.8 to 8.3 (m), 1 H from 10.4 to 10.8 (exchangeable).

| Elemental Analysis | | | | | |
|---|---|---|---|---|---|
| | C% | H% | Cl% | N% | O% |
| calculated: | 63.96 | 6.07 | 8.21 | 3.24 | 18.57 |
| found: | 63.50 | 6.04 | 8.30 | 3.45 | |

### 2-(4-CARBAMOYLPHENYL)-4-OXO-4H-[1]-BENZOPYRAN-8-ACETIC ACID

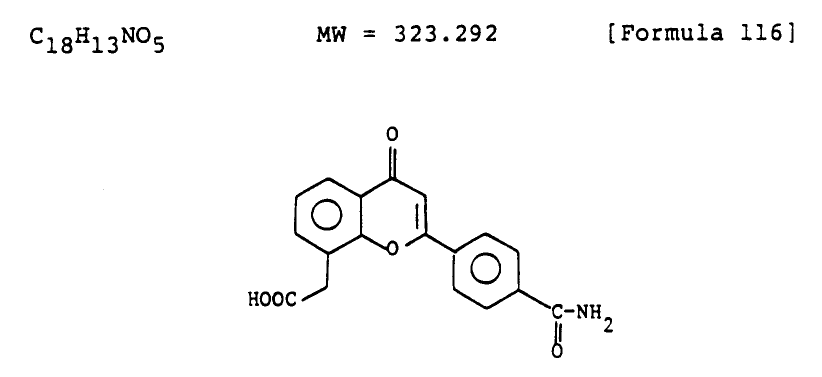

PF_{G} = 228-290°C; IR νC = 0 (acid) = 1710 cm⁻¹; νC = 0 (amide + pyrone) = 1640 cm⁻¹

| Elemental Analysis | | | | |
|---|---|---|---|---|
| | C% | H% | N% | O% |
| calculated: | 66.87 | 4.05 | 4.33 | 24.75 |
| found: | 66.35 | 4.35 | 4.46 | |

### [[methyl-2-thiazolyl-4]-4-phenyl]-2-OXO-4-4H[1]BENZOPYRAN-8-ACETIC ACID

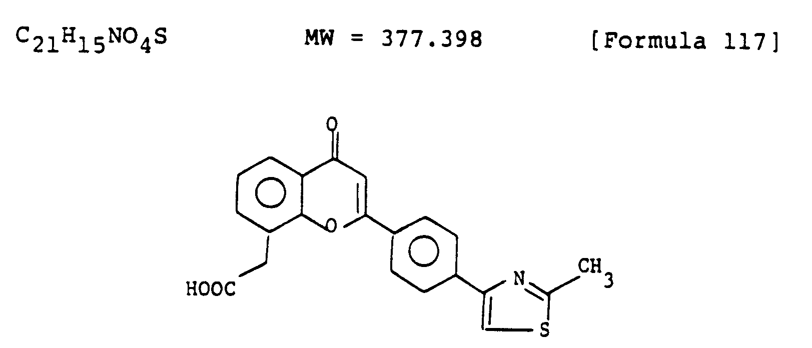

PF_{G} = 246-248°C; IR νC = 0 (acid) = 1720 cm⁻¹; νC = 0 (pyrone) = 1620 cm⁻¹; NMR (DMSO) δ in ppm relative to TMS: 3 H at 4 (s), 1 H at 7 (s), 8 H from 7.1 to 8.1 (m), 1 H at 12.6 (exchangeable).

| Elemental Analysis | | | | | |
|---|---|---|---|---|---|
| | C% | H% | N% | O% | S% |
| calculated: | 66.83 | 4.01 | 3.71 | 16.96 | 8.50 |
| found: | 66.73 | 3.95 | 3.66 | | 8.56 |

### 2-(2-CHLOROPHENYL)-4-OXO-4H-[1]-BENZOPYRAN-8-ACETIC ACID

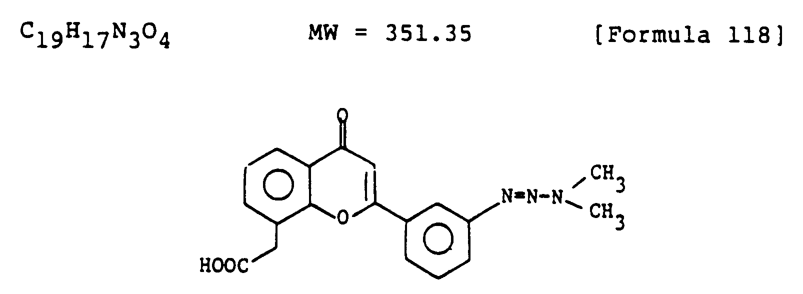

PF_{G} = 173-175°C; IR νC = 0 (acid) = 1710 cm⁻¹; νC = 0 (pyrone) = 1620 cm⁻¹; NMR (DMSO) δ in ppm relative to TMS: 6 H at 3.25 (s), 2 H at 4 (s), 1 H at 7 (s), 7 H from 7.2 to 8 (m), 1 H at 12.3 (exchangeable).

| Elemental Analysis | | | | |
|---|---|---|---|---|
| | C% | H% | N% | O% |
| calculated: | 64.95 | 4.88 | 11.96 | 18.22 |
| found: | 64.72 | 4.85 | 12.04 | |

### 2-(2-AMINO-4-THIAZOLYLPHENYL)-4-OXO-4H-[1]-BENZOPYRAN-8-ACETIC ACID

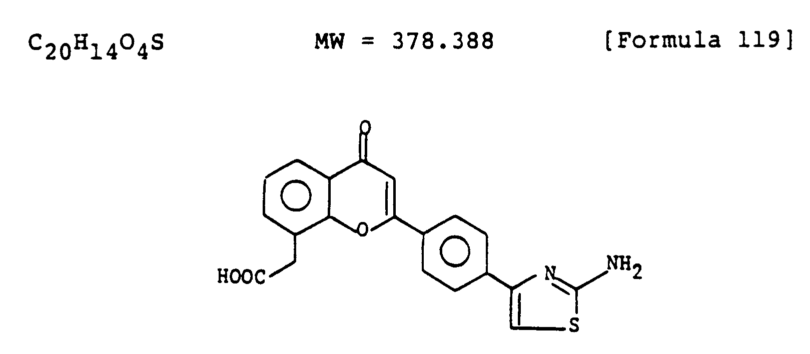

PF_{G} = 263-265°C; IR νC = 0 (acid) = 1710 cm⁻¹; νC = 0 (pyrone) = 1620 cm⁻¹; NMR (DMSO) δ in ppm relative to TMS: 2 H at 4 (s), 11 H from 7 to 8.2 (m, with 2 H exchangeable), 1 H at 12.9 (exchangeable).

| Elemental Analysis | | | |
|---|---|---|---|
| | C% | H% | O% |
| calculated: | 67.05 | 4.75 | 28.21 |
| found: | 67.70 | 4.54 | 7.12 |

### 2-(3,5 DIMETHOXYPHENYL)-4-OXO-4H-[1]-BENZOPYRAN-8-ACETIC ACID

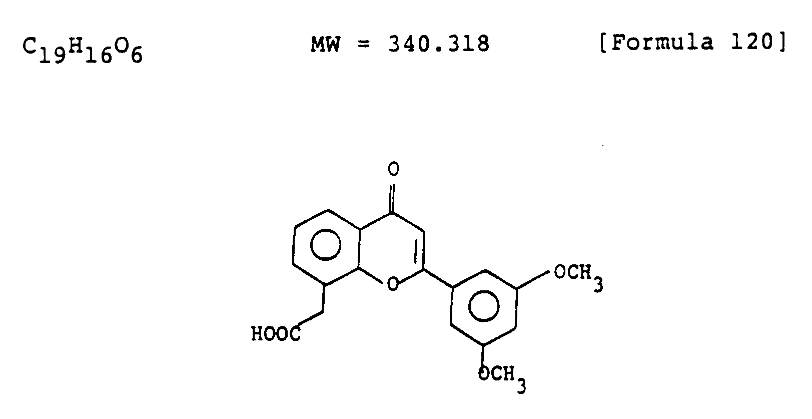

PF_{G} = 261-263°C; IR νC = 0 (acid) = 1720 cm⁻¹; νC = 0 pyrone) = 1630 cm⁻¹; NMR (DMSO) δ in ppm relative to TMS: 6 H at 3.9 (s), 2 H at 4 (s), 7 H from 6.5 to 8 (m), 1 H at 12.9 (exchangeable).

| Elemental Analysis | | | |
|---|---|---|---|
| | C% | H% | O% |
| calculated: | 67.05 | 4.74 | 28.21 |
| found: | 67.20 | 4.54 | |

### 2-(4-PYRIDYL)-4-OXO-4H-[1]-BENZOPYRAN-8-ACETIC ACID

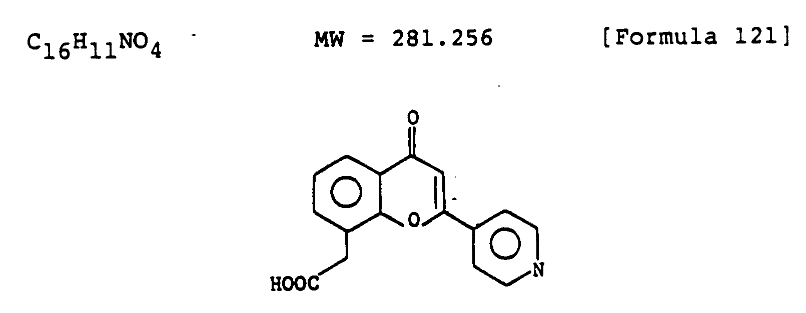

PF_{G} = 275-277°C; IR νC = 0 (acid) = 1720 cm⁻¹; νC = 0 (pyrone) = 1600 cm⁻¹; NMR (DMSO + CF₃COOD) δ in ppm relative to TMS: 2 H at 4 (s), 8 H from 7.3 to 8.8 (m).

| Elemental Analysis | | | | |
|---|---|---|---|---|
| | C% | H% | N% | O% |
| calculated: | 68.32 | 3.94 | 4.98 | 22.76 |
| found: | 67.94 | 4.09 | 5.12 | |

### 2-(2-PYRIDYL)-4-OXO-4H-[1]-BENZOPYRAN-8-ACETIC ACID

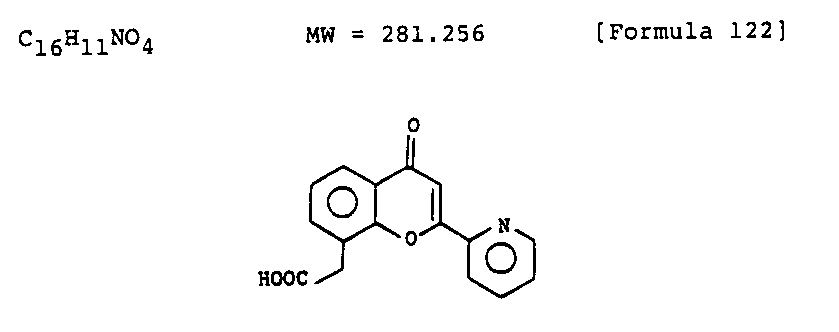

PF_{G} = 221-223°C; IR νC = 0 (acid) = 1720-1740 cm⁻¹; νC = 0 (pyrone) = 1640 cm⁻¹; NMR (DMSO) δ in ppm relative to TMS: 2 H at 4.1 (s), 1 H at 7.2 (s), 8 H from 7.25 to 9 (m), 1 H at 13 (exchangeable).

| Elemental Analysis | | | | |
|---|---|---|---|---|
| | C% | H% | N% | O% |
| calculated: | 68.32 | 3.94 | 4.98 | 22.76 |
| found: | 68.50 | 3.89 | 4.86 | |

### 2-(4-HEXYLPHENYL)-4-OXO-4H-[1]-BENZOPYRAN-8-ACETIC ACID

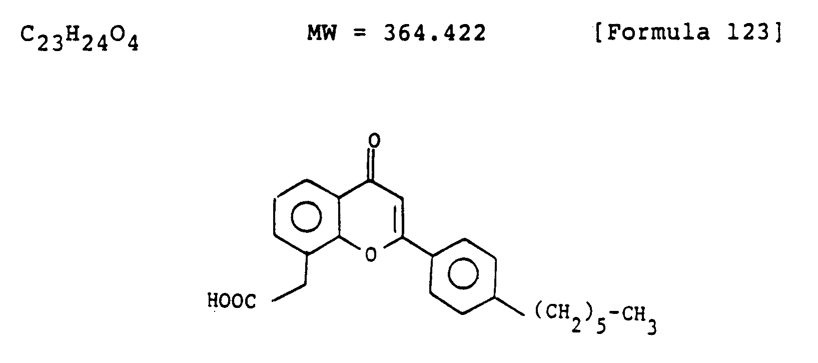

PF_{G} = 154-156°C; IR νC = 0 (acid) = 1720 cm⁻¹; νC = 0 (pyrone) = 1620 cm⁻¹; NMR (DMSO) δ in ppm relative to TMS: 13 H from 0.7 to 2.8 (m), 2 H at 4 (s), 1 H at 7 (s), 7 H from 7.2 to 8.1 (m), 1 H at 12.9 (exchangeable).

| Elemental Analysis | | | |
|---|---|---|---|
| | C% | H% | O% |
| calculated: | 75.80 | 6.64 | 17.56 |
| found: | 75.50 | 6.49 | |

### 2-(3-METHYLPHENYL)-4-OXO-4H-[1]-BENZOPYRAN-8-ACETIC ACID

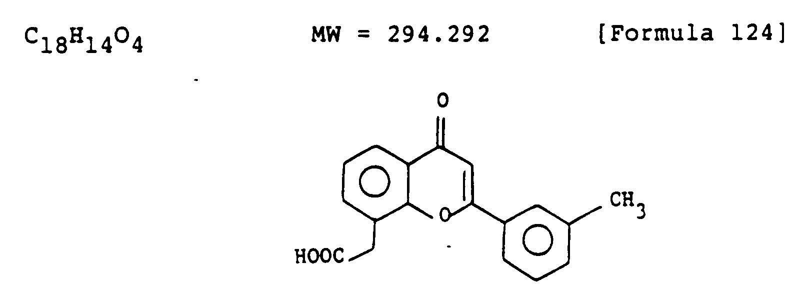

PF_{G} = 252-254°C; IR νC = 0 (acid) = 1720 cm⁻¹; νC = 0 (pyrone) = 1620 cm⁻¹; NMR (CF₃COOD) δ in ppm relative to TMS: 2 H at 2.55 (s), 2 H at 4.5 (m), 8 H from 7.5 to 8.6 (m).

| Elemental Analysis | | | |
|---|---|---|---|
| | C% | H% | O% |
| calculated: | 73.46 | 4.80 | 21.75 |
| found: | 73.74 | 4.86 | |

### 2-(4-BENZOYLPHENYL)-4-OXO-4H-[1]-BENZOPYRAN-8-ACETIC ACID

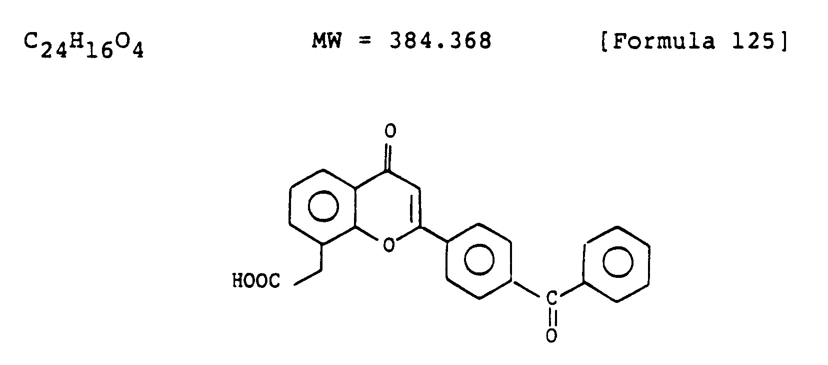

PF_{G} = 257-259°C; IR νC = 0 (acid) = 1720 cm⁻¹; νC = 0 (benzoyl) = 1650 cm⁻¹; νC = 0 (pyrone) = 1620 cm⁻¹; NMR (CF₃COOD) δ in ppm relative to TMS: 2 H at 4.5 (s), 13 H from 7.5 to 8.7 (m).

| Elemental Analysis | | | |
|---|---|---|---|
| | C% | H% | O% |
| calculated: | 74.99 | 4.20 | 20.81 |
| found: | 75.11 | 4.09 | |

### 2-(4-UNDECYLPHENYL)-4-OXO-4H-[1]-BENZOPYRAN-8-ACETIC ACID

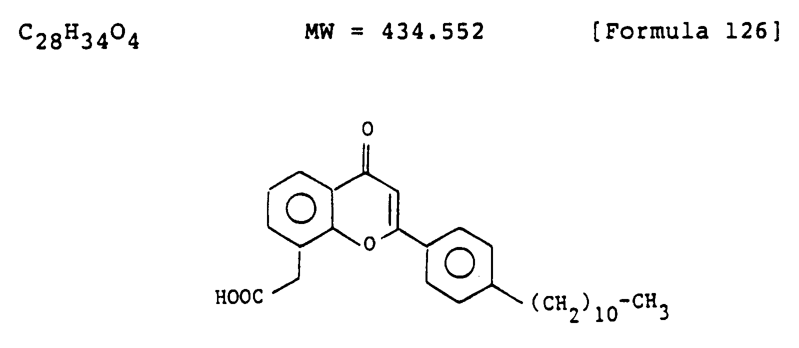

PF_{G} = 150-152°C; IR νC = 0 (acid) = 1710 cm⁻¹; νC = 0 (pyrone) = 1620 cm⁻¹; NMR (CF₃COOD) δ in ppm relative to TMS: 23 H from 0.6 to 1.7 (m), 2 H at 4.5 (s), 8 H from 7.5 to 8.4 (m).

| Elemental Analysis | | | |
|---|---|---|---|
| | C% | H% | O% |
| calculated: | 77.39 | 7.89 | 14.73 |
| found: | 77.34 | 7.87 | |

### NITRO-3, PHENYL-4-PHENYL)-2-OXO-4-4H-[1]BENZOPYRAN-8-ACETIC ACID

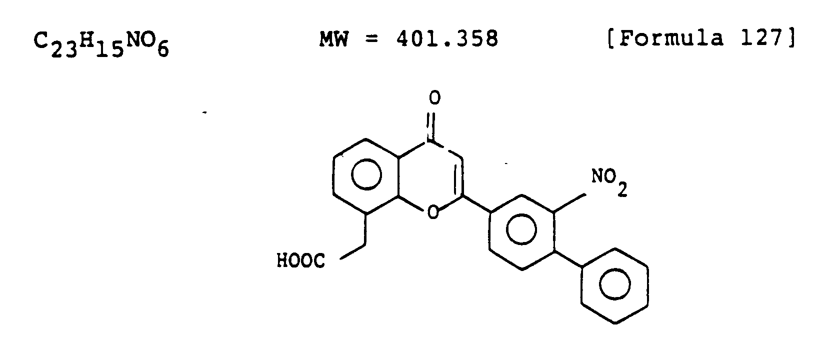

PF_{G} = 270-272°C; IR νC = 0 (acid) = 1720 cm⁻¹; νC = 0 (pyrone) = 1620 cm⁻¹; NMR (DMSO) δ in ppm relative to TMS: 2 H at 4 (s), 12 H from 7.2 to 8.7 (m), 1 H at 12.9 (exchangeable).

| Elemental Analysis | | | | |
|---|---|---|---|---|
| | C% | H% | N% | O% |
| calculated: | 68.88 | 3.77 | 3.49 | 23.92 |
| found: | 68.72 | 3.66 | 3.35 | |

### 2-(4-TRIFLUOROMETHYLPHENYL)-4-OXO-4H-[1]-BENZOPYRAN-8-ACETIC ACID

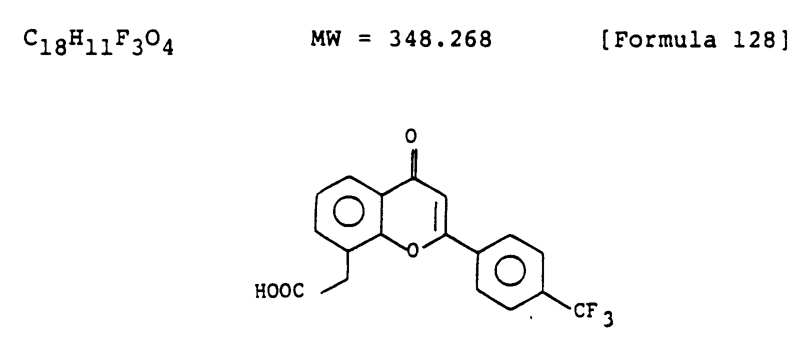

PF_{G} = 216-218°C; IR νC = 0 (acid) = 1720 cm⁻¹; νC = 0 pyrone) = 1640 cm⁻¹; NMR (DMSO) δ in ppm relative to TMS: 2 H at 4 (s), 8 H from 7.1 to 8.4 (m), 1 H at 12.8 (exchangeable).

| Elemental Analysis | | | | |
|---|---|---|---|---|
| | C% | H% | F% | O% |
| calculated: | 63.07 | 3.18 | 16.37 | 18.38 |
| found: | 63.02 | 3.32 | 16.37 | |

### 2-(4-DIMETHYLTRIAZENYLPHENYL)-4-OXO-4H-[1]-BENZOPYRAN-8-ACETIC ACID

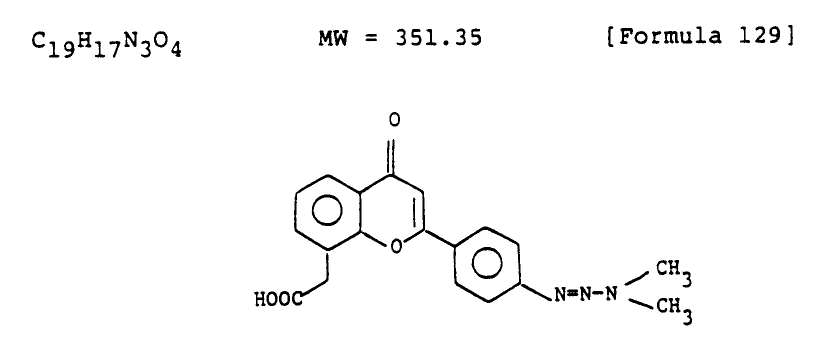

PF_{G} = 209-211°C; IR νV = 0 (acid) = 1720 cm⁻¹; νC = 0 (pyrone) = 1620 cm⁻¹; NMR (DMSO) δ in ppm relative to TMS: 6 H at 3.3 (m), 2 H at 4 (s), 1 H at 7 (s), 8 H from 7.2 to 8.1 (m), 1 H at 12.8 (exchangeable).

| Elemental Analysis | | | | |
|---|---|---|---|---|
| | C% | H% | N% | O% |
| calculated: | 64.95 | 4.88 | 11.96 | 18.22 |
| found: | 64.75 | 4.95 | 12.25 | |

### 2-(3-NITRO-4-METHOXYPHENYL)-4-OXO-4H-[1]-BENZOPYRAN-8-ACETIC ACID

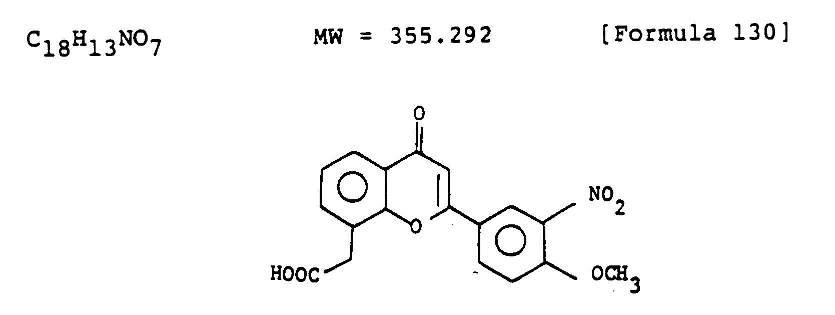

PF_{G} = 254-256°C; IR νC = 0 (acid) = 1720 cm⁻¹; νC = 0 (pyrone) = 1620 cm⁻¹; NMR (CF₃COOD): 3 H at 4.1 (s), 2 H at 4.3 (s), 7 H from 7.1 to 9 (m).

| Elemental Analysis | | | | |
|---|---|---|---|---|
| | C% | H% | N% | O% |
| calculated: | 60.85 | 3.69 | 3.94 | 31.52 |
| found: | 61.07 | 3.68 | 4.16 | |

### 2-(4-TERBUTYLPHENYL)-4-OXO-4H-[1]-BENZOPYRAN-8-ACETIC ACID

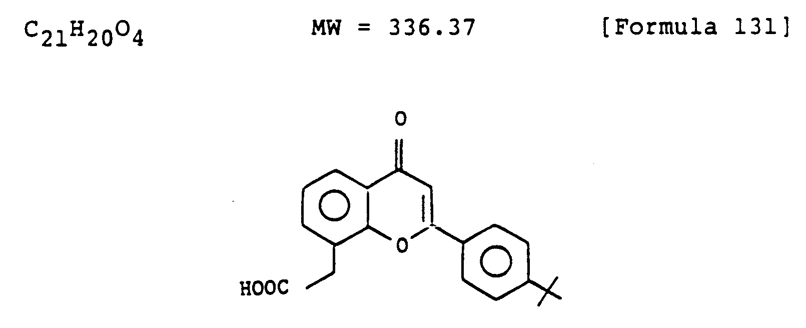

PF_{G} = 240-242°C; IR νC = 0 (acid) = 1720 cm⁻¹; νC = 0 (pyrone) = 1610 cm⁻¹; NMR (DMSO) δ in ppm relative to TMS: 6 H at 1.2 (s), 2 H at 4.5 (s), 8 H from 7.1 to 8.3 (m), 1 H at 12.9 (exchangeable).

| Elemental Analysis | | | |
|---|---|---|---|
| | C% | H% | O% |
| calculated: | 74.98 | 5.99 | 19.03 |
| found: | 74.76 | 5.85 | |

## Claims

1. A compound of the formula (I): 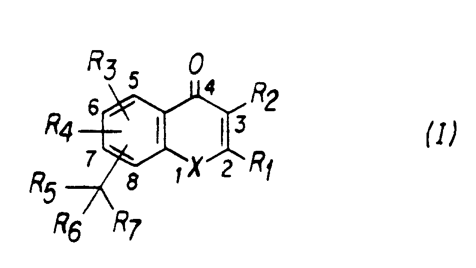 wherein:
X is N, O, Se, or S(O)ₙ, wherein n is 0, 1 or 2;
R₁ is phenyl; phenyl substituted by at least one member selected from the group consisting of halogens, C₁₋₁₂ alkyl, trifluoromethyl, hydroxyl, C₁₋₆ alkoxy, (̵C₁₋₆-alkylene)̵COOR₁₀, nitro, C₁₋₆(̵alkyl)̵carboylamino, benzoyl, C₁₋₆(̵alkyl)carboyl, CONR₁₀R₁₁, (where R₁₀ and R₁₁ are each independently H or C₁₋₆ alkyl), NR₁₀R₁₁, -N=N-NR₁₀R₁₁, phenyl, -O(̵C₁₋₆ alkylene)̵NR₁₀R₁₁, thiazolyl, and thiazolyl substituted by C₁₋₆ alkyl or amino; or R₁ is pyridyl; pyridyl substituted by at least one member selected from the group consisting of C₁₋₆ alkyls and halogens; trifluoromethyl; benzoyl or benzyl;
R₂ is H; phenyl; OH; C₁₋₃ alkyl; or C₁₋₃ alkoxy; or
R₁ and R₂ together form a naphthalene ring fused to to the flavonoid nucleus;
R₃ is H; OH; or halogen;
R₄ is H;
R₅ is H or C₁₋₃ alkyl;
R₆ is H; OH; or -O-CO(̵C₁₋₆ alkyl);
or R₅ and R₆ together are a group =CR₁₀R₁₁, or a group =NOH, or a group =O or a group =CHR₁₂ (where R₁₂ is phenyl, pyridyl, phenyl substituted by at least one member selected from the group consisting of halogen atoms, trifluoromethyl and C₁₋₃ alkyls or pyrridyl substituted by at least one member selected from the group consisting of halogen atoms, trifluoromethyl and C₁₋₃ alkyls);
R₇ is H; COOR₁₀; -P(O)(OR₁₀R₁₁)₂; NR₁₃R₁₄ (where R₁₃ and R₁₄ are independently H; phenyl; phenyl substituted by a halogen atom or a C₁₋₃ alkyl group or a group -COOR₁₀, -CO-O-CH(CH₃)-COOR₁₀, morpholinyl, -C(CH₂OH)₂(CH₃), imidazolinyl, (̵C₁₋₆ alkylene)̵OH, (̵C₁₋₆ alkylene)̵COOR₁₀, or C₁₋₃ alkoxy, or wherein R₁₃ and R₁₄ together with the nitrogen atom to which they are both bound from an imidazole or a N(̵C₁₋₃ alkyl)̵piperazinyl); or
R₇ is -CO(̵C₁₋₆ alkyl); -S-(C₁₋₆ alkyl); -SH; -S-CO(̵C₁₋₃ alkyl); 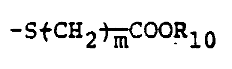 (with 0 < m ≦ 6); -O(̵C₁₋₆ alkylene)̵NR₁₀R₁₁; -NR₁₀NR₁₀R₁₁; C₁₋₆ alkyl; thiazolyl; thiazolyl substituted by at least one member selected from the group consisting of -NH₂, C₁₋₃ alkyl, phenyl, and COOR₁₀; -NR-CO(̵C₁₋₃-alkyl); or (̵C₁₋₃-alkylene)̵CH(NH₂(COOH); or
-CR₅R₆R₇ is a group of the formula 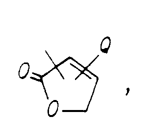 wherein Q is at least one member selected from the group consisting of H; COOR₁₀; phenyl; -O(̵C₁₋₃-alkylene)̵COOR₁₀; C₁₋₃ alkyl; -O-CS-NR₁₀R₁₁; -O(̵C₁₋₃-alkylene)̵NR₁₀R₁₁; OH; C₁₋₃ alkoxy; and NR₁₀R₁₁; or
wherein any two of R₃, R₄, R₅, R₆ and R₇ together form a benzene ring; or a benzene ring substituted by (̵C₁₋₃-alkylene)̵COOR₁₀; (̵C₁₋₃-alkyl)̵OH, COOR₁₀, or (̵C₁₋₃-alkylene)̵O-CO(̵C₁₋₃-alkyl); or a naphthalene system; or a naphthalene system substituted by (̵C₁₋₃-alkylene)̵COOR₁₀, (̵C₁₋₃-alky)̵OH, COOR₁₀, or (̵C₁₋₃-alkylene)̵O-CO(̵C₁₋₃-alkyl); and
physiologically acceptable salts thereof, with the proviso that
when R₂, R₃, R₄, R₅ and R₆ are all H and R₇ is COOR₁₀, R₁ is other than phenyl, 2-thenyl, 3-methoxy phenyl, 3,4-dimethoxy phenyl, 2-furyl, para-tolyl, 2-naphthyl, 4-methoxy phenyl, cyclohexyl, benzyl, or methyl.

2. The compound of claim 1, wherein:
R₁ is phenyl substituted by C₁₋₃ alkyl, halogen, trifluoromethyl, hydroxy, C₁₋₃ alkoxy or nitro.

3. The compound of Claim 1, wherein R₁ is phenyl substituted by one halogen atom.

4. The compound of Claim 1, wherein R₇ is -COOR₁₀, -P(o)(OR₁₀R₁₁), -CH₂CH₂COOR₁₀ or -CONR₁₀R₁₁.

5. The compound of Claim 1, wherein said compound has the formula (IV): 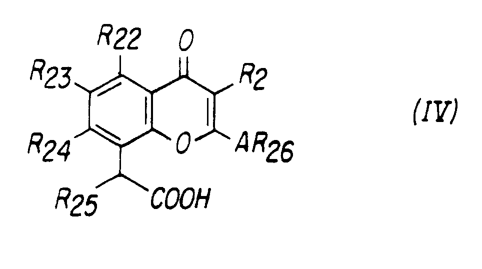 wherein:
AR₂₆ is phenyl, substituted phenyl or biphenyl;
R₂ is hydrogen, hydroxyl, or C₁₋₃ alkoxy;
R₂₂ is hydrogen or hydroxyl;
R₂₃ is hydrogen or fluoro;
R₂₄ is hydrogen or hydroxy;
R₂₅ is hydrogen, 2-methylpyridyl, benzylidene, 4-methylenepyridyl or methylene; or
R₂₂ and R₂₃ together form a benzene ring fused to the flavonoid nucleus; or
R₂₃ and R₂₄ together form a benzene ring fused to the flavonoid nucleus; or
R₂₅ and R₂₄ together form a benzene ring fused to the flavonoid nucleus.

6. A pharmaceutical composition, comprising a pharmaceutically acceptable carrier and a compound of the formula (I) of claim 1.

7. A pharmaceutical composition comprising a compound of Claim 2, 3 or 4 and a pharmaceutically acceptable carrier.

8. A pharmaceutical composition comprising a compound of Claim 5, and a pharmaceutically acceptable carrier.

9. The compound of Claim 1, wherein said compound is

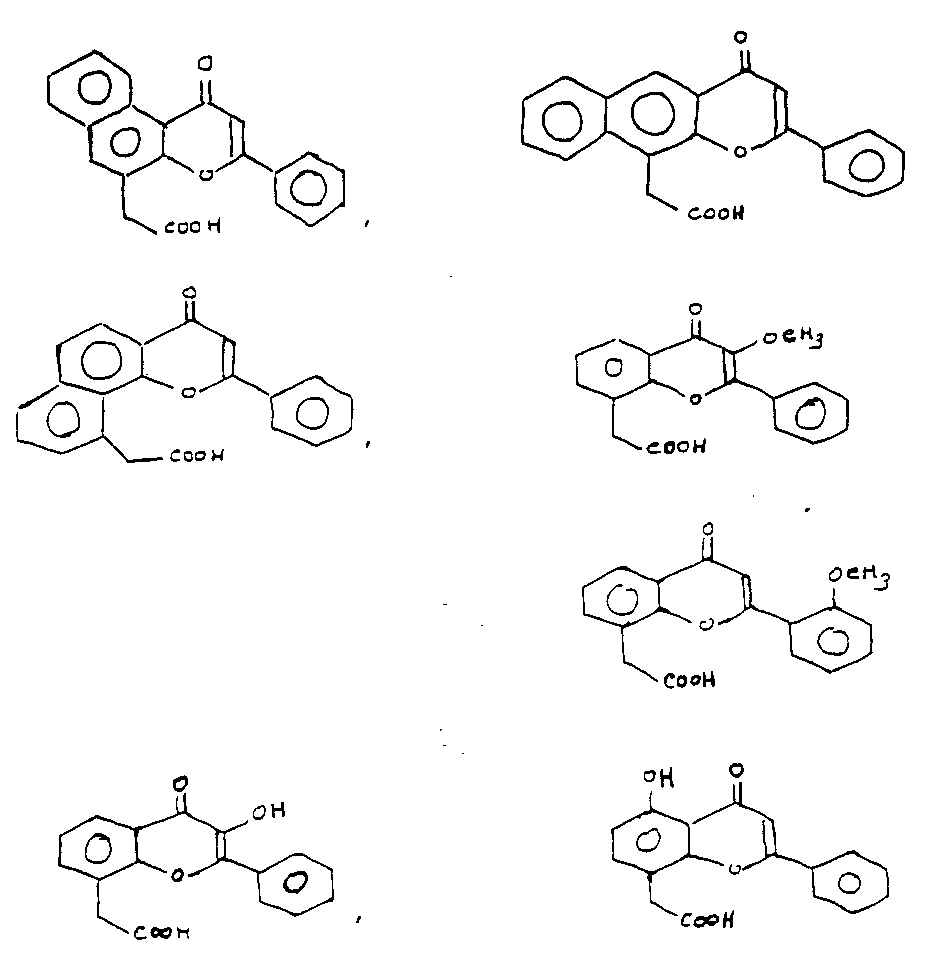

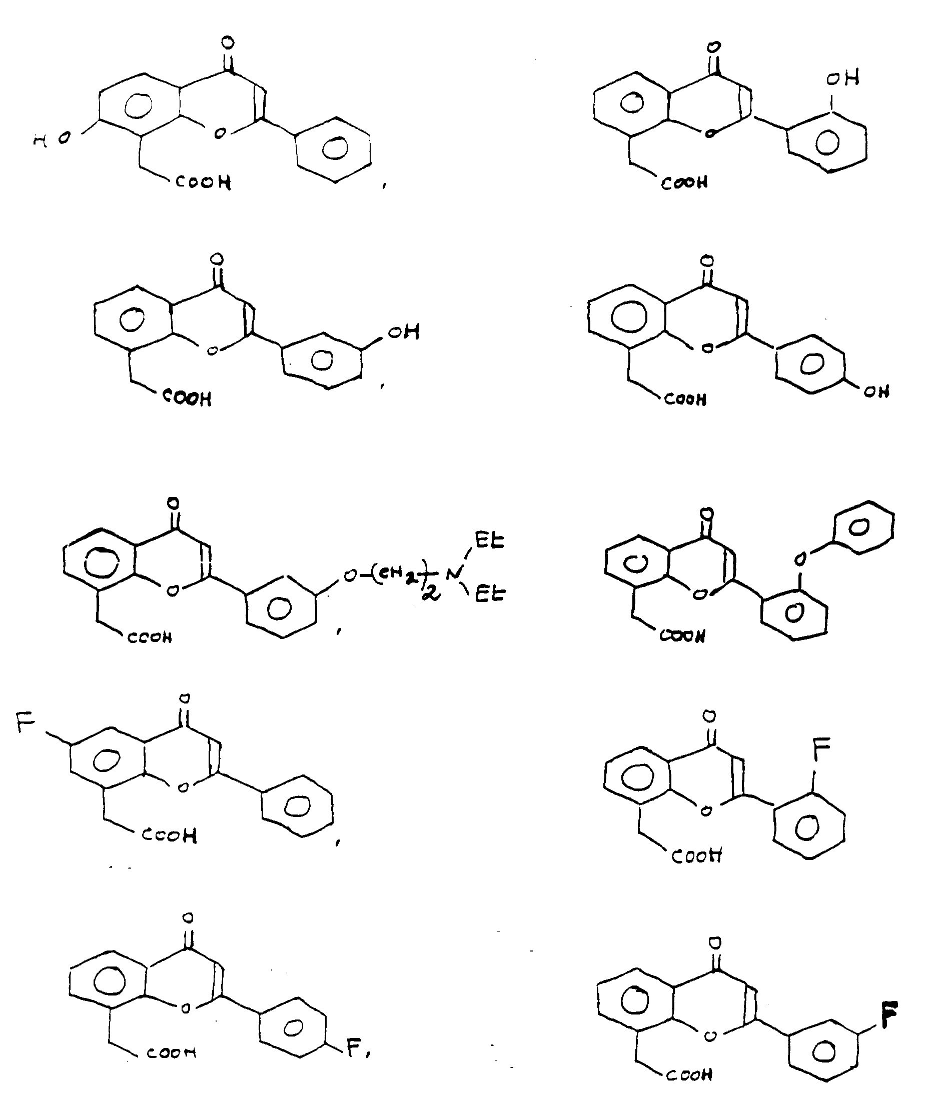

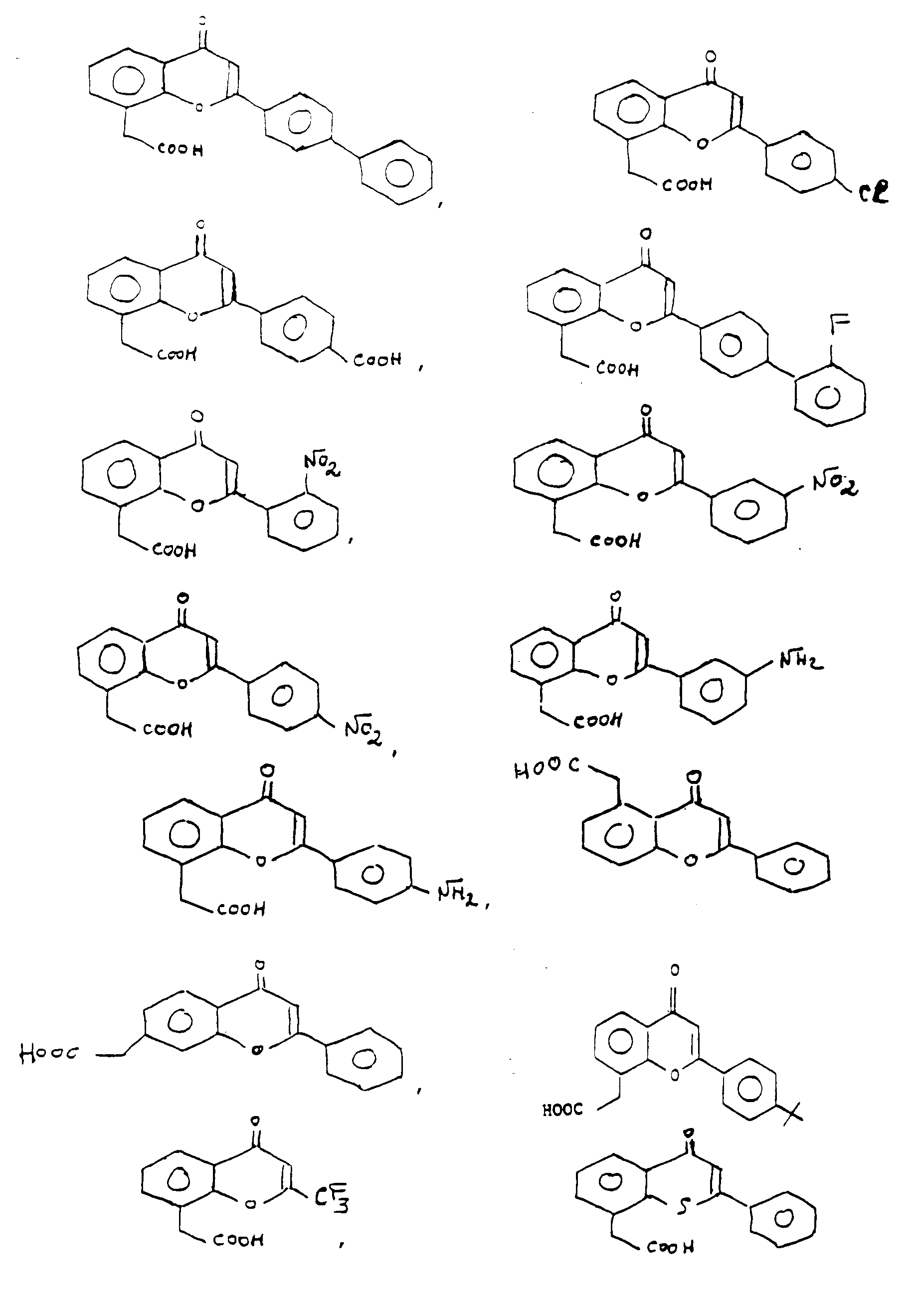

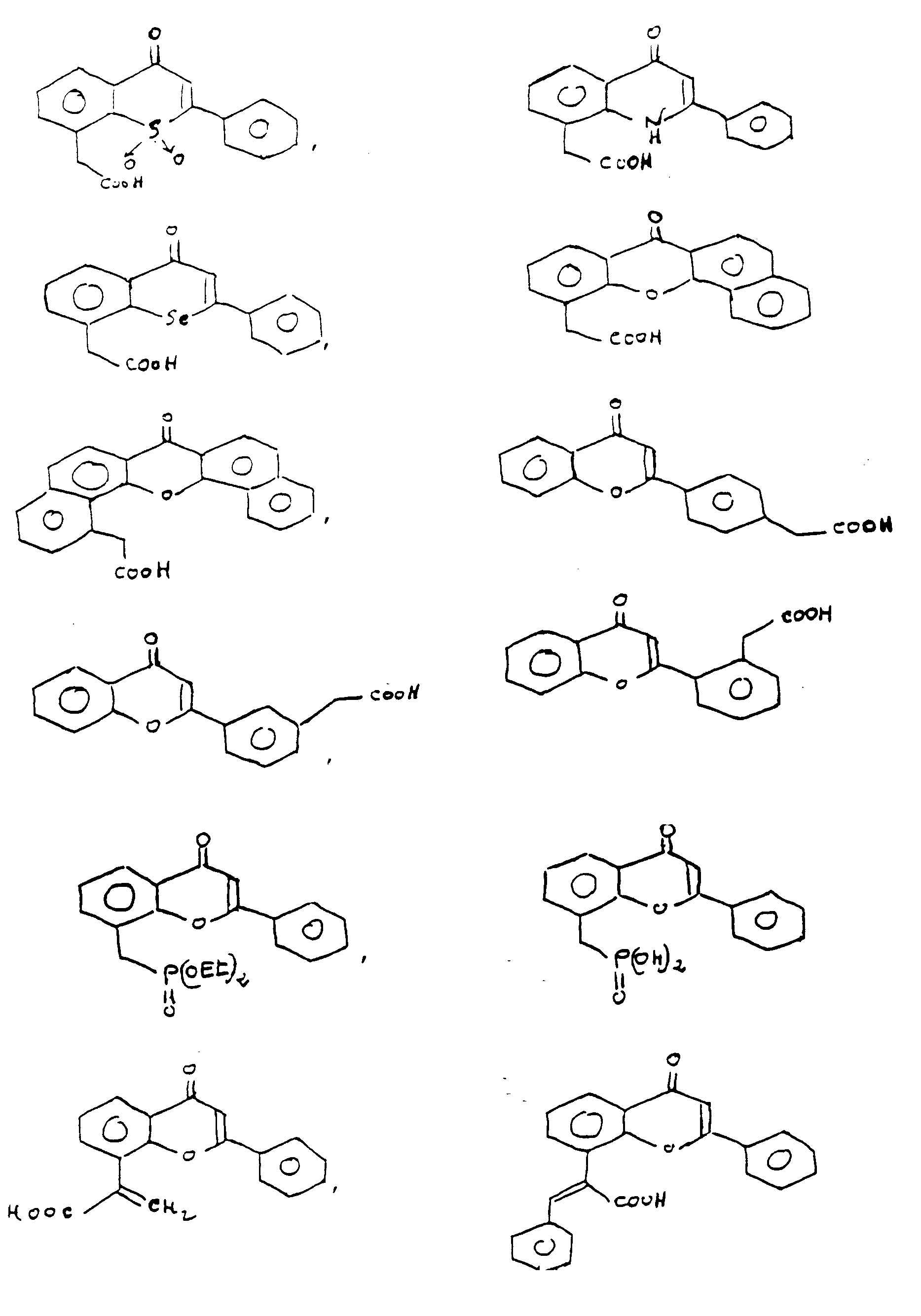

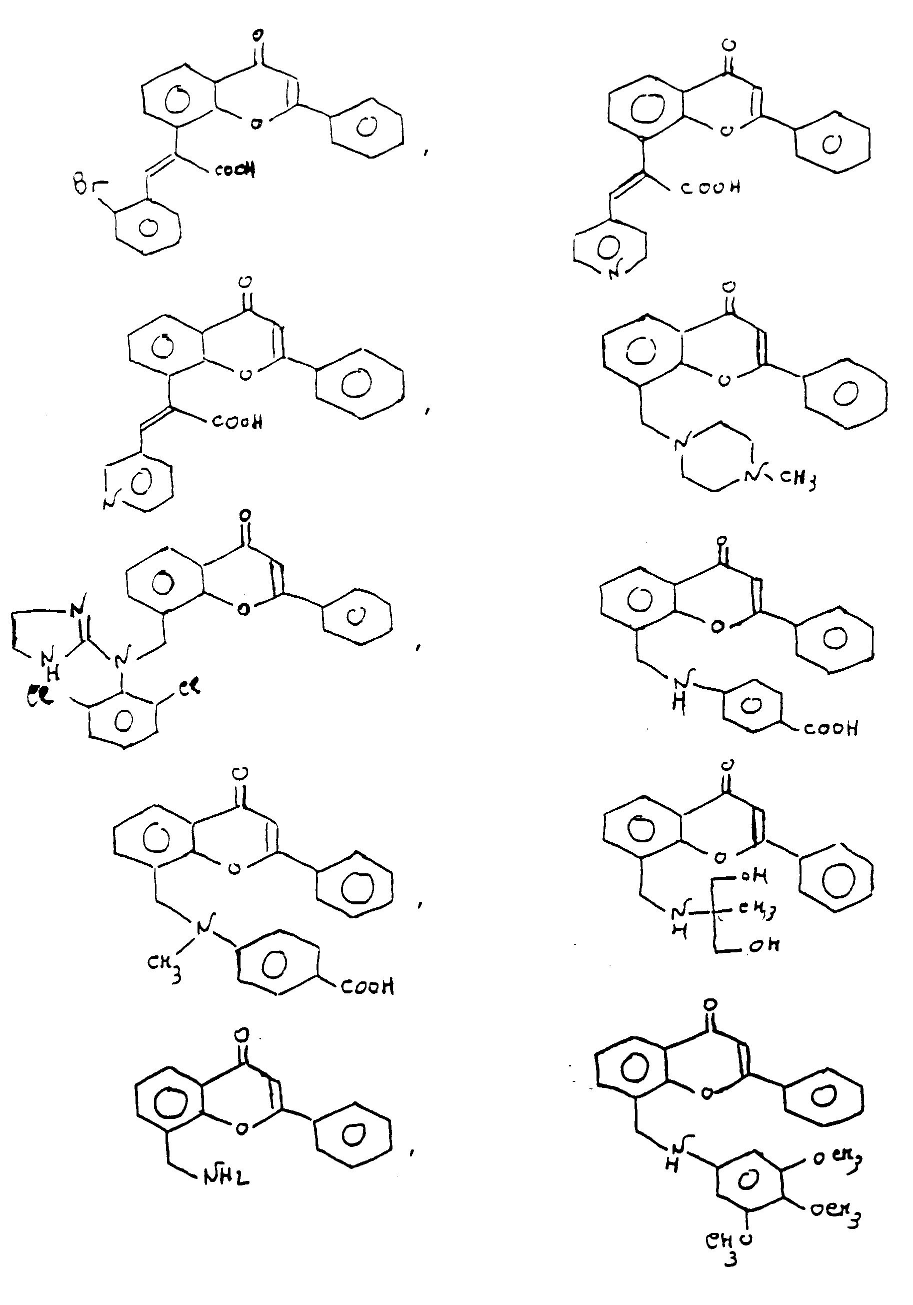

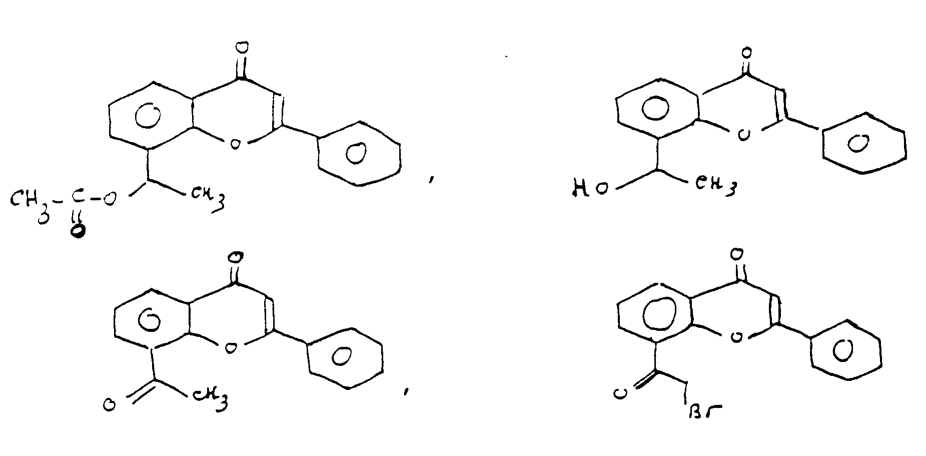

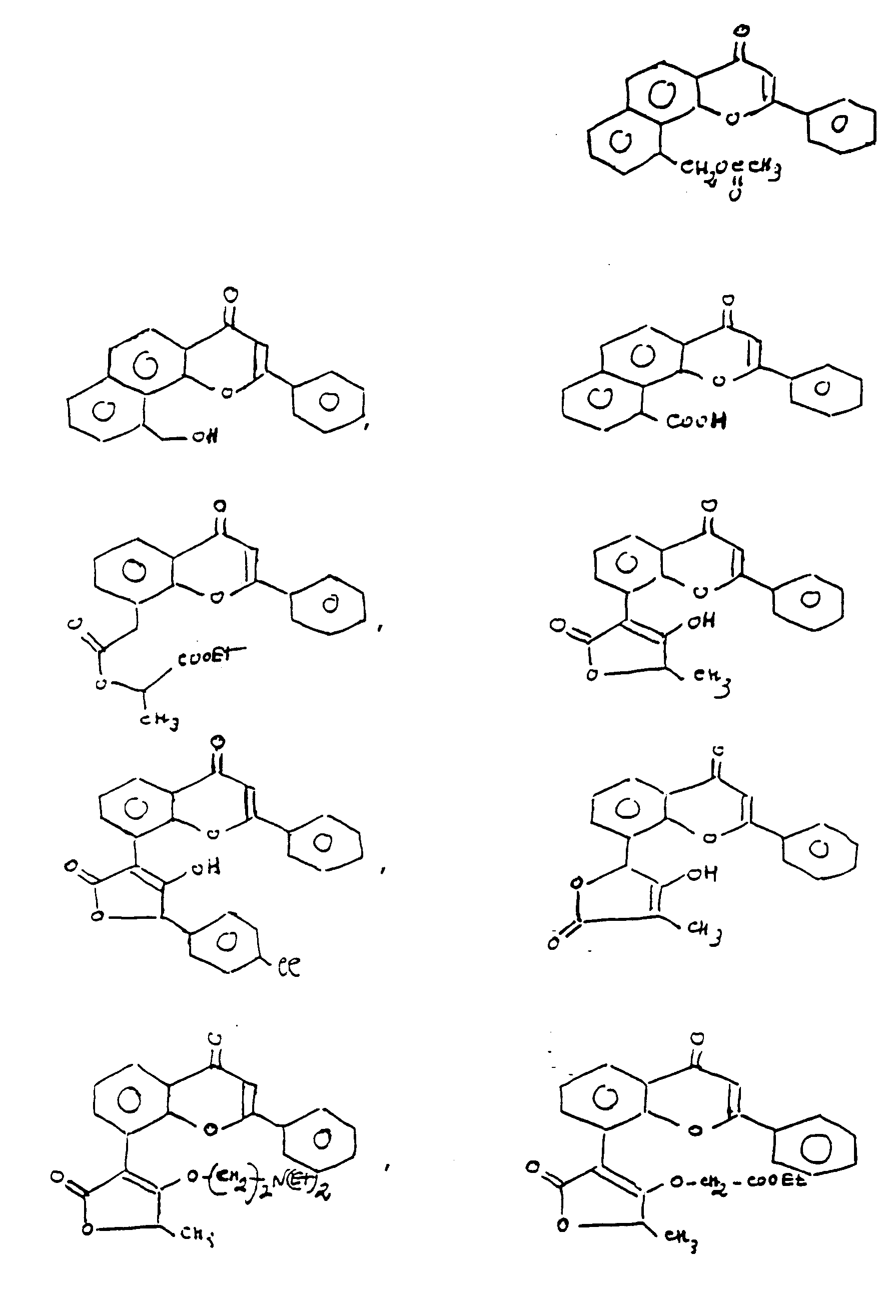

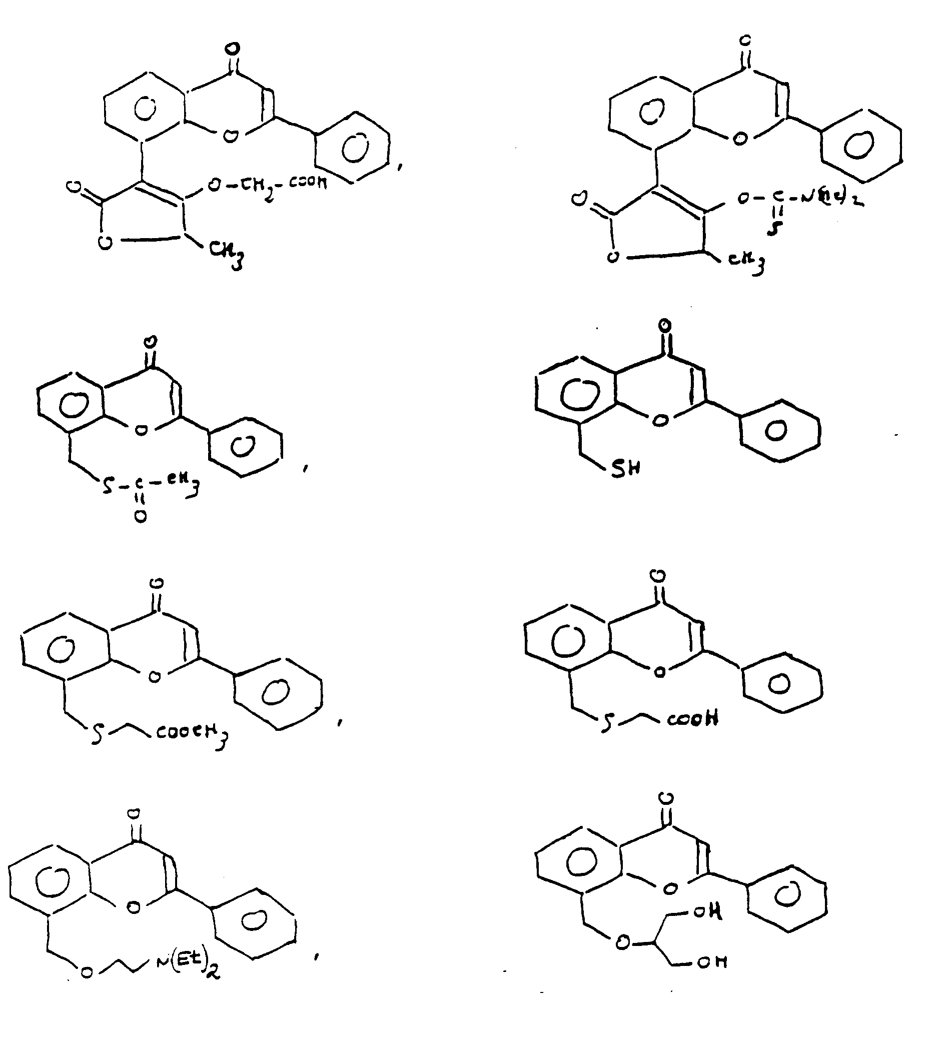

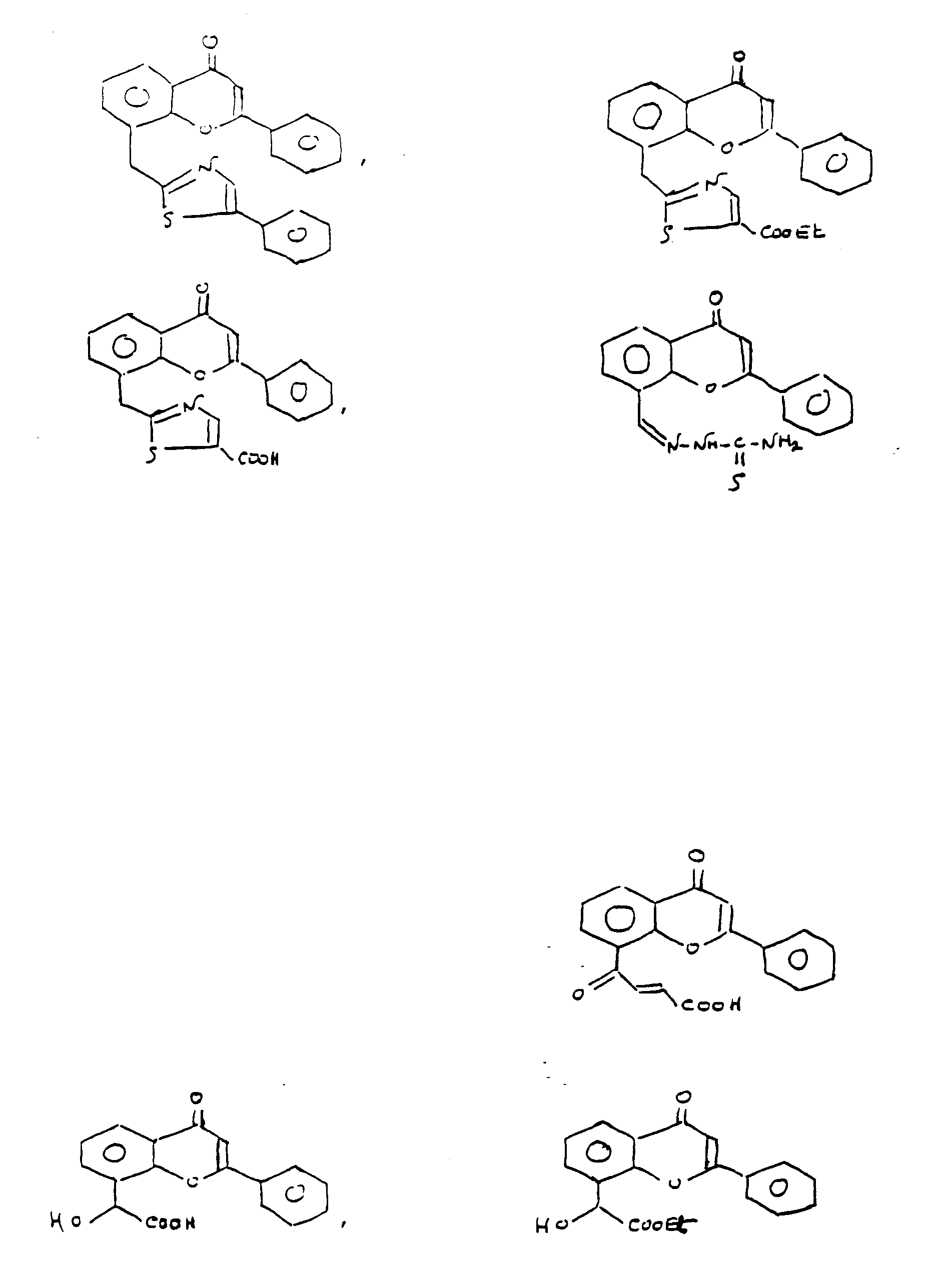

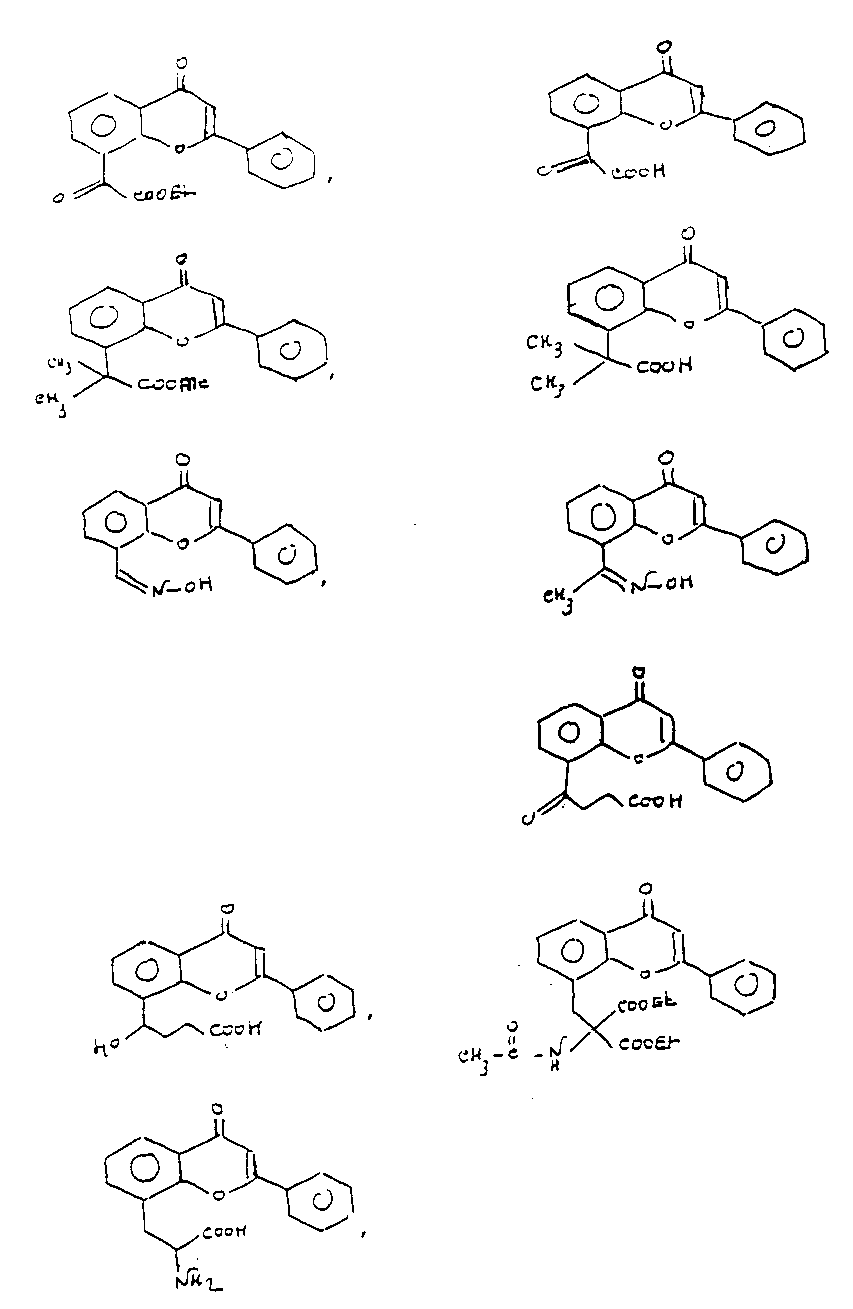

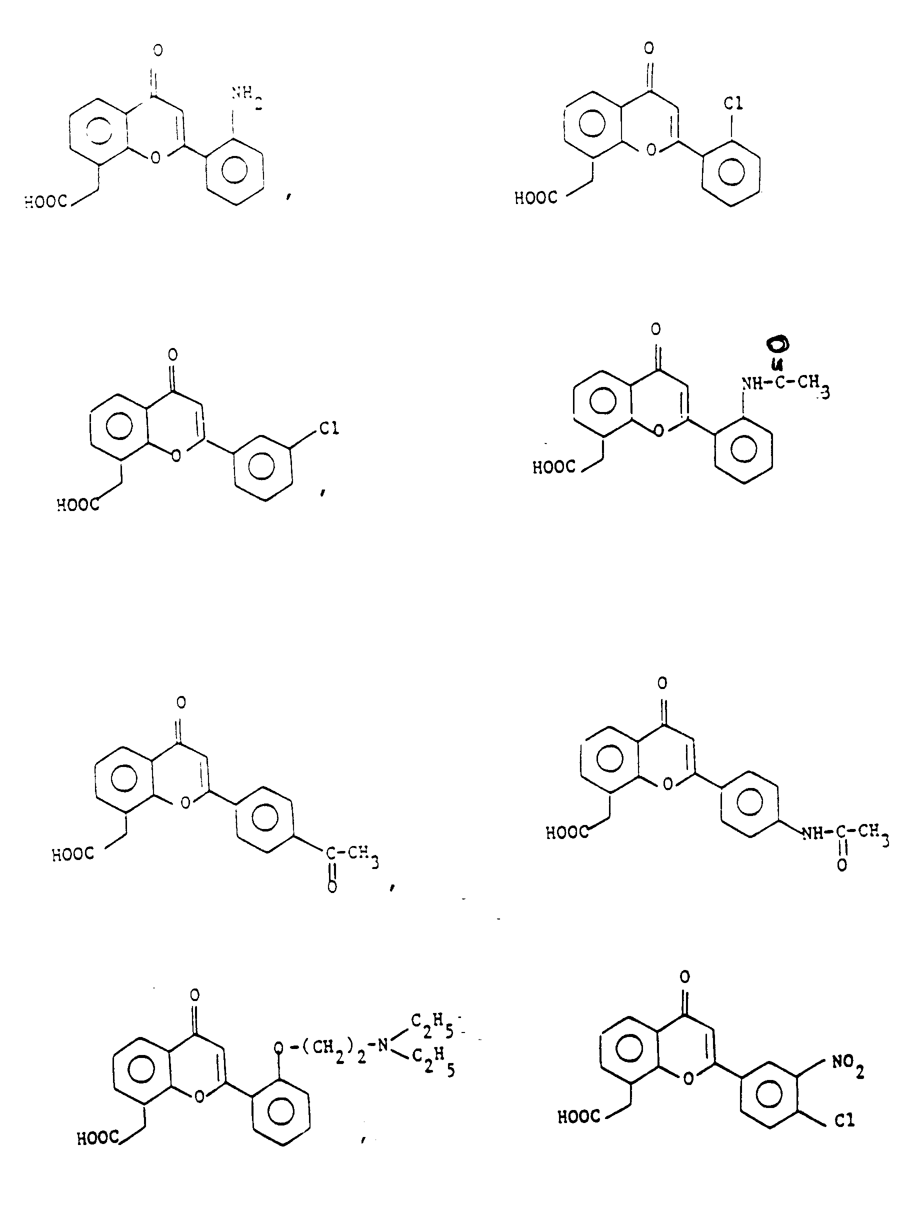

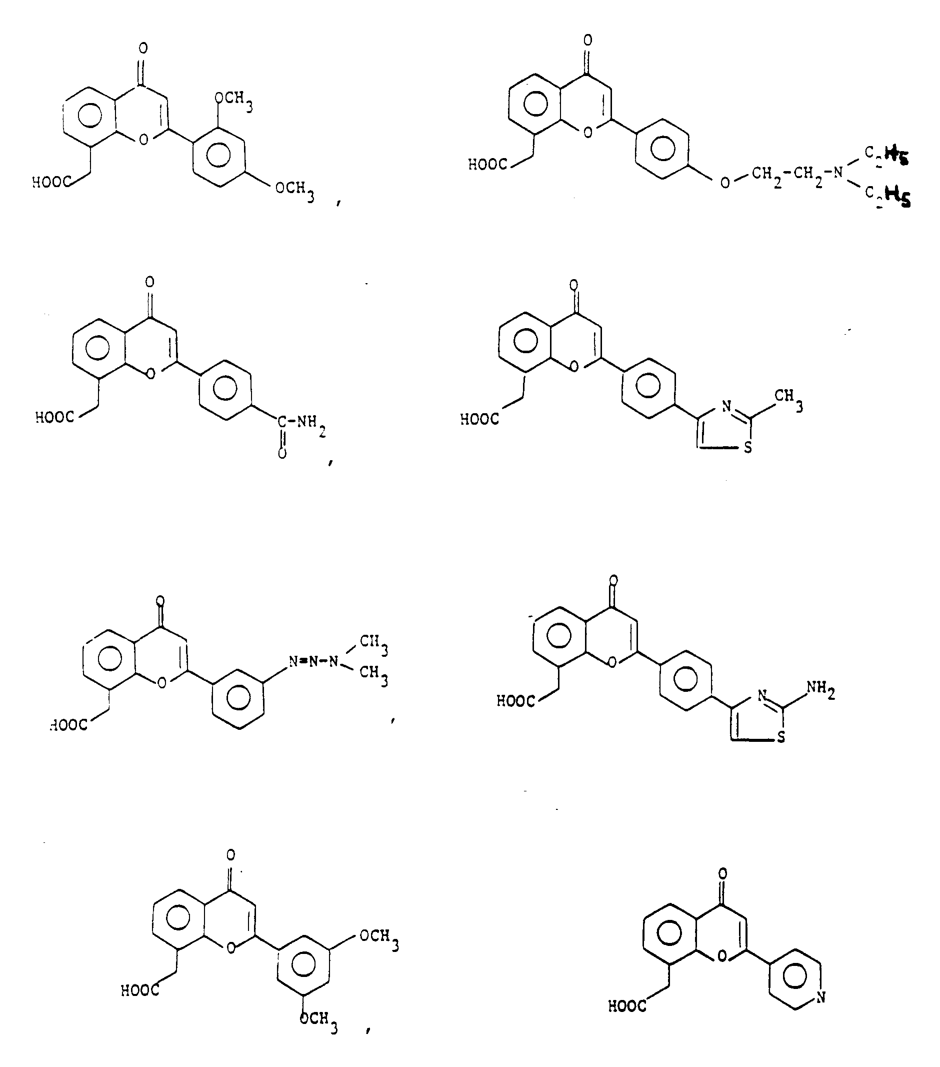

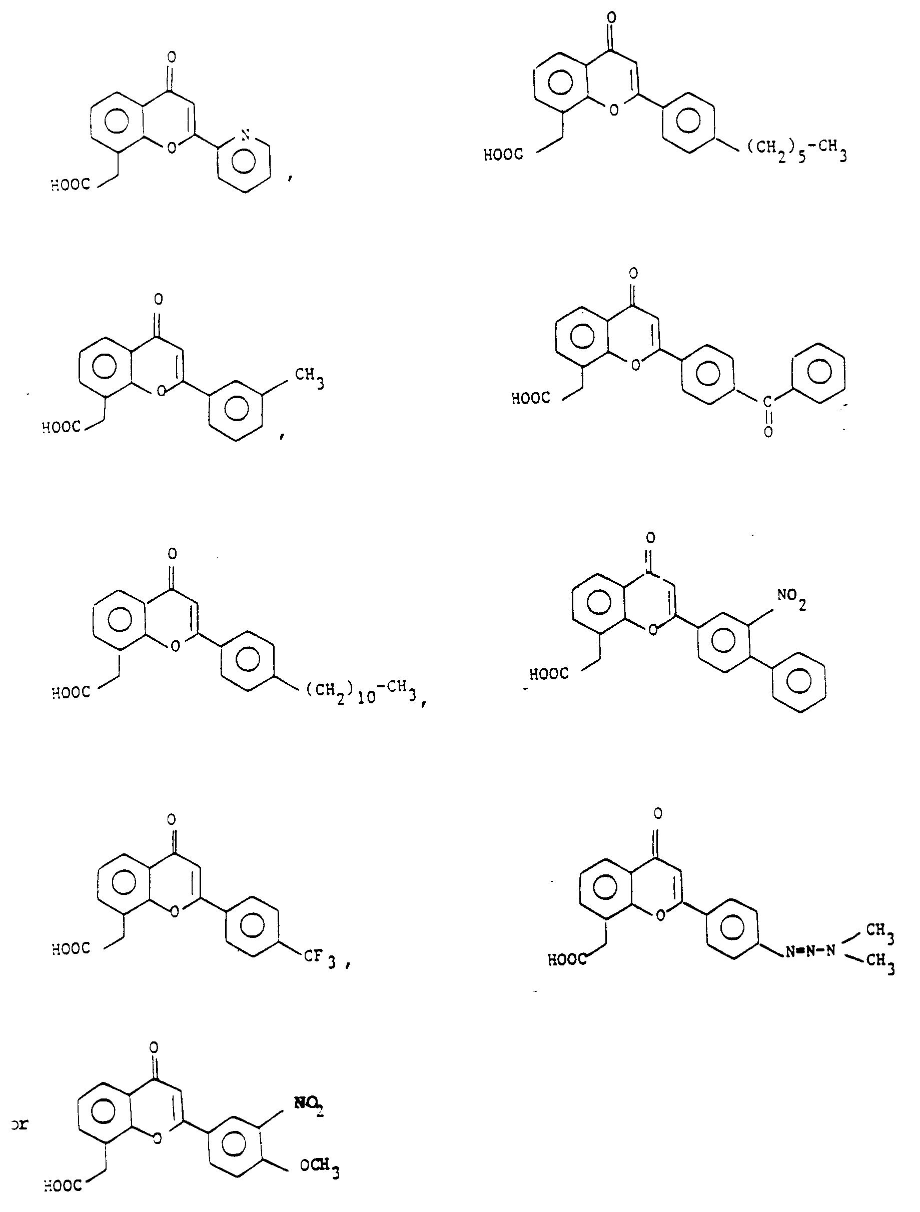

10. The compound of Claim 1, wherein said compound is

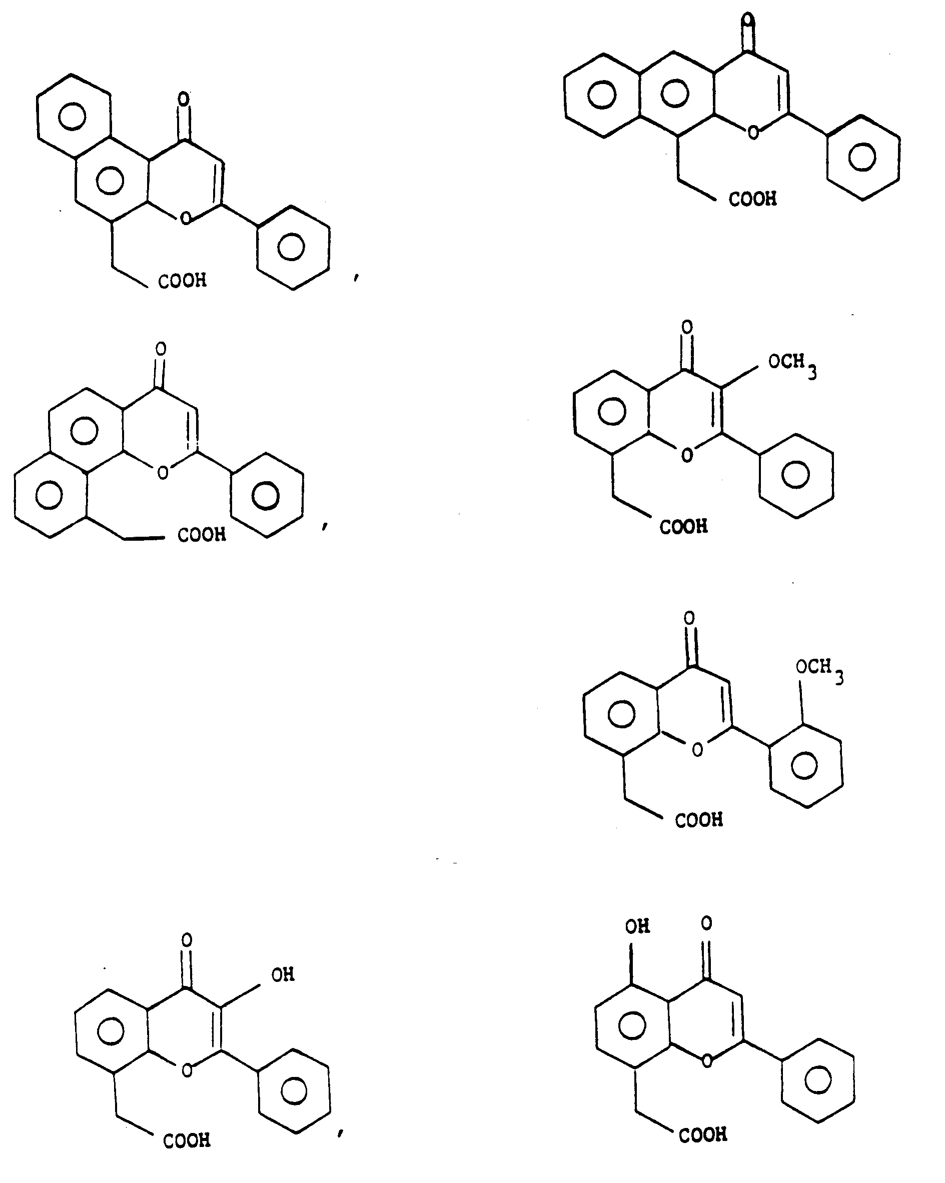

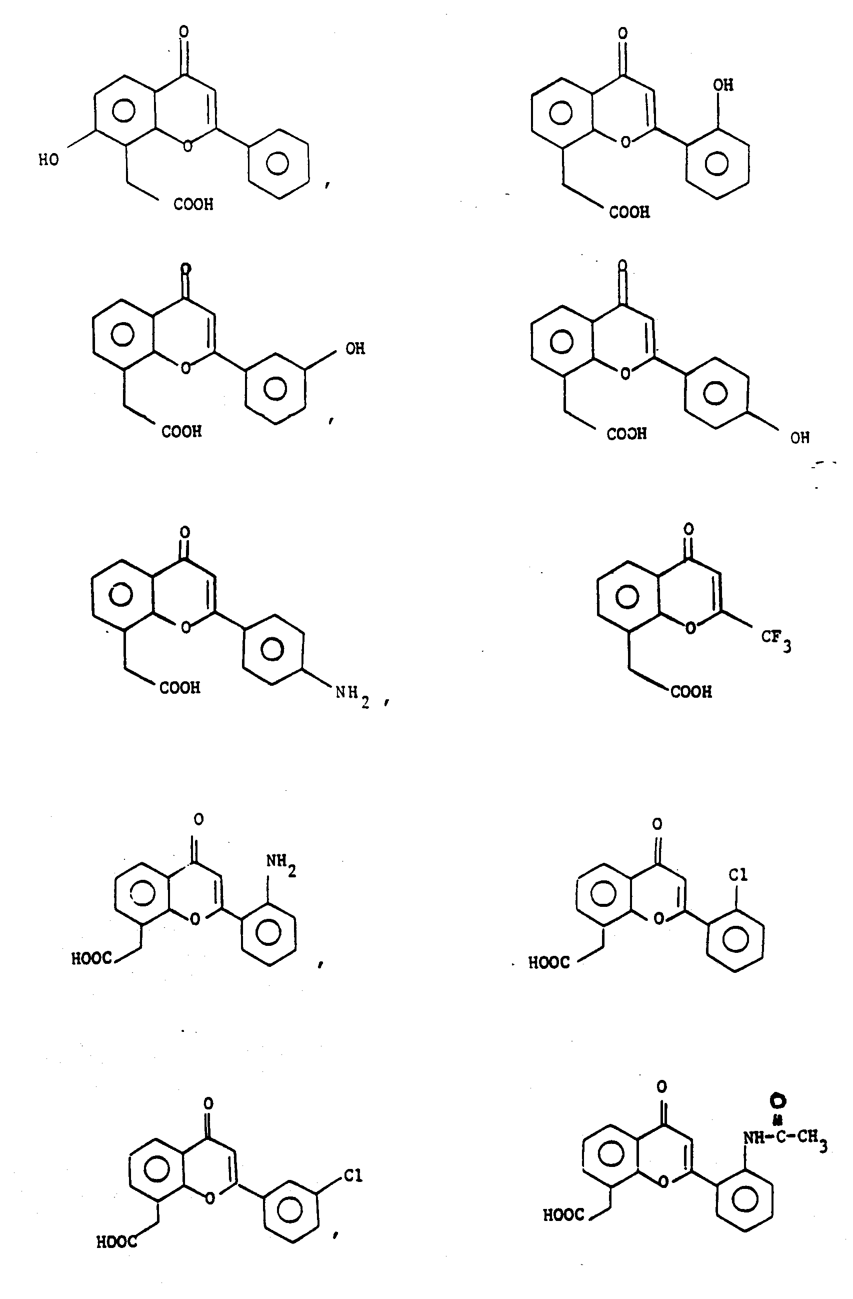

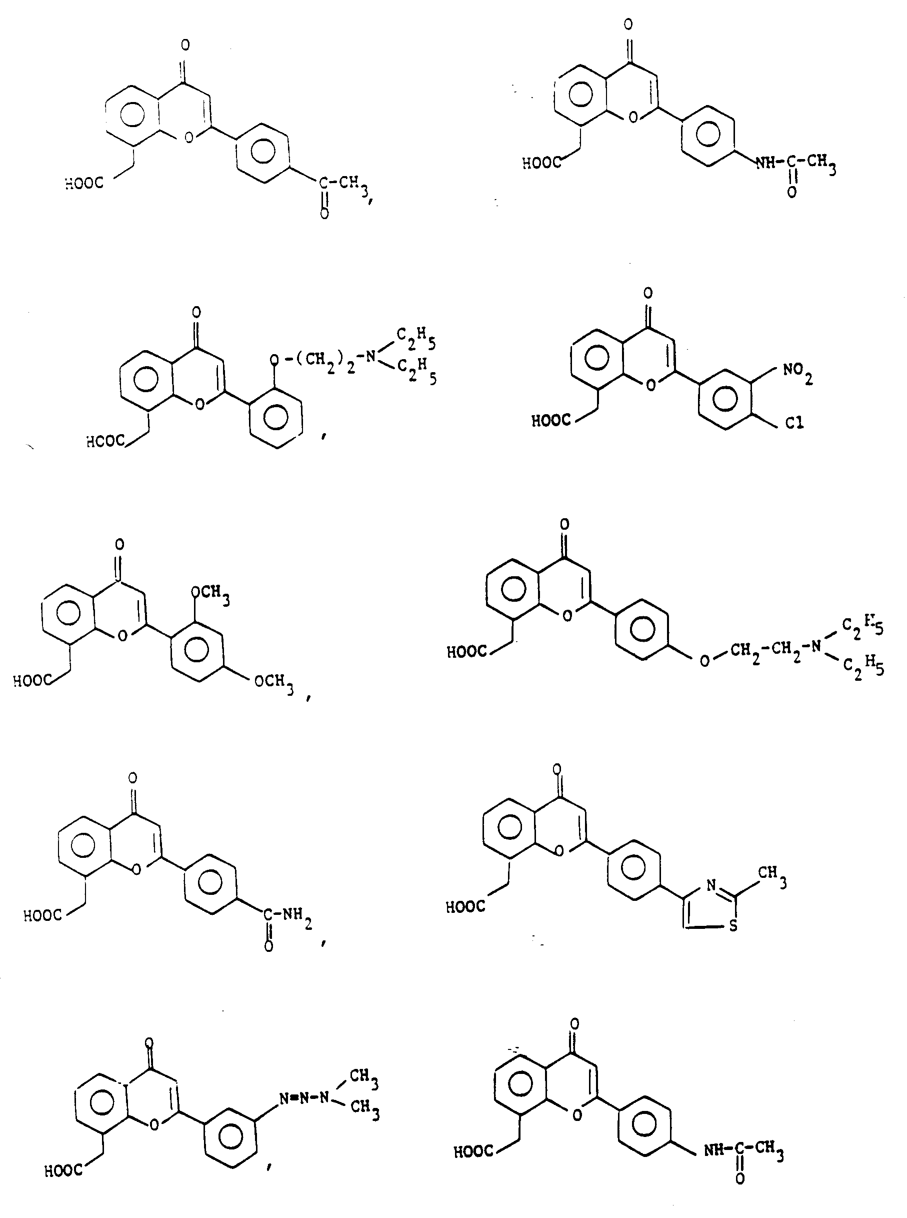

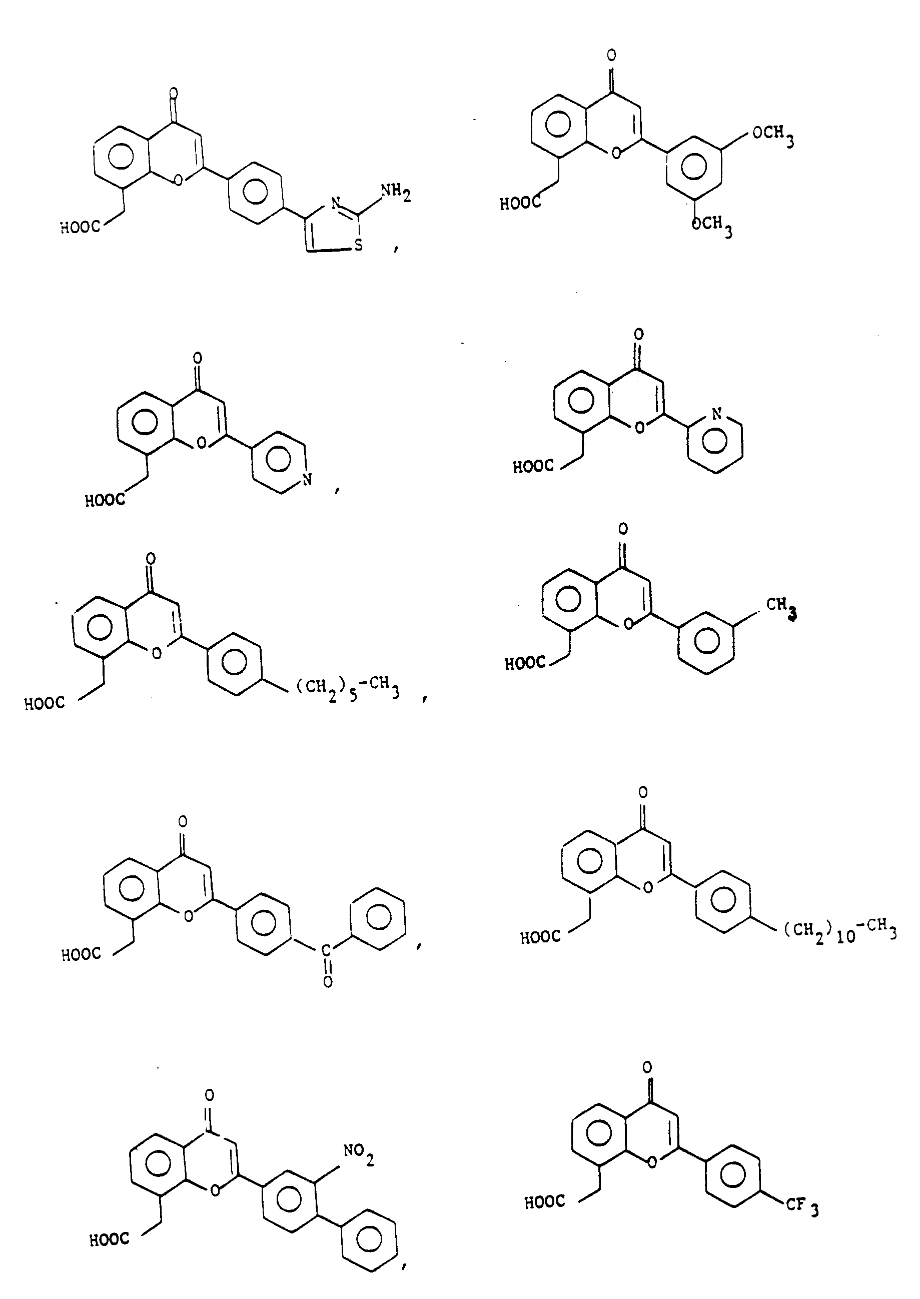

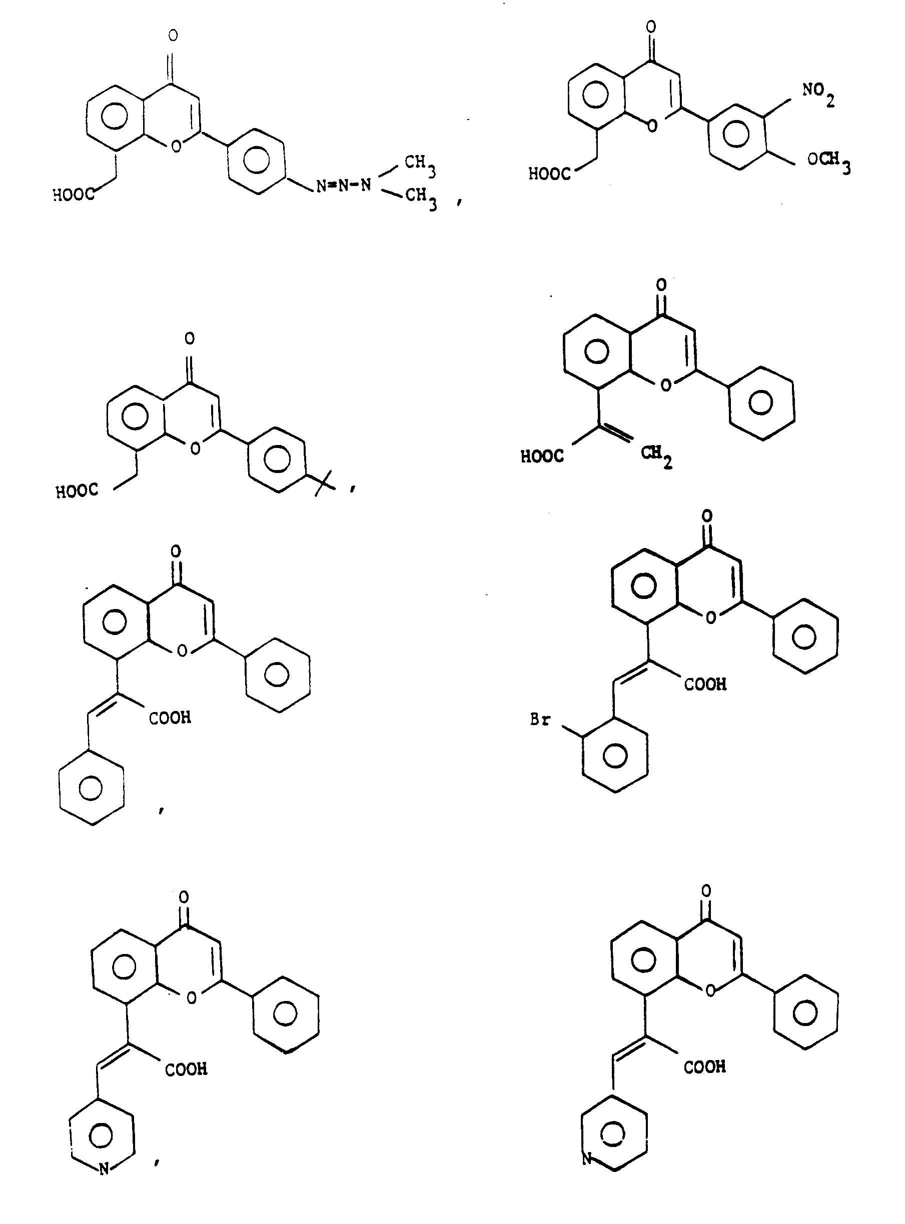

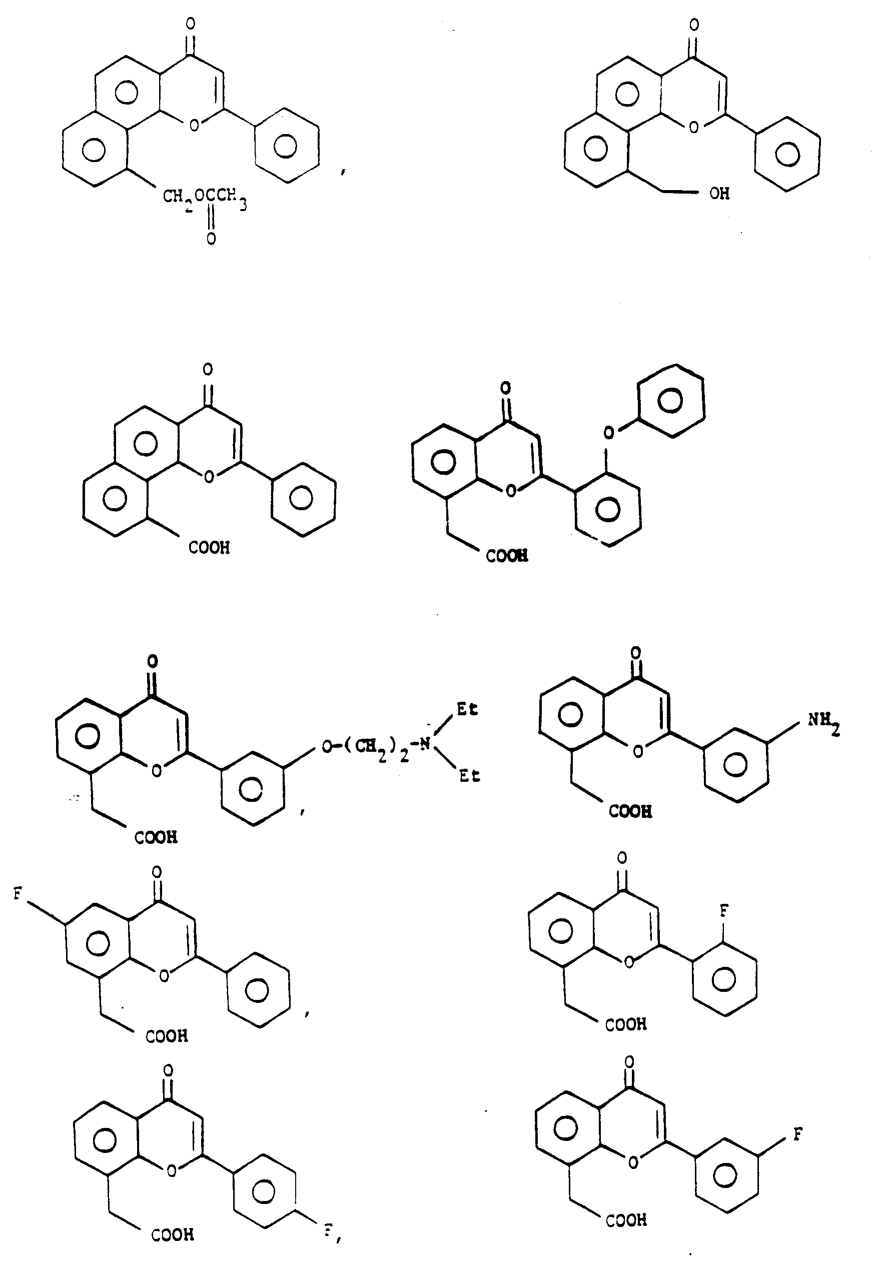

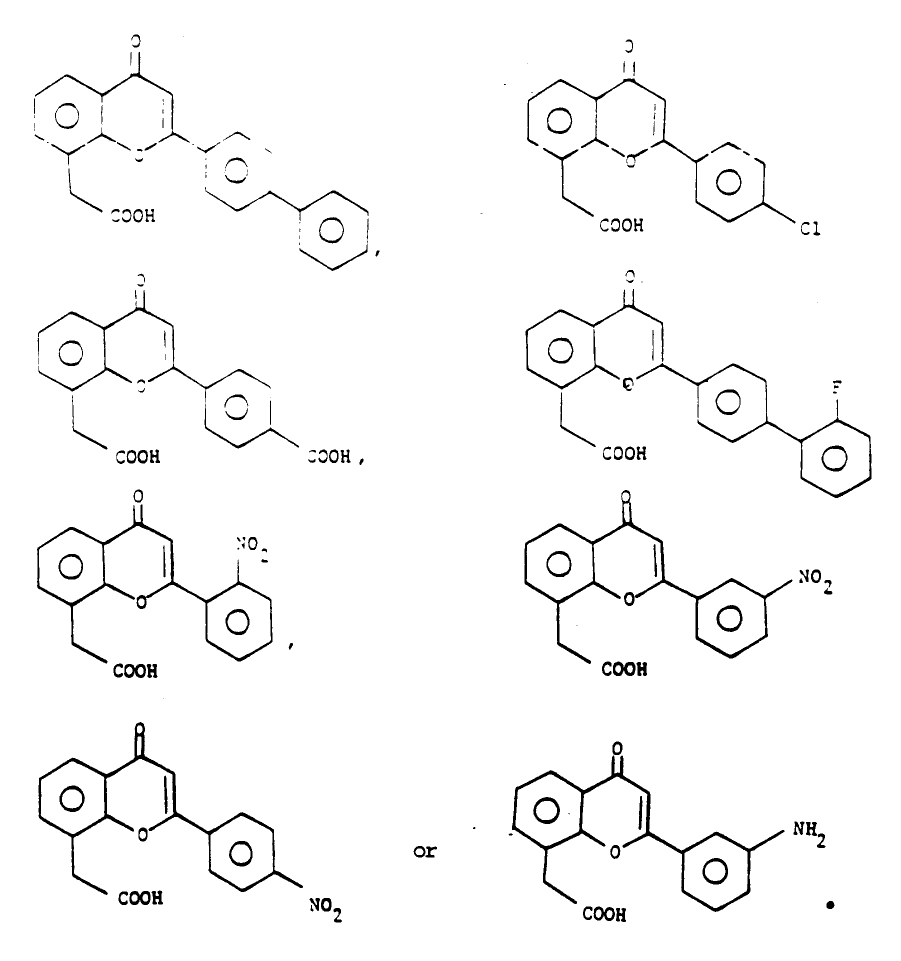

## Patentansprüche

1. Verbindung der Formel (I) 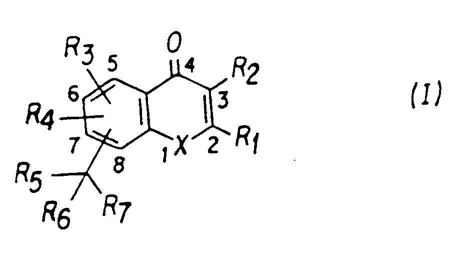 in der:
X für N, O, Se oder S(O)ₙ steht, wobei n 0, 1 oder 2 ist;
R₁ für Phenyl oder Phenyl, das durch mindestens einen Substituenten aus der Gruppe der Halogene, C₁₋₁₂-Alkyl, Trifluormethyl, Hydroxy, C₁₋₆-Alkoxy, (̵C₁₋₆-Alkylen)̵ COOR₁₀, Nitro, C₁₋₆(̵Alkyl)̵carboylamino, Benzoyl, C₁₋₆(̵Alkyl)-carboyl, CONR₁₀R₁₁, (wobei R₁₀ und R₁₁ jeweils unabhängig voneinander H oder C₁₋₆-Alkyl sind), NR₁₀R₁₁, -N=N-NR₁₀R₁₁, Phenyl, -O(̵C₁₋₆-Alkylen)̵NR₁₀R₁₁, Thiazolyl und durch C₁₋₆-Alkyl oder Amino substituiertes Thiazolyl substituiert ist, steht; oder R₁ für Pyridyl; durch mindestens einen Substituenten aus der Gruppe der C₁₋₆-Alkyle und Halogene substituiertes Pyridyl; Trifluormethyl; Benzoyl oder Benzyl steht;
R₂ für H; Phenyl; OH; C₁₋₃-Alkyl oder C₁₋₃-Alkoxy steht; oder
R₁ und R₂ gemeinsam für einen an den Flavonoidkern ankondensierten Naphthalinring stehen;
R₃ für H; OH oder Halogen steht;
R₄ für H steht;
R₅ für H oder C₁₋₃-Alkyl steht;
R₆ für H; OH oder -O-CO(̵C₁₋₆-Alkyl) steht;
oder R₅ und R₆ gemeinsam für eine Gruppe =CR₁₀R₁₁, oder eine Gruppe =NOH, oder eine Gruppe =O oder eine Gruppe =CHR₁₂ stehen (wobei R₁₂ für Phenyl, Pyridyl, Phenyl, das durch mindestens einen Substituenten aus der Gruppe der Halogenatome, Trifluormethyl und C₁₋₃-Alkyle substituiert ist, oder Pyridyl, das durch mindestens einen Substituenten aus der Gruppe der Halogenatome, Trifluormethyl und C₁₋₃-Alkyle substituiert ist, steht);
R₇ für H; COOR₁₀; -P(O)(OR₁₀R₁₁)₂; NR₁₃R₁₄ (wobei R₁₃ und R₁₄ unabhängig voneinander für H; Phenyl; Phenyl, das durch ein Halogenatom, eine C₁₋₃-Alkylgruppe, oder eine -COOR₁₀-, -CO-O-CH(CH₃)-COOR₁₀-, Morpholinyl-, -C(CH₂OH)₂(CH₃)-, Imidazolinyl-, (̵C₁₋₆-Alkylen)̵OH-, (̵C₁₋₆-Alkylen)̵COOR₁₀- bzw. C₁₋₃-Alkoxy-Gruppe substituiert ist, stehen, oder wobei R₁₃ und R₁₄ gemeinsam mit dem Stickstoffatom, an das beide gebunden sind, Imidazol oder N(̵C₁₋₃-Alkyl)̵piperazinyl bilden) steht; oder
R₇ für -CO(̵C₁₋₆-Alkyl); -S-(C₁₋₆-Alkyl); -SH; -S-CO(̵₁₋₃-Alkyl); 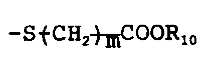 (wobei 0 < m ≦ 6); -O(̵C₁₋₆-Alkylen)̵NR₁₀R₁₁; -NR₁₀NR₁₀R₁₁; C₁₋₆-Alkyl; Thiazolyl; Thiazolyl, das durch mindestens einen Substituenten aus der Gruppe -NH₂, C₁₋₃-Alkyl, Phenyl und COOR₁₀ substituiert ist; -NH-CO(̵C₁₋₃-Alkyl) oder (̵C₁₋₃ Alkylen)̵CH(NH₂(COOH)- steht;
oder
-CR₅R₆R₇ für eine Gruppe der Formel 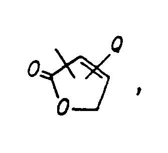 steht,
worin Q für mindestens einen Substituenten aus der Gruppe H; COOR₁₀; Phenyl; -O(̵C₁₋₃-Alkylen)̵COOR₁₀; C₁₋₃-Alkyl; -O-CS-NR₁₀R₁₁; -O(̵C₁₋₃-Alkylen)̵NR₁₀R₁₁; OH; C₁₋₃-Alkoxy; und NR₁₀R₁₁ steht; oder
in der jeweils zwei Reste aus der Gruppe R₃, R₄, R₅, R₆ und R₇ gemeinsam einen Benzolring oder einen durch (̵C₁₋₃-Alkylen)̵COOR₁₀; (̵C₁₋₃-Alkyl)̵OH, COOR₁₀ oder (̵C₁₋₃-Alkylen)̵-O-CO(C₁₋₃-Alkyl) substituierten Benzolring; ein Naphthalinsystem oder ein durch (̵C₁₋₃-Alkylen)̵COOR₁₀, (̵C₁₋₃-Alkyl)̵OH, COOR₁₀ oder (̵C₁₋₃-Alkylen)̵O-CO(̵C₁₋₃-Alkyl) substituiertes Naphthalinsystem bilden; und
deren physiologisch unbedenkliche Salze,
unter der Voraussetzung, daß, wenn alle Reste R₂, R₃, R₄, R₅ und R₆ H und R₇ COOR₁₀ darstellen, R₁ nicht Phenyl, 2-Thienyl, 3-Methoxyphenyl, 3,4-Dimethoxyphenyl, 2-Furyl, para-Tolyl, 2-Naphthyl, 4-Methoxyphenyl, Cyclohexyl, Benzyl oder Methyl bedeutet.

2. Verbindung nach Anspruch 1, in der:
R₁ für durch C₁₋₃-Alkyl, Halogen, Trifluormethyl, Hydroxyl, C₁₋₃-Alkoxy oder Nitro substituiertes Phenyl steht.

3. Verbindung nach Anspruch 1, in der R₁ für durch ein Halogenatom substituiertes Phenyl steht.

4. Verbindung nach Anspruch 1, in der R₇ für -COOR₁₀, -P(O)(OR₁₀R₁₁), -CH₂CH₂COOR₁₀ oder -CONR₁₀R₁₁ steht.

5. Verbindung nach Anspruch 1, wobei diese Verbindung der Formel (IV) entspricht: 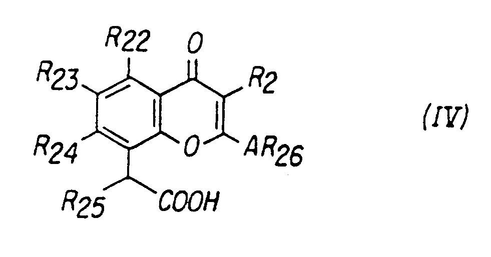 in der:
AR₂₆ für Phenyl, substituiertes Phenyl oder Biphenylyl steht;
R₂ für Wasserstoff, Hydroxyl oder C₁₋₃-Alkoxy steht;
R₂₂ für Wasserstoff oder Hydroxyl steht;
R₂₃ für Wasserstoff oder Fluor steht;
R₂₄ für Wasserstoff oder Hydroxyl steht;
R₂₅ für Wasserstoff, 2-Methylpyridyl, Benzyliden, 4-Methylenpyridyl oder Methylen steht; oder
R₂₂ und R₂₃ gemeinsam einen an den Flavonoidkern ankondensierten Benzolring bilden; oder
R₂₃ und R₂₄ gemeinsam einen an den Flavonoidkern ankondensierten Benzolring bilden; oder
R₂₅ und R₂₄ gemeinsam einen an den Flavonoidkern ankondensierten Benzolring bilden.

6. Pharmazeutische Zusammensetzung mit einem pharmazeutisch unbedenklichen Trägor und einer Verbindung der Formel (I) nach Anspruch 1.

7. Pharmazeutische Zusammensetzung mit einer Verbindung nach Anspruch 2, 3 oder 4 und einem pharmazeutisch unbedenklichen Träger.

8. Pharmazeutische Zusammensetzung mit einer Verbindung nach Anspruch 5 und einem pharmazeutisch unbedenklichen Träger.

9. Verbindung nach Anspruch 1, wobei es sich bei dieser Verbindung um

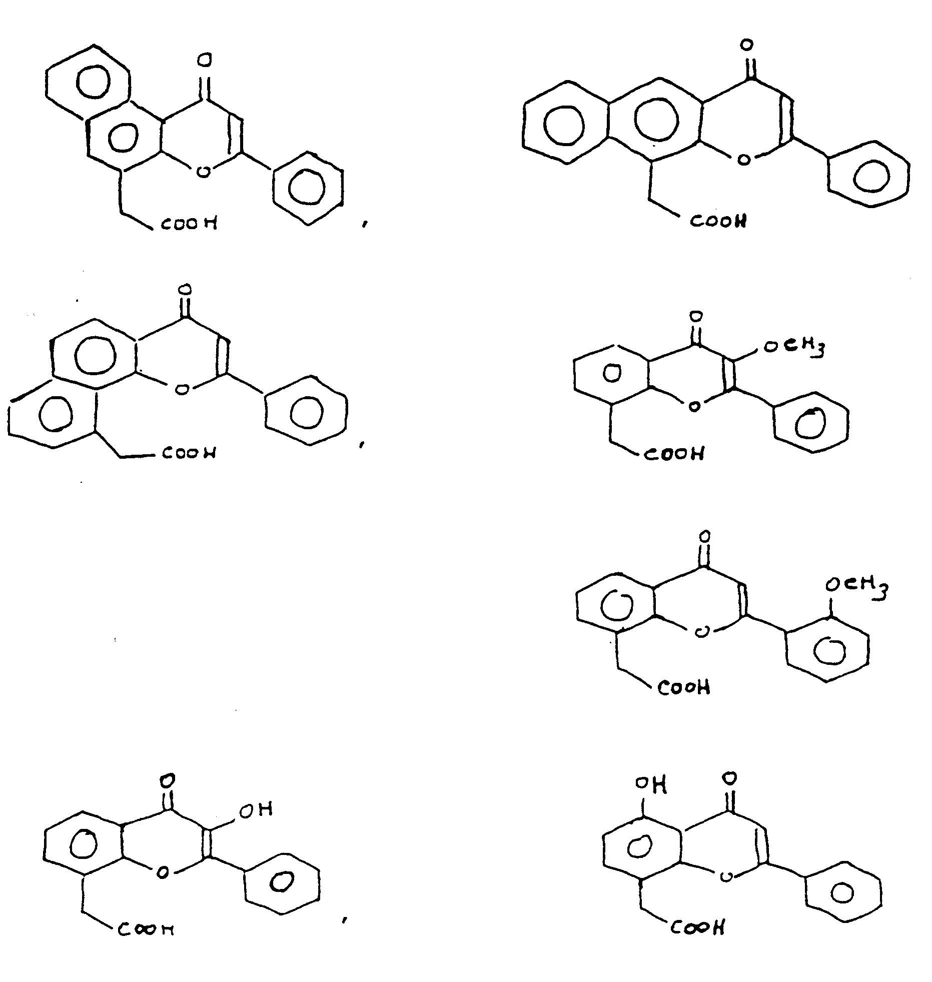

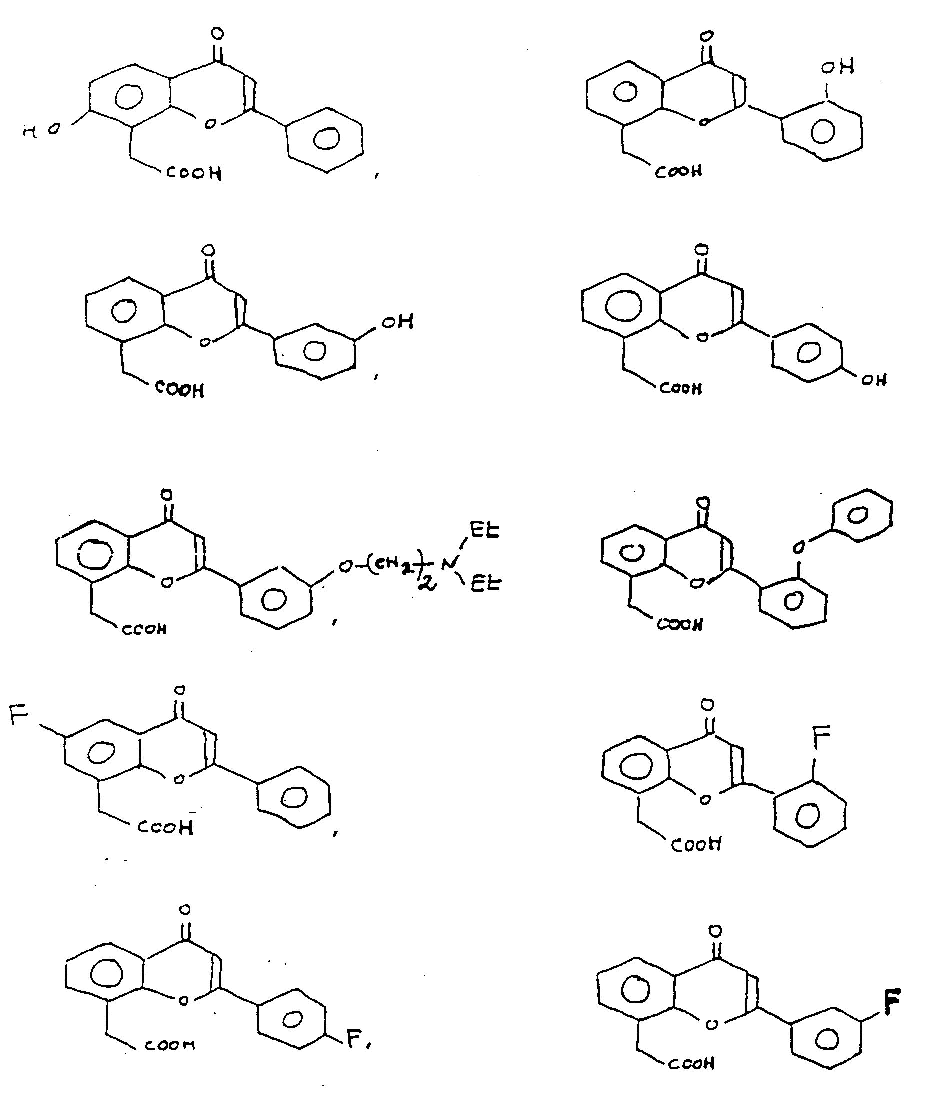

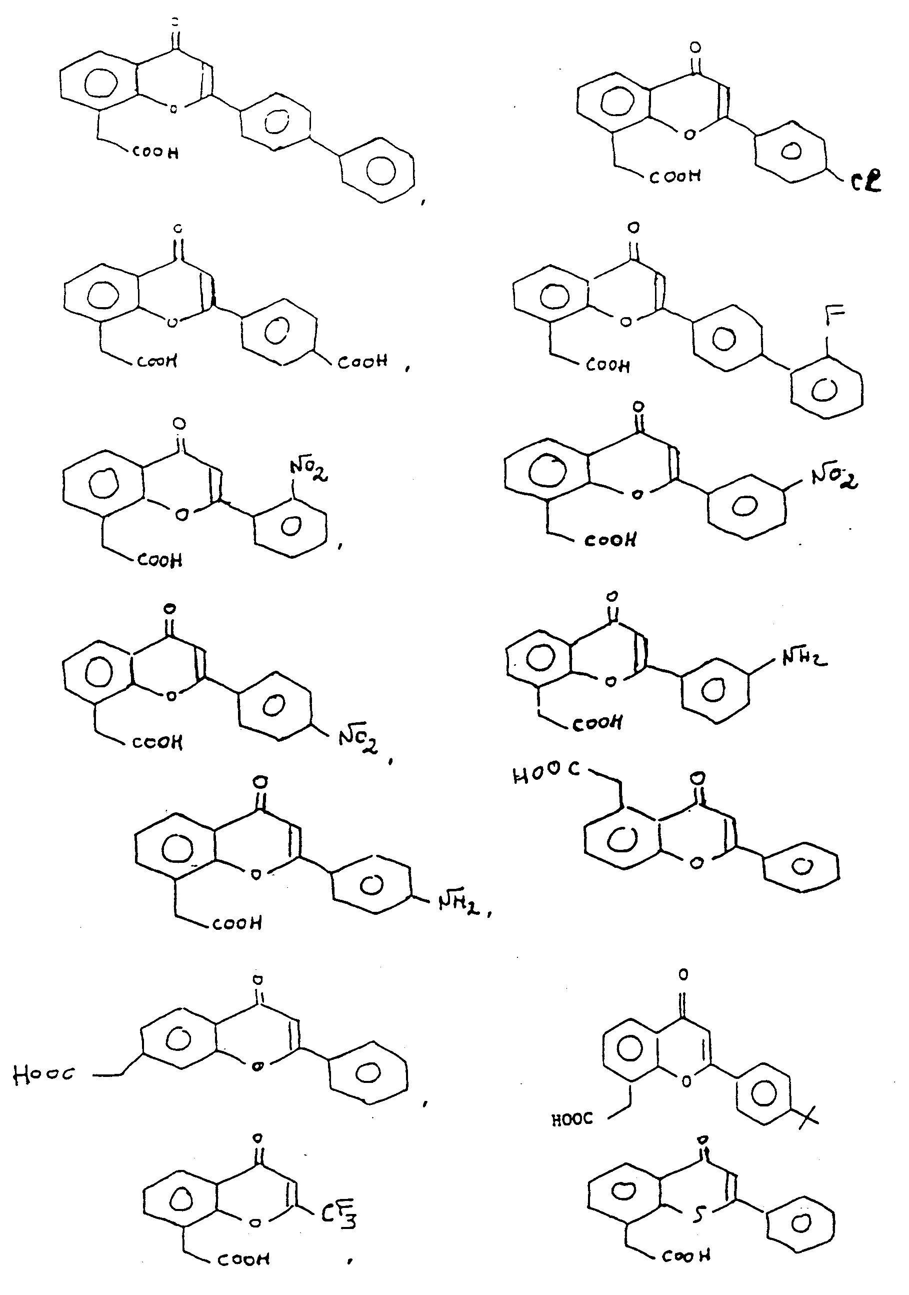

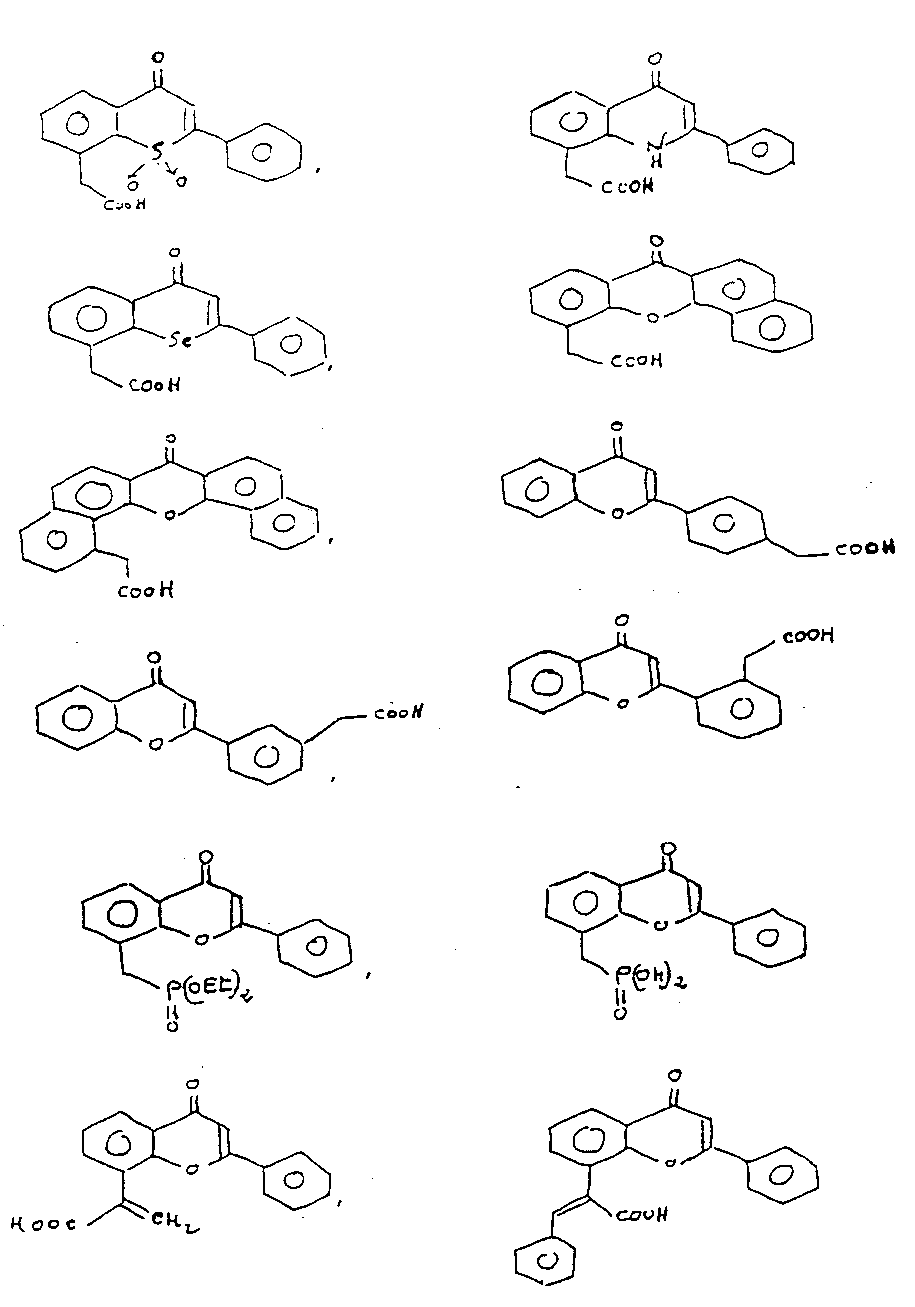

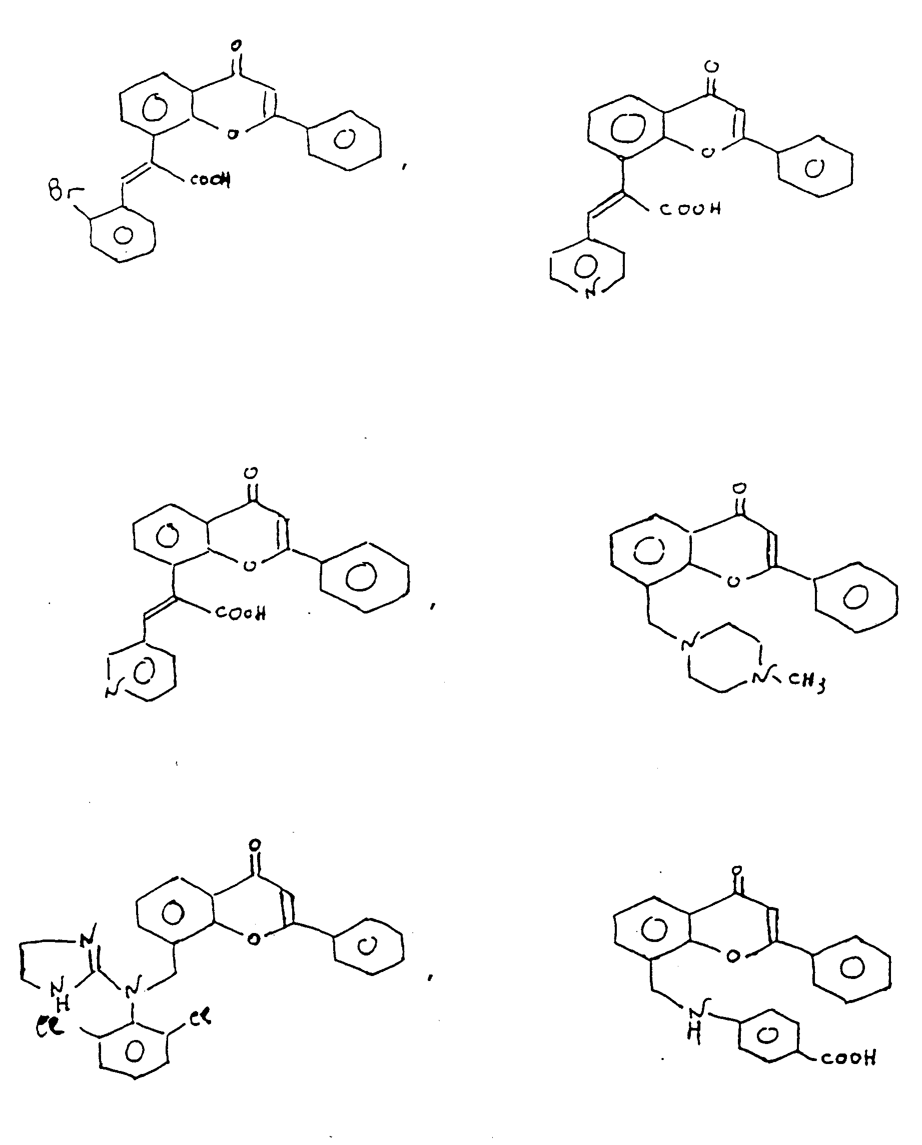

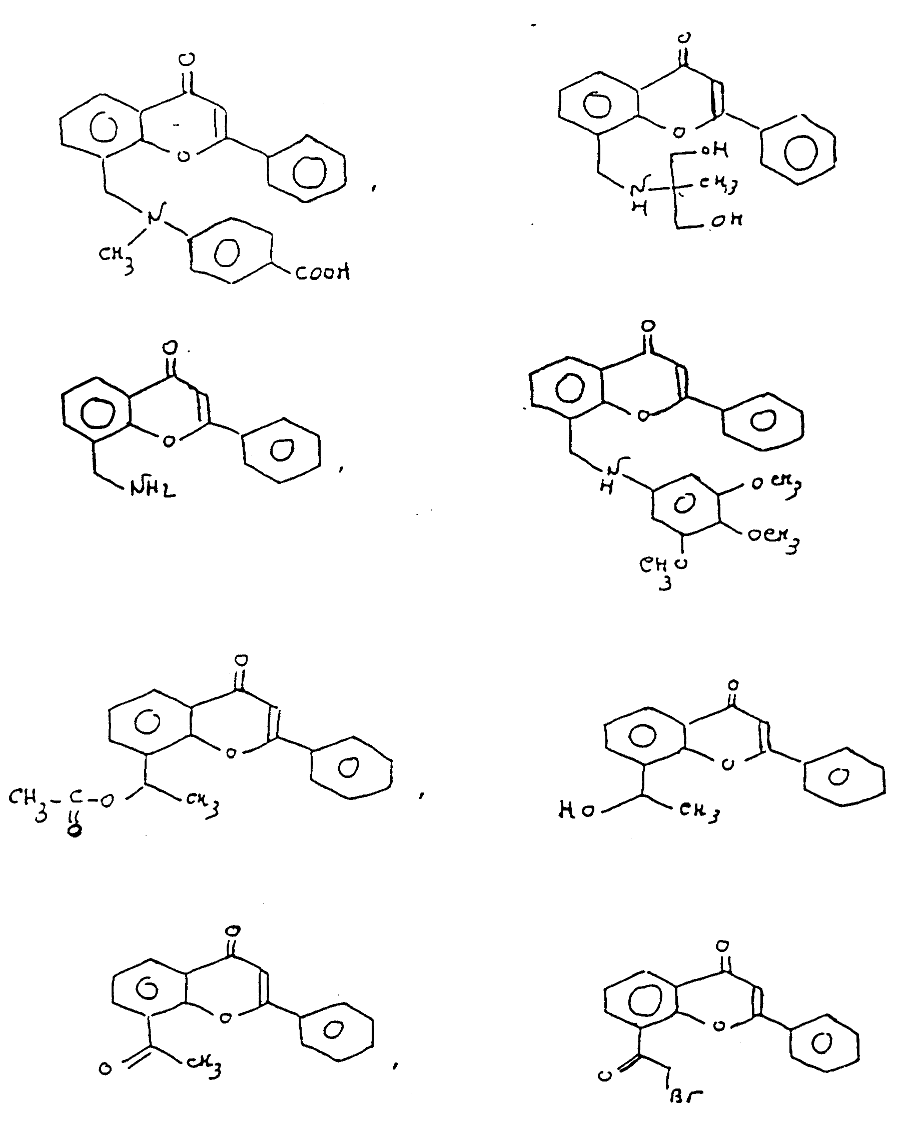

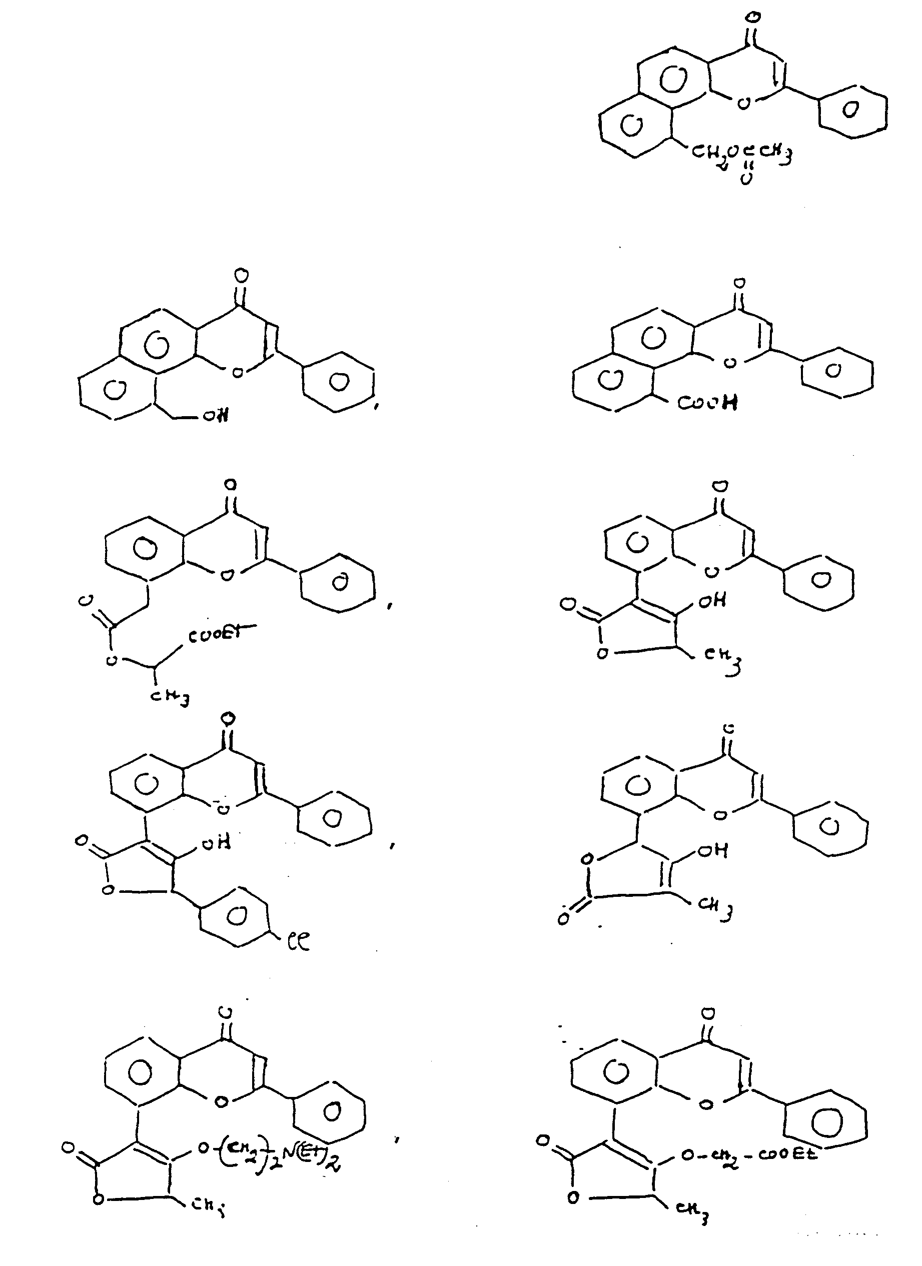

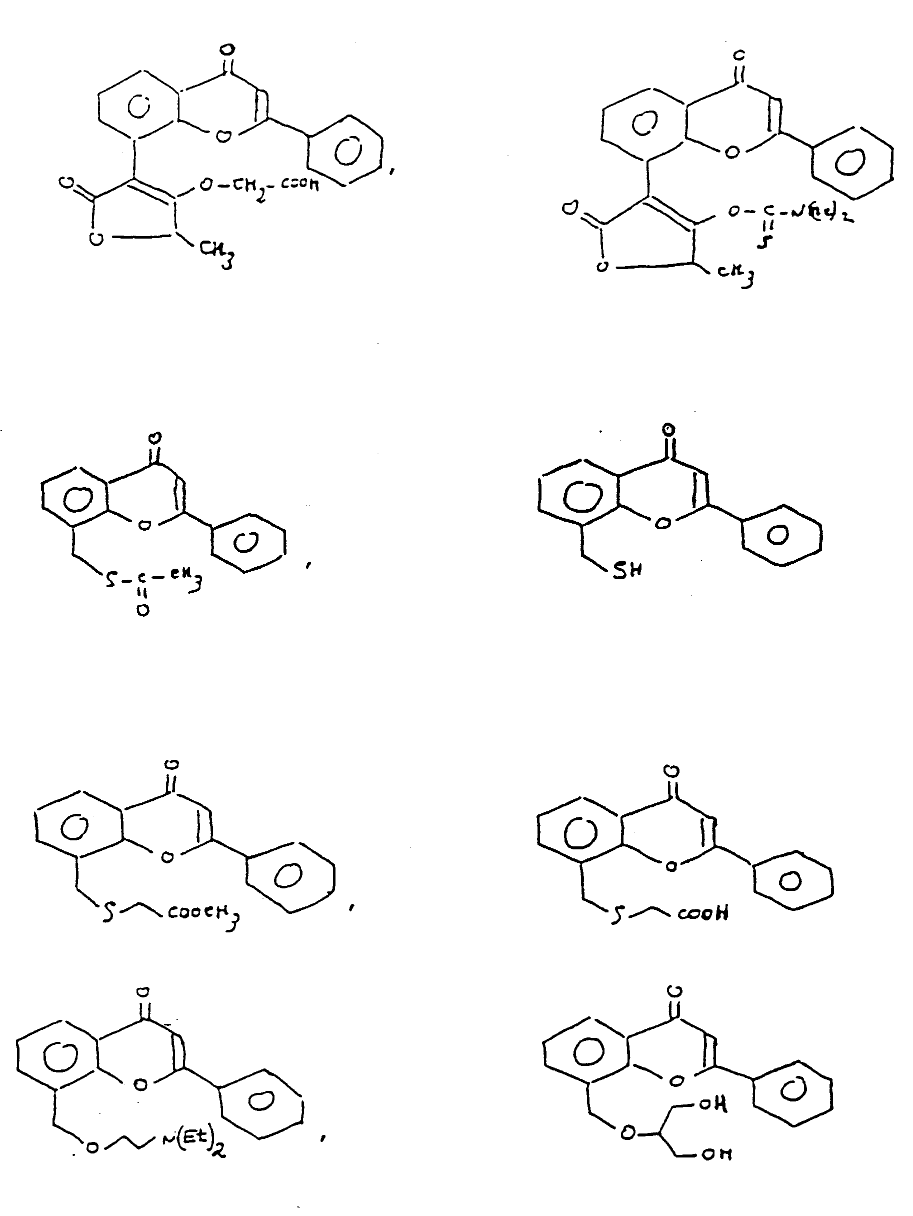

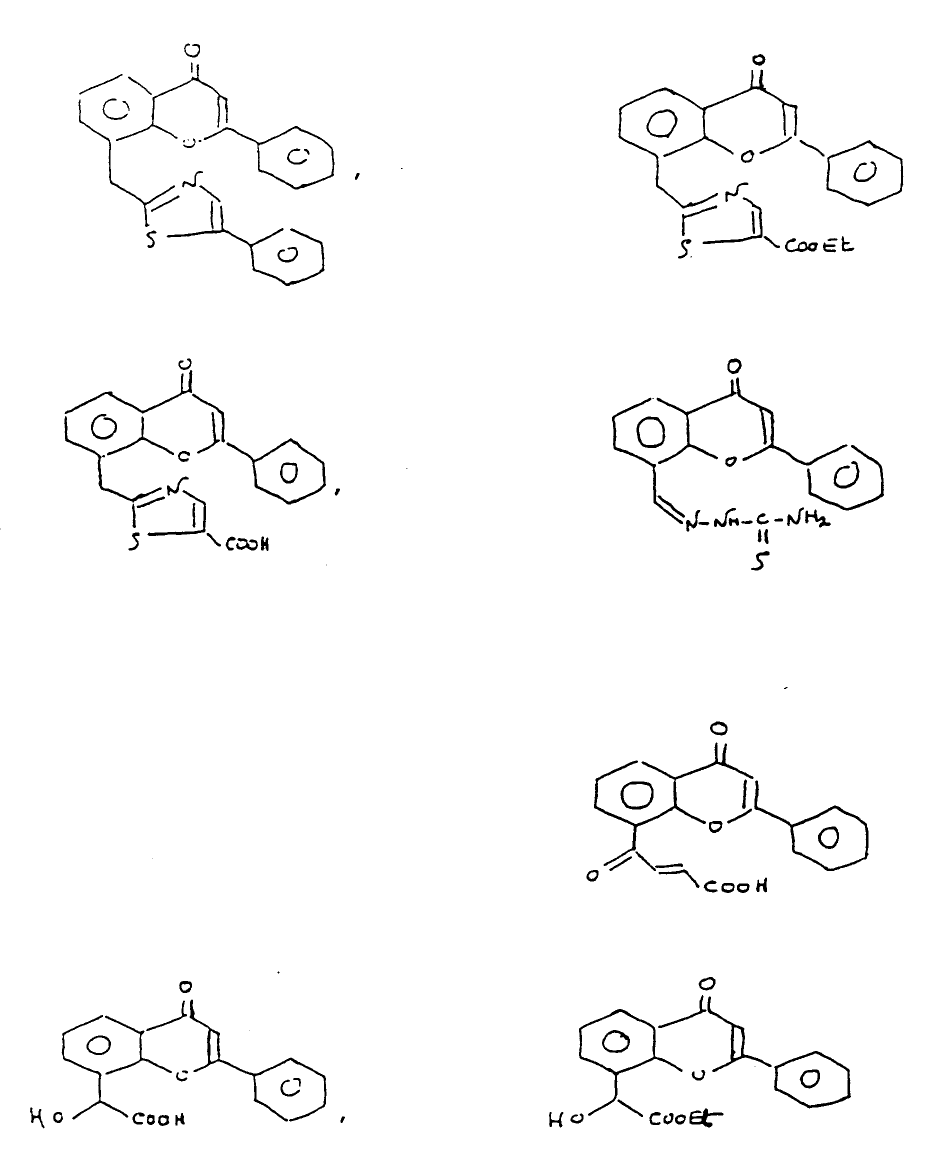

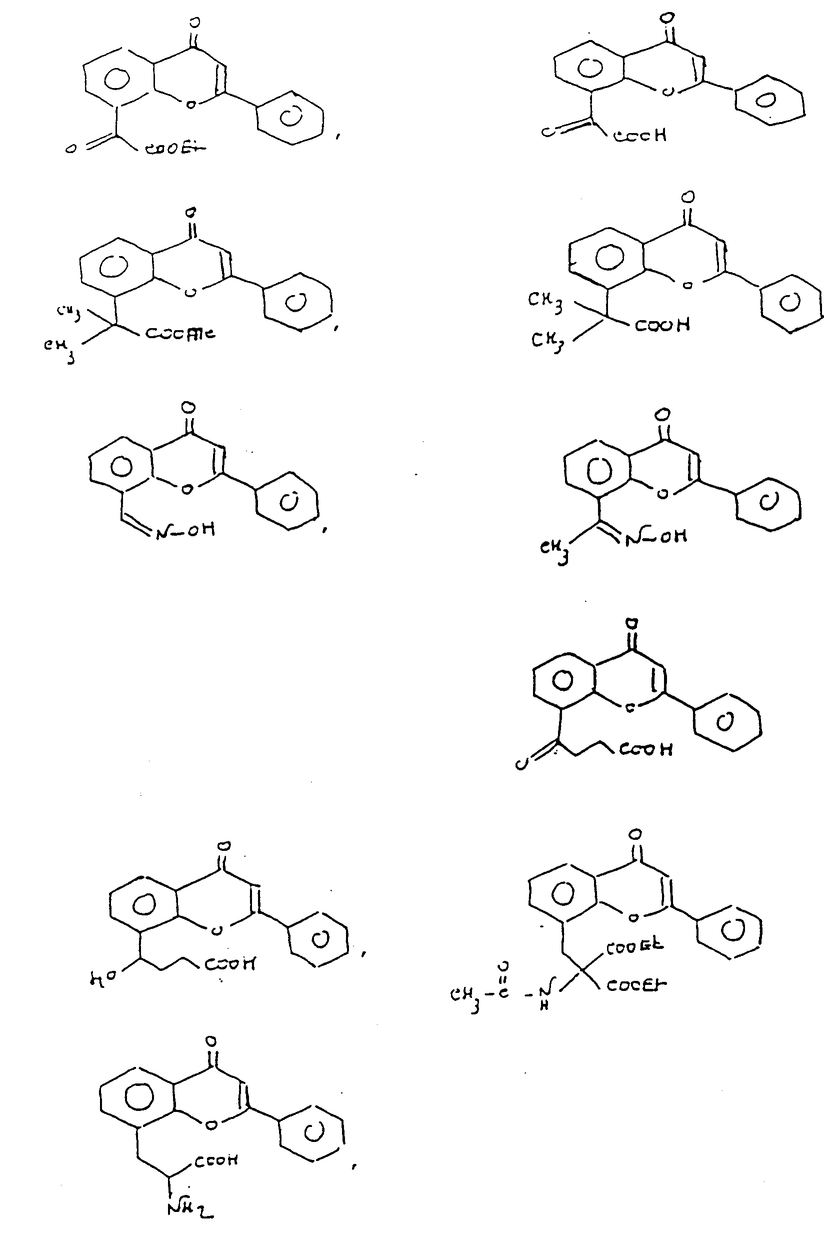

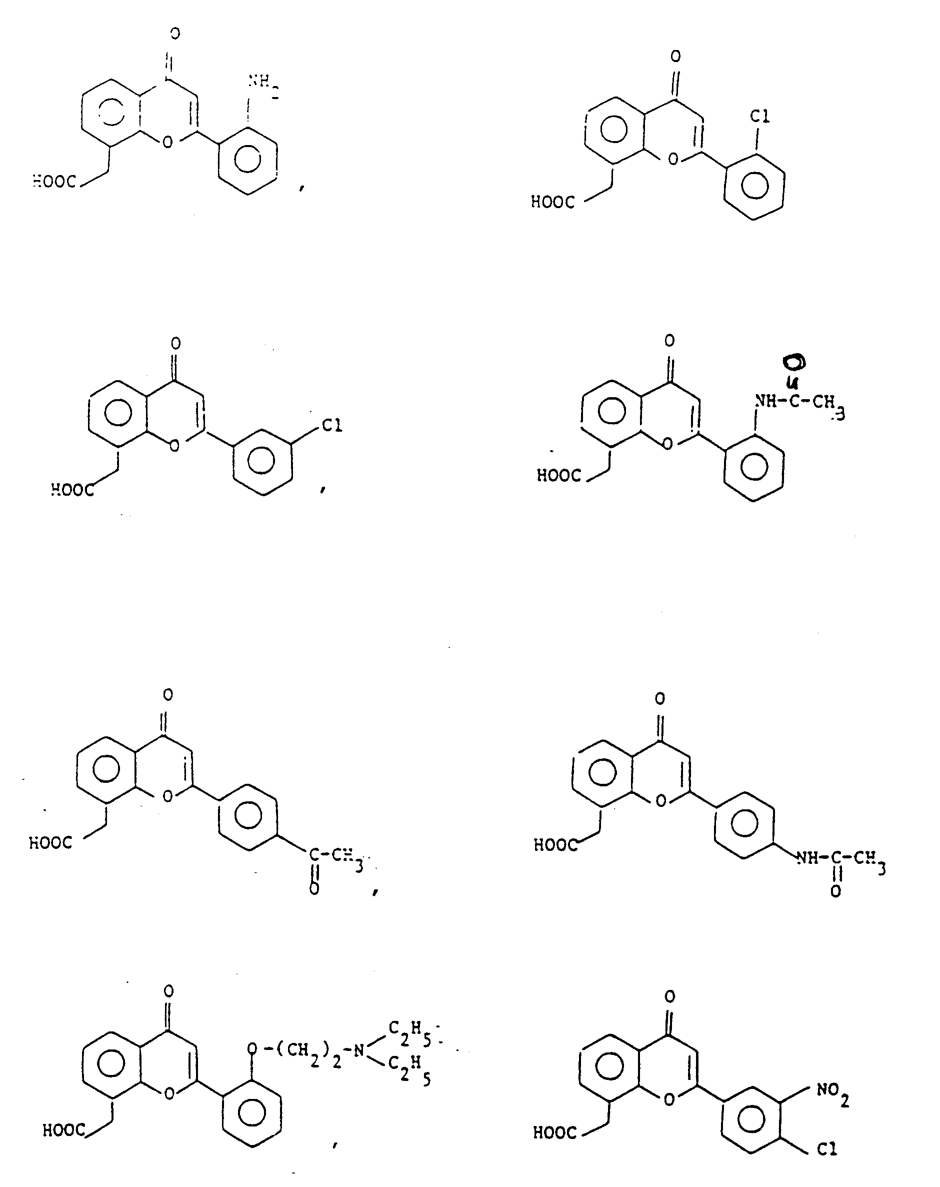

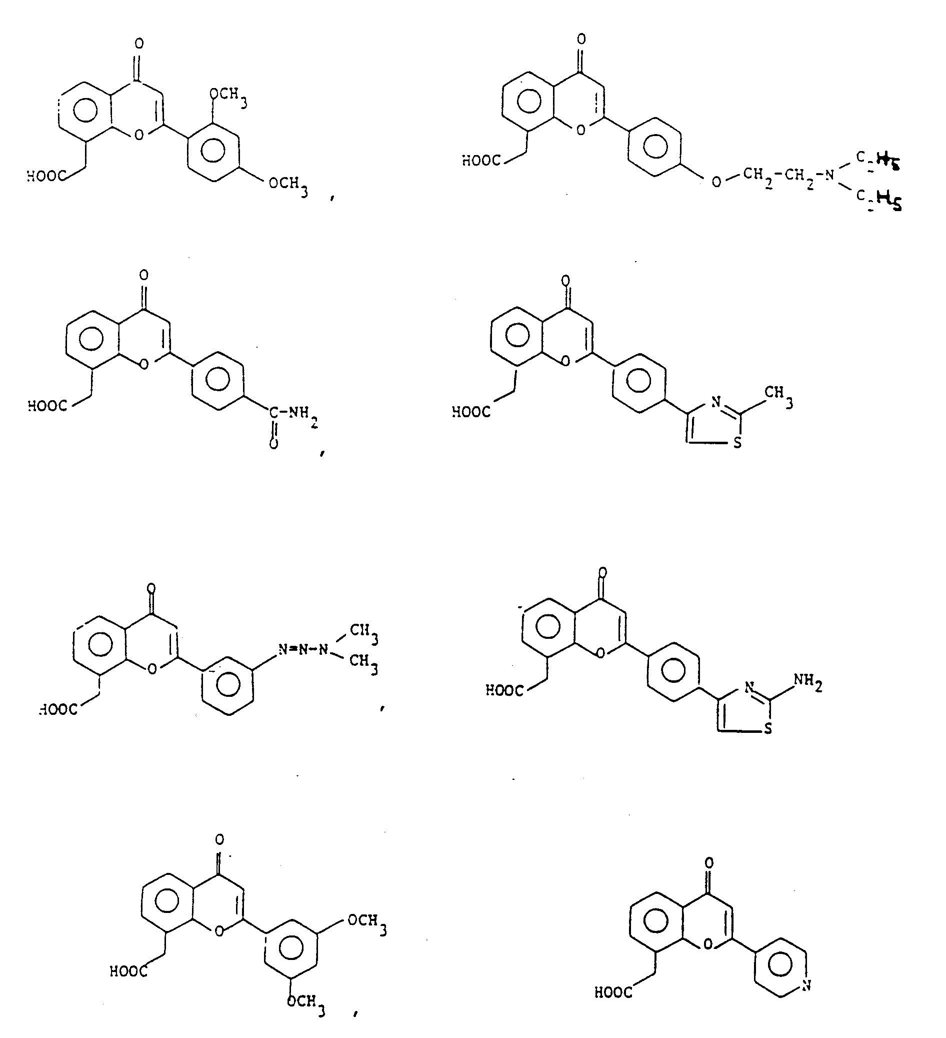

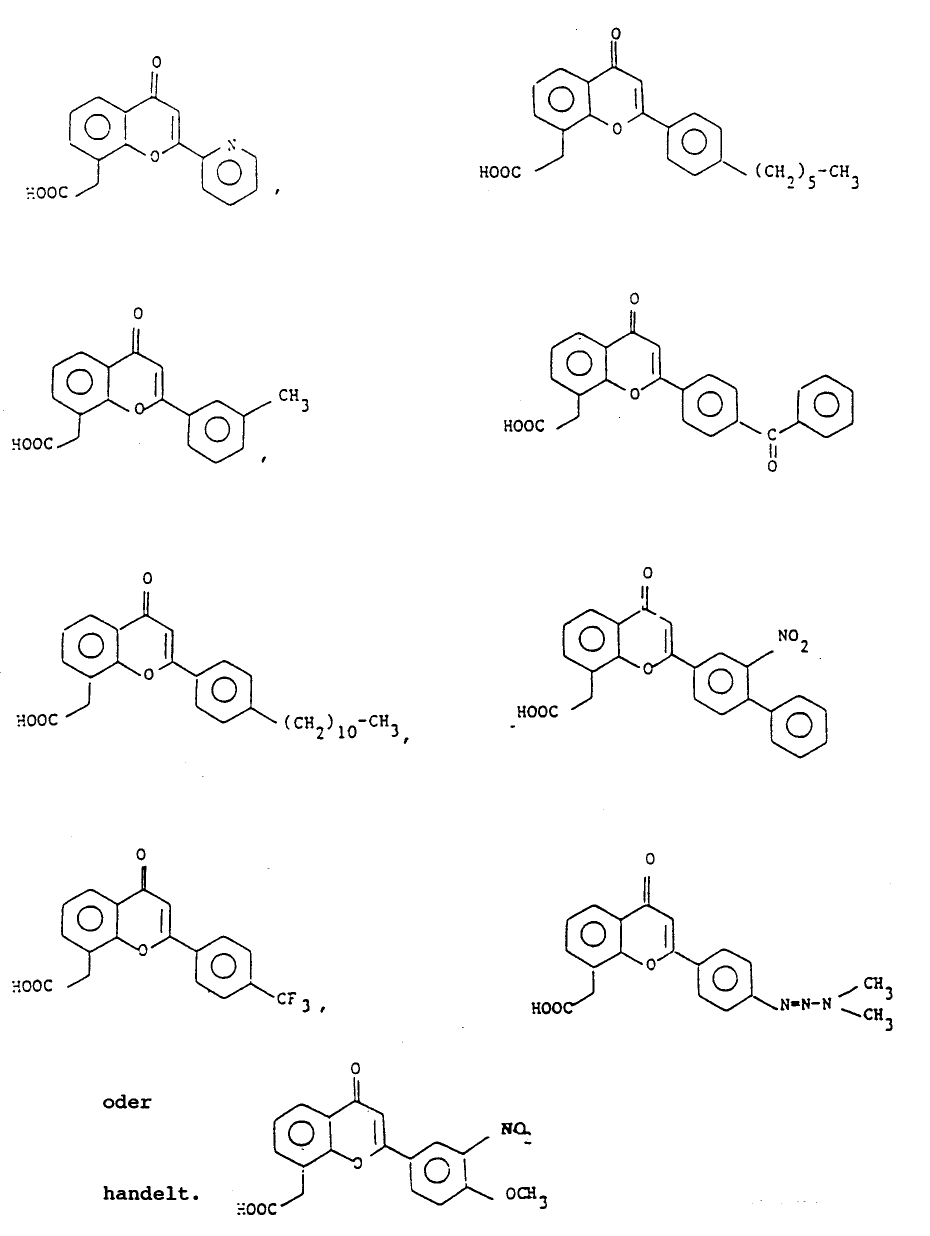

10. Verbindung nach Anspruch 1, wobei es sich bei dieser Verbindung um

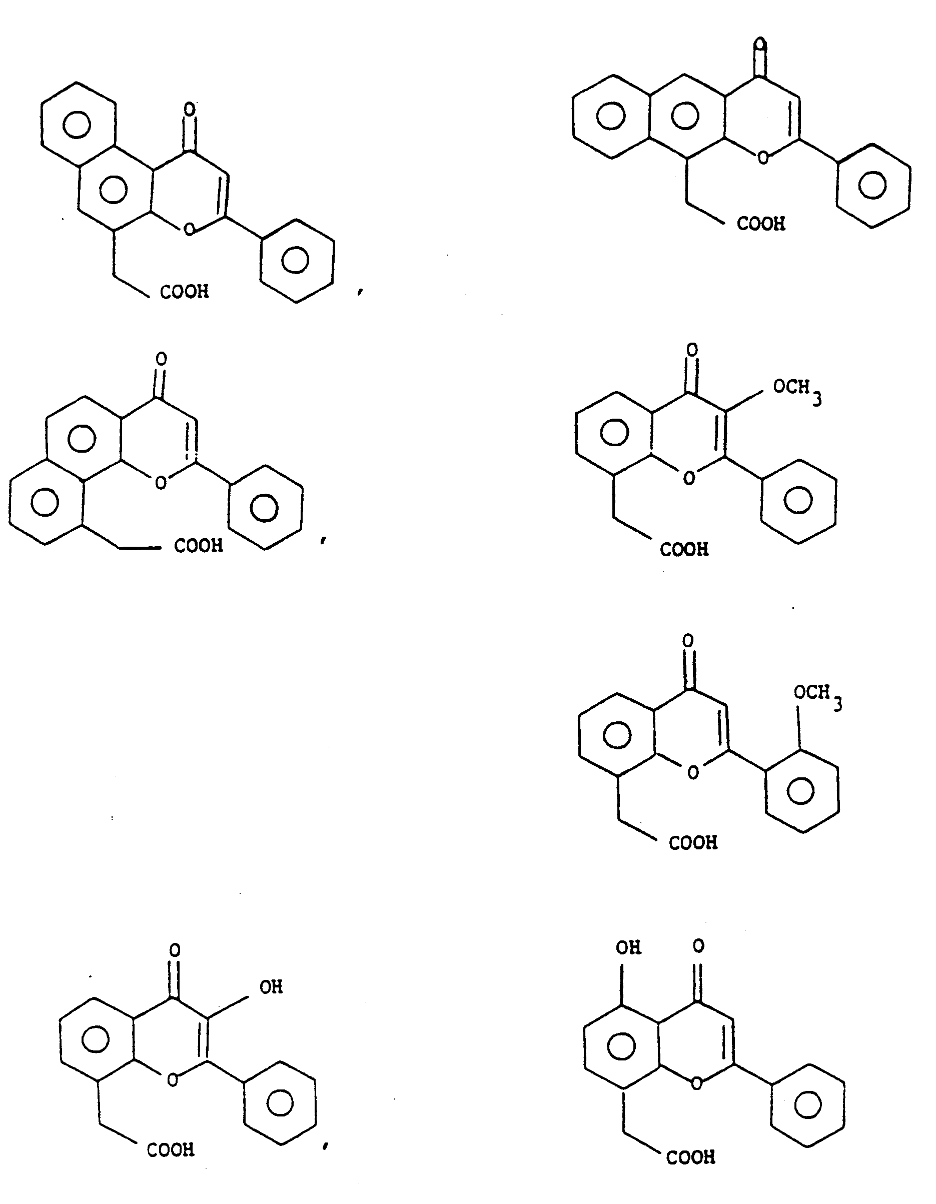

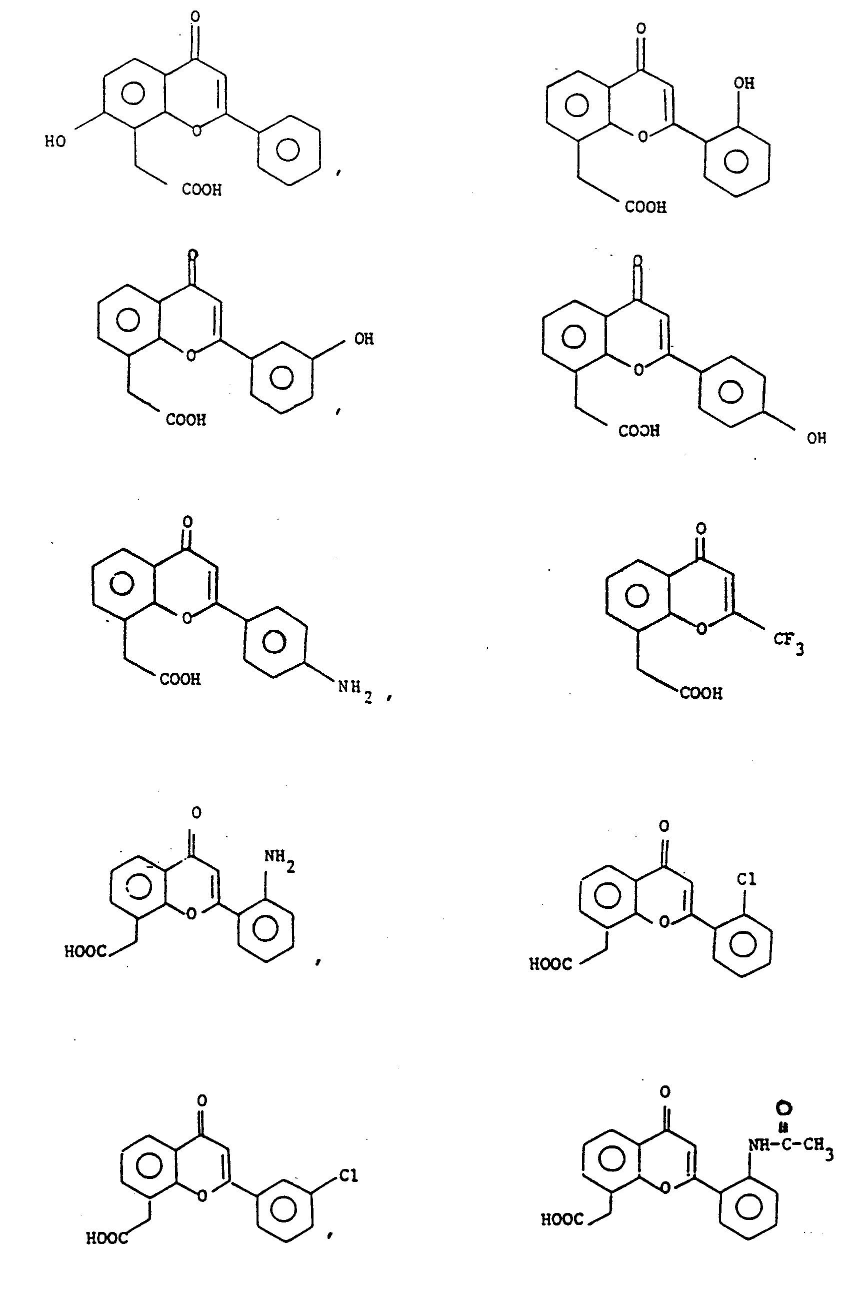

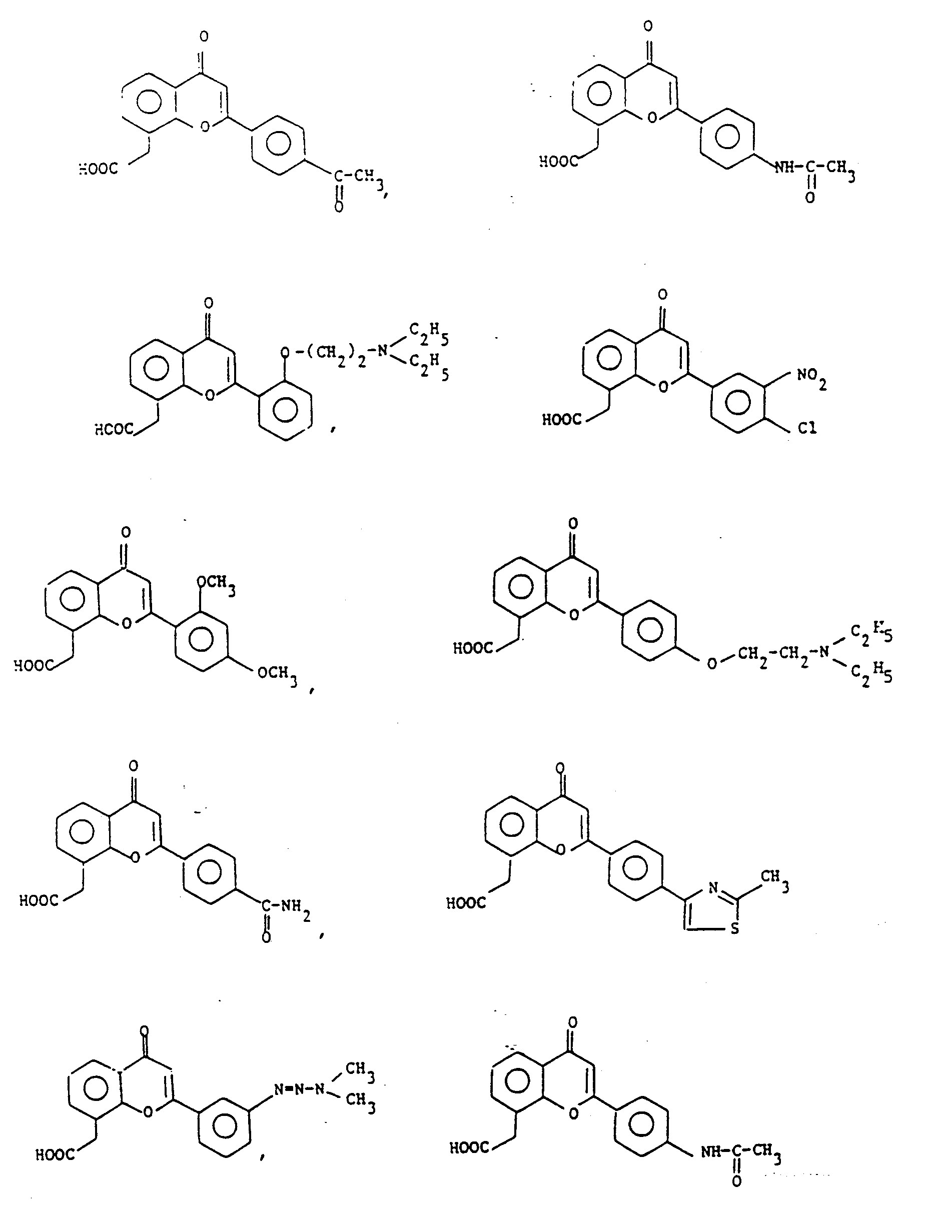

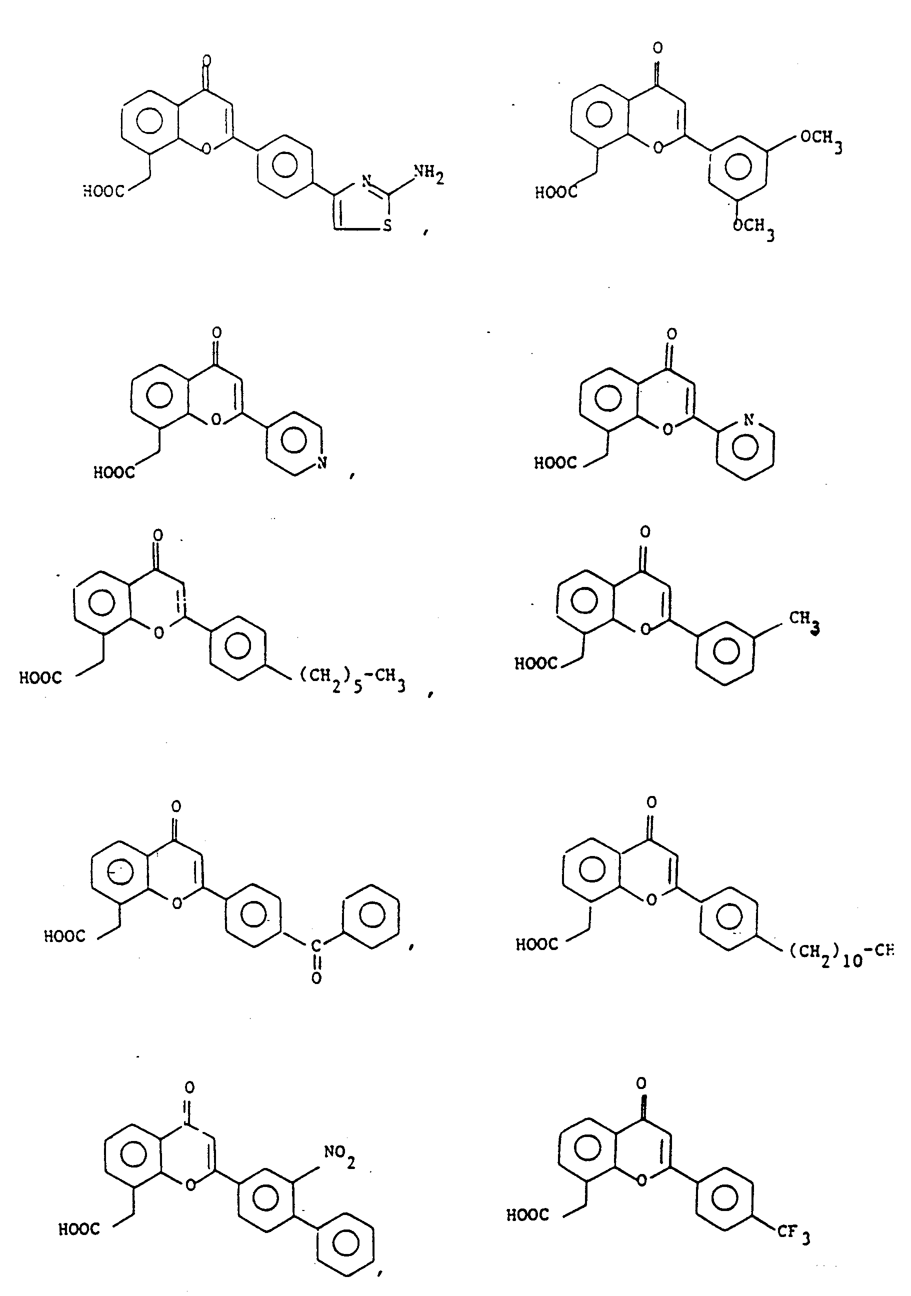

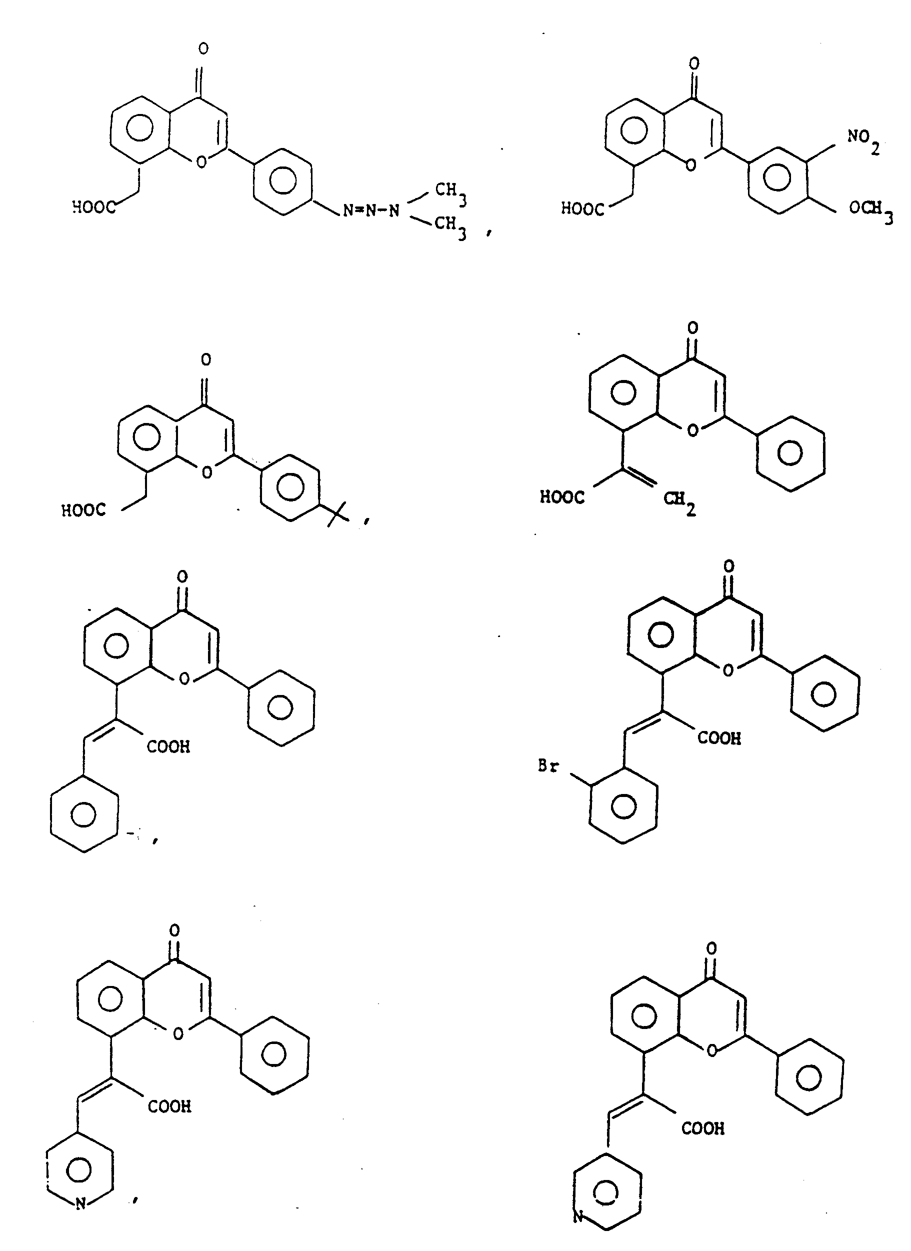

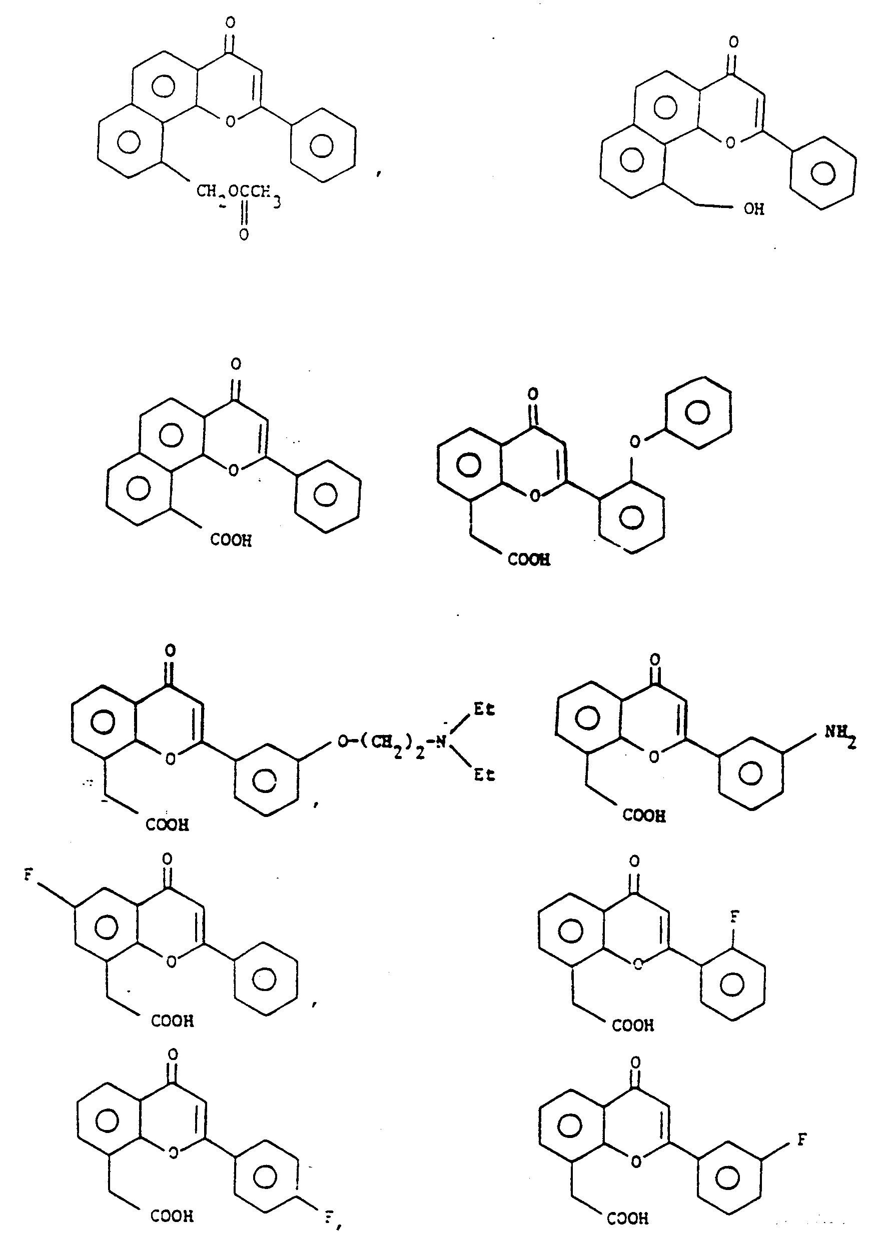

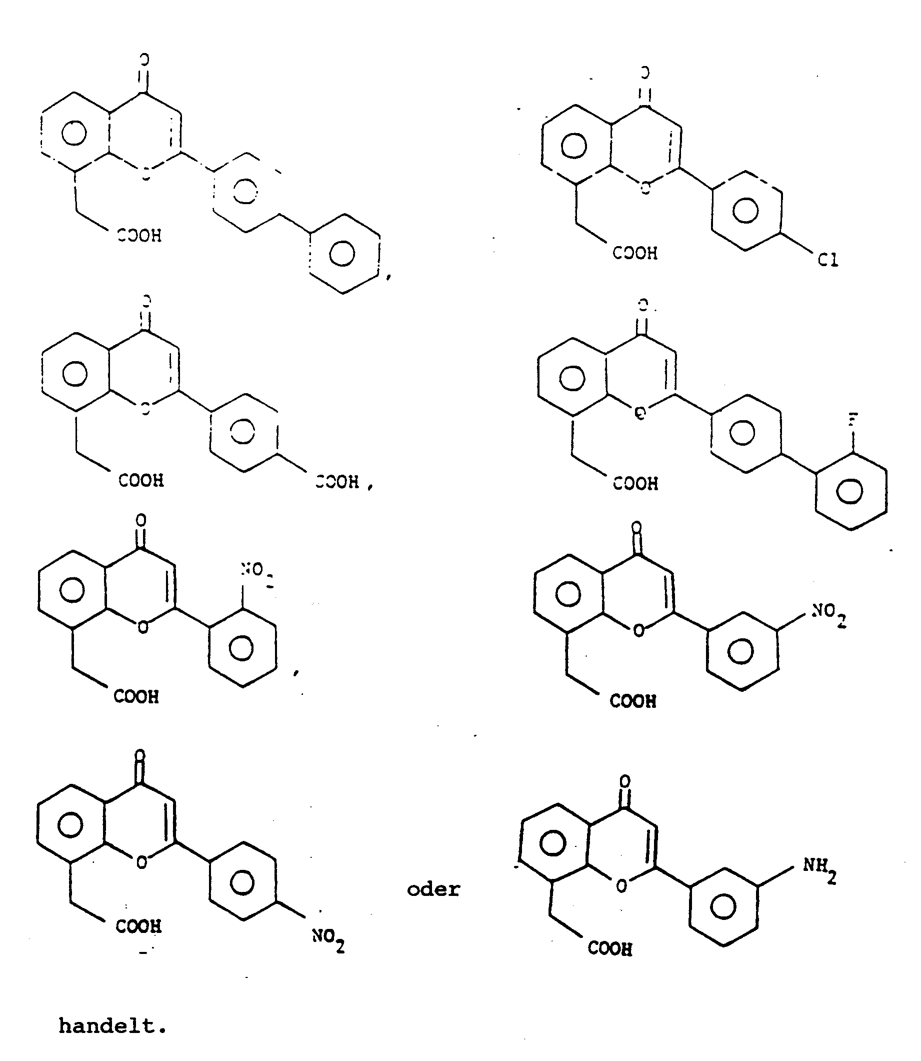

## Revendications

1. Composé de formule (I): 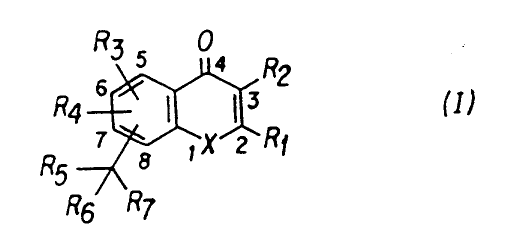 dans laquelle :
X est N, O, Se ou S(O)ₙ où n est 0, 1 ou 2 ;
R₁ est un groupe phényle ; phényle substitué par au moins un membre de la classe formée par les halogènes, les groupes alkyle en C₁-C₁₂, trifluorométhyle, hydroxyle, alcoxy en C₁-C₆, (̵alkylène en C₁-C₆)̵COOR₁₀, nitro, (alkyle en C₁-C₆)̵carbonylamino, benzoyle, (alkyle en C₁-C₆)̵carbonyle, CONR₁₀R₁₁ (où R₁₀ et R₁₁ sont chacun indépendamment H ou un groupe alkyle en C₁-C₆), NR₁₀R₁₁, -N=N-NR₁₀R₁₁, phényle, -O(̵alkylène en C₁-C₆)̵NR₁₀R₁₁, thiazolyle et thiazolyle substitué par un groupe alkyle en C₁-C₆ ou amino ; ou bien R₁ est un groupe pyridyle ; pyridyle substitué par au moins un membre de la classe formée par les groupes alkyle en C₁-C₆ et les halogènes ; trifluorométhyle ; benzoyle ; ou benzyle ;
R₂ est H ; un groupe phényle ; OH ; alkyle en C₁-C₃ ; ou alcoxy en C₁-C₃ ; ou bien
R₁ et R₂ forment ensemble un noyau naphtalénique accolé au noyau de flavonoïde ;
R₃ est H ; OH ; ou un halogène ;
R₄ est H ;
R₅ est H ou un groupe alkyle en C₁-C₃ ;
R₆ est H ; OH ; ou un groupe -O-CO(̵alkyle en C₁-C₆)̵ ;
ou bien R₅ et R₆ forment ensemble un groupe =CR₁₀R₁₁, un groupe =NOH, un groupe =O ou un groupe =CHR₁₂ (où R₁₂ est un groupe phényle, pyridyle, phényle substitué par au moins un membre de la classe formée par les atomes d'halogènes, le groupe trifluorométhyle et les groupes alkyle en C₁-C₃, ou pyridyle substitué par au moins un membre de la classe formée par les atomes d'halogènes, le groupe trifluorométhyle et les groupes alkyle en C₁-C₃) ;
R₇ est H ; COOR₁₀ ; -P(O)(OR₁₀R₁₁)₂ ; NR₁₃R₁₄ (où R₁₃ et R₁₄ sont indépendamment H ; un groupe phényle ; phényle substitué par un atome d'halogène ou un groupe alkyle en C₁-C₃ ou un groupe -COOR₁₀, -CO-O-CH(CH₃)COOR₁₀, morpholinyle, -C(CH₂OH)₂(CH₃), imidazolinyle, (̵alkylène en C₁-C₆)̵OH, (̵alkylène en C₁-C₆)̵COOR₁₀ ou alcoxy en C₁-C₃, ou bien R₁₃ et R₁₄ forment, avec l'atome d'azote auquel ils sont tous deux liés, un cycle d'imidazole ou un groupe N(̵alkyle en C₁-C₃)̵pipérazinyle) ; ou bien
R₇ est un groupe -CO(̵alkyle en C₁-C₆) ; -S(̵alkyle en C₁-C₆) ; -SH ; -S-CO(̵alkyle en C₁-C₃) ; 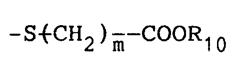 (avec 0 < m ≦ 6) ; -O(̵alkylène en C₁-C₆)̵NR₁₀R₁₁ ; -NR₁₀NR₁₀R₁₁ ; alkyle en C₁-C₆ ; thiazolyle ; thiazolyle substitué par au moins un membre de la classe formée par -NH₂, un groupe alkyle en C₁-C₃, phényle et COOR₁₀ ; -NH-CO(̵alkyle en C₁-C₃) ; ou (̵alkylène en C₁-C₃)̵CH(NH₂)(COOH) ; ou bien
-CR₅R₆R₇ est un groupe de formule 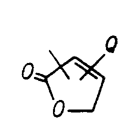 où Q est au moins un membre de la classe formée par H ; un groupe COOR₁₀ ; phényle ; -O(̵alkylène en C₁-C₃)̵COOR₁₀ ; alkyle en C₁-C₃ ; -O-CS-NR₁₀R₁₁ ; -O(̵alkylène en C₁-C₃)̵NR₁₀R₁₁ ; OH ; alcoxy en C₁-C₃ ; et NR₁₀R₁₁ ; ou bien
où deux quelconques de R₃, R₄, R₅, R₆ et R₇ forment ensemble un noyau benzénique ; ou un noyau benzénique substitué par un groupe (̵alkylène en C₁-C₃)̵COOR₁₀ ; (̵alkylène en C₁-C₃)̵OH, COOR₁₀ ou (̵alkylène en C₁-C₃)̵O-CO(̵alkyle en C₁-C₃) ; ou un système naphtalénique ; ou un système naphtalénique substitué par un groupe (̵alkylène en C₁-C₃)̵COOR₁₀, (̵alklène en C₁-C₃)̵OH, COOR₁₀ ou (̵alkylène en C₁-C₃)̵O-CO(̵alkyle en C₁-C₃) ;
et ses sels physiologiquement acceptables,
à condition que si R₂, R₃, R₄, R₅ et R₆ sont tous H et R₇ est COOR₁₀, R₁ soit autre chose qu'un groupe phényle, 2-thiényle, 3-méthoxyphényle, 3,4-diméthoxyphényle, 2-furyle, para-tolyle, 2-naphtyle, 4-méthoxyphényle, cyclohexyle, benzoyle ou méthyle.

2. Composé de la revendication 1, dans lequel :
R₁ est un groupe phényle substitué par au moins un halogène, alkyle en C₁-C₃, trifluorométhyle, hydroxyle, alcoxy en C₁-C₃ ou nitro.

3. Composé de la revendication 1, dans lequel R₁ est un groupe phényle substitué par un seul atome d'halogène.

4. Composé de la revendication 1, dans lequel R₇ est -COOR₁₀, -P(O)(OR₁₀R₁₁), -CH₂CH₂COOR₁₀ ou -CONR₁₀R₁₁.

5. Composé de la revendication 1, ledit composé répondant à la formule (IV) : 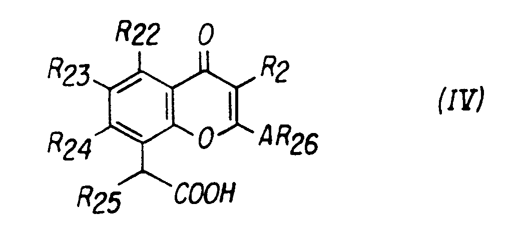 dans laquelle :
AR₂₆ est un groupe phényle, phényle substitué ou biphénylyle ;
R₂ est l'hydrogène, un groupe hydroxyle ou alcoxy en C₁-C₃ ;
R₂₂ est l'hydrogène ou un groupe hydroxyle ;
R₂₃ est l'hydrogène ou le fluor ;
R₂₄ est l'hydrogène ou un groupe hydroxyle ;
R₂₅ est l'hydrogène, un groupe 2-méthylpyridyle, benzylidène, 4-méthylène-pyridyle ou méthylène ; ou bien
R₂₂ et R₂₃ forment ensemble un noyau benzénique accolé au noyau de flavonoïde ; ou bien
R₂₃ et R₂₄ forment ensemble un noyau benzénique accolé au noyau de flavonoïde ; ou bien
R₂₅ et R₂₄ forment ensemble un noyau benzénique accolé au noyau de flavonoïde.

6. Composition pharmaceutique, comprenant un support pharmaceutiquement acceptable et un composé de formule (I) de la revendication 1.

7. Composition pharmaceutique comprenant un composé de la revendication 2, 3 ou 4 et un support pharmaceutiquement acceptable.

8. Composition pharmaceutique comprenant un composé de la revendication 5 et un support pharmaceutiquement acceptable.

9. Composé de la revendication 1, ledit composé étant

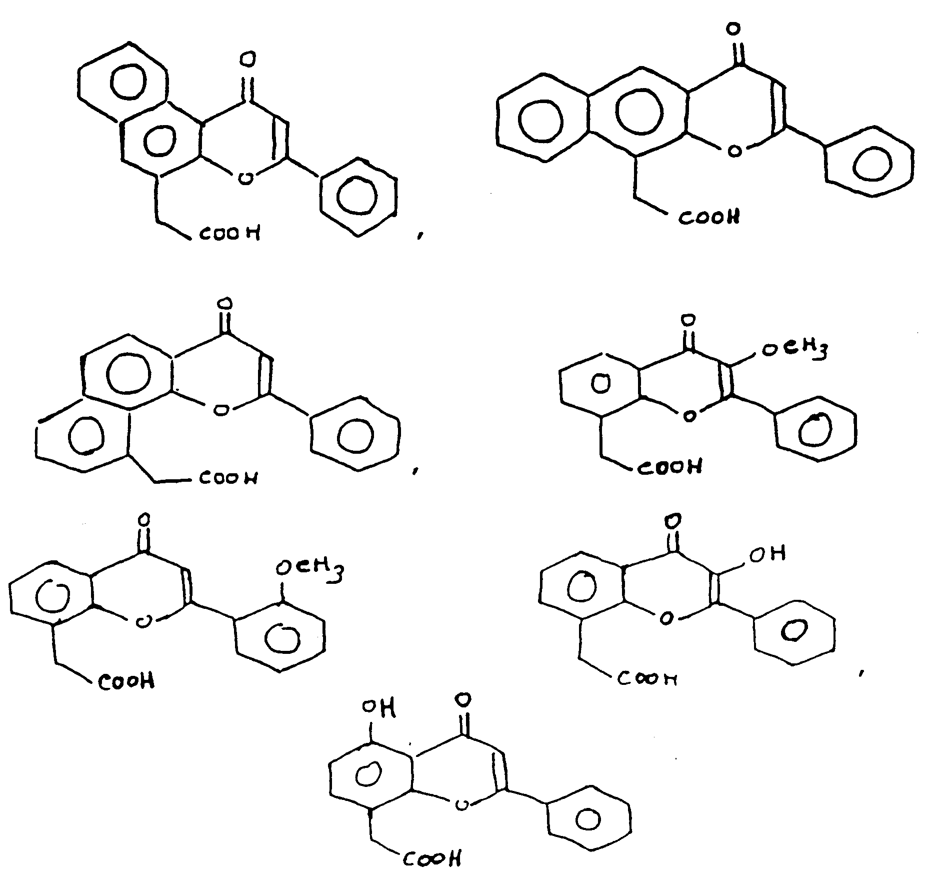

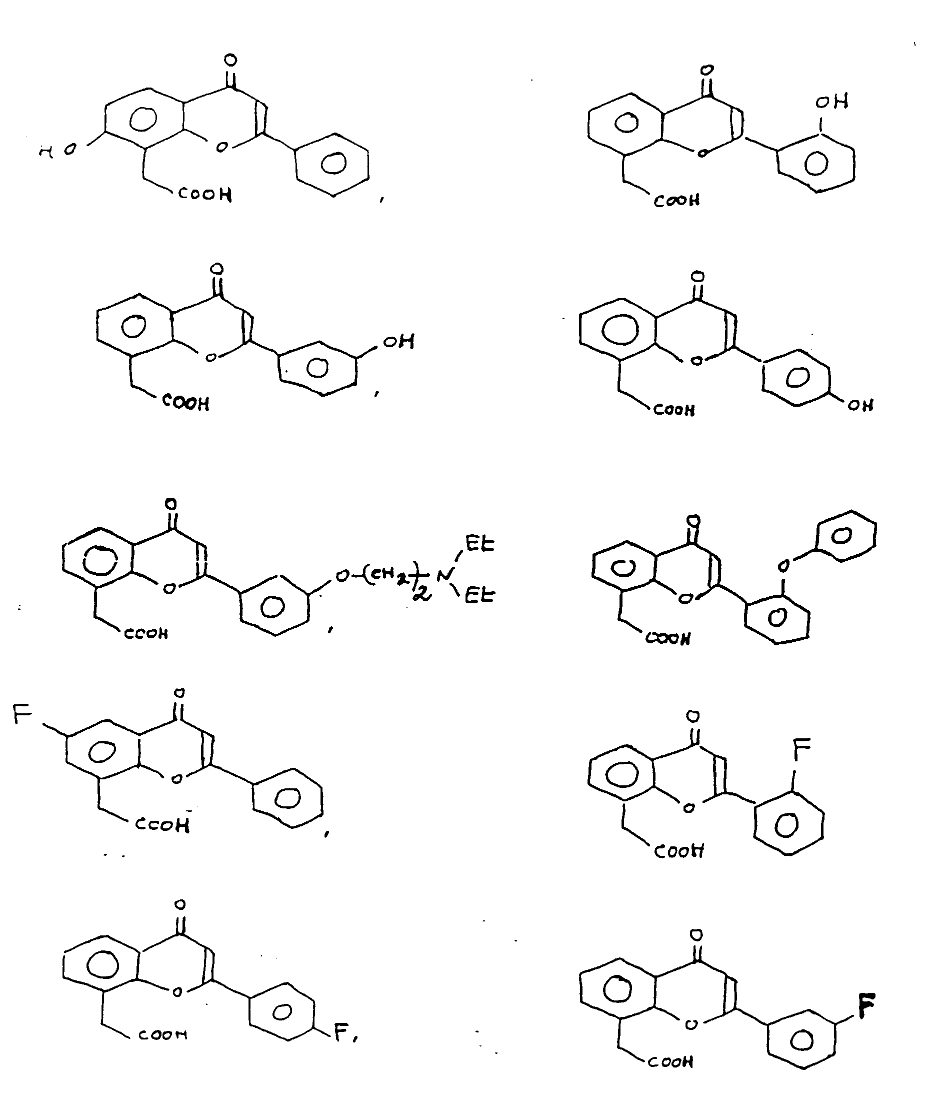

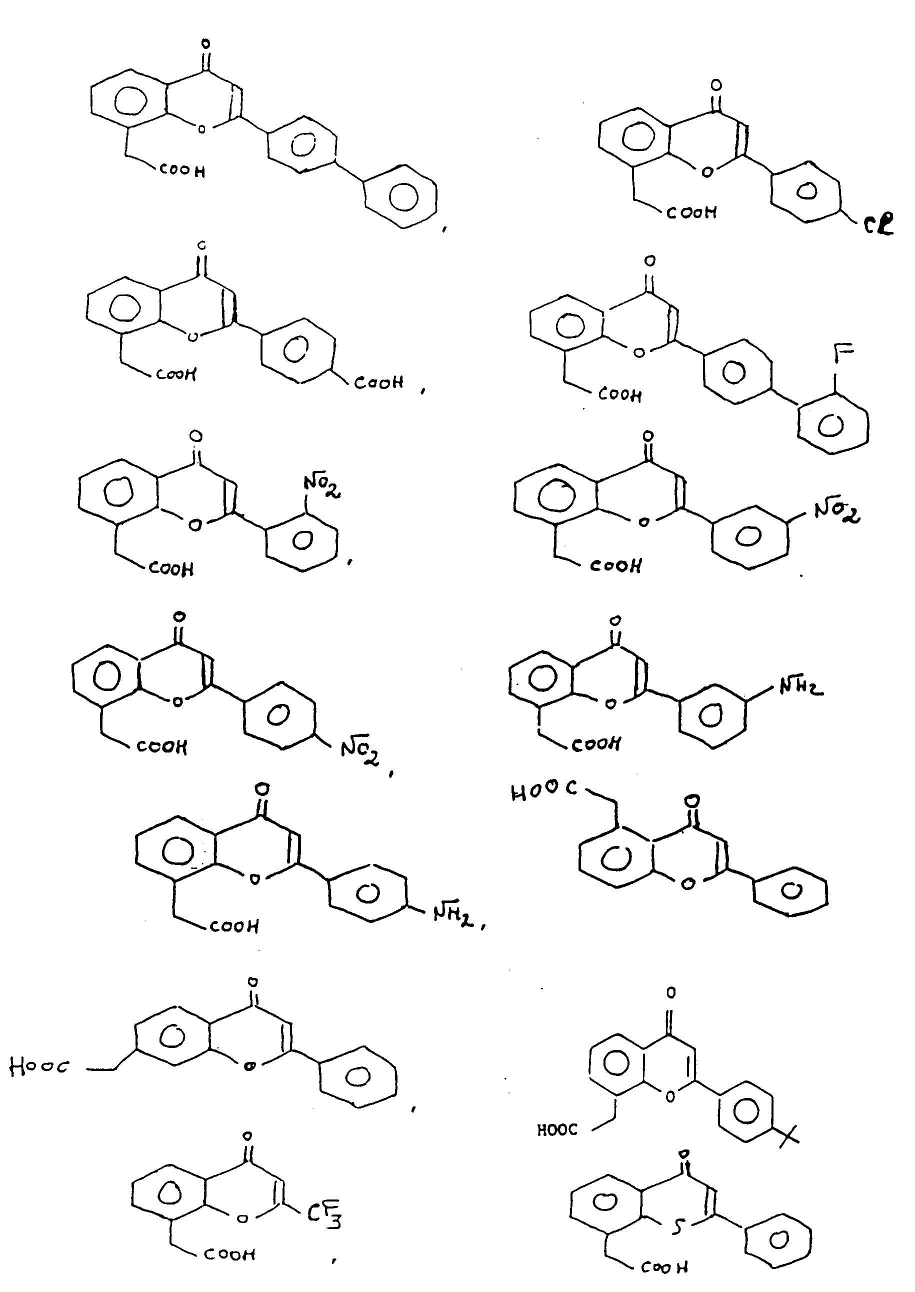

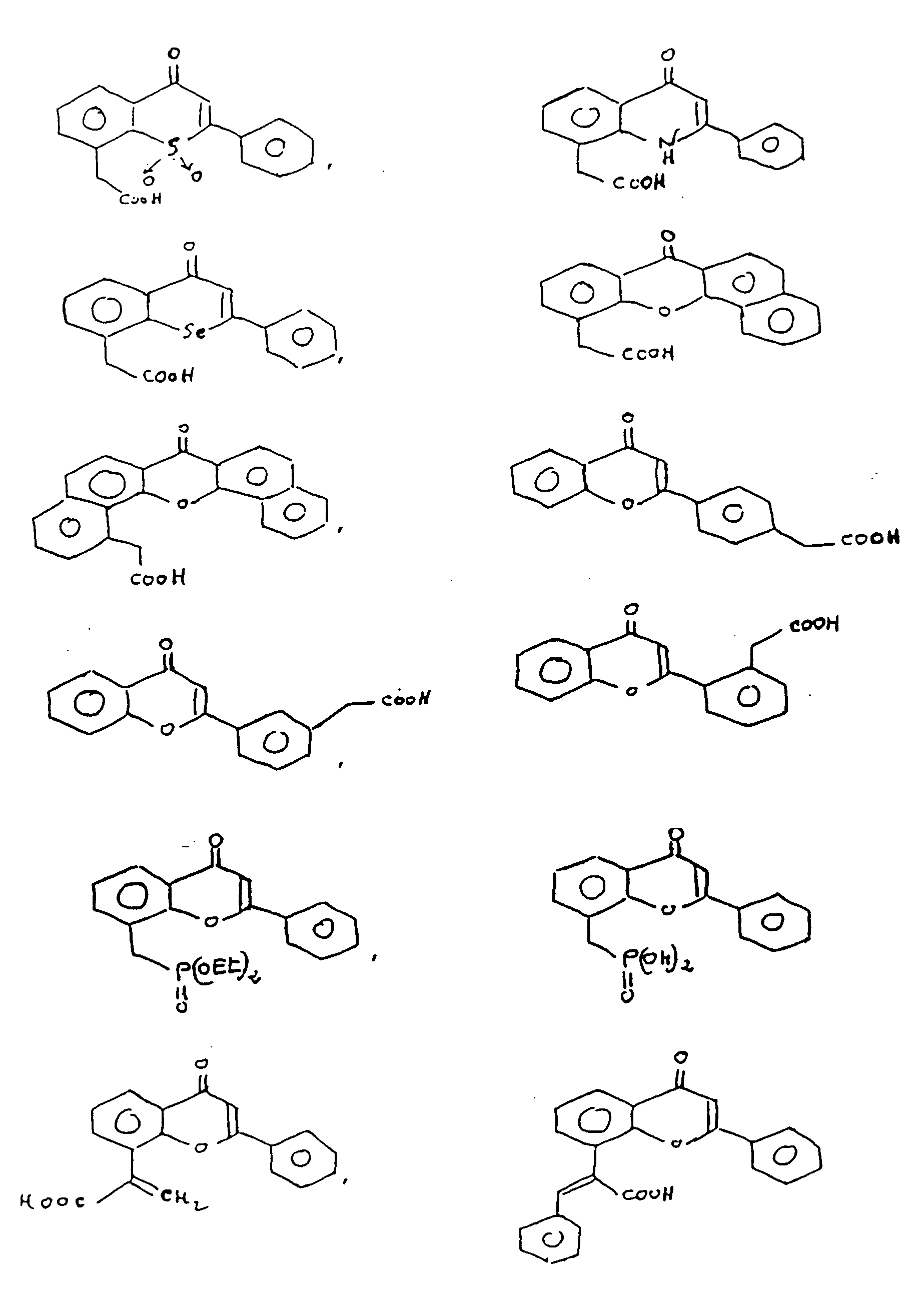

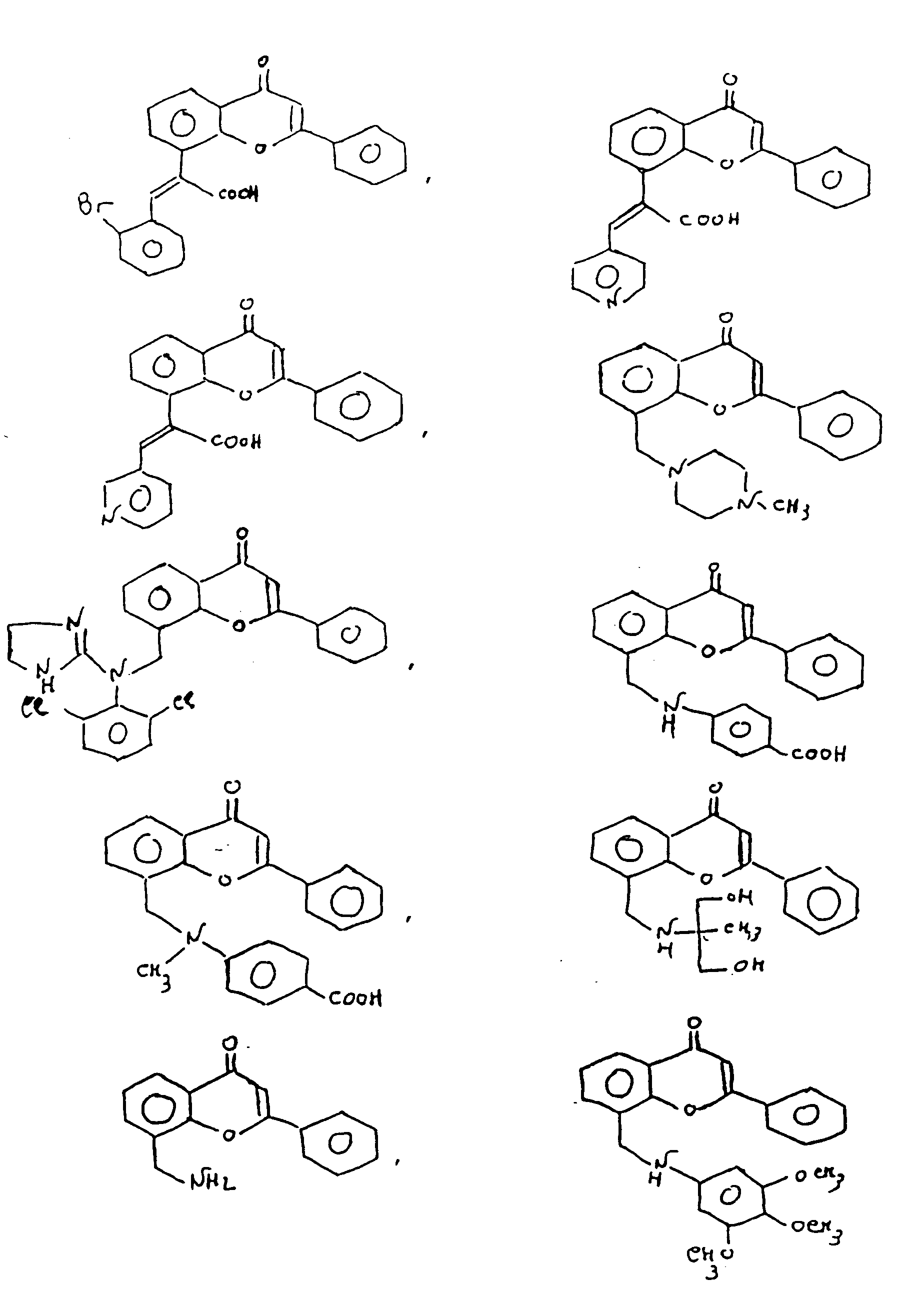

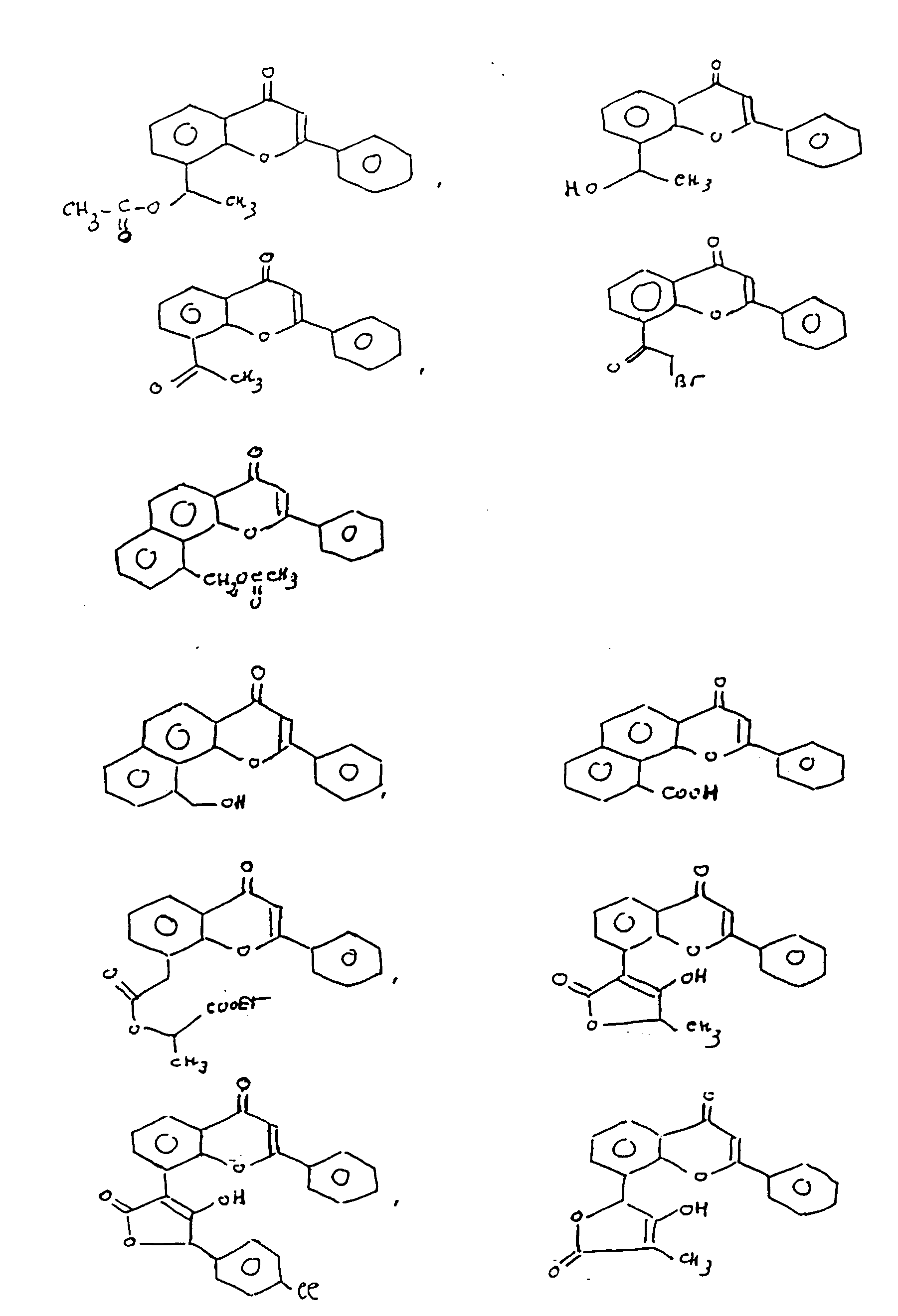

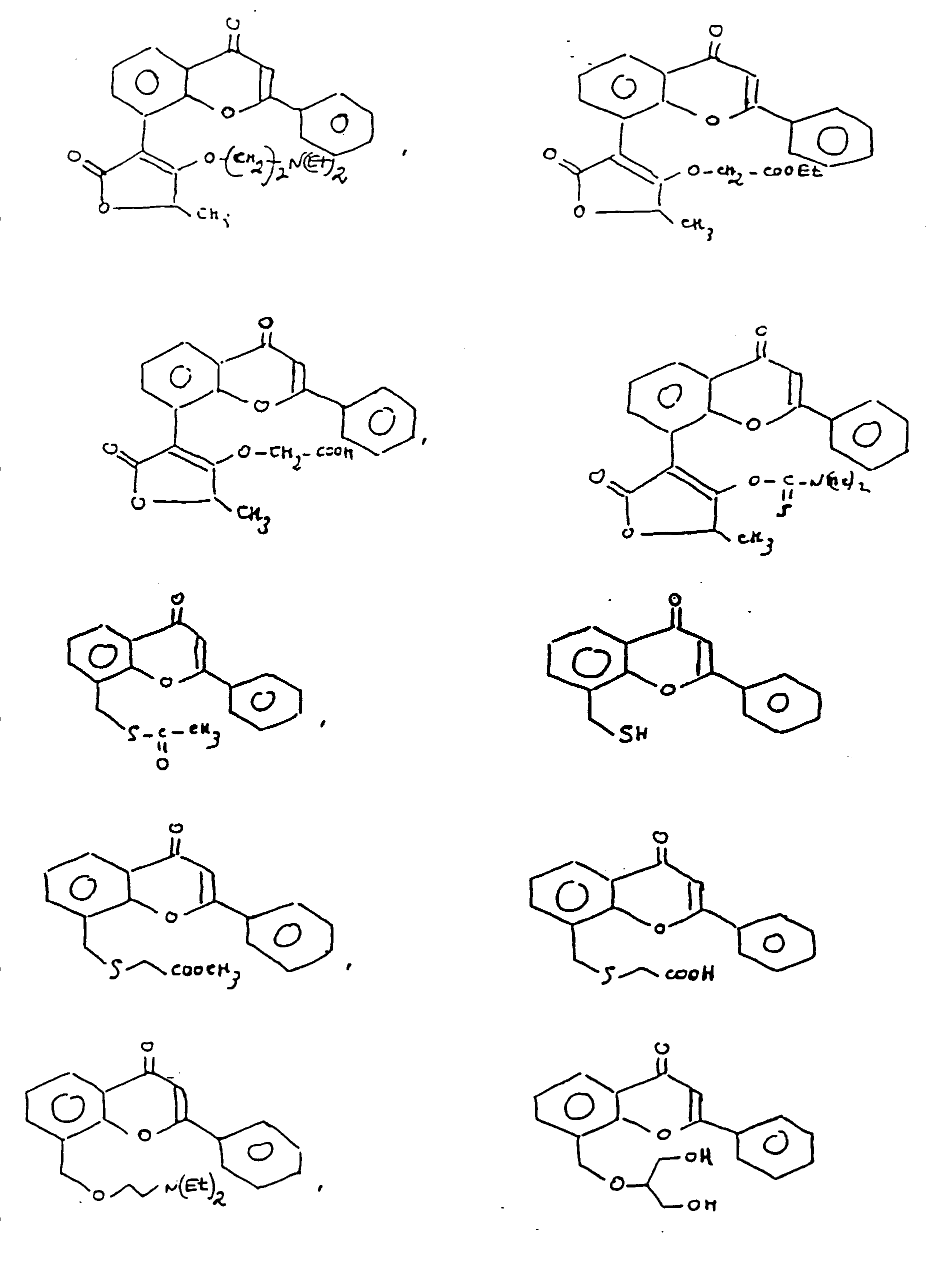

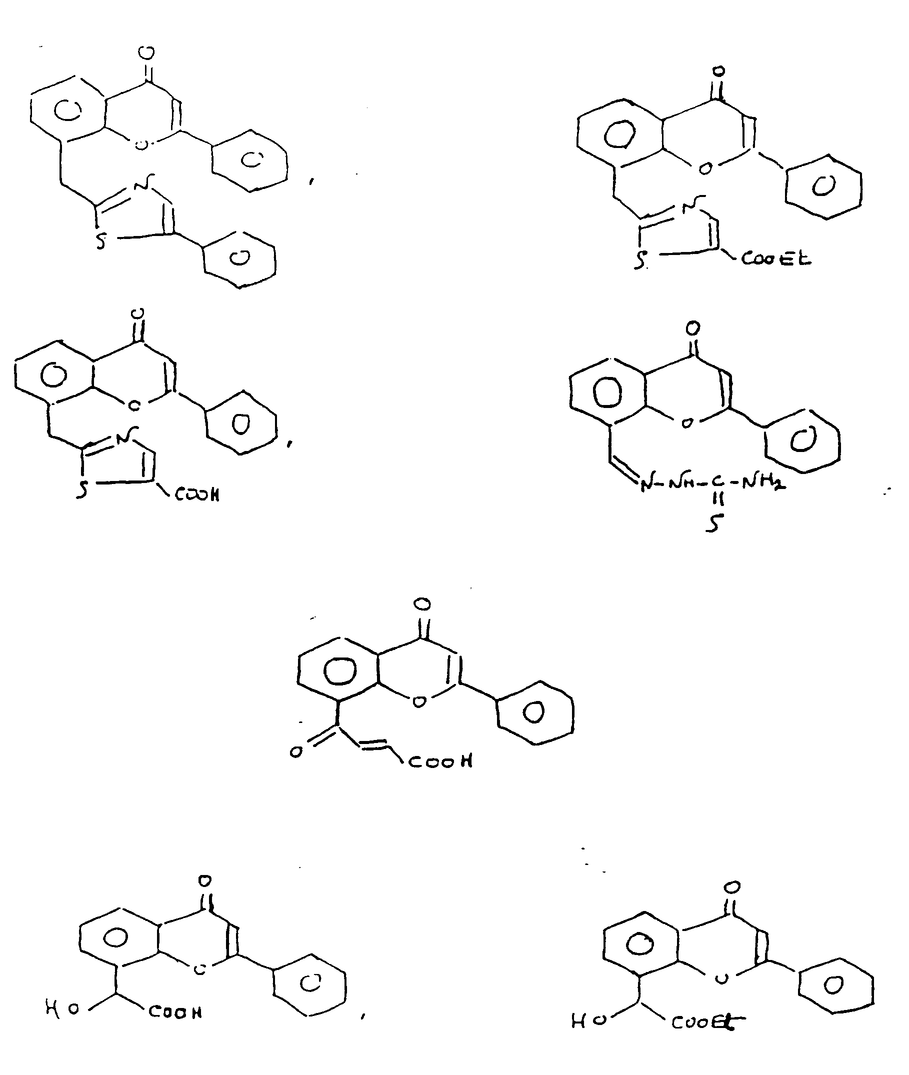

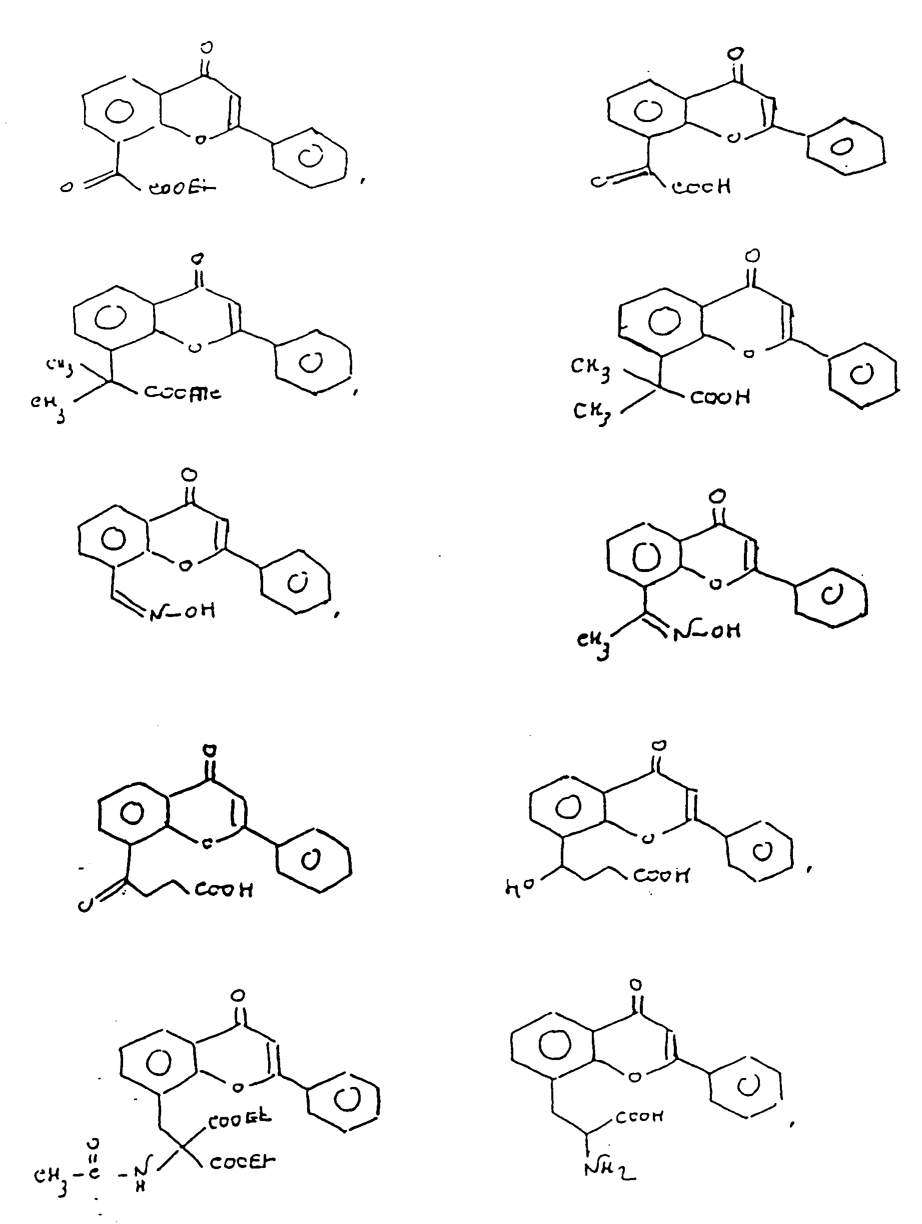

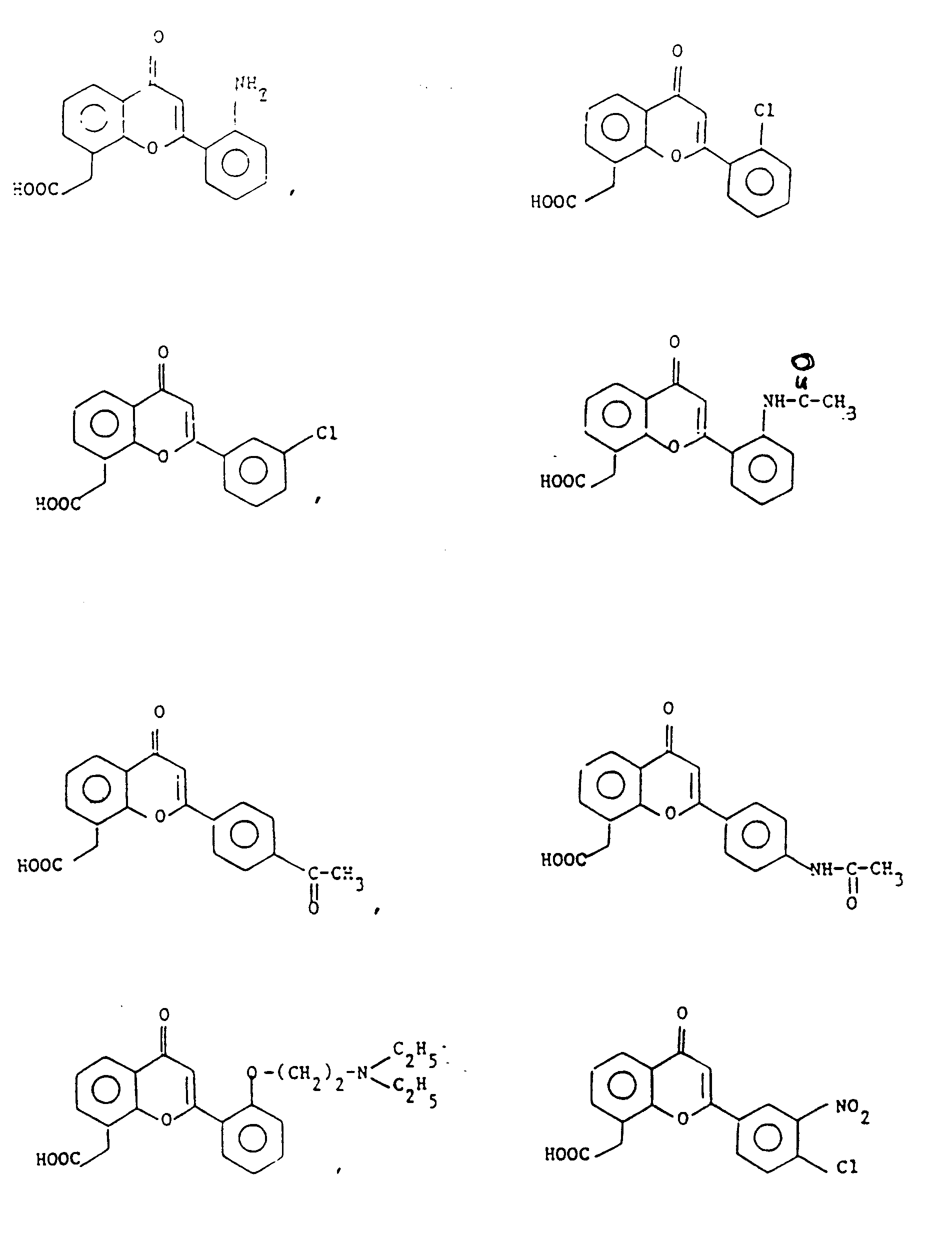

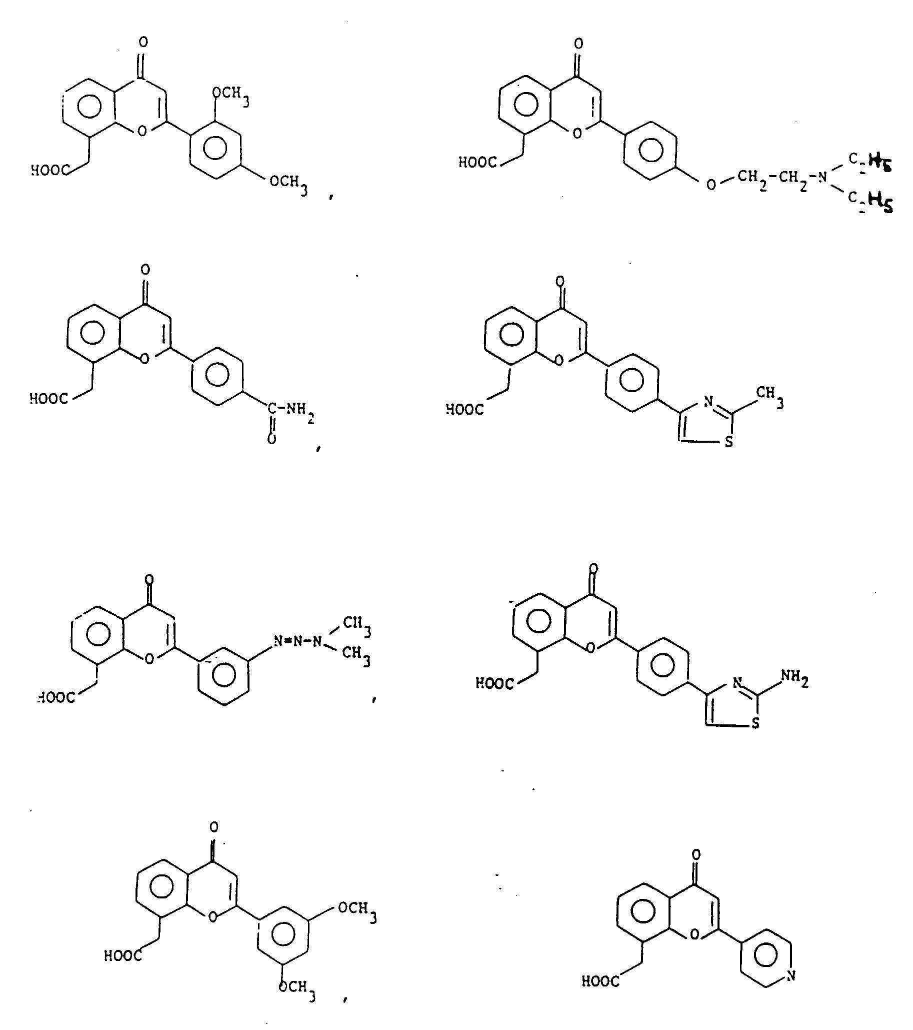

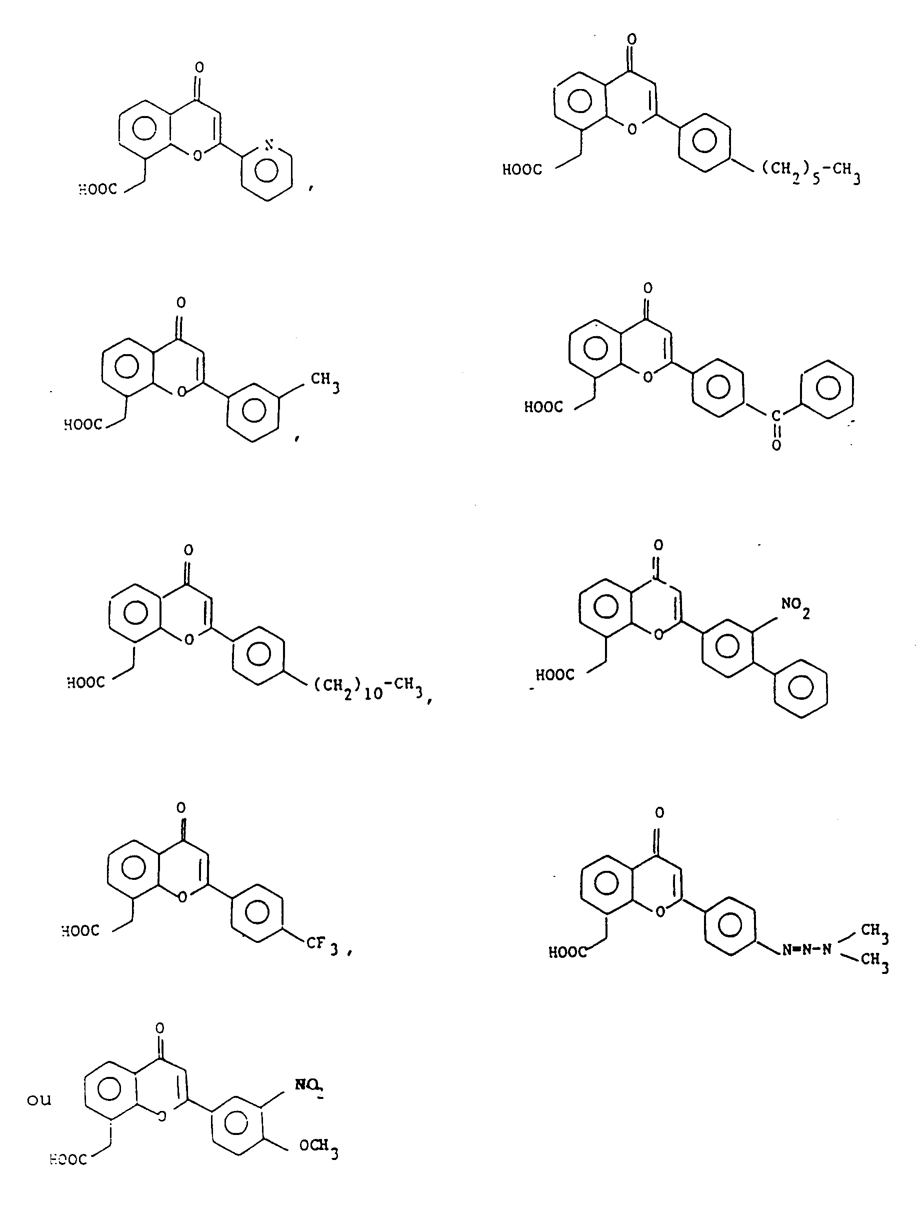

10. Composé selon la revendication 1, ledit composé étant
